# EUROPÄISCHE PATENTSCHRIFT

(11) **EP 0 877 744 B1**
(45) Veröffentlichungstag und Bekanntmachung des Hinweises auf die Patenterteilung: **11.08.2004**
(21) Anmeldenummer: 97901000.6
(22) Anmeldetag: 10.01.1997
(51) Int. Cl.: C07D 403/12, C07D 403/04, C07D 243/08, C07D 409/12, C07D 213/73, C07D 403/14, C07D 401/14, A61K 31/505

(54) **SUBSTITUIERTE AZA- UND DIAZACYCLOHEPTAN- UND -CYCLOOCTANVERBINDUNGEN UND DEREN VERWENDUNG**
SUBSTITUTED AZA- AND DIAZACYCLOHEPTANE AND CYCLOOCTANE COMPOUNDS AND THEIR USE
COMPOSES AZA- ET DIAZACYCLOHEPTANE ET -CYCLO-OCTANE SUBSTITUES ET LEUR UTILISATION

(30) Priorität: 12.01.1996 DE 19600934
(43) Veröffentlichungstag der Anmeldung: 18.11.1998
(73) Patentinhaber: Abbott GmbH & Co. KG, 65205 Wiesbaden (DE)
(72) Erfinder: TREIBER, Hans-Jörg, D-68782 Brühl (DE); BLANK, Stefan, D-67065 Ludwigshafen (DE); STARCK, Dorothea, D-67059 Ludwigshafen (DE); UNGER, Liliane, D-67065 Ludwigshafen (DE); TESCHENDORF, Hans-Jürgen, D-67373 Dudenhofen (DE); WICKE, Karsten, D-67112 Altrip (DE)
(74) Vertreter: Kinzebach, Werner, Dr.
(86) Internationale Anmeldenummer: PCT/EP1997/000106
(87) Internationale Veröffentlichungsnummer: WO 1997/025324

(56) Entgegenhaltungen:
- EP-A- 0 361 271
- BIOORGANIC &MEDICINAL CHEMISTRY LETTERS., Bd. 5, Nr. 3, 1995, ENGLAND, Seiten 219-222, XP000646526 P. JOHN MURRAY ET AL.: "A NOVEL SERIE OF ARYLPIPERAZINES WITH HIGH AFFINITY AND SELECTIVITY FOR THE DOPAMINED3 RECEPTOR." in der Anmeldung erwähnt

## Beschreibung

Die Erfindung betrifft substituierte Aza- und Diazacycloheptanund -cyclooctanverbindungen und die Verwendung derartiger Verbindungen. Die erwähnten Verbindungen besitzen wertvolle therapeutische Eigenschaften und sind insbesondere zur Behandlung von Erkrankungen brauchbar, die auf Dopamin-D₃-Liganden ansprechen.

Verbindungen der hier in Rede stehenden Art mit physiologischer Aktivität sind bereits teilweise bekannt. So beschreiben DE 21 39 082 und DE 22 58 561 basisch substituierte Pyrimidinderivate bzw. Pyrimidonderivate als Arzneimittel zur Senkung des Blutdrucks. Diese Pyrimidin- bzw. Pyrimidonderivate besitzen die Formeln: 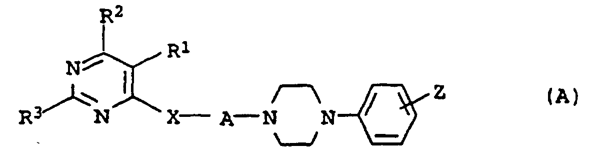 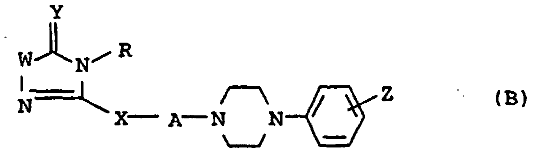 worin in (A) X unter anderem ein Schwefelatom bedeutet, A eine C₁-C₆-Alkylengruppe bedeutet und R¹, R², R³ und Z für verschiedene Substituenten stehen. In (B) stehen X und Y für ein Sauerstoffoder Schwefelatom, A für eine C₂-C₆-Alkylengruppe, W für eine Vinylengruppe und R und Z für verschiedene Substituenten.

Die EP-A-361271 beschreibt Pyridyl- und Pyrimidylderivate der Formel: 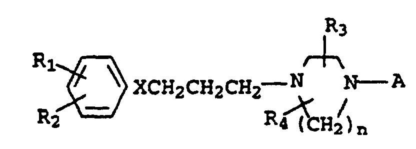 worin R₁ für Halogen oder Wasserstoff steht und R₂ für Halogen steht; X für Sauerstoff, Schwefel oder Methylen steht; R₃ und R₄, die gleich oder verschieden sind, für Wasserstoff oder Niedrigalkyl stehen; n für 2 oder 3 steht: A für eine 2-Pyrimidylgruppe oder eine 2- oder 3-Pyridylgruppe steht, wobei diese Gruppen substituiert sein können.

Diese Verbindungen sind zur Behandlung von mentalen Störungen brauchbar.

Die EP-A-454498 beschreibt Verbindungen der Formel 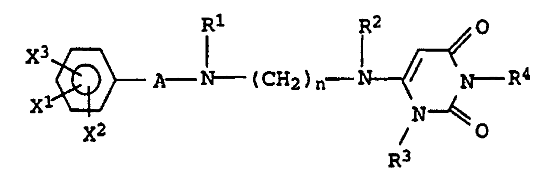 worin A unter anderem für -(CH₂)ₘ- oder -B-(CH₂)ₖ- steht, wobei B für O, S, eine gegebenenfalls substituierte Aminogruppe, -CONH- oder -COO- steht, R¹ und R² unter anderem zusammen eine Alkylenkette bilden können, R³ und R⁴ ein Wasserstoffatom oder eine Niedrigalkylgruppe bedeuten und X¹, X² und X³ verschiedene Substituenten bedeuten. Diese Verbindungen sind zur Behandlung von Herzarrhythmien brauchbar.

Die EP-A-452107 und EP-A-369627 beschreiben strukturell ähnliche Verbindungen, die ebenfalls zur Behandlung von Herzarrhythmien brauchbar sind.

Außerdem beschreibt die BE-A-628 766 Verbindungen der Formel 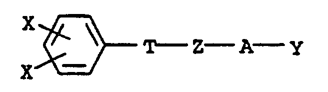 worin X ein Halogenatom oder einen Niedrigalkylrest bedeutet, T für Piperazin, Methylpiperazin, Homopiperazin oder Methylhomopiperazin steht; Z für Alkylen oder Alkenylen steht; A für O oder S steht; und Y für einen Naphthyl-, Halogennaphthyl oder einen gegebenenfalls mit 1 bis 3 Resten substituierten Phenylrest steht. Diese Verbindungen sind zur Behandlung von Schistosomiasis brauchbar.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der Etiologie von Schizophrenie und der Parkinson'schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁ und D₂-Rezeptoren.

In jüngerer Zeit wurde ein dritter Subtyp gefunden, nämlich der D₃-Rezeptor, der einige Effekte der Antipsychotika zu vermitteln scheint. (J.C. Schwanz et al., The Dopamine D₃ Receptor as a Target for Antipsychotics, in Novel Antipsychotic Drugs, H.Y. Meltzer, Ed. Raven Press, New York 1992, Seiten 135-144)

D₃-Rezeptoren werden hauptsächlich im limbischen System exprimiert. Es wird daher angenommen, daß ein selektiver D₃-Antagonist wohl die antipsychotischen Eigenschaften der D₂-Antagonisten, nicht aber ihre neurologischen Nebenwirkungen haben sollte. (P. Solokoff et al., Localization and Function of the D₃ Dopamine Receptor, Arzneim, Forsch./Drug Res. 42(1), 224 (1992); P. Solokoff et al. Molecular Cloning and Characterization of a Novel Dopamine Receptor (D₃) as a Target for Neuroleptics, Nature, 347, 146 (1990)).

P.J. Murray et al., Bioorganic & Medicinal Chemistry Letters, Vol. 5, No. 3, 219-222 (1995), beschreiben bereits Arylpiperazine der Formel 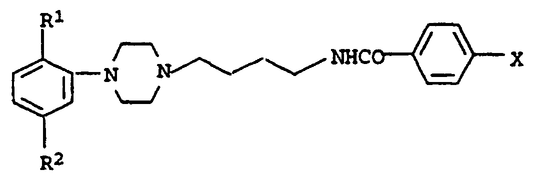 worin R¹ und R² für H oder CH₃O stehen und X für Br, 4-Acetylphenyl, 4-Methylsulfonylphenyl oder 4-Aminophenyl steht, mit hoher Affinität und Selektivität gegenüber dem Dopamin D₃-Rezeptor.

Überraschenderweise wurde nun gefunden, daß bestimmte Aza- und Diazacycloheptan- und -cyclooctanverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung sind daher die Verbindungen der allgemeinen Formel I:

Ar¹-A-B-Ar² (I)

worin
Ar¹ für 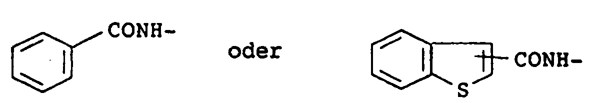 oder einen 5- oder 6-gliedrigen aromatischen Heteromonocyclus mit 1, 2 oder 3 Heteroatomen, die unabhängig voneinander ausgewählt sind unter O, N und S steht, wobei Ar¹ gegebenenfalls 1, 2, 3 oder 4 Substituenten aufweist, die unabhängig voneinander ausgewählt sind unter OR¹, C₁-C₈-Alkyl, das gegebenenfalls durch OH, OC₁-C₈-Alkyl oder Halogen substituiert ist, C₂-C₆-Alkenyl, C₂-C₆-Alkinyl, C₃-C₆- Cycloalkyl, Halogen, CN, CO₂R¹, NO₂, NR¹R², SR¹, CF₃, CHF₂, Phenyl, das gegebenenfalls durch C₁-C₆-Alkyl, OC₁-C₆-Alkyl, HCO, C₁-C₆-Alkyl-CO, Phenyl, Amino, Nitro, Cyano oder Halogen substituiert ist, Phenoxy, das gegebenenfalls durch C₁-C₆-Alkyl, OC₁-C₆-Alkyl oder Halogen substituiert ist, C₁-C₆-Alkanoyl oder Benzoyl;
R¹ für H, C₁-C₈-Alkyl, das gegebenenfalls durch OH, OC₁-C₆-Alkyl, Phenyl oder Halogen substituiert ist, steht;
R² die für R¹ angegebenen Bedeutungen besitzt oder für COR¹ oder CO₂R¹ steht;
A für eine C₃-C₁₅-Alkylengruppe steht, wenn Ar¹ für C₆H₅CONH steht, oder, wenn Ar¹ einen 5- oder 6-gliedrigen aromatischen Heteromonocyclus bedeutet, für eine C₄-C₁₅-Alkylengruppe oder eine C₃-C₁₅-Alkylengruppe steht, die wenigstens eine Gruppe Z umfaßt, die ausgewählt ist unter O, S, NR¹, einer Doppel- und einer Dreifachbindung, wobei R¹ wie oben definiert ist,
B für einen 7- oder 8-gliedrigen gesättigten Ring mit einem oder zwei Stickstoffheteroatomen steht, wobei sich die Stickstoffheteroatome in 1,4- oder 1,5-Position befinden und der Ring in 1-Position an den Rest A und in 4- oder 5-Position an den Rest Ar² gebunden ist und wobei der Ring außerdem eine Doppelbindung in 3- oder 4-Position im Monoazacyclus und in 6-Position im 1,4-Diazacyclus aufweisen kann;
Ar² für Phenyl, Pyridyl, Pyrimidinyl oder Triazinyl steht, wobei Ar² gegebenenfalls 1, 2, 3 oder 4 Substituenten aufweisen kann, die unabhängig voneinander ausgewählt sind unter OR¹, C₁-C₈-Alkyl, C₂-C₆-Alkenyl, C₂-C₆-Alkinyl, C₁-C₈-Alkoxy-C₁-C₈-alkyl, C₁-C₈, Halogenalkyl, Halogen, CN, CO₂R¹, NO₂, SO₂R¹, NR¹R², SO₂NR¹R², SR¹, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter O, S und N, wobei der carbocyclische oder der heterocyclische Ring gegebenenfalls substituiert ist durch C₁-C₈-Alkyl, Phenyl, Phenoxy, Halogen, OC₁-C₈-Alkyl, OH, NO₂ oder CF₃, wobei R¹ und R² die oben angegebenen Bedeutungen besitzen und Ar² gegebenenfalls auch mit einem carbocyclischen Ring der oben definierten Art kondensiert sein kann und wobei Ar² nicht für einen Pyrimidinylrest stehen kann, der mit 2 Hydroxygruppen substituiert ist, und die Salze davon mit physiologisch verträglichen Säuren.

Bei den erfindungsgemäßen Verbindungen handelt es sich um selektive Dopamin-D₃-Rezeptor-Liganden, die regioselektiv im limbischen System angreifen und aufgrund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Rezeptorantagonisten bzw. -agonisten ansprechen, z.B. zur Behandlung von Erkrankungen des zentralen Nervensystems insbesondere Schizophrenie, Depressionen, Neurosen und Psychosen.

Im Rahmen der vorliegenden Erfindung besitzen die nachfolgenden Ausdrücke die anschließend angegebenen Bedeutungen:

C₁-C₈-Alkyl (auch in Resten wie Alkoxy, Alkylamino etc) bedeutet eine geradkettige oder verzweigte Alkylgruppe mit 1 bis 8 Kohlenstoffatomen, vorzugsweise 1 bis 6 Kohlenstoffatomen und insbesondere 1 bis 4 Kohlenstoffatomen. Die C₁-C₈-Alkylgruppe kann einen oder mehrere Substituenten aufweisen, die unabhängig voneinander ausgewählt sind unter OH und OC₁-C₈-Alkyl.

Beispiele für eine C₁-C₈-Alkylgruppe sind Methyl, Ethyl, n-Propyl, i-Propyl, n-Butyl, iso-Butyl, t-Butyl, etc.

C₃-C₆-Cycloalkyl steht für Cyclopropyl, Cyclobutyl, Cyclopentyl und Cyclohexyl.

Alkylen steht für geradkettige oder verzweigte Reste mit vorzugsweise 4 bis 15 Kohlenstoffatomen, besonders bevorzugt 4 bis 10 Kohlenstoffatomen, bzw. mit 3 bis 15, insbesondere 3 bis 10 Kohlenstoffatomen, wenn die Alkylengruppe eine der genannten Gruppen umfaßt.

Die Alkylengruppen können gegebenenfalls wenigstens eine der oben bei der Definition von A angegebenen Gruppen Z umfassen. Diese kann - ebenso wie die erwähnte Doppel- oder Dreifachbindung - in der Alkylenkette an beliebiger Stelle oder in Position 1 oder 2 der Gruppe A (vom Rest Ar¹ her gesehen) angeordnet sein. Besonders bevorzugt steht A für Verbindungen nach der Formel I, worin A für - Z-C₃-C₆-Alkylen, insbesondere -Z-CH₂CH₂CH₂-, -Z-CH₂CH₂CH₂CH₂-, - Z-CH₂CH=CHCH₂-, -Z-CH₂C(CH₃)=CHCH₂-, -Z-CH₂C(=CH₂)CH₂-, - Z-CH₂CH(CH₃)CH₂- oder für einen linearen -Z-C₇-C₁₀-Alkylenrest steht. Dabei steht A besonders bevorzugt für -Z-C₃-C₆-Alkylen, wenn Ar¹ für einen gegebenenfalls substituierten Pyrimidin- oder Triazolrest steht und für einen linearen -Z-C₇-C₁₀-Alkylenrest, wenn Ar¹ für einen gegebenenfalls substituierten Thiadiazolrest steht. Z kann dabei auch CH₂ bedeuten und steht vorzugsweise für CH₂, O und insbesondere S.

Halogen bedeutet F, Cl, Br oder I.

C₁-C₈-Halogenalkyl kann ein oder mehrere, insbesondere 1, 2 oder 3 Halogenatome umfassen, die sich an einem oder mehreren C-Atomen befinden können, vorzugsweise in α- oder ω-Position. Besonders bevorzugt sind CF₃, CHF₂, CF₂Cl oder CH₂F.

C₁-C₆-Alkyl-CO steht insbesondere für Acetyl.
Wenn Ar¹ substituiert ist, kann sich der Substituent auch an dem Stickstoffheteroatom befinden.

Vorzugsweise steht Ar¹ für 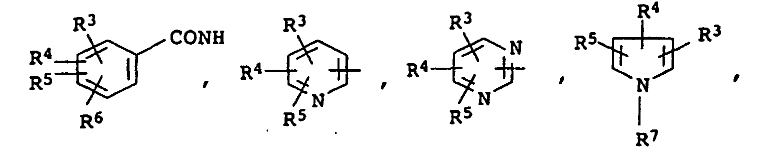 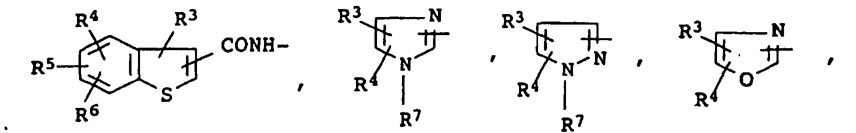 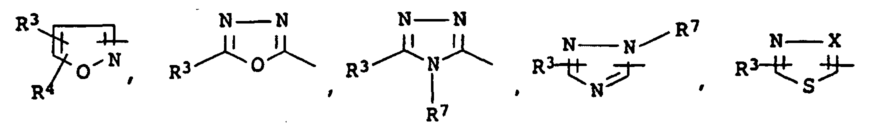 worin
R³ bis R⁶ für H oder die oben genannten Substituenten des Restes Ar¹ stehen,
R⁷ die oben für R² angegebenen Bedeutungen besitzt und
X für N oder CH steht. wenn der Benzamidrest substituiert ist, stehen die Substituenten vorzugsweise in m- oder p-Stellung.

Besonders bevorzugt steht Ar¹ für 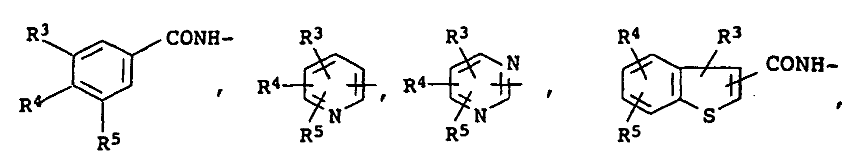 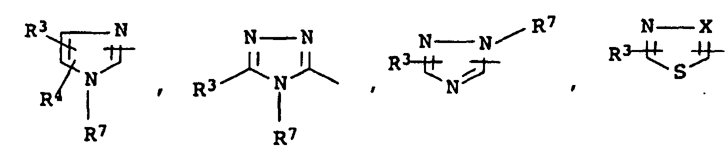 worin R³ bis R⁵, R⁷ und X die oben angegebenen Bedeutungen besitzen und insbesondere für 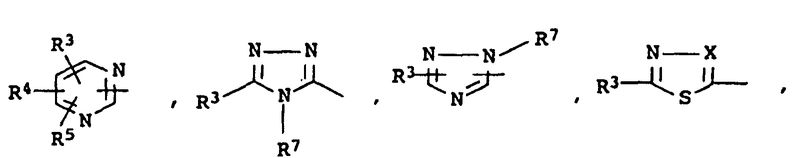 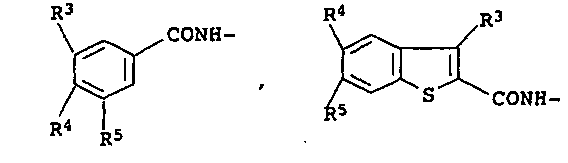 worin R³ bis R⁵, R⁷ und X die oben angegebenen Bedeutungen besitzen. Vorzugsweise steht dann R³, R⁴ und R⁵ unabhängig voneinander für H, OR¹, C₁-C₈-Alkyl, NR¹R², Halogen, Phenoxy, CN, Phenyl, das gegebenenfalls durch C₁-C₆-Alkyl, HCO, C₁-C₆-Alkyl-CO oder Halogen substituiert ist, oder COOR¹; stehen R¹ und R² unabhängig voneinander für H, C₁-C₈-Alkyl oder Benzyl; steht R⁷ für H, C₁-C₈-Alkyl oder C₃-C₆-Cycloalkyl; und steht X steht für N oder CH steht.

Die Reste R³ bis R⁶ stehen vorzugsweise für H, C₁-C₆-Alkyl, OR¹, NR¹R², SR¹, Phenyl, das gegebenenfalls durch C₁-C₆-Alkyl, HCO, C₁-C₆-Alkyl-CO oder Halogen substitutiert ist, und Halogen, wobei R¹ und R² die oben angegebenen Bedeutungen besitzen.

In einer bevorzugten Ausführungsform der Erfindung sind R³ bis R⁵ unabhängig voneinander ausgewählt unter H, C₁-C₆-Alkyl, OR¹, NR¹R², Phenyl, das gegebenenfalls durch C₁-C₆-Alkyl, HCO, C₁-C₆-Alkyl-CO oder Halogen substituiert ist, und Halogen, wobei R¹ und R² die oben angegebenen Bedeutungen besitzen, R⁷ für H oder C₁-C₈-Alkyl steht und X für N steht.

In einer weiteren bevorzugten Ausführungsform der Erfindung steht Ar¹ für Pyrimidinyl, das gegebenenfalls durch OH, OC₁-C₈-Alkyl oder OBenzyl substituiert ist.

In einer weiteren bevorzugten Ausführungsform der Erfindung steht Ar¹ für 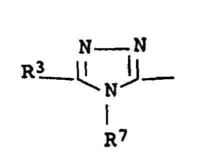 worin R³ für NR¹R² steht, wobei R¹ und R² unabhängig voneinander für H, C₁-C₈-Alkyl oder Benzyl stehen und R⁷ für H oder C₁-C₈-Alkyl steht.

In einer weiteren bevorzugten Ausführungsform der Erfindung steht Ar¹ für Thiadiazol, das gegebenenfalls durch NR¹R² substituiert ist, wobei R¹ und R² unabhängig voneinander für H, C₁-C₈-Alkyl oder Benzyl stehen.

In einer weiteren bevorzugten Ausführungsform der Erfindung steht Ar¹ für 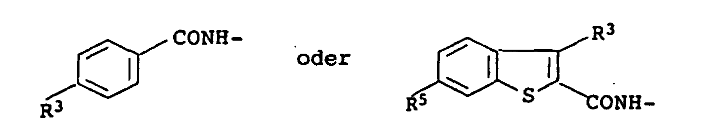 worin R³ und R⁵ unabhängig voneinander für H oder Halogen, C₁-C₈-Alkyl oder Phenyl stehen.

In einer weiteren Ausführungsform der Erfindung steht A für - Z-C₃-C₆-Alkylen, insbesondere -Z-CH₂CH₂CH₂-, -Z-CH₂CH₂CH₂CH₂-, - Z-CH₂CH=CHCH₂-, -Z-CH₂C(CH₃)=CHCH₂-, -Z-CH₂C(=CH₂)CH₂-, -Z-CH₂CH(CH₃)CH₂- oder für einen linearen -Z-C₇-C₁₀-Alkylenrest steht, wobei Z an Ar¹ gebunden ist und für CH₂, O oder S steht.

Der Rest B steht vorzugsweise für

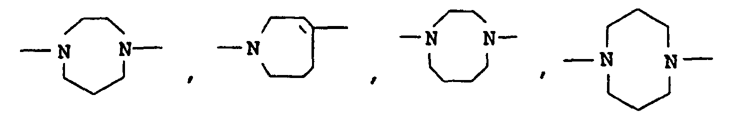

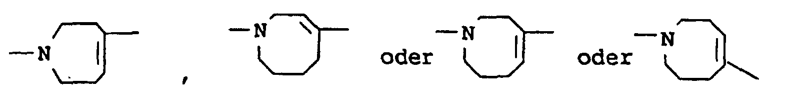

Der Rest Ar² kann einen, zwei, drei oder vier Substituenten, vorzugsweise einen oder zwei Substituenten, die sich insbesondere in m-Stellung und/oder p-Stellung befinden, aufweisen. Vorzugsweise sind sie unabhängig voneinander ausgewählt unter C₁-C₆-Alkyl, C₁-C₈-Halogenalkyl, NO₂, Halogen, insbesondere Chlor, Phenyl, Pyrrolyl, Imidazolyl, Pyrazolyl, Thienyl, Cyclopentyl und Cyclohexyl. Wenn einer der Substituenten für C₁-C₈-Alkyl steht, ist eine verzweigte Gruppe und insbesondere Isopropyl oder t-Butyl bevorzugt.

In einer weiteren bevorzugten Ausführungsform der Erfindung steht B für

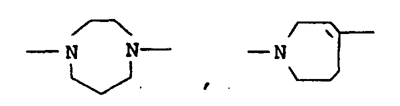

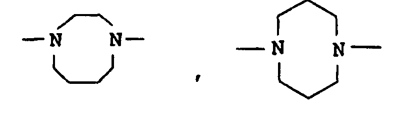

In einer weiteren Ausführungsform der Erfindung steht Ar² für Phenyl, Pyridinyl oder Pyrimidinyl, das gegebenenfalls einen oder zwei Substituenten aufweist, die unabhängig voneinander ausgewählt sind unter C₁-C₆-Alkyl, C₂-C₆-Alkinyl, Halogen, CN, C₁-C₈-Halogenalkyl, OC₁-C₈-Alkyl, NO₂, Phenyl, Pyrrolyl, Imidazolyl, Pyrazolyl, Thienyl, Cyclopentyl und Cyclohexyl. Vorzugsweise sind der oder die Substituenten unabhängig voneinander ausgewählt unter C₁-C₆-Alkyl, NO₂ und C₁-C₈-Halogenalkyl, insbesondere CF₃, CHF₂ und CF₂Cl.

Vorzugsweise steht Ar² für gegebenenfalls substituiertes Phenyl, 2-, 3- oder 4-Pyridinyl oder 2-, 4(6)- oder 5- Pyrimidinyl.

Wenn einer der Substituenten des Restes Ar² für einen 5- oder 6-gliedrigen heterocyclischen Ring steht, so handelt es sich beispielsweise um einen Pyrrolidin-, Piperidin-, Morpholin-, Piperazin-, Pyridin-, 1,4-Dihydropyridin-, Pyrimidin-, Triazin-, Pyrrol-, Thiophen-, Thiazol-, Imidazol-, Oxazol-, Isoxazol-, Pyrazol-, oder Thiadiazolrest, wobei ein Pyrrol-, Imidazol-, Pyrazoloder Thienylrest bevorzugt ist.

Wenn einer der Substituenten des Restes Ar² für einen carbocyclischen Rest steht, handelt es sich insbesondere um einen Phenyl-, Cyclopentyl- oder Cyclohexylrest.

Wenn Ar² mit einem carbocyclischen Rest kondensiert ist, handelt es sich insbesondere um einen Naphthalin-, Di- oder Tetrahydronaphthalinrest.

Die Erfindung umfaßt auch die Säureadditionssalze der erfindungsgemäßen Verbindungen, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff, Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Das Verfahren zur Herstellung der Verbindungen (I) besteht darin, daß man
a) eine Verbindung der allgemeinen Formel II

   Ar¹ - Z - A - Y¹ (II)

   worin Y¹ für eine übliche Abgangsgruppe wie beispielsweise Hal, Alkansulfonyloxy, Arylsulfonyloxy etc. steht, und Z die oben genannten Bedeutungen besitzt,
   mit einer Verbindung der allgemeinen Formel (III)

   H - B - Ar² (III)

   umsetzt; oder
b) eine Verbindung der allgemeinen Formel (IV)

   Ar¹ - A¹ - Z¹H (IV)

   worin Z¹ für O, NR¹ oder S und A¹ für C₁-C₁₅-Alkylen oder eine Bindung steht, mit einer Verbindung der allgemeinen Formel V

   Y¹ - A² - B - Ar² (V)

   wobei Y¹ die oben angegebene Bedeutung besitzt und A² für C₂-C₁₅-Alkylen steht, wobei A¹ und A² zusammen 3 bis 15 C-Atome aufweisen,
   umsetzt; oder
c) eine Verbindung der allgemeinen Formel (VI)

   Ar¹ - Y¹ (VI)

   worin Y¹ die oben angegebene Bedeutung besitzt, mit einer Verbindung der allgemeinen Formel VII

   H - Z¹ - A - B - Ar² (VII)

   worin Z¹ die oben angegebenen Bedeutungen besitzt, umsetzt; oder
d) eine Verbindung der Formel (VIII)

   NC - A - B - Ar² (VIII)

   in eine Verbindung des Typs (IX) 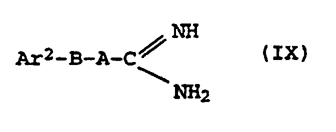 überführt,
   und diese mit einer Dicarbonyl-Verbindung in bekannter Weise umsetzt; oder
e) zur Herstellung einer Verbindung der Formel I, worin Ar¹ für einen Benzamidrest steht:
   eine Verbindung der allgemeinen Formel (X) 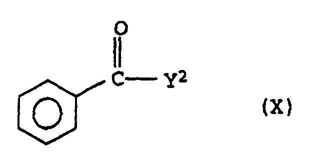 worin Y² für OH, OC₁-C₄-Alkyl, Cl oder zusammen mit CO für eine aktivierte Estergruppe steht, mit einer Verbindung der Formel (XI)

   Z² - A² - B - Ar² (XI)

   worin A² die oben angegebenen Bedeutungen besitzt und Z² für OH oder NH₂ steht, umsetzt.

Die Verbindungen der Formel III sind Ausgangsverbindungen zur Herstellung von Verbindungen der Formeln V, VII, VIII und werden hergestellt, indem man
a) eine Verbindung der allgemeinen Formel (XII)

   HB¹ (XII)

   worin B¹ für 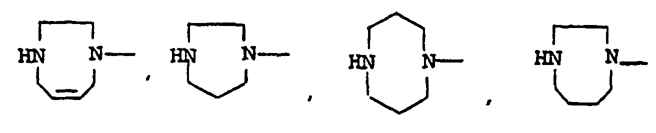 mit einer Verbindung der allgemeinen Formel (XIII)

   Y¹ - Ar² (XIII)

   worin Y¹ für eine der vorstehend erwähnten Abgangsgruppen und Ar² die oben angegebenen Bedeutung besitzt,
   in bekannter Weise umsetzt; oder
b) eine Verbindung der allgemeinen Formel (XIV)

   H - B²

   worin B² für 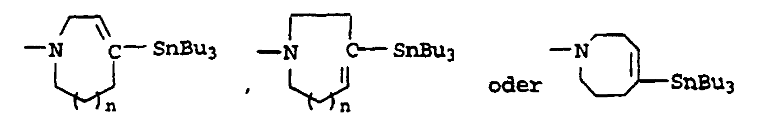 mit n = 1, oder 2 steht,
   mit einer Verbindung der allgemeinen Formel (XV)

   Y² - Ar²

   worin Y² für Br, Cl, I steht und Ar² obige Bedeutungen besitzt, nach bekannten Verfahren umsetzt, wie beschrieben z.B. von S.C. Buchwald et al., Angew. Chem. 1995, 107, 1456 oder J.F. Hartweg et al., Tetrahedron Lett 1995, 36, 3604 bzw. J.K. Stille et al., Angew. Chem. 1986, 98, 504 oder Pereyre M. et al., Tin in Organic Synthesis, Butterworth 1987; oder
c) eine Verbindung der allgemeinen Formel (XVI) 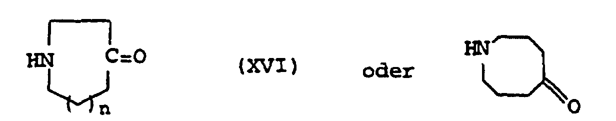 wobei n = 1 oder 2,
   mit einer Verbindung M-Ar², worin M für ein Metall wie z.B. Li, MgY² steht, umsetzt. MAr² kann erhalten werden aus Verbindungen der Formel XV nach literaturbekannten Methoden.

Verbindungen des Typs Ar¹ sowie Ar² sind entweder bekannt oder können nach bekannten Verfahren hergestellt werden wie z.B. beschrieben in A.R. Katritzky, CW. Rees (ed.) "Comprehensive Heterocyclic Chemistry", Pergamon Press, oder "The Chemistry of Heterocyclic Compounds", J. Wiley & Sons Inc. NY und der dort zitierten Literatur.

Verbindungen des Typs B sind entweder bekannt oder sie können analog zu bekannten Verfahren hergestellt werden, wie z.B.
- 1,4- und 1,5-Diazacycloalkane:: L. Börjeson et al. Acta Chem. Scand. 1991, 45, 621 Majahrzah et al Acta Pol. Pharm., 1975, 32, 145
- 1,4-Diazacyclooct-6ene:: W. Schroth et al. Z. Chem. 1969, 9, 143
- 1-Azacyclooctanone:: N.J. Leonard et al. J. Org. Chem. 1964, 34, 1066
- 1-Azacyclo-heptanone:: A. Yokoo et al. Bull Chem. Soc. Jpn. 1956, 29, 631

Die Herstellung der erfindungsgemäßen Verbindungen und der Ausgangsmaterialien und Zwischenprodukte kann auch analog zu den in den eingangs genannten Patentpublikationen beschriebenen Methoden erfolgen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ethylacetat, Tetrahydrofuran, Dimethylformamid, Dimethylsulfoxid, Dimethoxyethan, Toluol, Xylol, ein Keton, wie Aceton oder Methylethylketon oder ein Alkohol, wie Ethanol oder Butanol.

Gewünschtenfalls arbeitet man in Gegenwart eines säurebindenden Mittels. Geeignete säurebindende Mittel sind anorganische Basen, wie Natrium- oder Kaliumcarbonat, Natriummethylat, Natriumethylat, Natriumhydrid oder metallorganische Verbindungen wie Butyllithium oder Alkylmagnesium Verbindungen, oder organische Basen, wie Triethylamin oder Pyridin. Letztere können gleichzeitig als Lösungsmittel dienen.

Die Umsetzungen erfolgen gegebenenfalls unter Verwendung eines Katalysators, wie z.B. Übergangsmetalle und deren Komplexe, z.B. Pd(PPh₃)₄, Pd(OAc)₂ oder Pd(P(oTol)₃)₄, oder eines Phasen-Transfer-Katalysators, z.B. Tetrabutylammoniumchlorid oder Tetrapropylammoniumbromid.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### Beispiel 1

1-[2-t-Butyl-6-trifluormethyl-pyrimidin-4-yl]-4-[3-[4-hydroxypyrimidin-2-yl)-mercaptopropyl]-hexahydro-(1H)-1,4-diazepin-fumarat

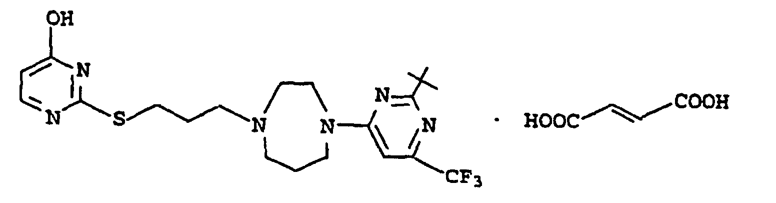

### Herstellung der Ausgangsprodukte:

a) 2-t-Butyl-4-hydroxy-6-trifluormethyl-pyrimidin.

Der Aufbau des obigen Pyrimidins erfolgte in an sich bekannter Weise durch Kondensation von 2,2-Dimethylpropion-amidin mit Trifluoracetessigsäureethylester und Natriumethylat in Ethanol, siehe Heterocyclic Compounds, Vol. 52, The Pyrimidines, S. 189 ff., D.J. Brown et al. (Eds.) John Wiley and Sons, 1994.

Fp. 187-188°C

In analoger Weise wurden die 4-Hydroxypyrimidine der Formel 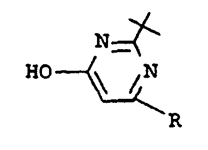 erhalten.

| R | Fp [°C] |
|---|---|
| t-C₄H₉ | 169 |
| n-C₃H₇ | 120 |
| CF₂Cl | 135-136 |

b) 2-t-Butyl-4-chlor-6-trifluormethylpyrimidin

Das Hydroxypyrimidin aus Stufe a) wurde mit Phosphoroxychlorid oder Thionylchlorid in an sich bekannter Weise in die Chlorverbindung überführt, siehe Heterocyclic Compounds, Vol. 52, The Pyrimidines, S. 329 ff., John Wiley and Sons, 1994. Die Verbindung liegt als gelbliches Öl vor.

In analoger Weise wurden die 4-Chlorpyrimidine der Formel 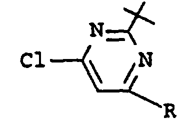 erhalten:

| R | Fp [°C] |
|---|---|
| t-C₄H₉ | öl |
| n-C₃H₇ | Öl |
| CF₂Cl | Öl |

c) 1-[2-t-Butyl-6-trifluormethylpyrimidin-4-yl]-hexahydro-(1H)-1,4-diazepin

18 g (0,18 Mol) Homopiperazin wurden in 25 ml Ethanol gelöst und bei Rückflußtemperaturen eine Lösung von 7,2 g (0,03 Mol) des nach b) erhaltenen Chlorids, gelöst in 10 ml Ethanol, innerhalb 1 h zugetropft. Nach 30 min. Nachreaktion wurde der erkaltete Ansatz zur Aufarbeitung mit 200 ml Wasser versetzt und mehrfach mit insgesamt 200 ml Methylenchlorid extrahiert.
Die organische Phase wurde anschließend mit Wasser gewaschen, mit wasserfreiem Natriumsulfat getrocknet und eingeengt. Man erhielt die gewünschte Verbindung als gelbliches Öl, das roh weiter verarbeitet wurde.
Ausbeute: 98% d.Th.

Die folgenden Verbindungen wurden in entsprechender Weise erhalten:
1-Aryl-1,4-Diazepine der Formel:

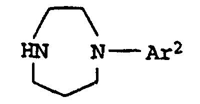

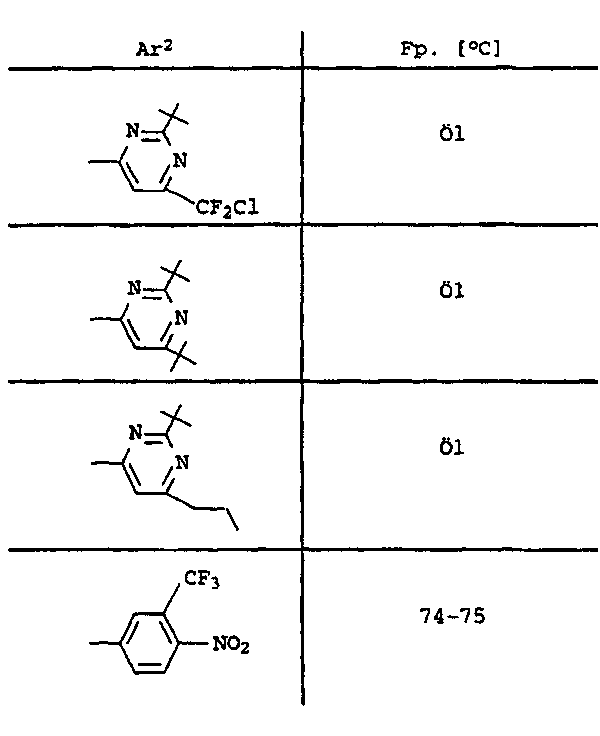

d) 1-[2-t-Butyl-6-trifluormethylpyrimidin-4-yl]-4-(3-chlorpropyl)hexahydro-(1H)-1,4-diazepin

5 g (0,0165 Mol) der vorstehend unter c) erhaltenen Verbindung wurden zusammen mit 2,5 g (0,025 Mol) Triethylamin und 3,15 g (0,02 Mol) 1-Brom-3-chlorpropan in 50 ml Tetrahydrofuran 10 h zum Sieden unter Rückfluß erhitzt. Anschließend wurde das Lösungsmittel abdestilliert, der Rückstand mit Wasser versetzt und mit Methylenchlorid extrahiert. Der nach dem Trocknen und Einengen erhaltene Rückstand wurde anschließend mittels Flash-Chromatographie (Kieselgel) gereinigt.
Ausbeute: 4,8 g (77% d.Th.) gelbes Öl

Die nachfolgend aufgeführten Verbindungen wurden in analoger Weise erhalten:

1-Axyl-4-halogenalkyl-1,4-diazepine der Formel

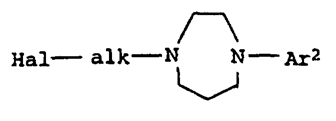

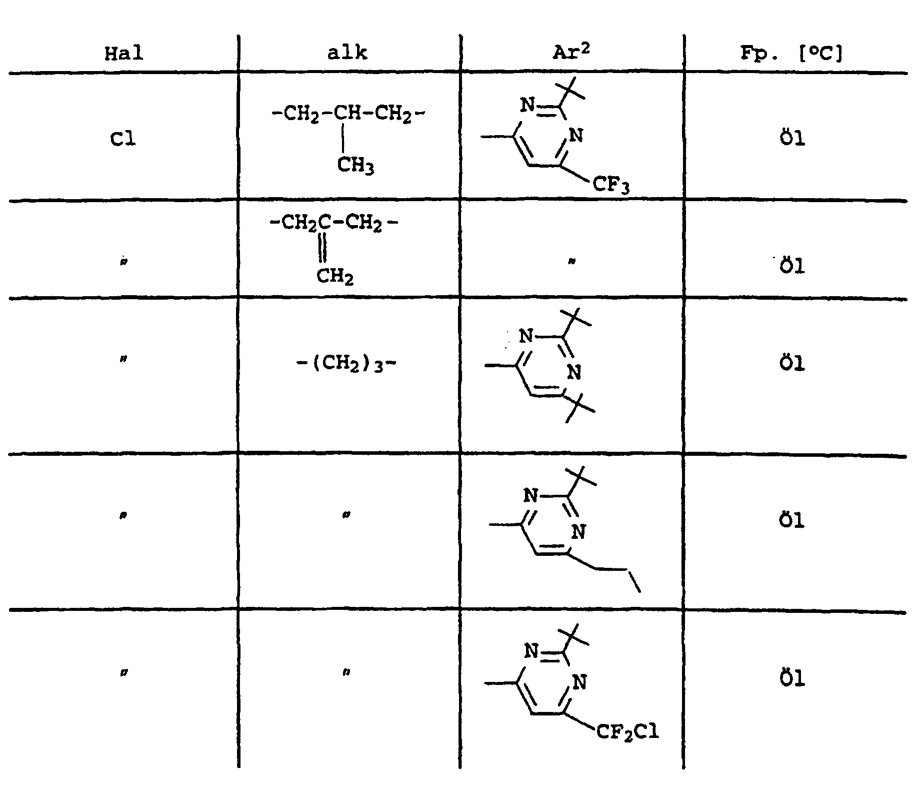

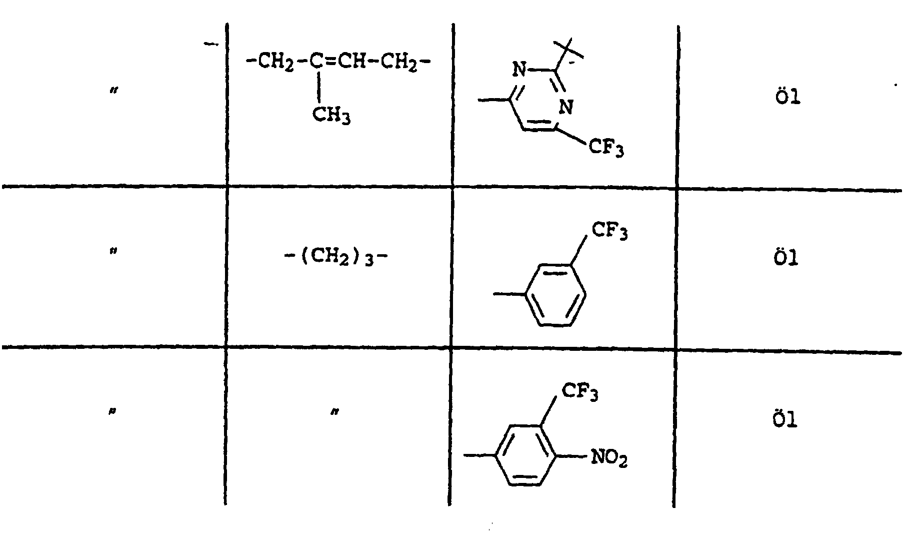

### Herstellung des Endproduktes

5 g (0,013 Mol) des nach d) erhaltenen Produktes wurden in 25 ml Dimethylformamid gelöst und bei 100°C unter Rühren zu einer Lösung von 2,03 g (0,016 Mol) 2-Thio-uracil, 0,38 g (0,016 Mol) Lithiumhydroxid und 1 g Natriumiodid in 50 ml Dimethylformamid innerhalb 1 h zugetropft. Nach 3-stündiger Reaktion wurde das Lösungsmittel im Vakuum abdestilliert, der Rückstand mit 150 ml Wasser versetzt und zweimal mit Essigsäureethylester extrahiert. Der nach Waschen mit Wasser, Trocknen mit Natriumsulfat und Einengen hinterbliebene Rückstand wurde chromatographisch gereinigt. (Flash-Chromatographie, Kieselgel, Laufmittel Methylenchlorid mit 2,5-5% Methanol)
Ausbeute: 4 g helles Öl
NMR:CDCl₃ . δ 1,3(s,9H); 1,85-2,25(m,4H); 2,6(m,4H); 2,8(m,2H); 3,2(t,2H); 3,5(m,2H); 4,0(m,2H); 6,2(d,1H); 6,5(s,1H); 7,8(d;1H)
Durch Versetzen einer ethanolischen Lösung mit Fumarsäure wurde die Substanz als Fumarat erhalten.
C₂₁H₂₉F₃N₆OS · C₄H₄O₄ MG 586,6
Fp. 188-189°C

Unter Verwendung verschiedener Halogenalkyl-1,4-Diazepine (wie z.B. 1d) und verschiedener mercaptosubstituierter Heterocyclen wie Thiouracil, 5-Amino-2-mercapto-triazole und 5-Amino-2-mercapto-thiadiazol wurden in analoger Weise die nachfolgend in Tabelle 1 aufgeführten Verbindungen erhalten:

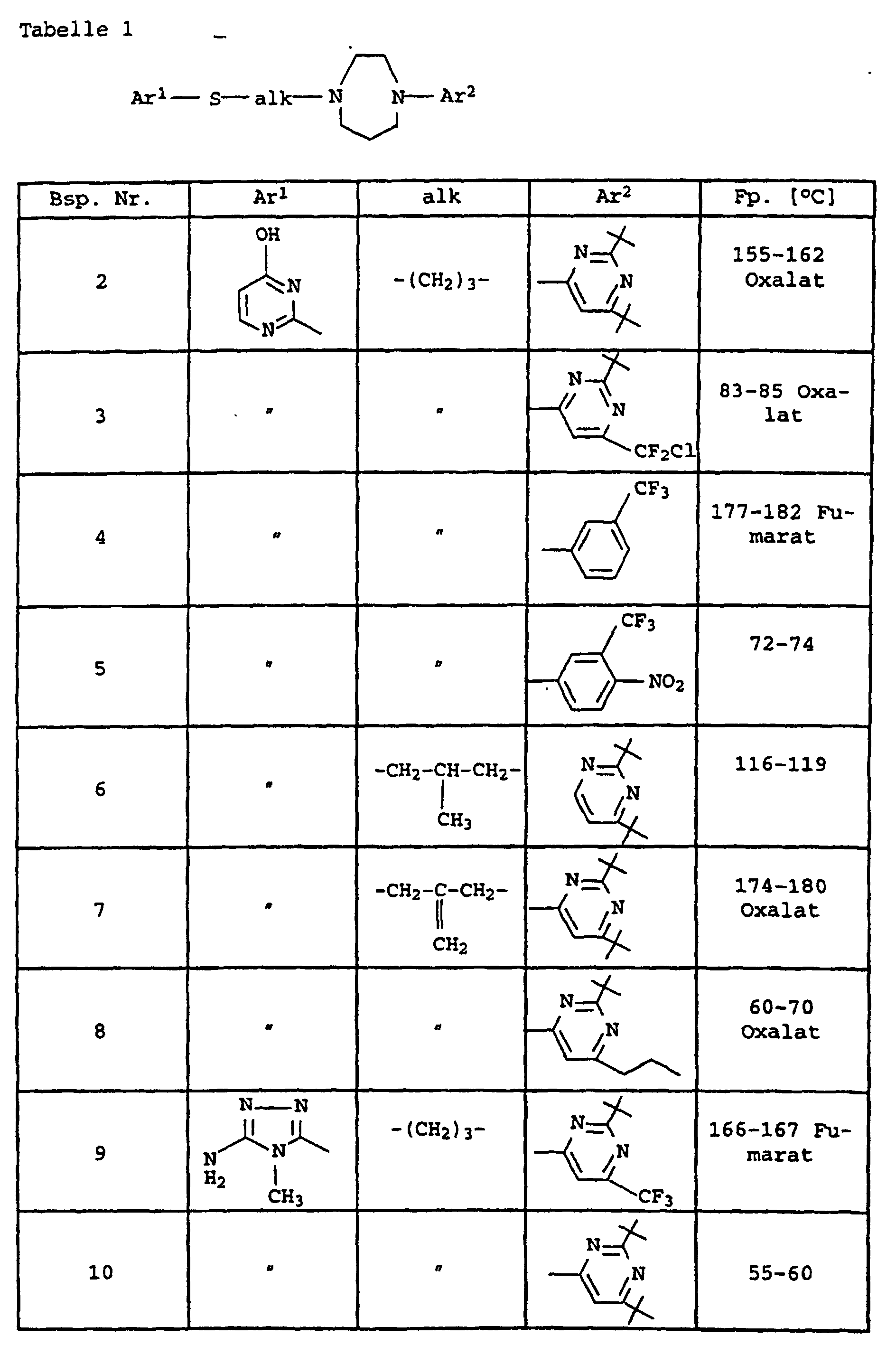

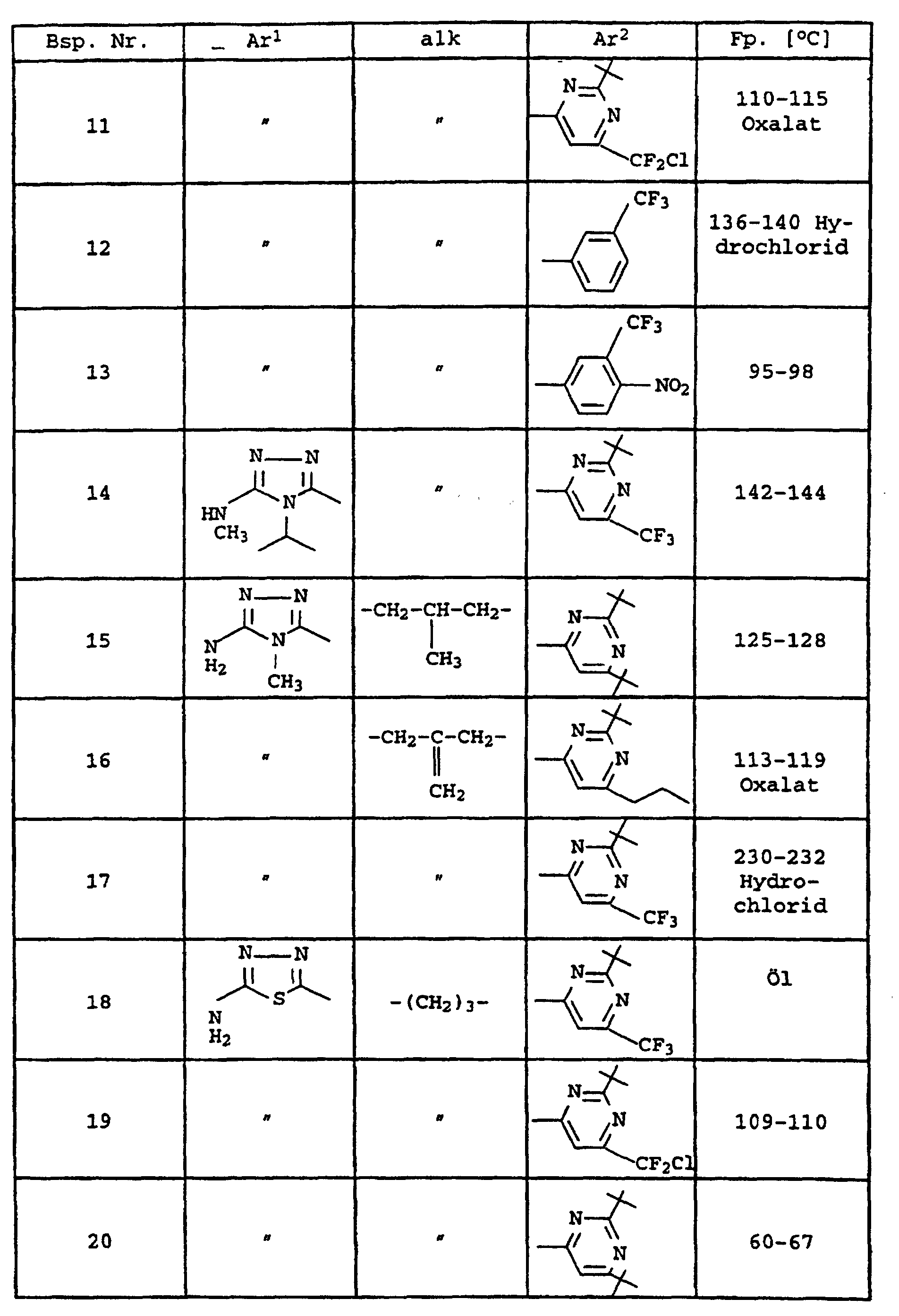

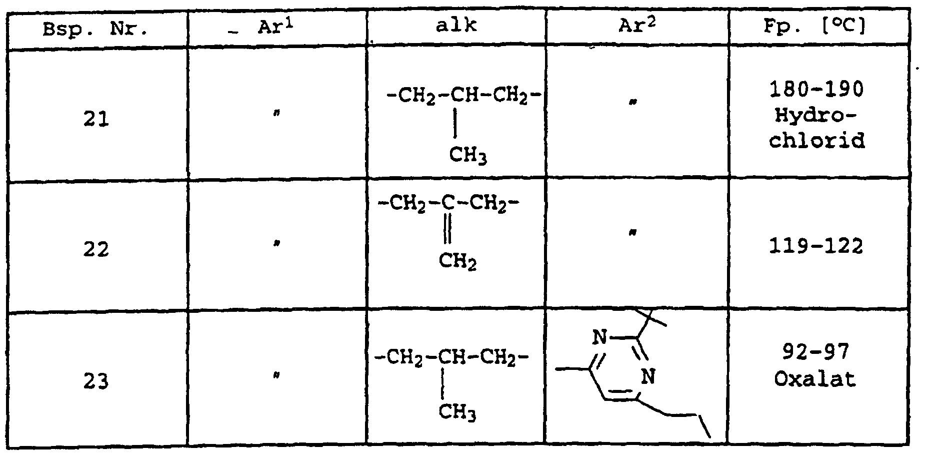

### Beispiel 24:

4-Brombenzoesäure-[4-{-4-(2,6-bis-t-butyl-pyrimidin-4-yl)}-(hexahydro-(1H)-1,4-diazepin-1-yl-)butyl]amid

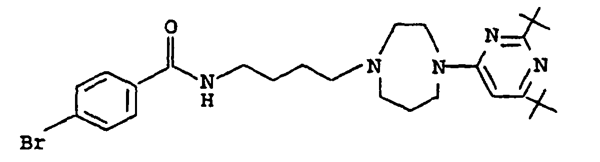

### Herstellung der Ausgangsprodukte

a) Hexahydro-1H-1-[2-t-butyl-6-trifluormethyl-pyrimidin-4-yl]-4-(4-phthalimido-butyl)-(1H)-1,4-diazepin
   10 g (0,033 Mol) des nach Beispiel 1c) hergestellten Diazepins wurden mit 9,8 g (0,035 Mol) N-(4-Brombutyl)-phthalimid und 9,1 g (0,066 Mol) Kaliumcarbonat in 120 ml Acetonitril 8 h zum Sieden unter Rückfluß erhitzt. Anschließend wurde filtriert und das Filtrat wurde eingeengt. Der Rückstand wurde roh weiterverarbeitet.
   Ausbeute: 16,2 g (98% d.Th.)
   Eine Probe wurde aus Ethanol umkristallisiert.
   Fp. 97-99°C
   Auf analoge Weise wurden erhalten: 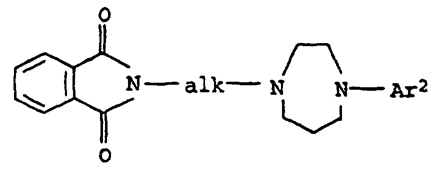 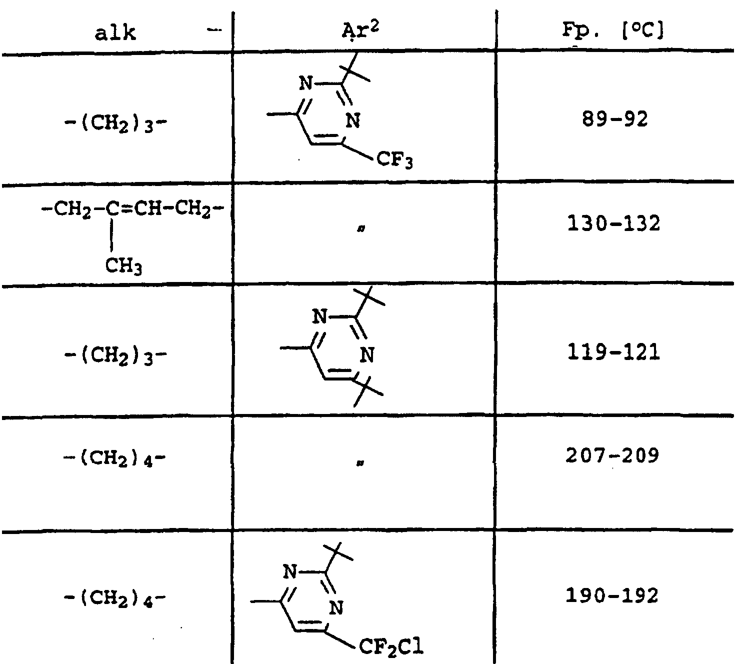
b) Hexahydro-1H-1-[2-t-butyl-6-trifluormethylpyrimidin-4-yl]-4-(4-aminobutyl)-1,4-diazepin
   15 g (0,03 Mol) des vorstehend unter a) beschriebenen Produkts wurden mit 6 g Hydrazinhydrat in 200 ml Ethanol 2 h unter Rühren zum Sieden erhitzt; anschließend wurde der Niederschlag abgesaugt und das Filtrat eingedampft. Der Rückstand wurde in Essigsäureethylester aufgenommen, nochmals filtriert, mit Wasser gewaschen, getrocknet und wiederum eingedampft.
   Es wurden erhalten:
   9,2 g (83% d.Th.) als Öl
   Auf analoge Weise wurden erhalten:

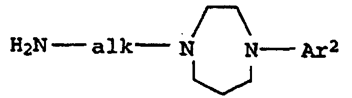

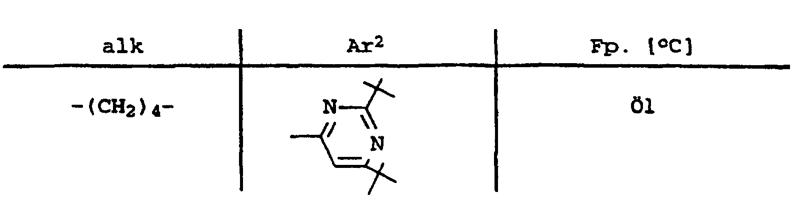

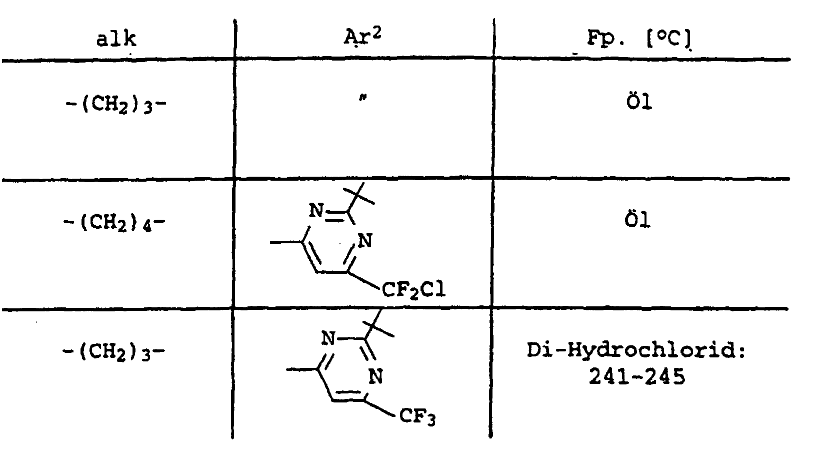

### Herstellung der Endprodukte:

3 g (0,0083 Mol) des vorstehend unter b) erhaltenen Produkts wurden mit 0,9 g (0,009 Mol) Triethylamin in 60 ml Tetrahydrofuran gelöst und eine Lösung von 2 g (0,009 Mol) 4-Brombenzoylchlorid in 10 ml Tetrahydrofuran wurde innerhalb 10 min. bei Raumtemperatur zugetropft. Nach 1 h wurde das Lösungsmittel im Vakuum abdestilliert, der Rückstand mit Wasser versetzt und zweimal mit Methylenchlorid extrahiert. Die getrocknete und eingeengte Lösungsmittelphase wurde mittels Flash-Chromatographie (Kieselgel, Laufmittel Methylenchlorid mit 3% Methanol) gereinigt.
Ausbeute: 4,2 g (93% d.Th.)
Fp. 125-127°C (aus Diisopropylether/Isopropanol)
C₂₈H₄₂BrN₅O (544,6)

Unter Verwendung verschiedener Aminoderivate (analog 24b) und bekannter Benzoylchloride wurden die nachstehend in Tabelle 2 aufgeführten Verbindungen erhalten.

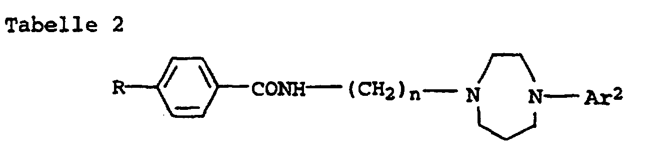

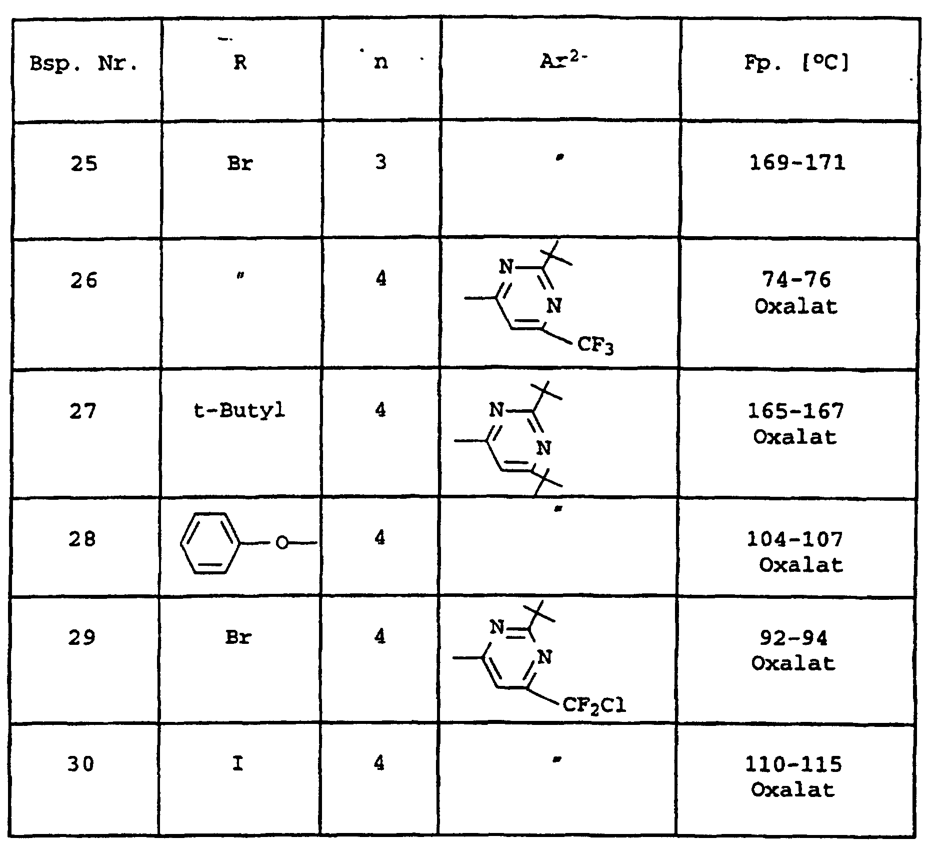

### Beispiel 31

4-[4-{4-Benzyloxy-pyrimidin-2-yl}aminobutyl]-1-[2-t-butyl-6-trifluormethyl-pyrimidin-4-yl]hexahydro-1H-1,4-diazepin-oxalat

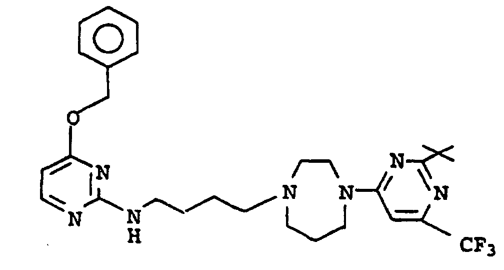

2,7 g (0,007 Mol) der nach Beispiel 24b) hergestellten Aminoverbindung wurden mit 0,3 g Natriumhydrid (0,009 Mol) in 20 ml Dimethylformamid vorgelegt. Nach 1 h Reaktionszeit wurden 1,6 g (0,006 Mol) 4-Benzyloxy-2-methylsulfonylpyrimidin (hergestellt durch Oxidation von 4-Benzyloxy-2-methylmercapto-pyrimidin), gelöst in 10 ml Dimethylformamid, zugegeben und 72 h bei Raumtemperatur gerührt.
Anschließend wurde mit Wasser versetzt, mit Essigsäureethylester extrahiert, getrocknet und die Lösung eingeengt. Der Rückstand wurde säulenchromatographisch gereinigt (Kieselgel, Methylenchlorid mit 4% Methanol)
Reinausbeute: 1,0 g (30% d.Th.)
Oxalat: Fp. 145-150°C
C₂₉H₃₈F₃N₇O · C₂H₂O₄ (647,7)

### Beispiel 32:

1-[2-t-butyl-6-trifluormethyl-pyrimidin-4-yl]-4-[4-{4-hydroxy-pyrimidin-2-yl}aminobutyl]hexahydro-(1H)-1,9-diazepin

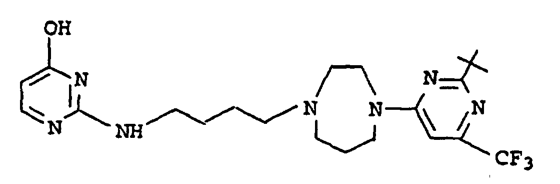

0,7 g (0,001 Mol) der im vorstehenden Beispiel beschriebenen Verbindung wurden in Methanol unter Normalbedingungen mit Palladium auf Kohle-Katalysator (10% Pd) hydriert.
Ausbeute: 0,6 g (100% d.Th.)
Fp. 111-115°C
C₂₂H₃₂F₃N₇O·C₂H₄O₄ (557,5)

### Beispiel 33:

1-[2-t-Butyl-6-trifluormethyl-pyrimidin-4-yl]-4-[4-(4-hydroxypyrimidin-2-yl)butyl-hexahydro-1H-1,4-diazepin 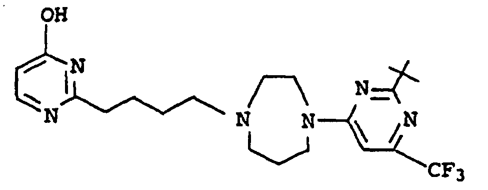
a) 1-[2-t-Butyl-6-trifluormethyl-pyrimidin-4-yl]-4-(4-cyanobutyl)-hexahydro-1,4-diazepin
   9,1 g (0,03 Mol) des Diazepins aus Beispiel 1c) wurden mit 3,5 g (0,03 Mol) 5-Chlorvaleronitril, 9,1 g Triethylamin (0,09 Mol) in 100 ml Dimethylformamid gelöst und 24 h auf 100° erwärmt.
   Anschließend wurde das Lösungsmittel im Vakuum abdestilliert, Wasser zugegeben, mit Essigsäureethylester extrahiert, diese Phase mit Natriumsulfat getrocknet und eingeengt. Der Rückstand wurde roh weiterverarbeitet.
   Ausbeute: 9,1 g als braunes Öl
b) 1-[2-t-Butyl-6-trifluormethyl-pyrimidin-4-yl]-4-(4-amiclinobutyl)-hexahydro-1,4-diazepinhydrochlorid
   9,1 g (0,024 Mol) des vorstehend beschriebenen Nitrils wurden in 2 ml Ethanol und 50 ml Methylenchlorid (beides wasserfrei) gelöst und bei 0-10° unter Kühlung wurde trockenes Chlorwasserstoffgas bis zur Sättigung eingeleitet. Nach Rühren über Nacht wurde der Niederschlag abgesaugt und das Filtrat eingeengt.
   Ausbeute: 7,6 g (58% d.Th.)

### Herstellung des Endprodukts

4,4 g (0,01 Mol) des vorstehend beschriebenen Amidins wurden mit der Natriumverbindung des Formylessigsäureethylesters (Herstellung J. Org. Chem. 35 (1970), 2515 ff) (2,8 g (0,02 Mol)) in 50 ml Wasser und 20 ml Tetrahydrofuran über Nacht gerührt. Der Reaktionsansatz wurde dann mehrfach mit Essigsäureethylester extrahiert, die organische Phase wurde getrocknet und eingeengt. Der Rückstand wurde säulenchromatographisch gereinigt (Kieselgel, Elutionsmittel Methylenchlorid mit 4% Methanol)
Ausbeute: 1,9 g (42%) Öl
NMR: (CDCl₃) δ: 1,3(s,9H); 1,8-2,0(m,4H); 2.0(m,2H); 2,4-2,6(m/br,6H); 2,5(t,2H); 3,5(m,1H); 4,0(m,2H); 6.2(d,1H); 6,5(s,1H); 7,8(d,1H)
C₂₂H₃₁F₃N₆O (425,5)
Oxalat: C₂₂H₃₁F₃N₆O · C₂H₂O₄ (542,5)
Fp. 173-177° (Z)

### Beispiel 34

1-[2-t-Butyl-6-trifluormethyl-pyrimidin-4-yl]-4-[3-{4-benzyloxypyrimidin-2-yl}oxypropyl]-hexahydro-(1H)-1,4-diazepin 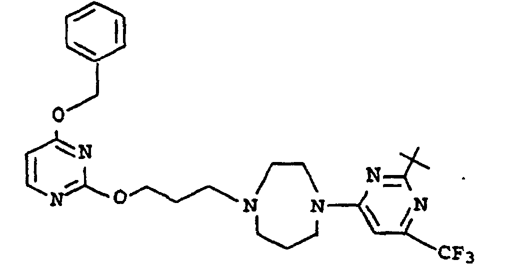
a) Ausgangsprodukt
   8,9 g (64,5 mmol) 3-Brom-propanol-1 wurde in 50 ml THF_{abs} aufgenommen und nacheinander mit 6,52 g (64,5 mmol) Triethylamin einer katalytischen Menge Natriumjodid sowie 16,2 g (53,7 mmol) des unter Beispiel 1c) hergestellten Azepins versetzt und 16 h unter Rückfluß erhitzt. Zur Aufarbeitung wurde von den ausgefallenen Salzen abfiltriert und die Mutterlauge i. Vak. eingeengt. Das erhaltene Öl nahm man in Dichlormethan auf, wusch die organische Phase mit Wasser, trocknete über Natriumsulfat und reinigte dann mittels Säulenchromatographie (SiO₂, Laufmittel CH₂Cl₂:MeOH=98:2) und erhielt ein farbloses Öl.
   Ausbeute: 10,11 g (53%)
b) Endprodukt
   2,45 g des vorstehend beschriebenen Produkts, gelöst in 25 ml DMF_{abs}, wurden bei Raumtemperatur unter Schutzgasatmosphäre mit 0,26 g (8,52 mmol) Natriumhydrid (80%) portionsweise versetzt und 30 min. nachgerührt. Dann tropfte man 1,5 g (5,68 mmol) 2-Methansulfonyl-4-benzyloxy-pyrimidin (wurde analog Literatur hergestellt: W.E. Barnett, R.F. Koebel Tetrahedron Lett. 1971, 20, 2867) gelöst in 15 ml DMF_{abs} zu. Nach 7 h wurde zur Aufarbeitung auf Wasser gegossen, mit tert.-Butyl-methylether extrahiert, die organische Phase mit Wasser gewaschen, über Natriumsulfat getrocknet, filtriert und i.Vak. eingeengt. Aufreinigung des erhaltenen Öls mittels Säulenchromatographie (SiO₂, Laufmittel CH₂Cl₂:MeOH = 98:2) ergab die Substanz als Öl.
   Ausbeute: 1,6 g (2,9 mmol, 52%)
   Zur Bildung des Hydrochlorids wurde das in Essigester/Et₂O gelöste Öl unter Schutzgas mit etherischer Salzsäure versetzt und das entstandene Salz abgesaugt.
   Fp.: 110-112°C
   C₂₈H₃₆ClF₃N₆O₂ (581,1)

### Beispiel 35

1-[2-t-Butyl-6-trifluormethyl-pyrimidin-2-yl]-4-[3-(4-hydroxypyrimidin-2-yl)oxypropyl]hexahydro-(1H)-1,4-diazepin

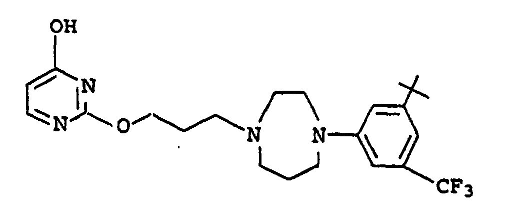

1,4 g (2,6 mmol) der Substanz des Beispiels 34, gelöst in 40 ml Essigester, wurden bei Raumtemperatur mit 0,2 g Pd/C (10% Pd) versetzt und bei 40-50°C unter Atmosphärendruck mit Wasserstoff hydriert. Nach beendeter Reaktion saugte man den Katalysator ab, wusch mit Essigester nach und engte i. Vak. ein.
Ausbeute: 1,2 g (100%)
Zur Bildung des Hydrochlorids wurde das in Essigester/Et₂O gelöste Öl unter Schutzgas mit etherischer Salzsäure versetzt und das entstandene Salz abgesaugt.
Fp.: 78-80°C
C₂₁H₃₀ClF₃N₆O₂ (491)

In analoger Weise erhält man die in den nachfolgenden Tabellen 3 bis 17 genannten Verbindungen.

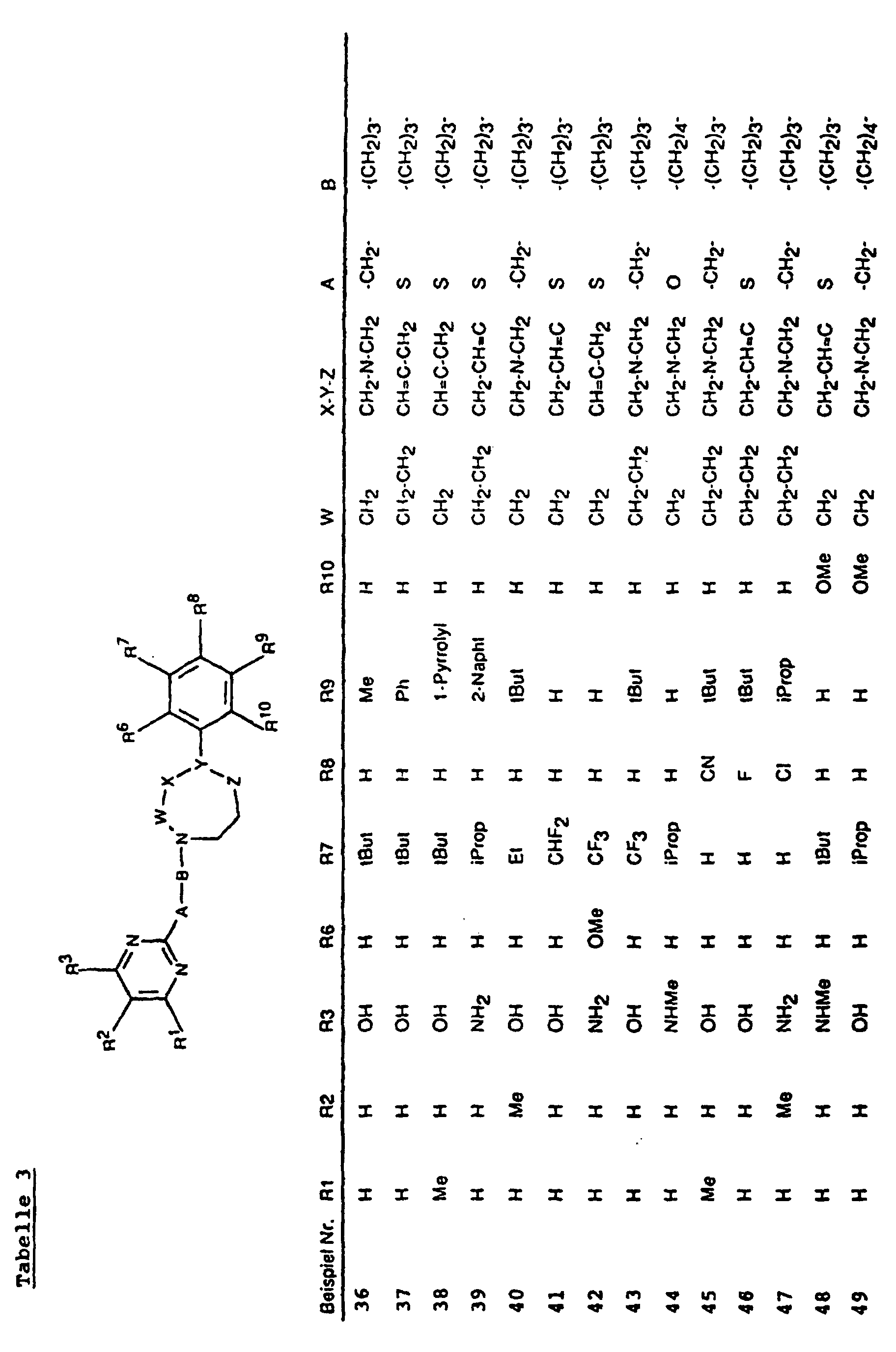

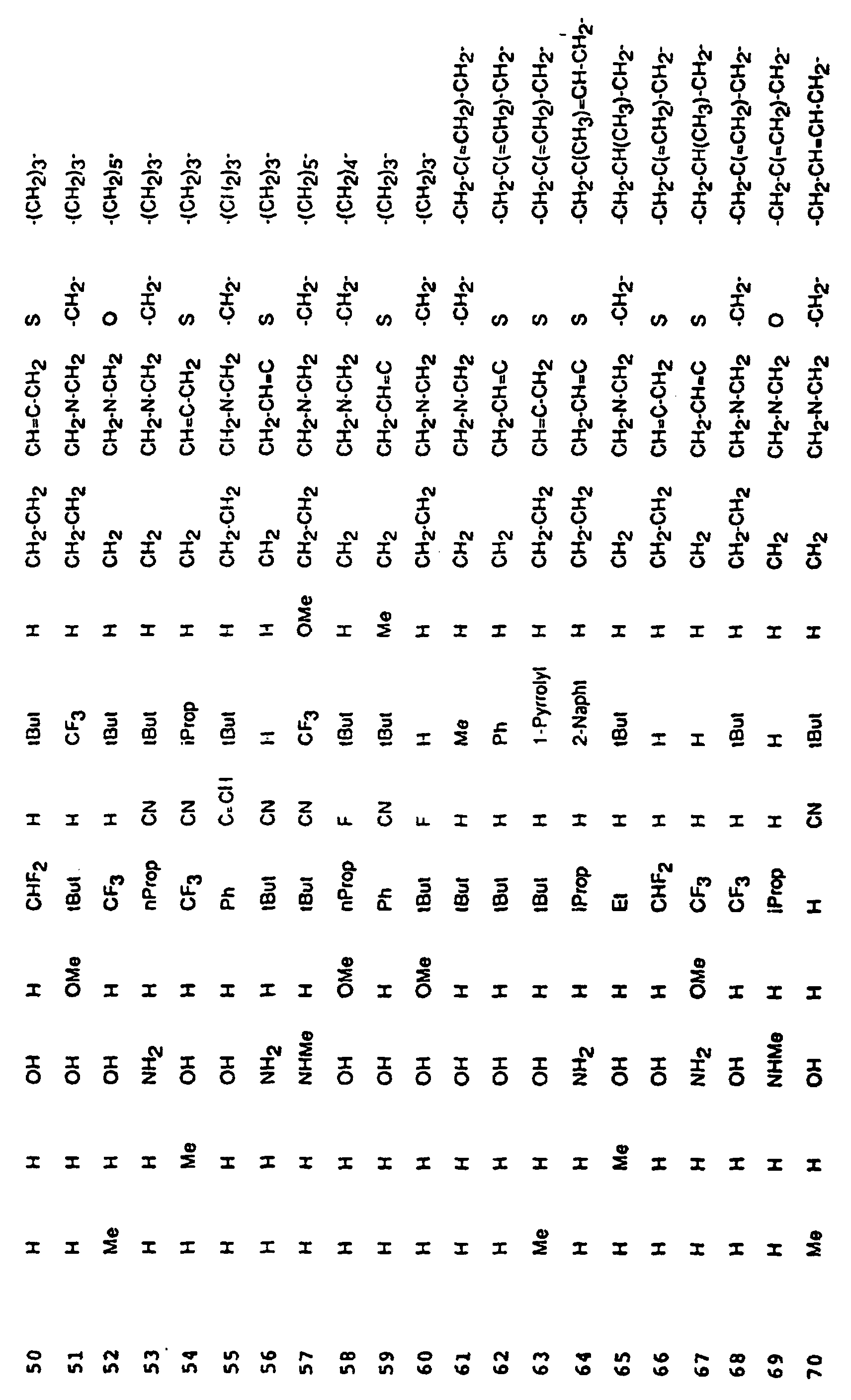

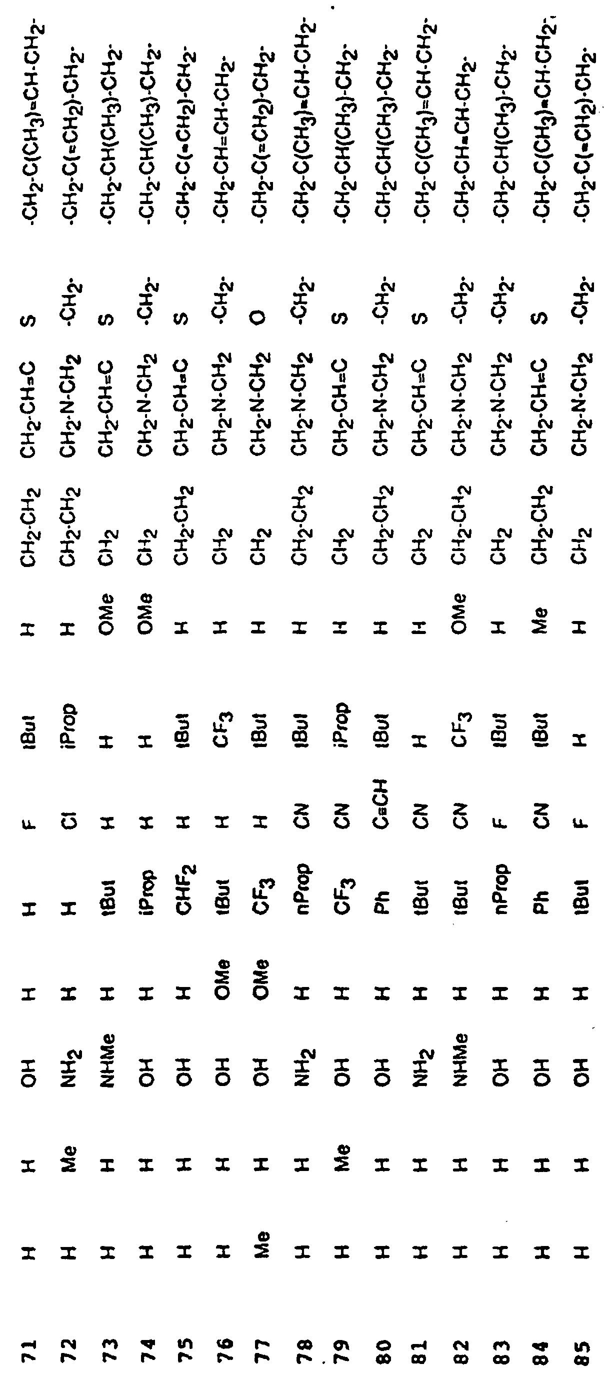

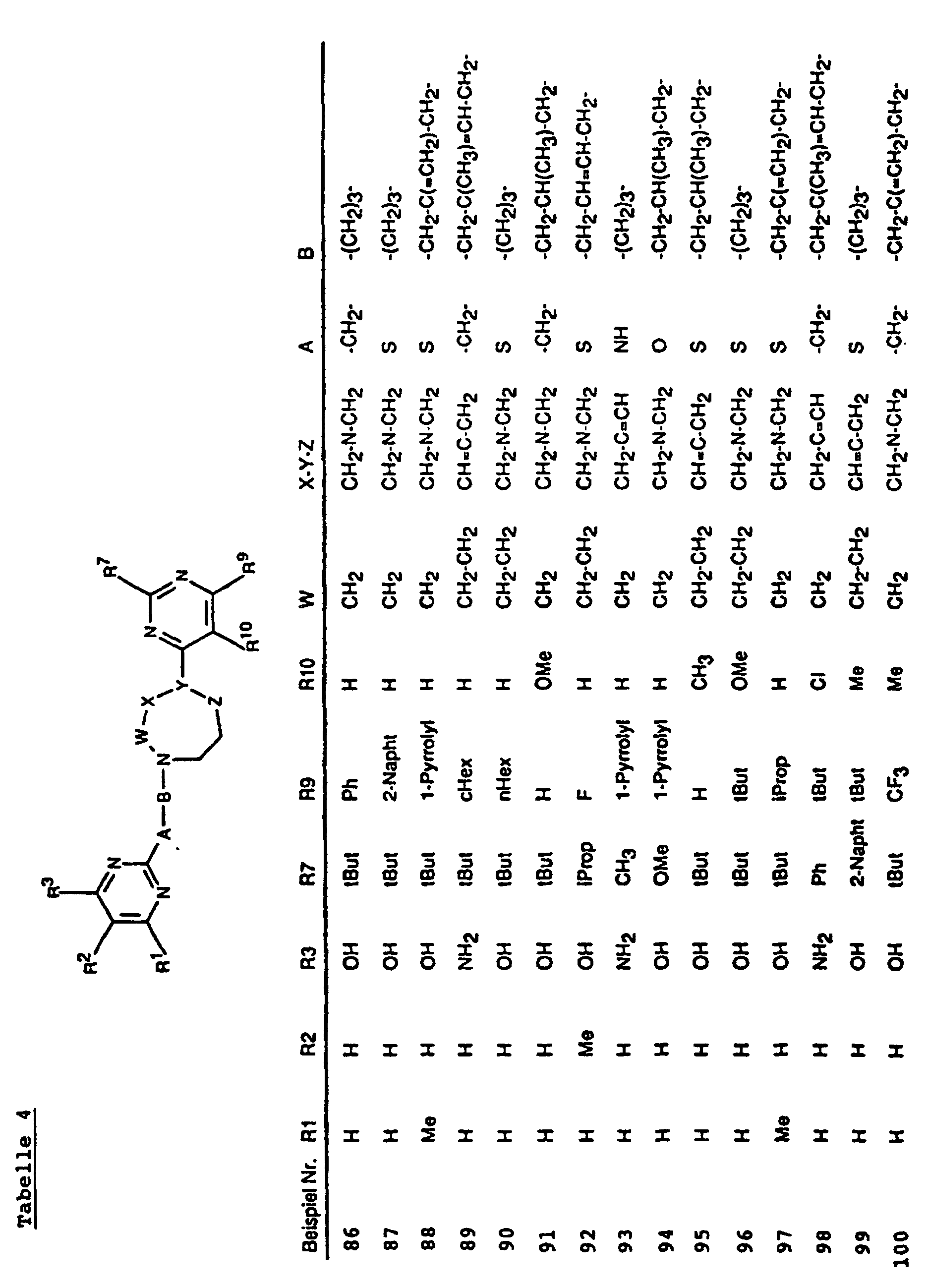

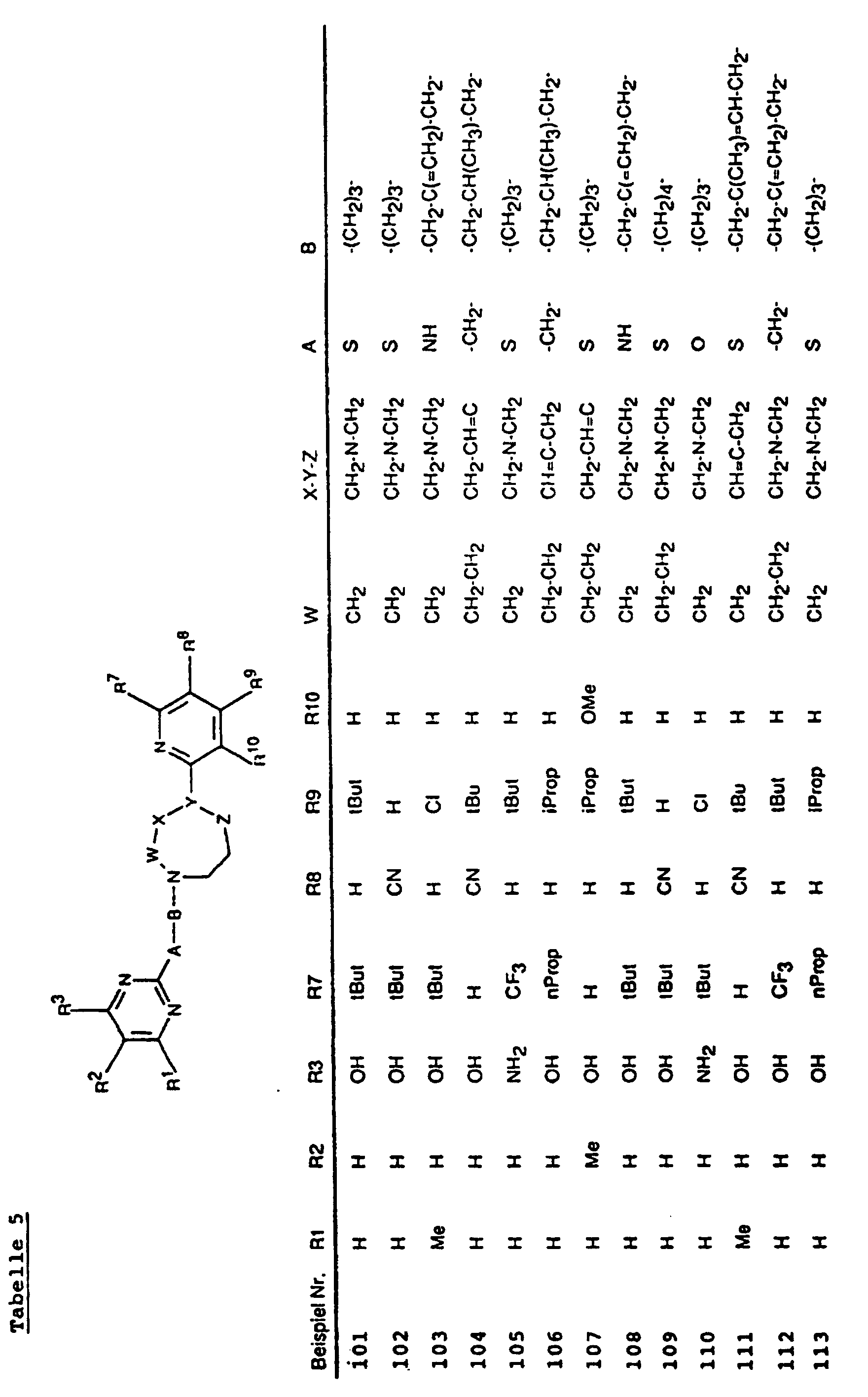

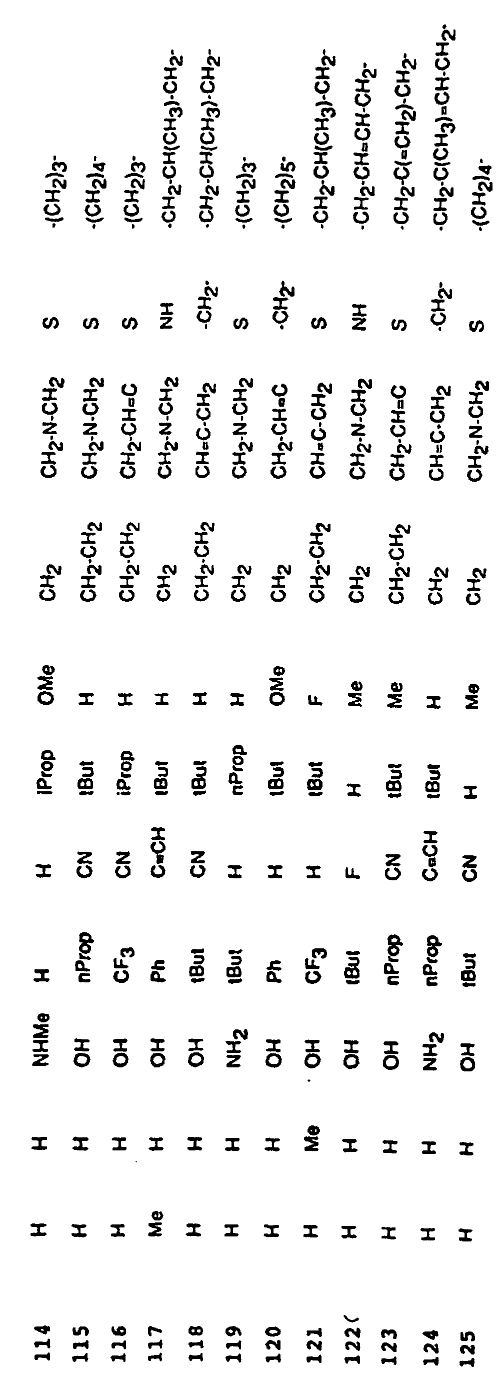

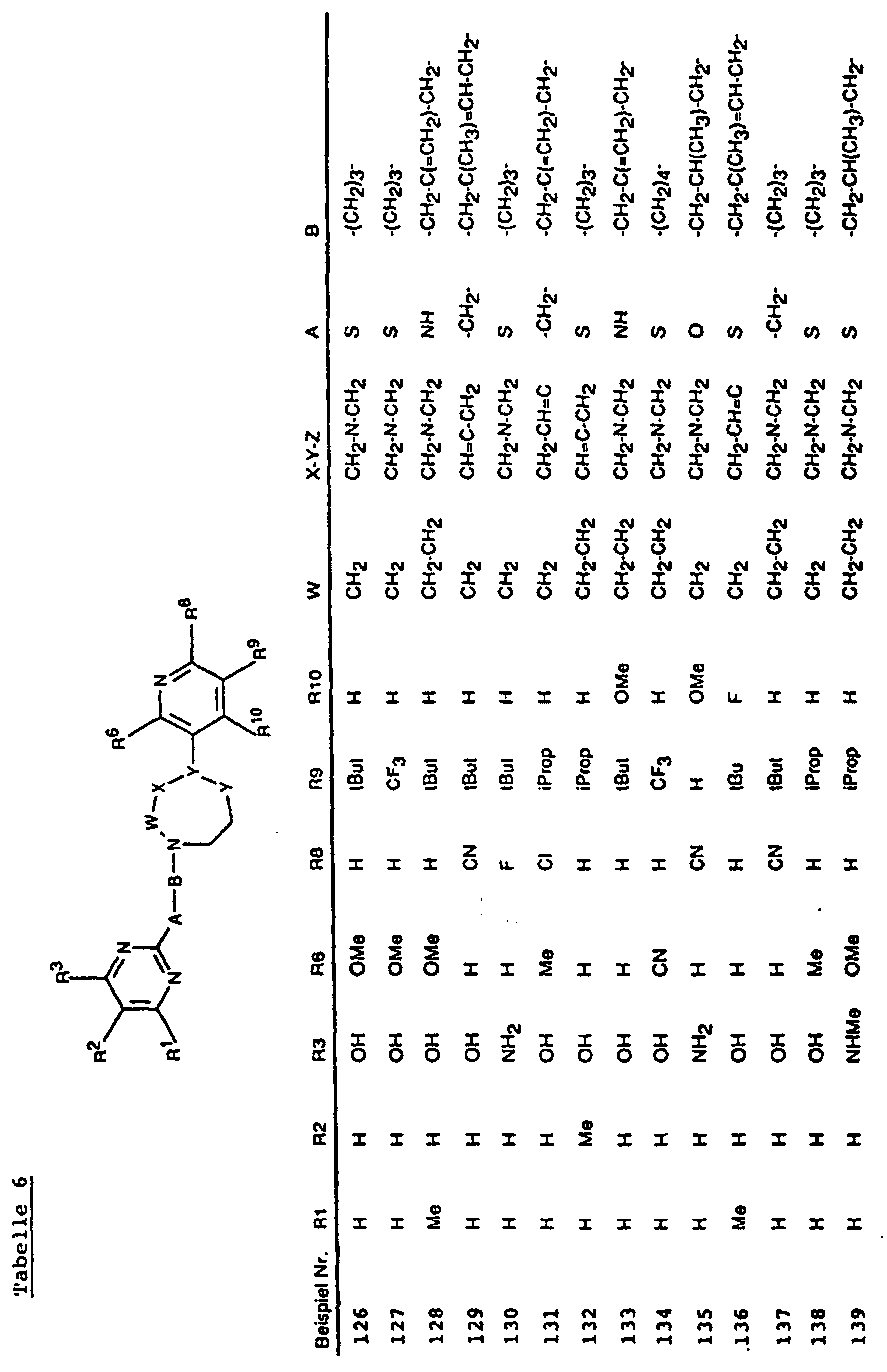

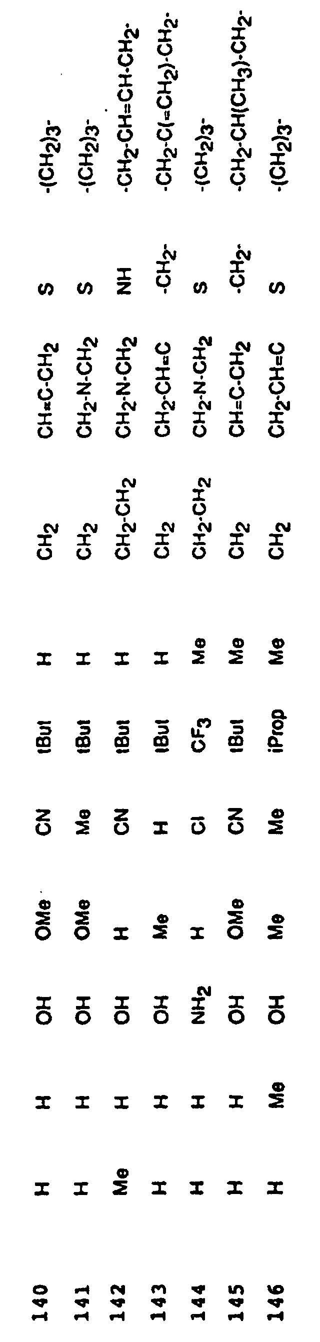

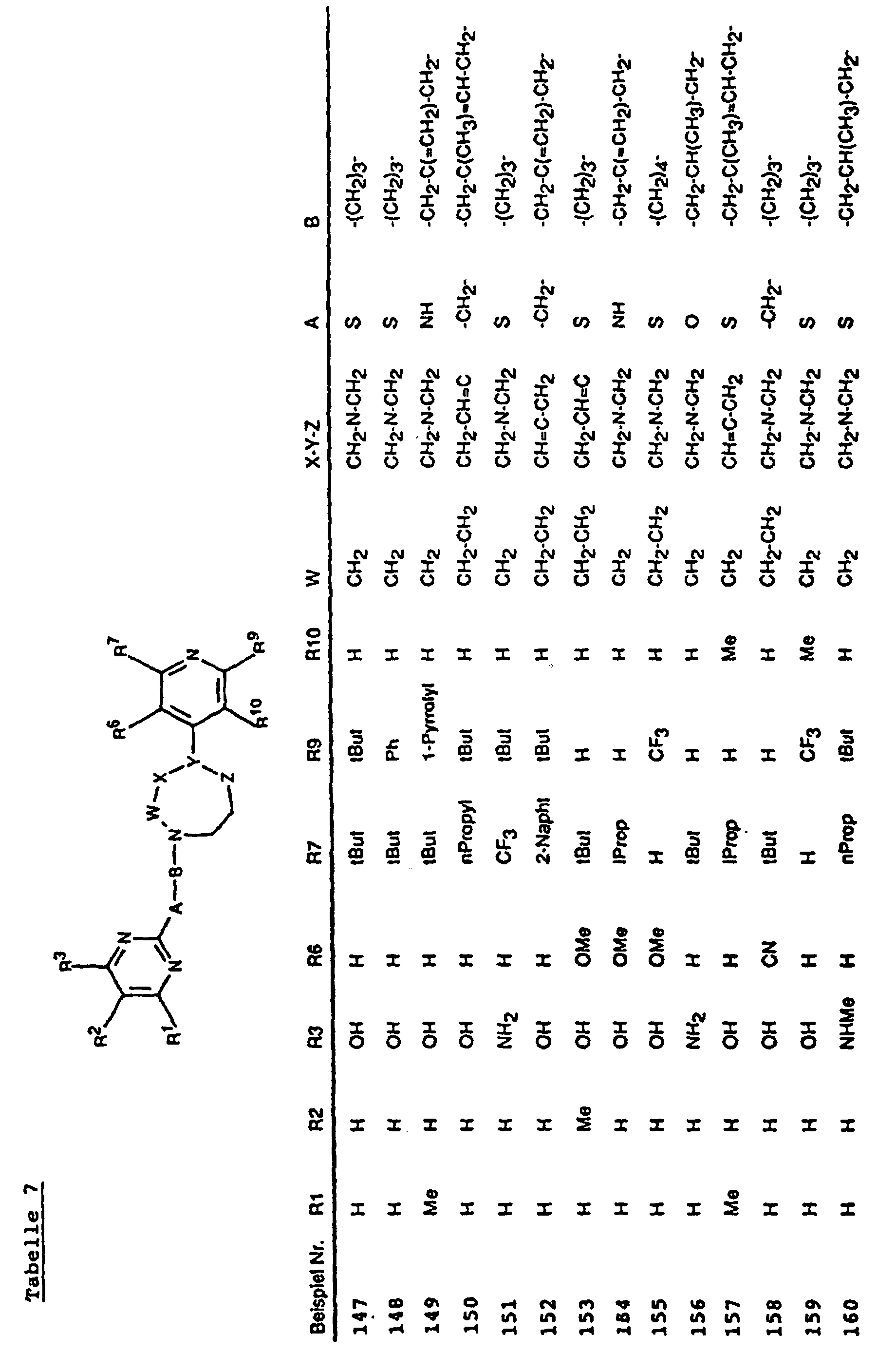

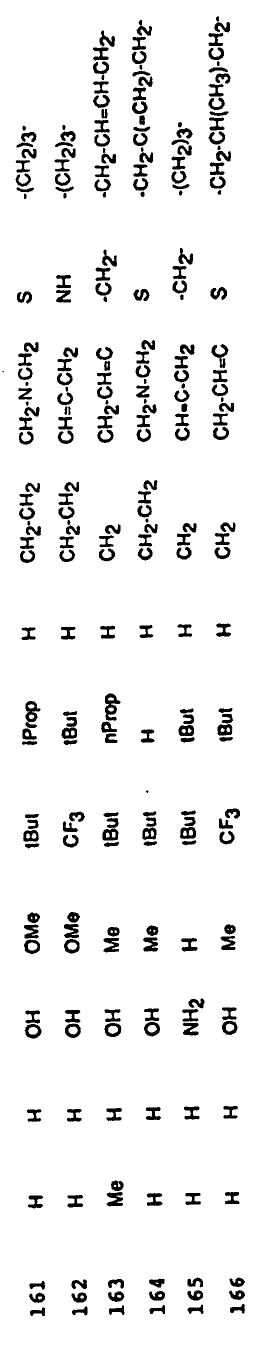

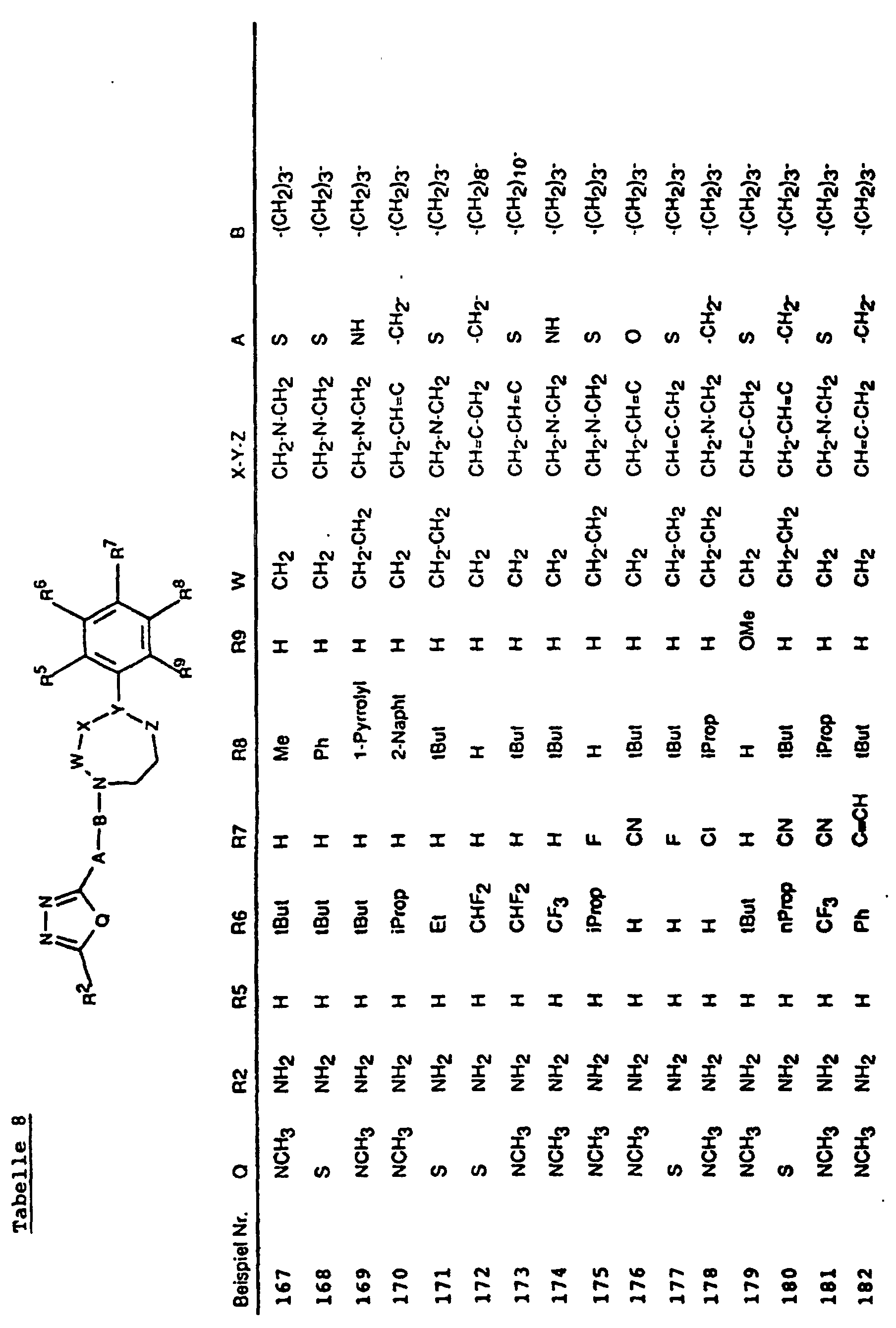

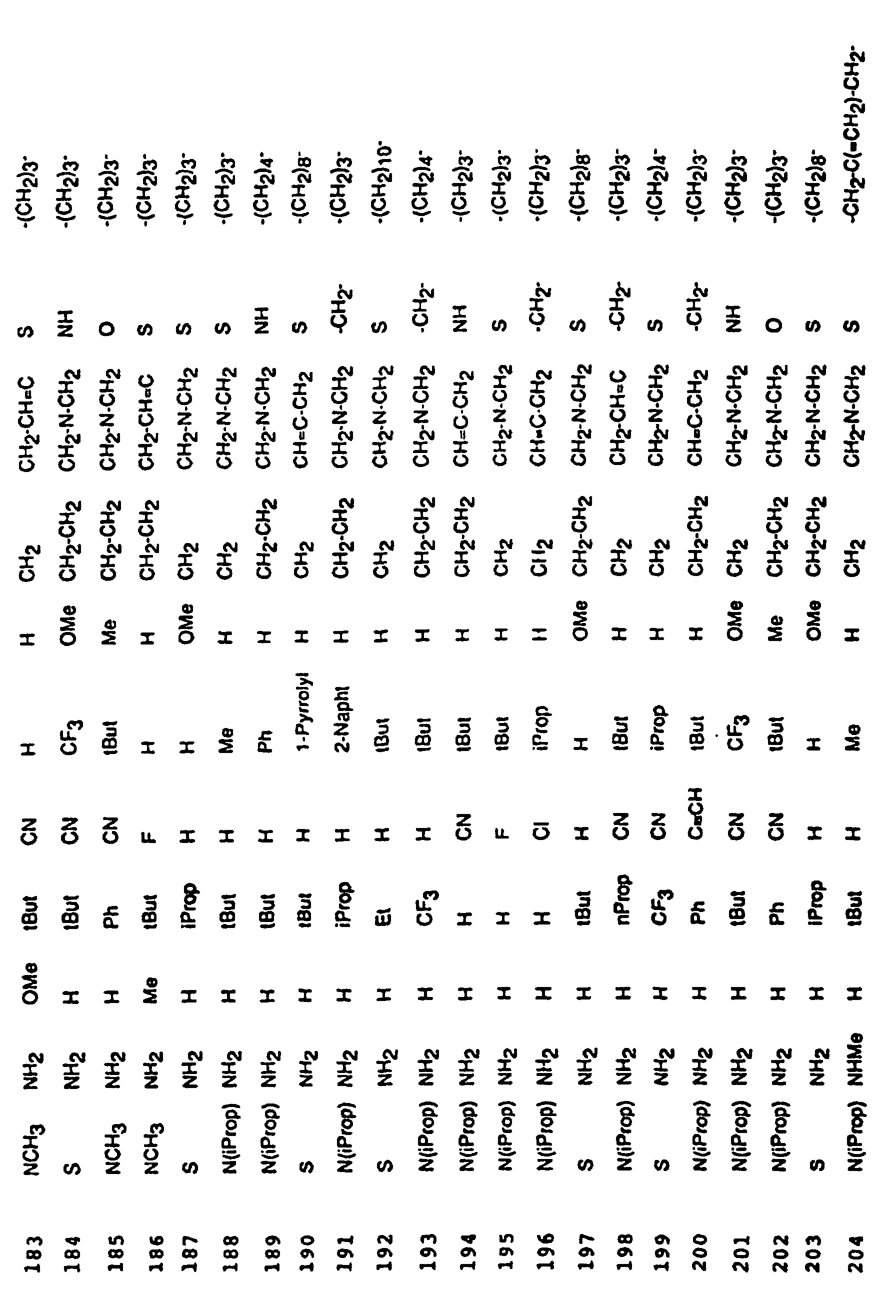

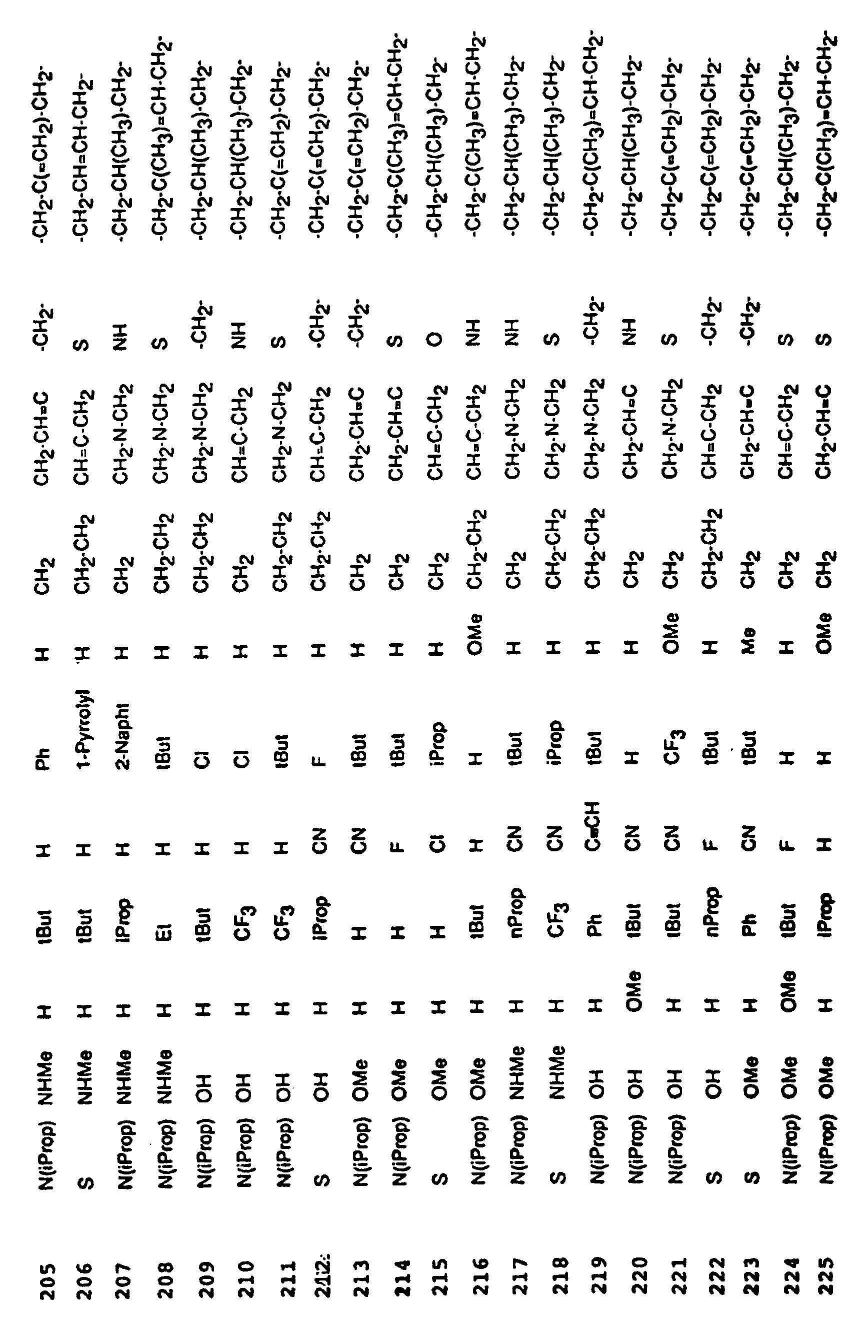

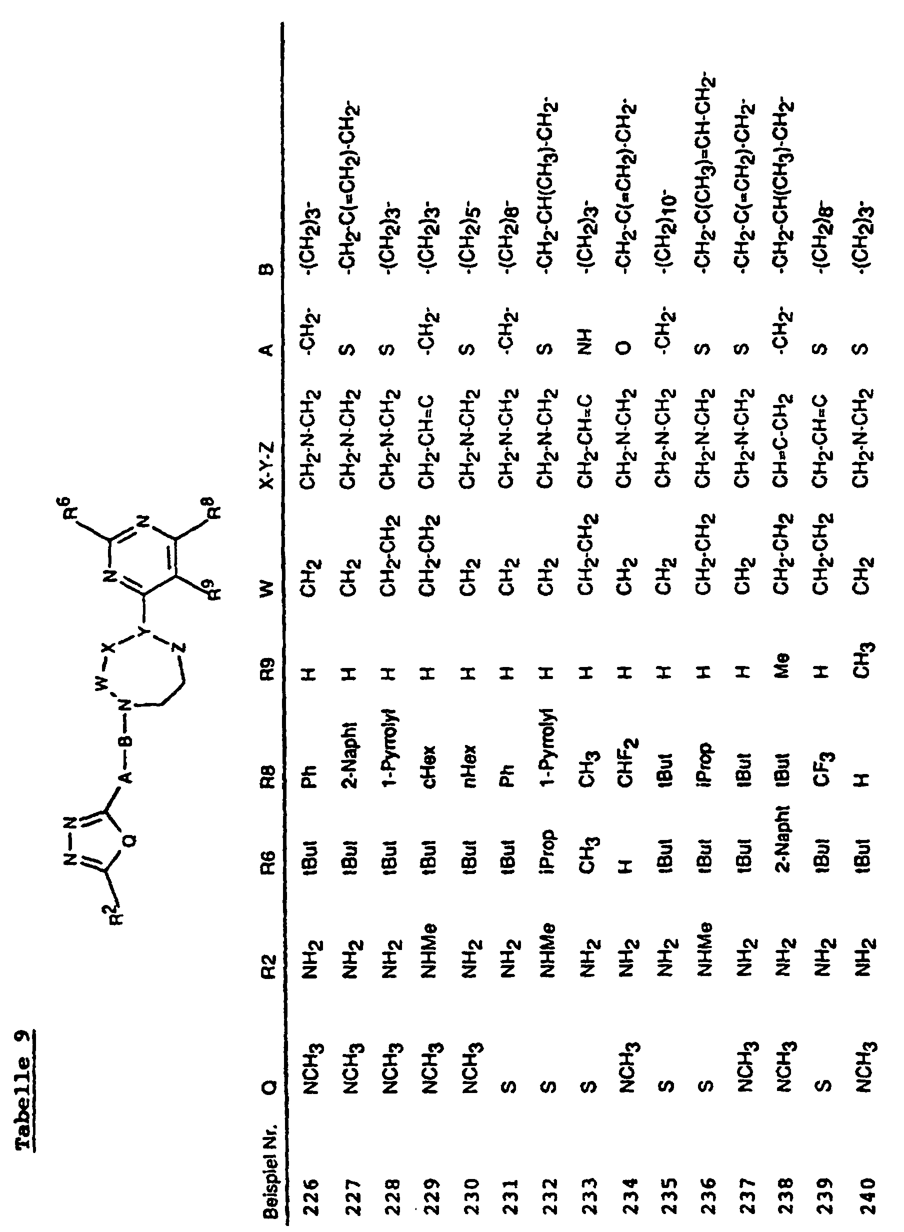

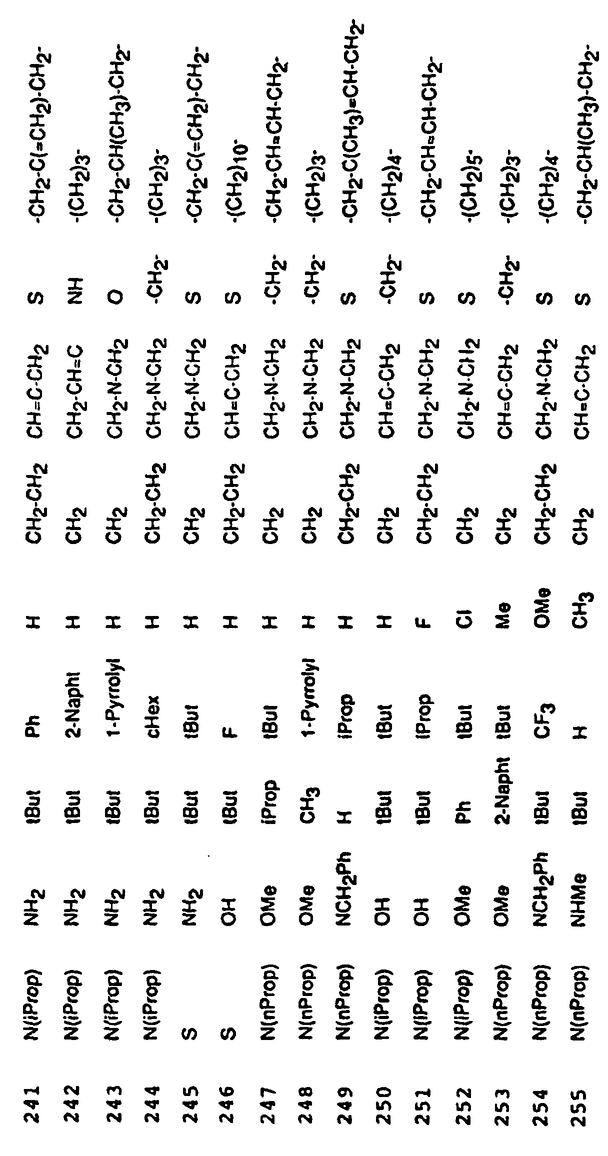

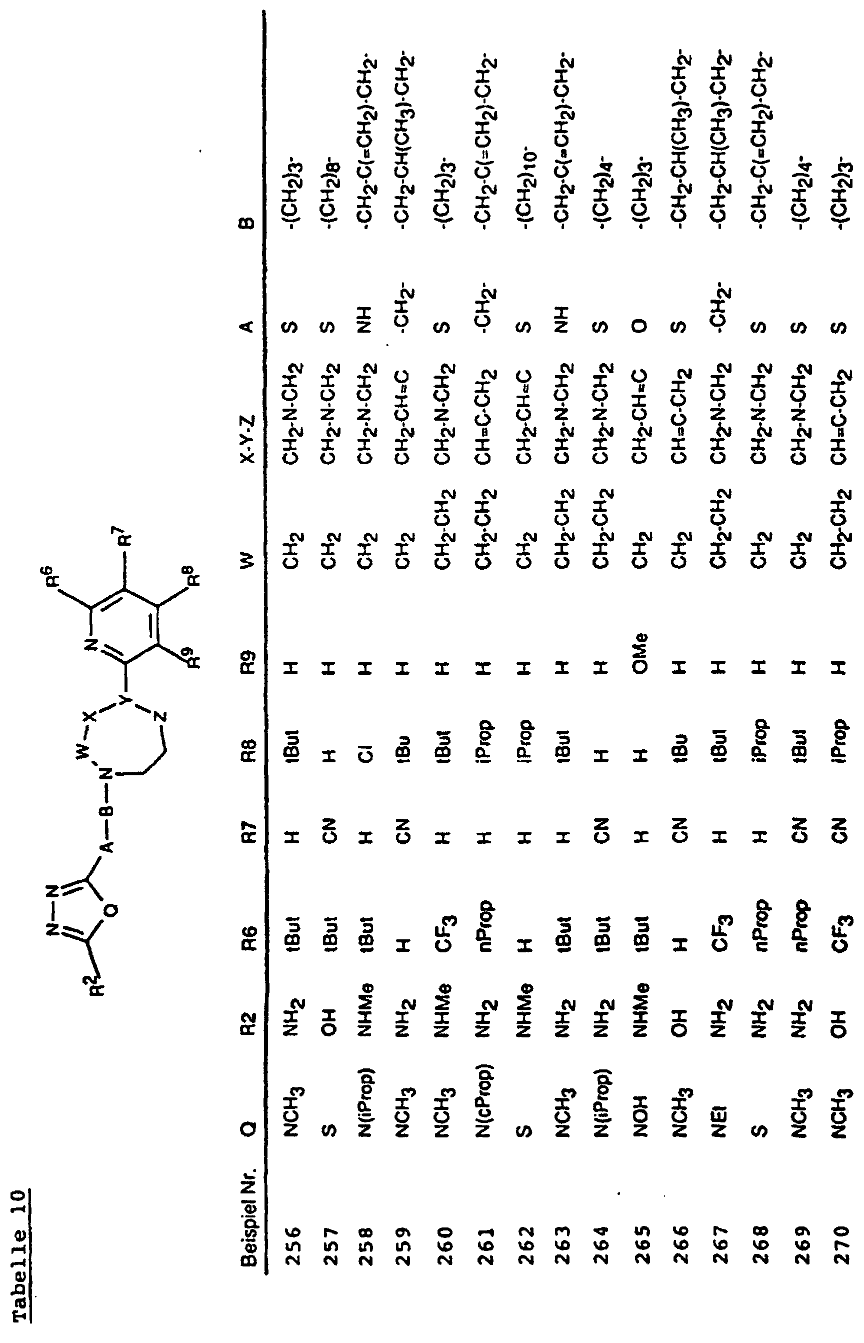

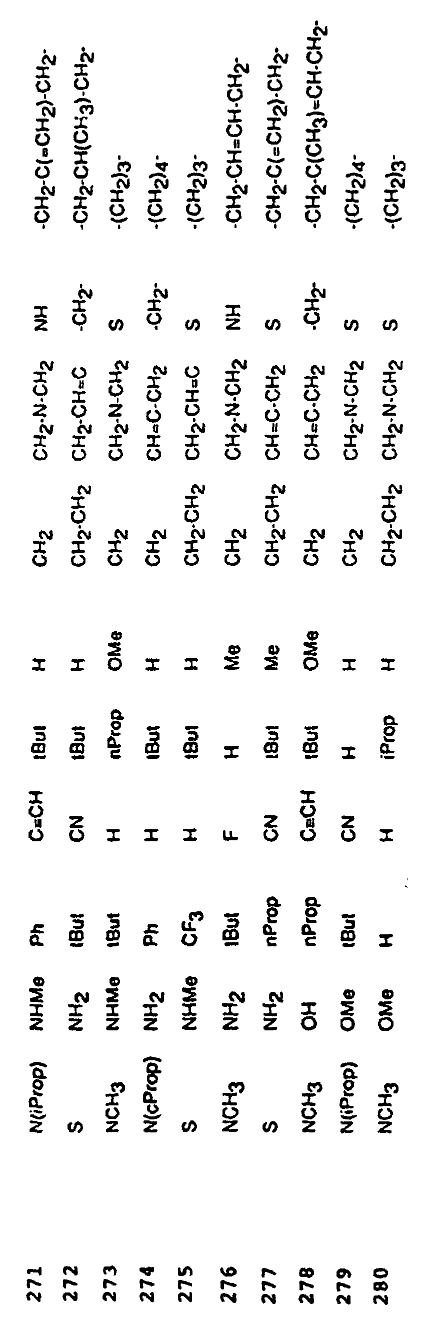

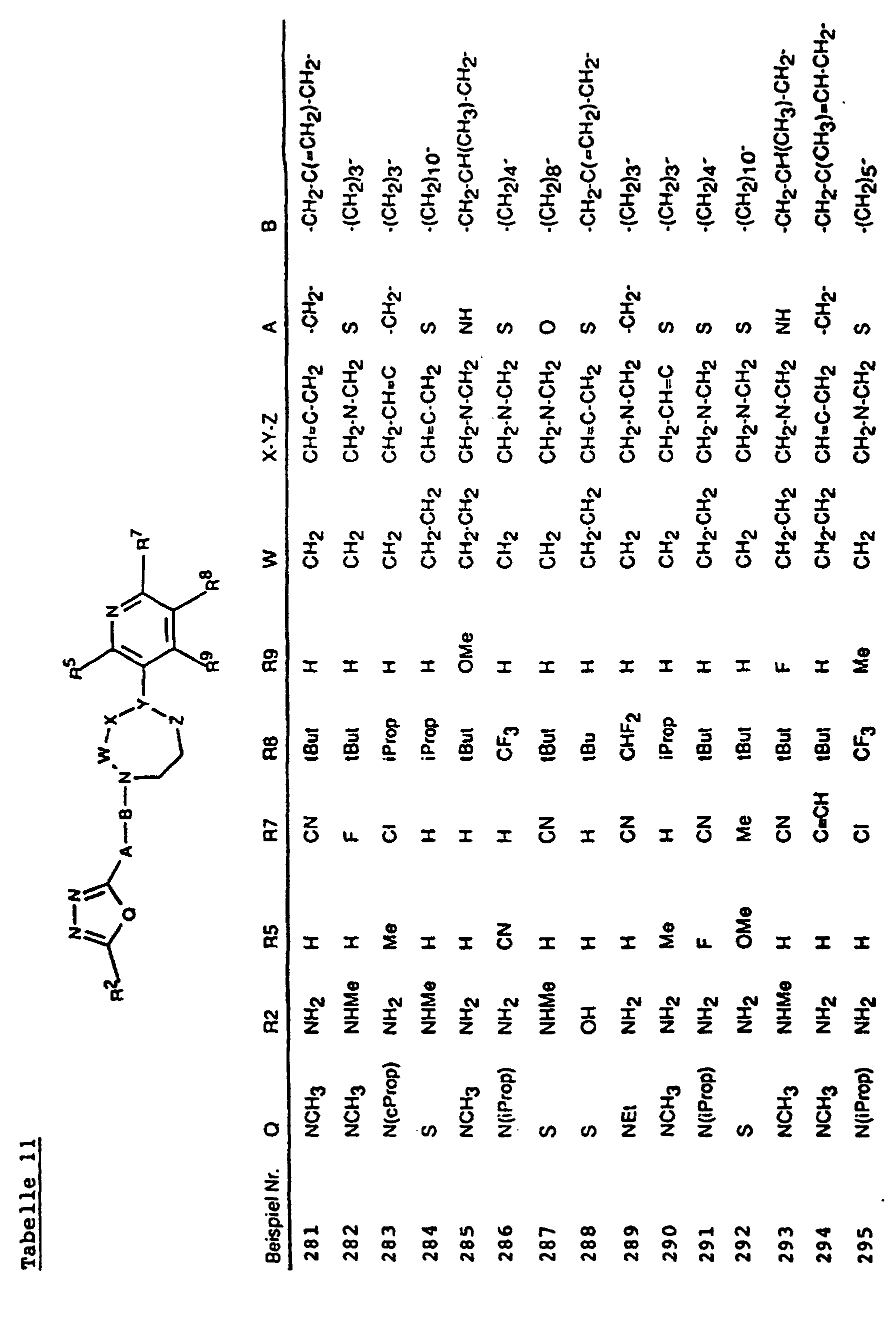

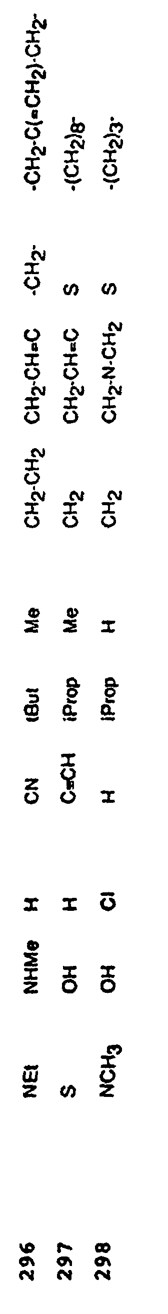

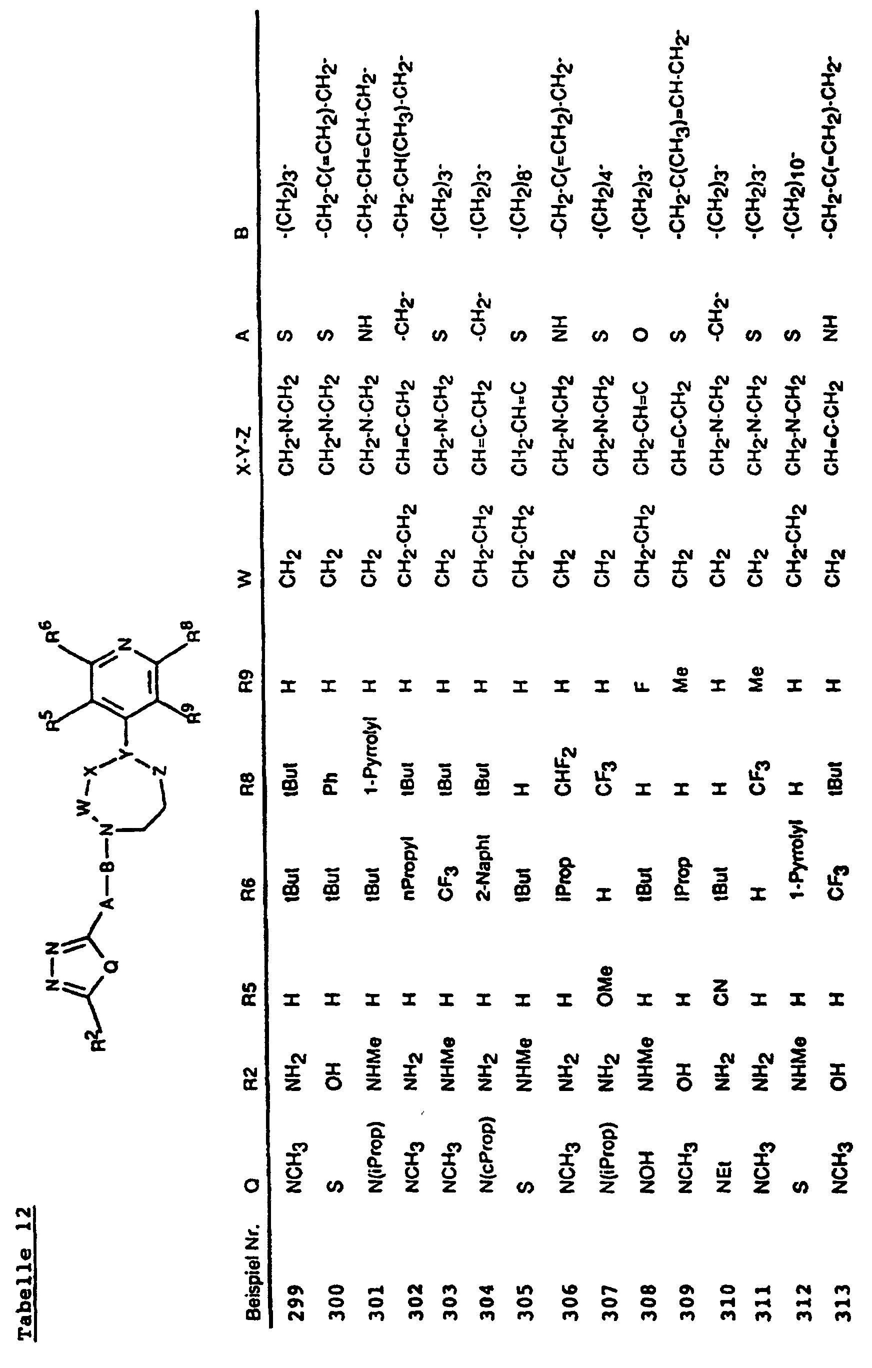

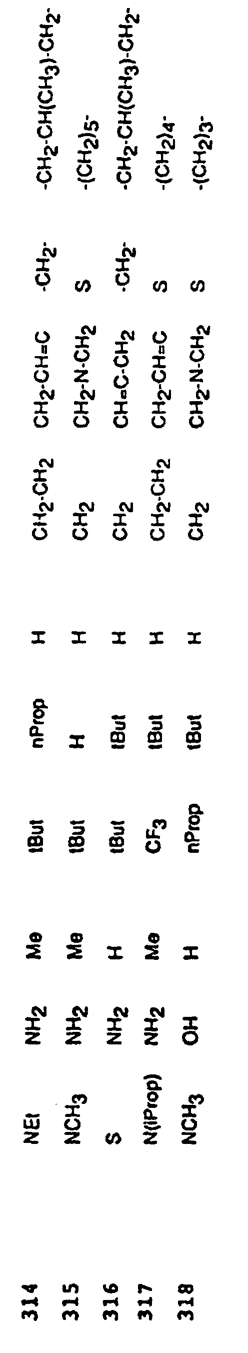

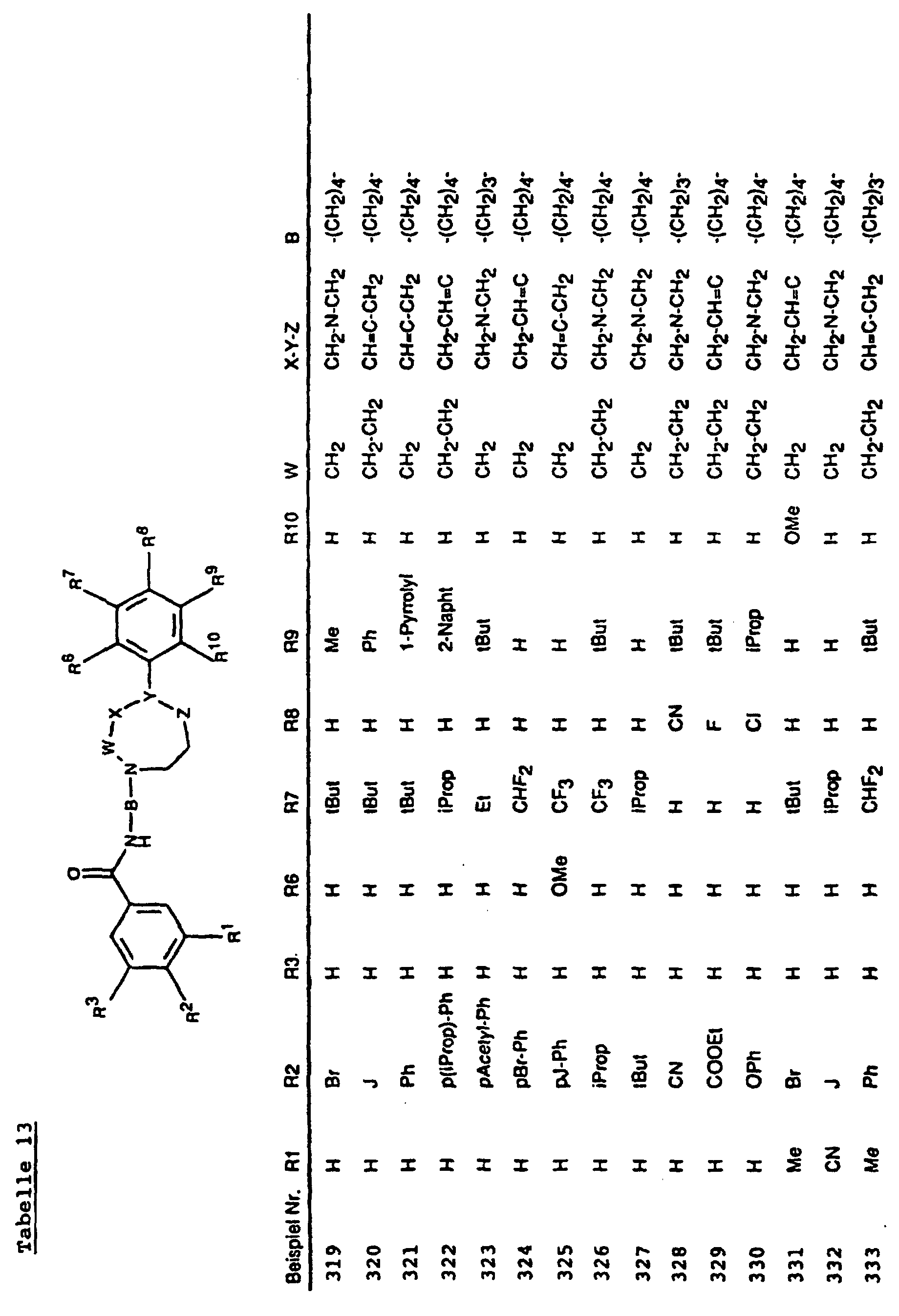

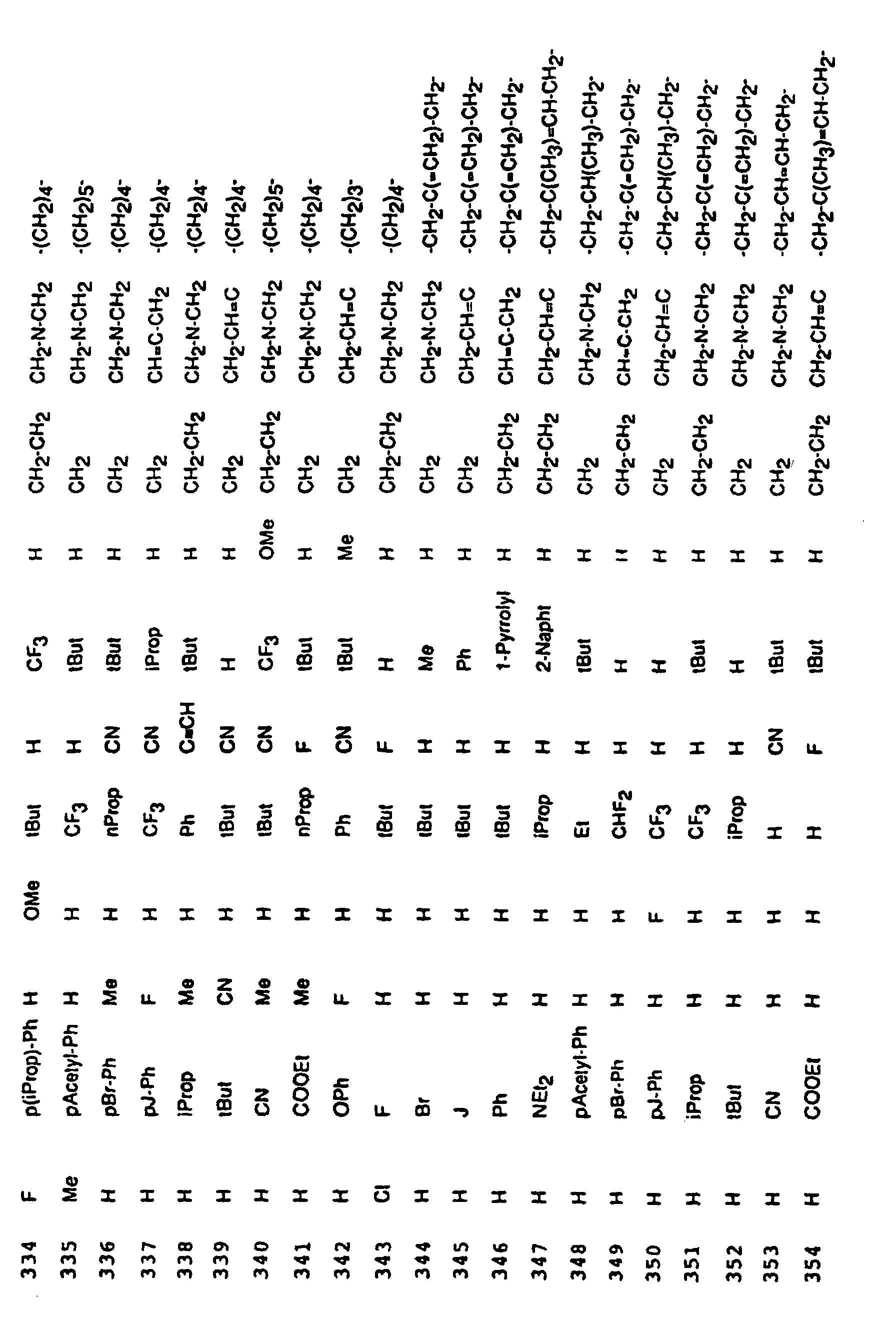

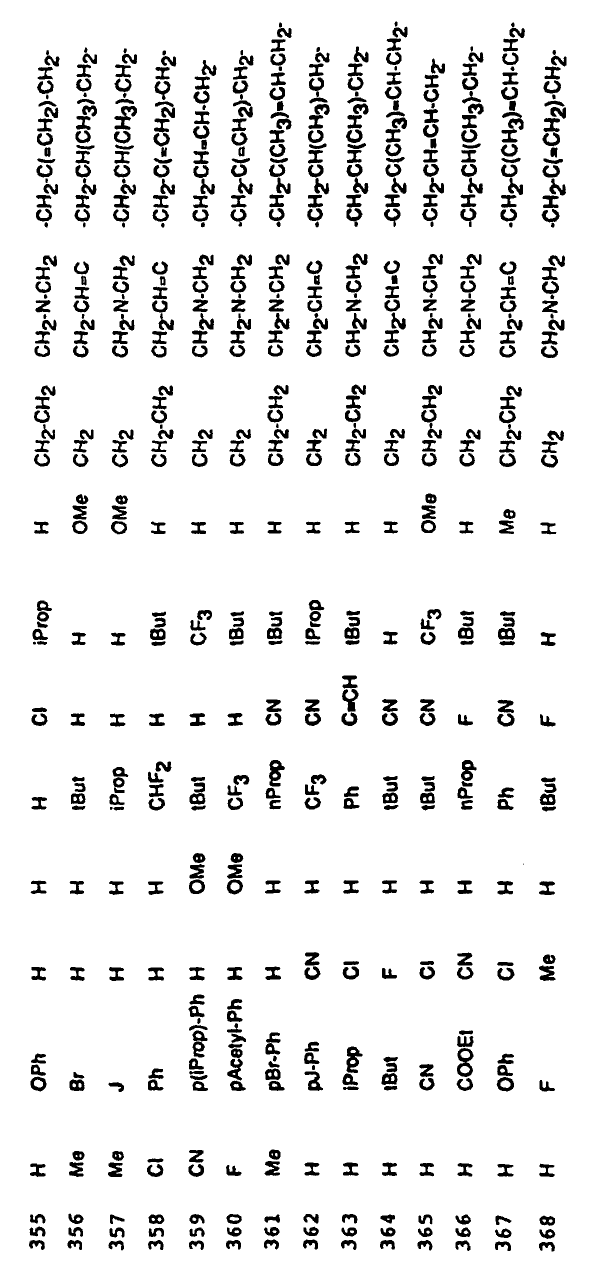

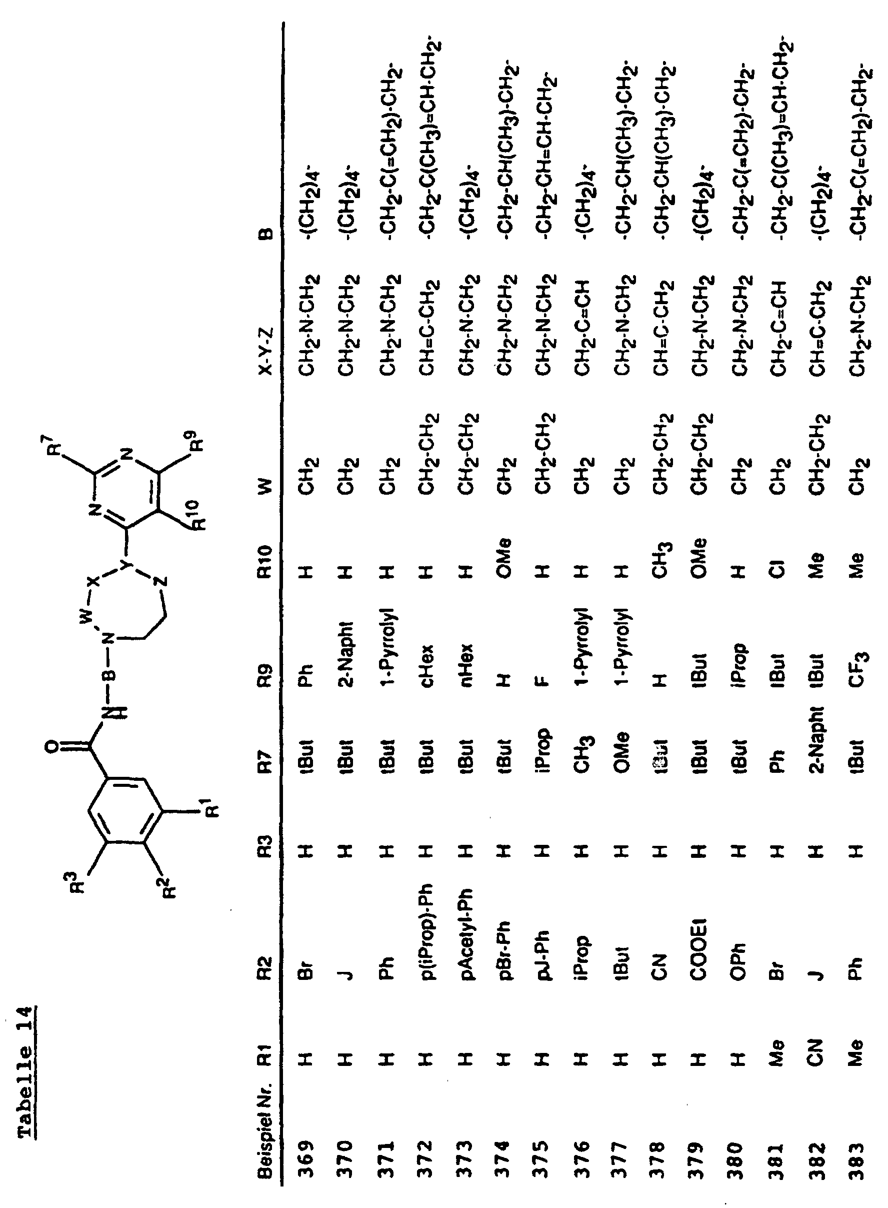

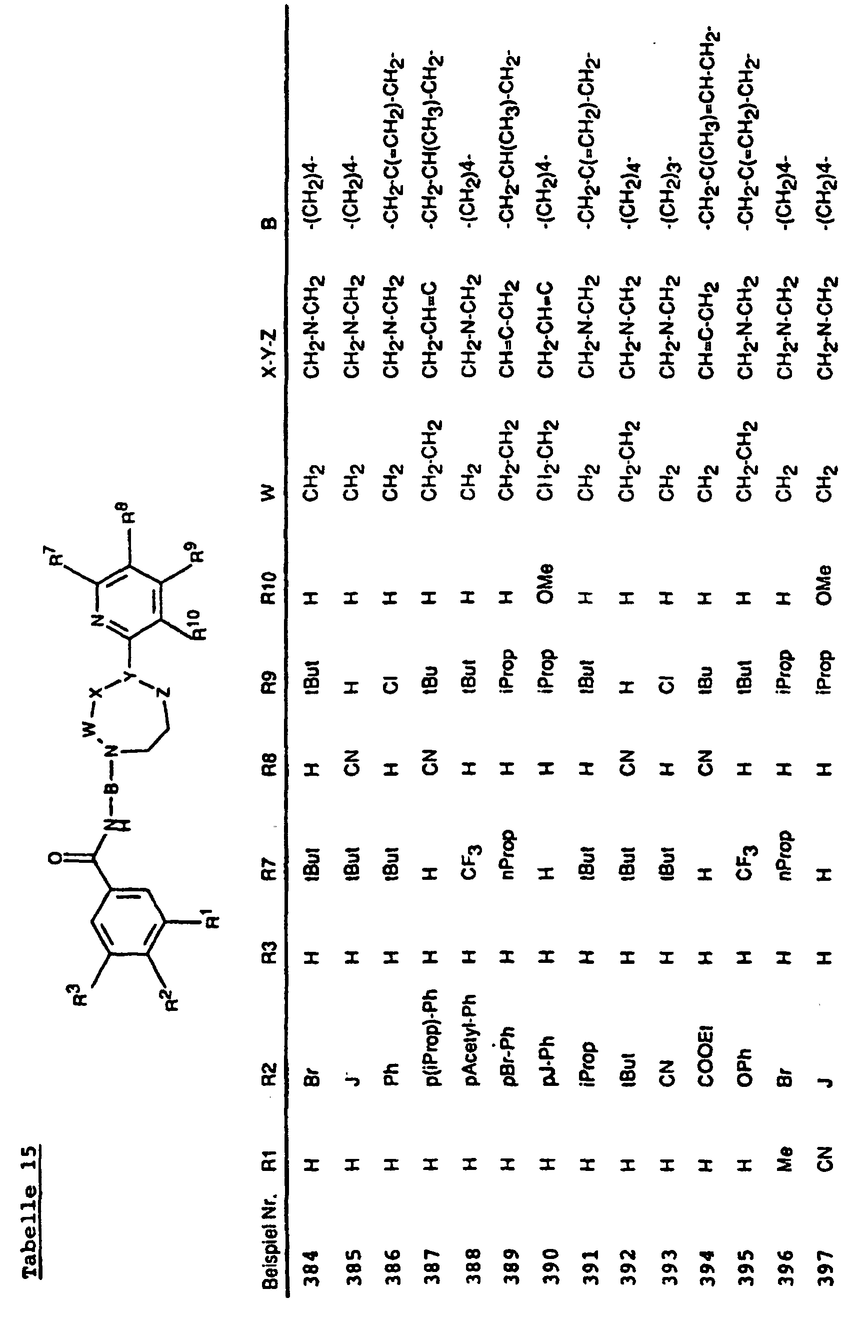

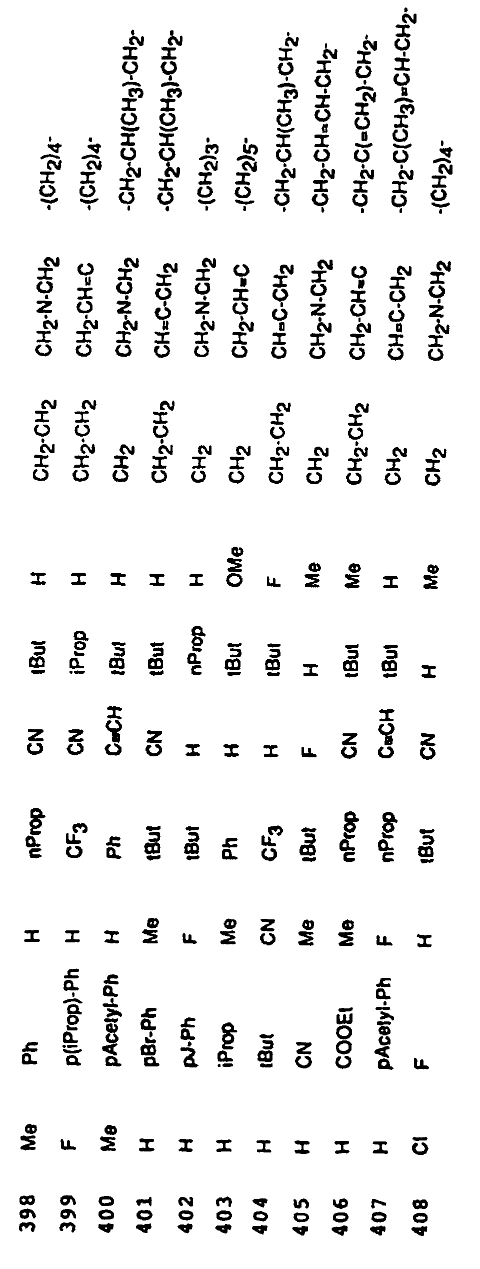

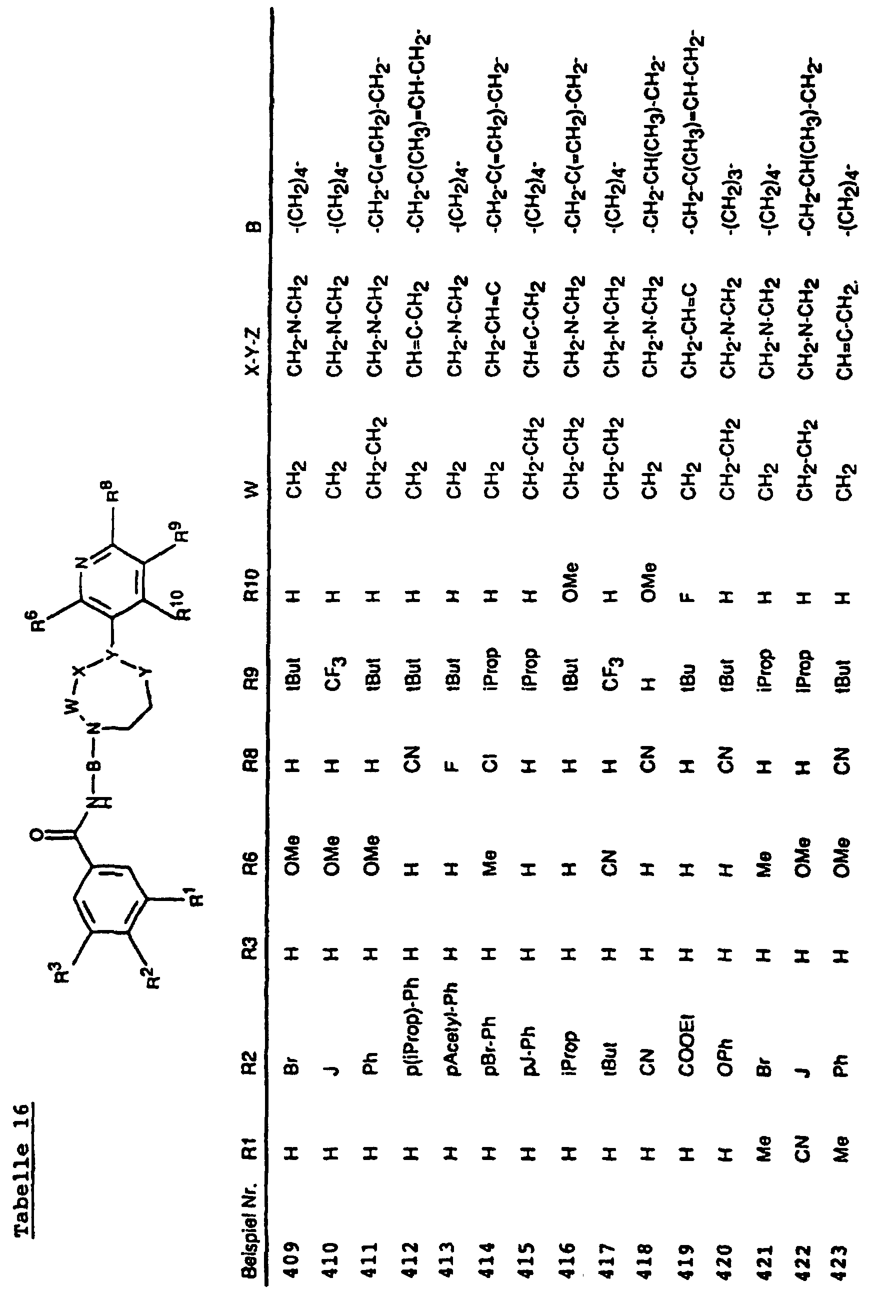

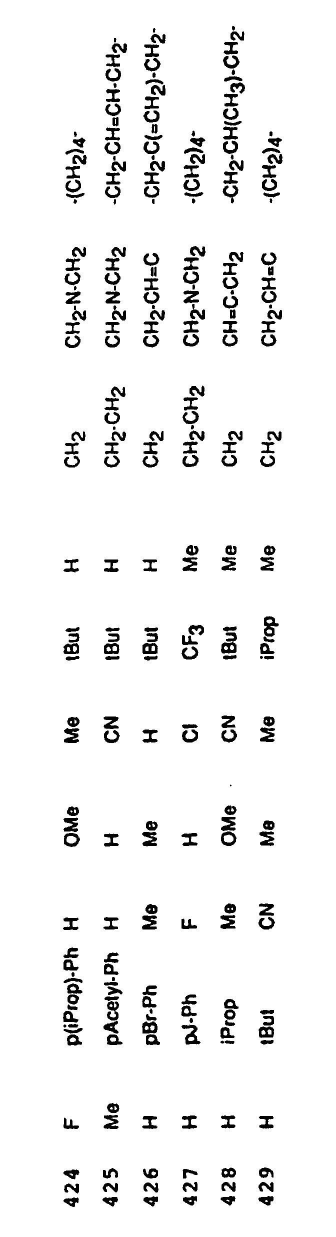

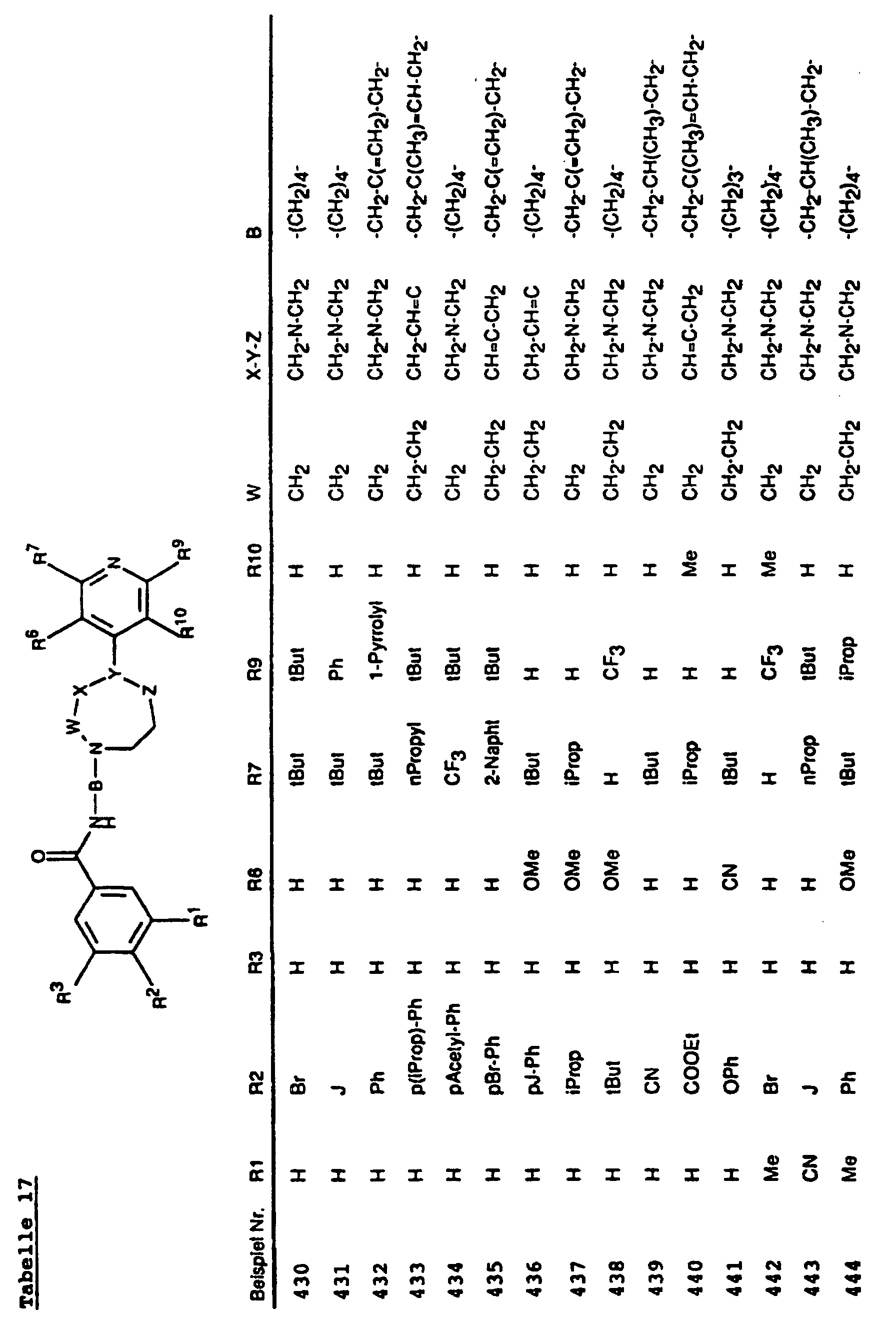

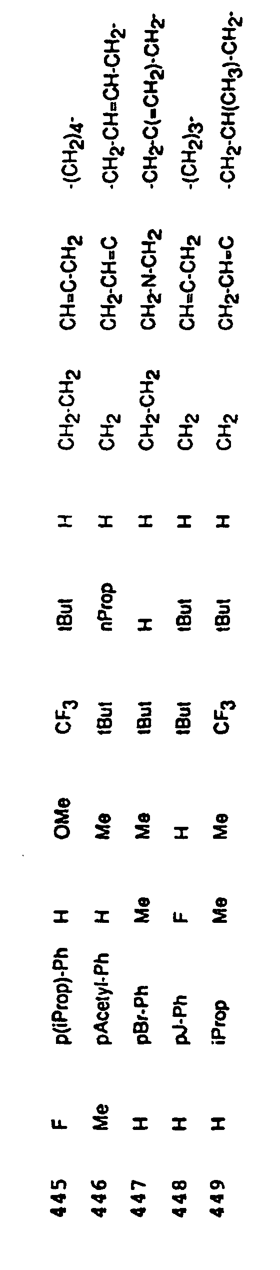

### Beispiele für galenische Applikationsformen

### A) Tabletten

Auf einer Tablettenpresse werden in üblicher Weise Tabletten folgender Zusammensetzung gepreßt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### B) Dragees

| | |
|---|---|
| 20 mg | Substanz des Beispiels 4 |
| 60 mg | Kernmasse |
| 70 mg | Verzuckerungsmasse |

Die Kernmasse besteht aus 9 Teilen Maisstärke, 3 Teilen Milchzukker und 1 Teil Vinylpyrrolidon-Vinylacetat-Mischpolymerisat 60:40. Die Verzuckerungsmasse besteht aus

5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 g gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷-Zellen /ml Lysispuffer 30 min bei 4°C inkubiert. Die Zellen wurden bei 200 g 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Ki-Werte erfolgte über nichtlineare Regressionsanalyse mit dem Programm LIGAND.

### 2) D₂-Bindungstest

### Zellkultur

HEK-293 Zellen mit stabil exprimierten humanen Dopamin-D2A-Rezeptoren wurden in RPMI 1640 mit Glutamax I™ und 25 mM HEPES mit 10% fötalem Kälberserumalbumin kultiviert. Alle Medien enthielten 100 Einheiten pro ml Penicillin und 100 µg/ml Streptomycin. Die Zellen wurden in feuchter Atmosphäre mit 5% CO₂ bei 37°C gehalten.

Die Zellpräparation für Bindungsstudien erfolgte durch Trypsinisierung (0,05% Trypsinlösung) für 3-5 Minuten bei Raumtemperatur. Danach wurden die Zellen bei 250 g 10 Minuten zentrifugiert und 30 Minuten bei 4°C mit Lysispuffer (5 mM Tris-HCl, 10% Glycerol, pH 7,4) behandelt. Nach Zentrifugation bei 250 g für 10 Minuten wurde der Rückstand bei -20°C bis zum Gebrauch aufbewahrt.

### Rezeptorbindungstests

### 1) Dopamin-D₂-Rezeptor "low affinity state" mit ¹²⁵I-Spiperon (81 TBq/mmol, Du Pont de Nemours, Dreieich)

Die Ansätze (1 ml) setzten sich zusammen aus 1 x 10⁵ Zellen in Inkubationspuffer (50 mM Tris, 120 mM NaCl,-5 mM KCl, 2 mM MgCl₂ und 2 mM CaCl₂, pH 7,4 mit HCl) und 0,1 nM ¹²⁵I-Spiperon (totale Bindung) oder zusätzlich 1 µM Haloperidol (unspezifische Bindung) oder Prüfsubstanz.

Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.

Die Auswertung erfolgte wie unter a).

Die Bestimmung der Ki-Werte erfolgte über nichtlineare Regressionsanalyse mit dem Programm LIGAND oder durch Umrechnung der IC₅₀-werte mit Hilfe der Formel von Cheng und Prusoff.

Die erfindungsgemäßen Verbindungen zeigen in diesen Tests sehr gute Affinitäten am D₃-Rezeptor und hohe Selektivitäten gegenüber dem D₃-Rezeptor.

Die folgenden Verbindungen wurden durch die oben beschriebenen Verfahren erhalten:

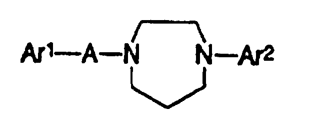

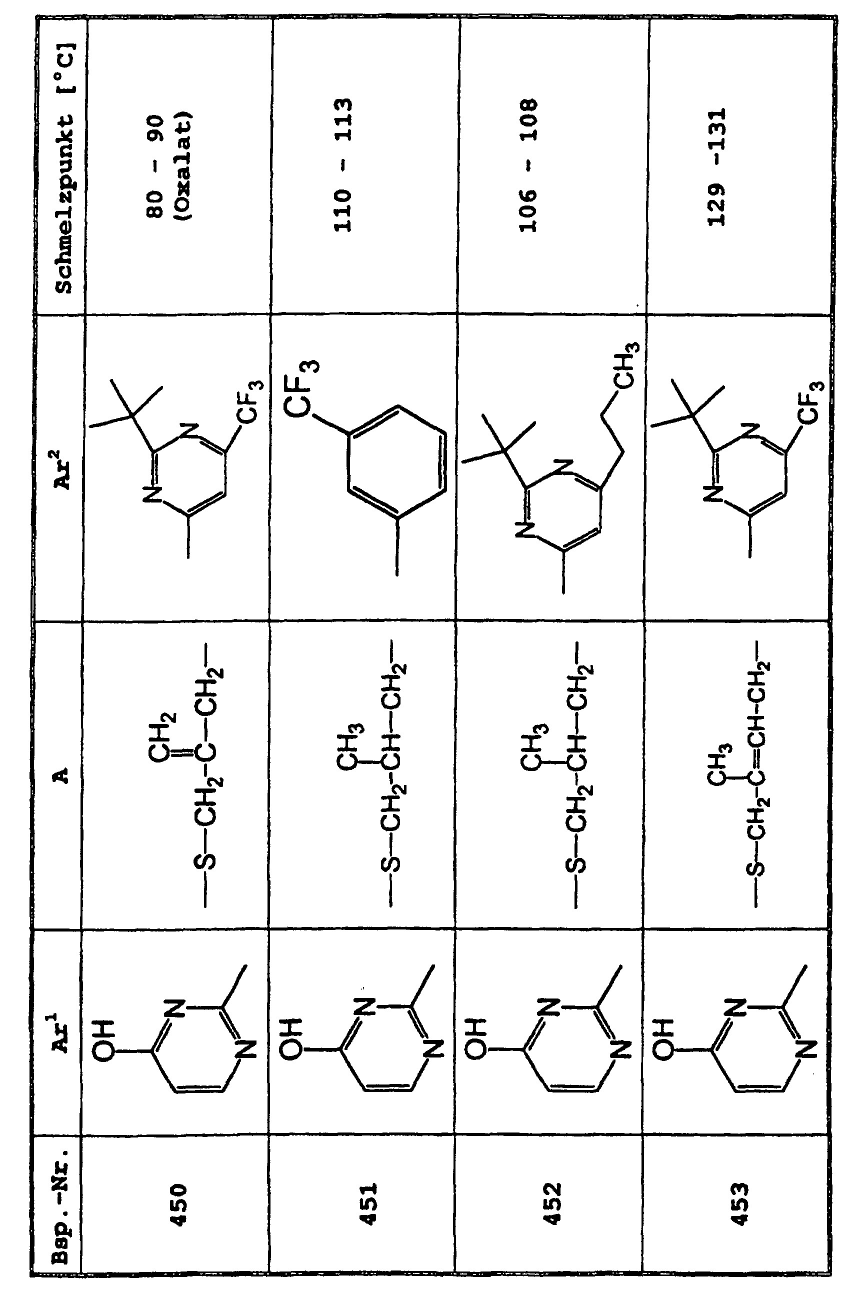

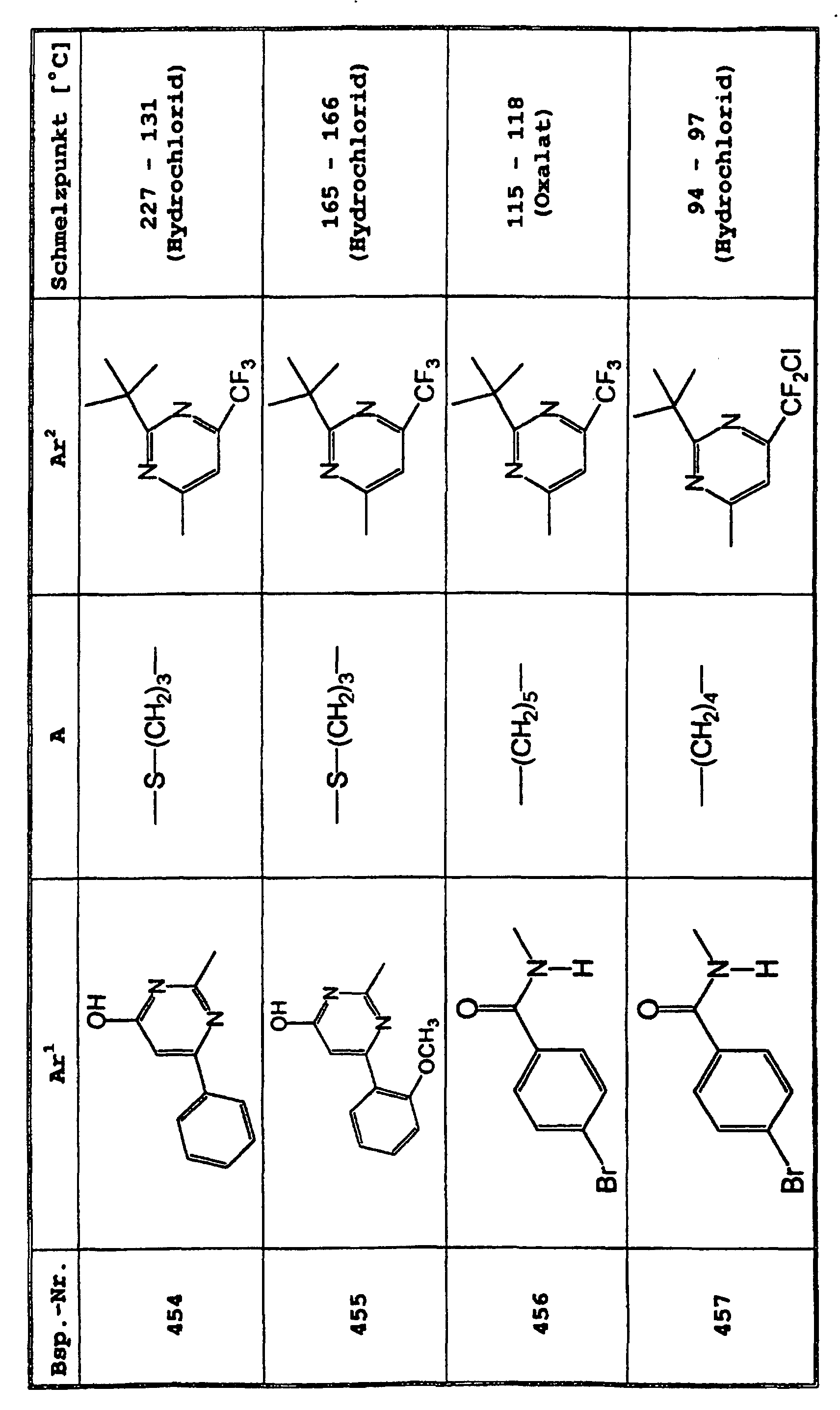

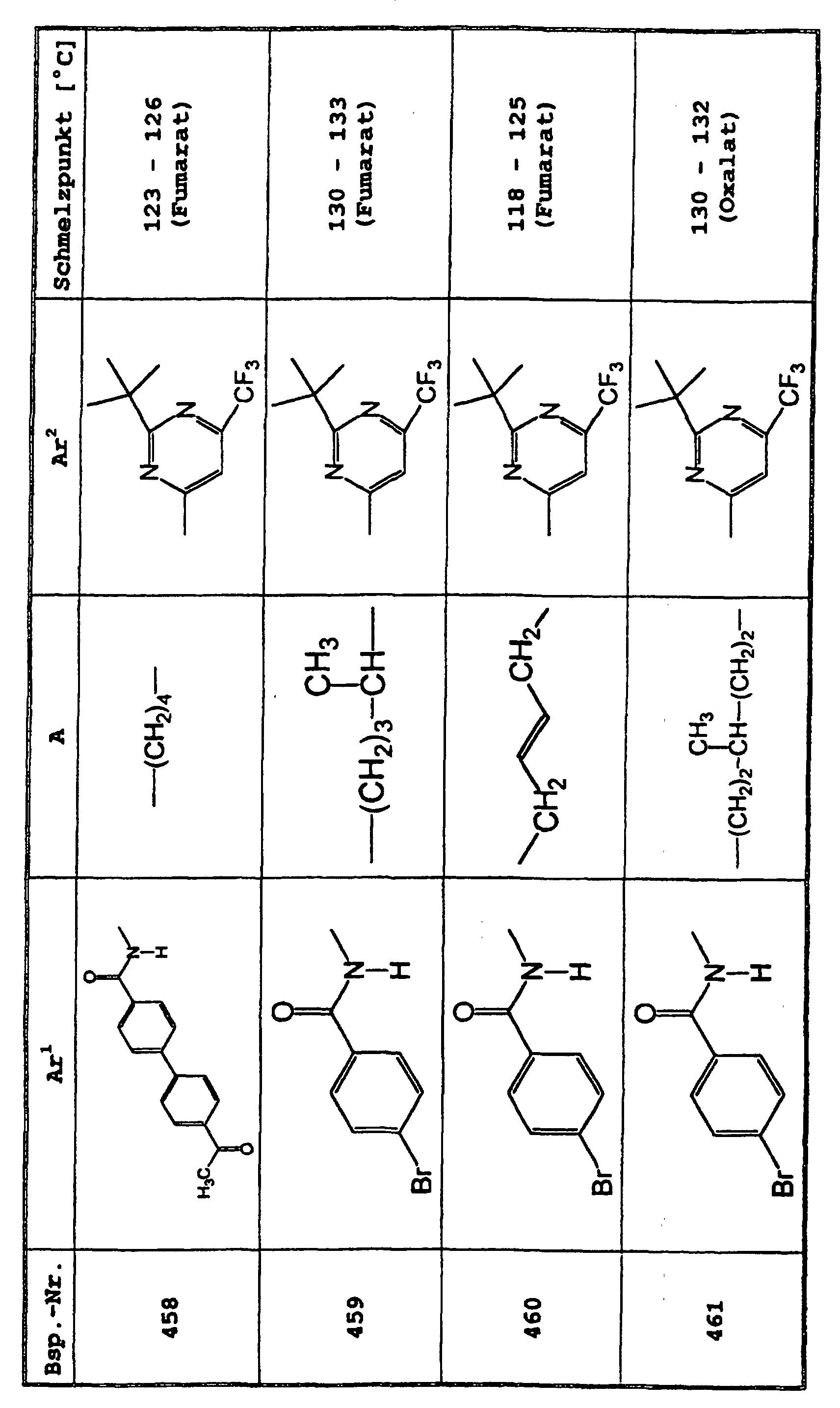

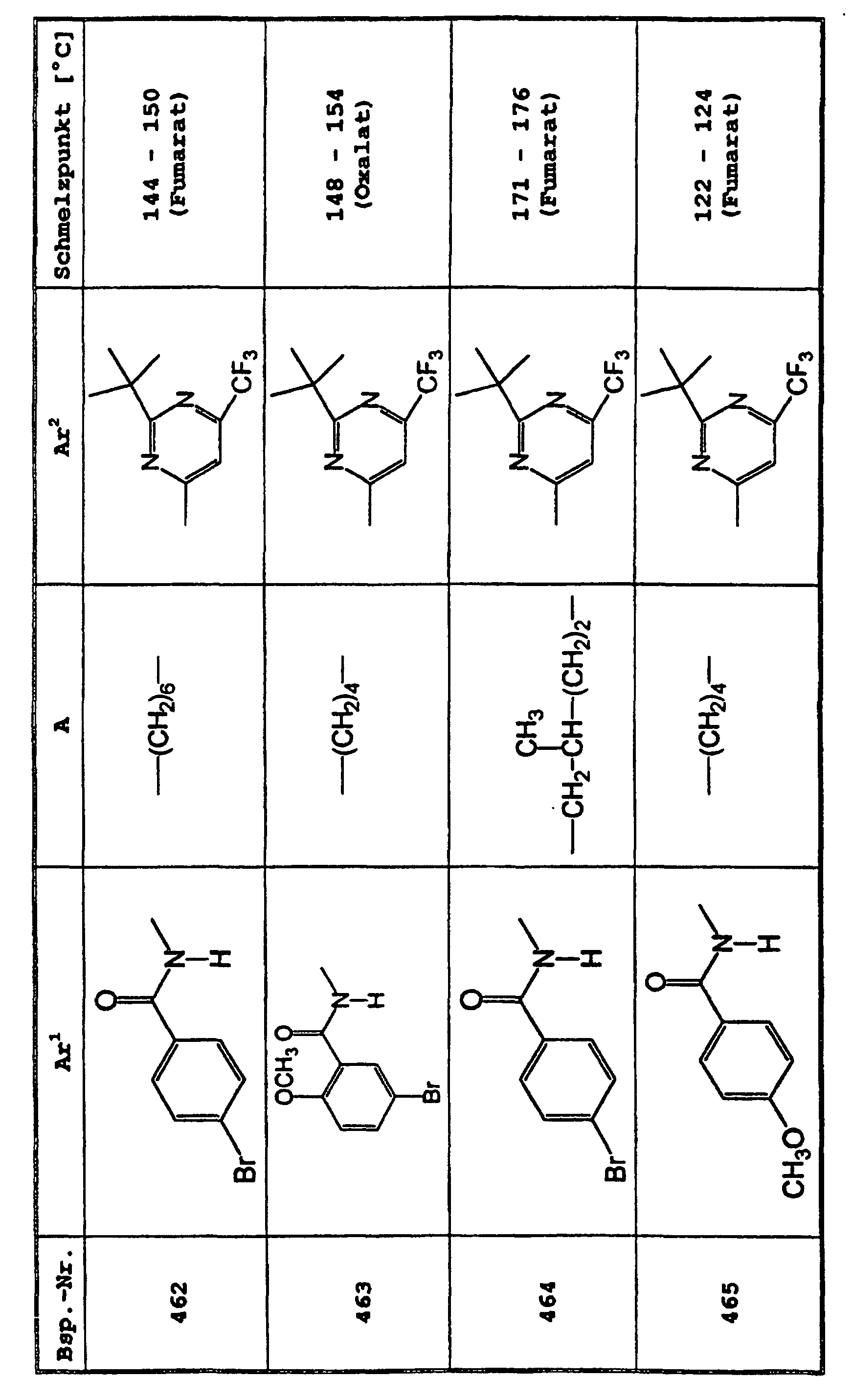

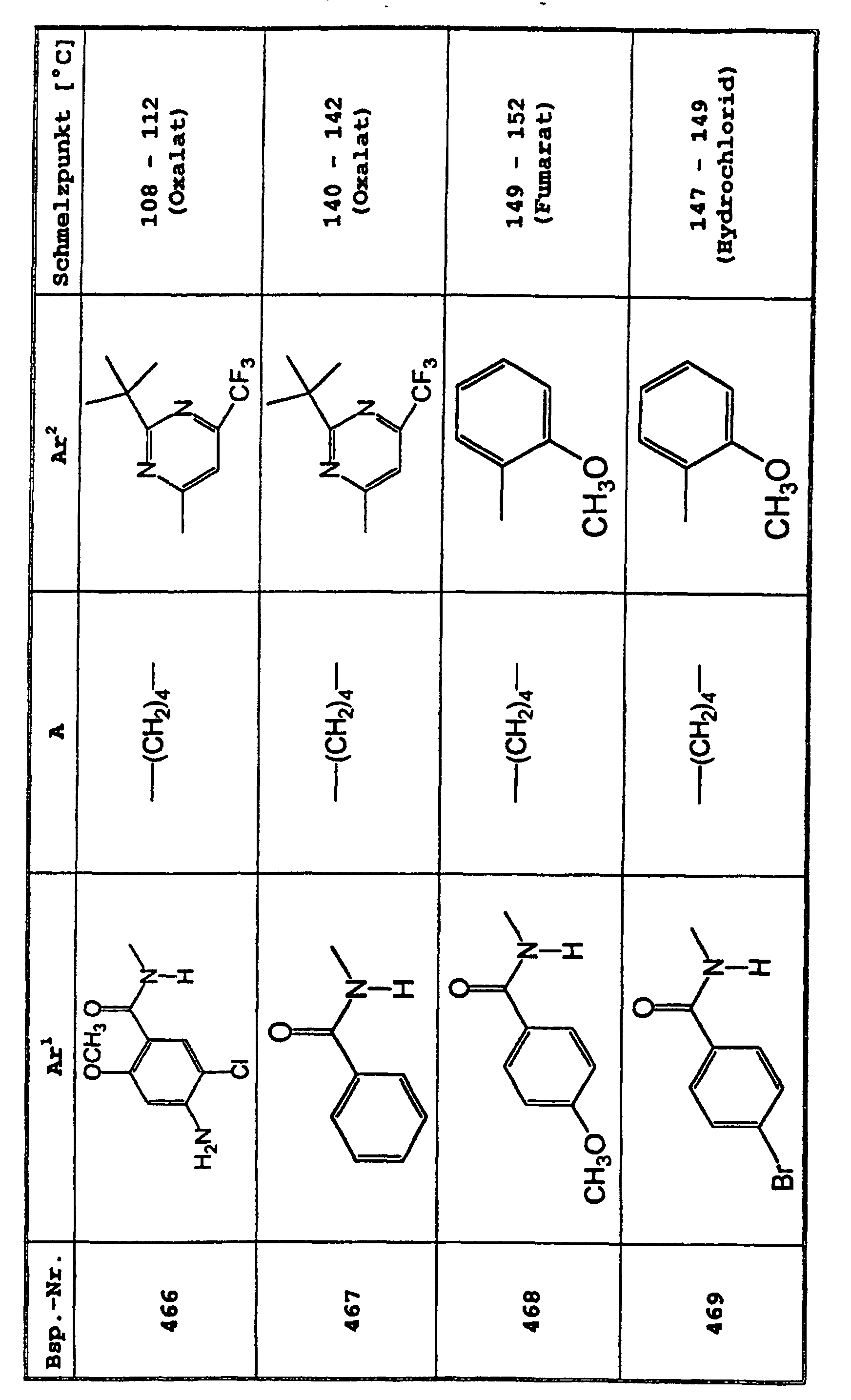

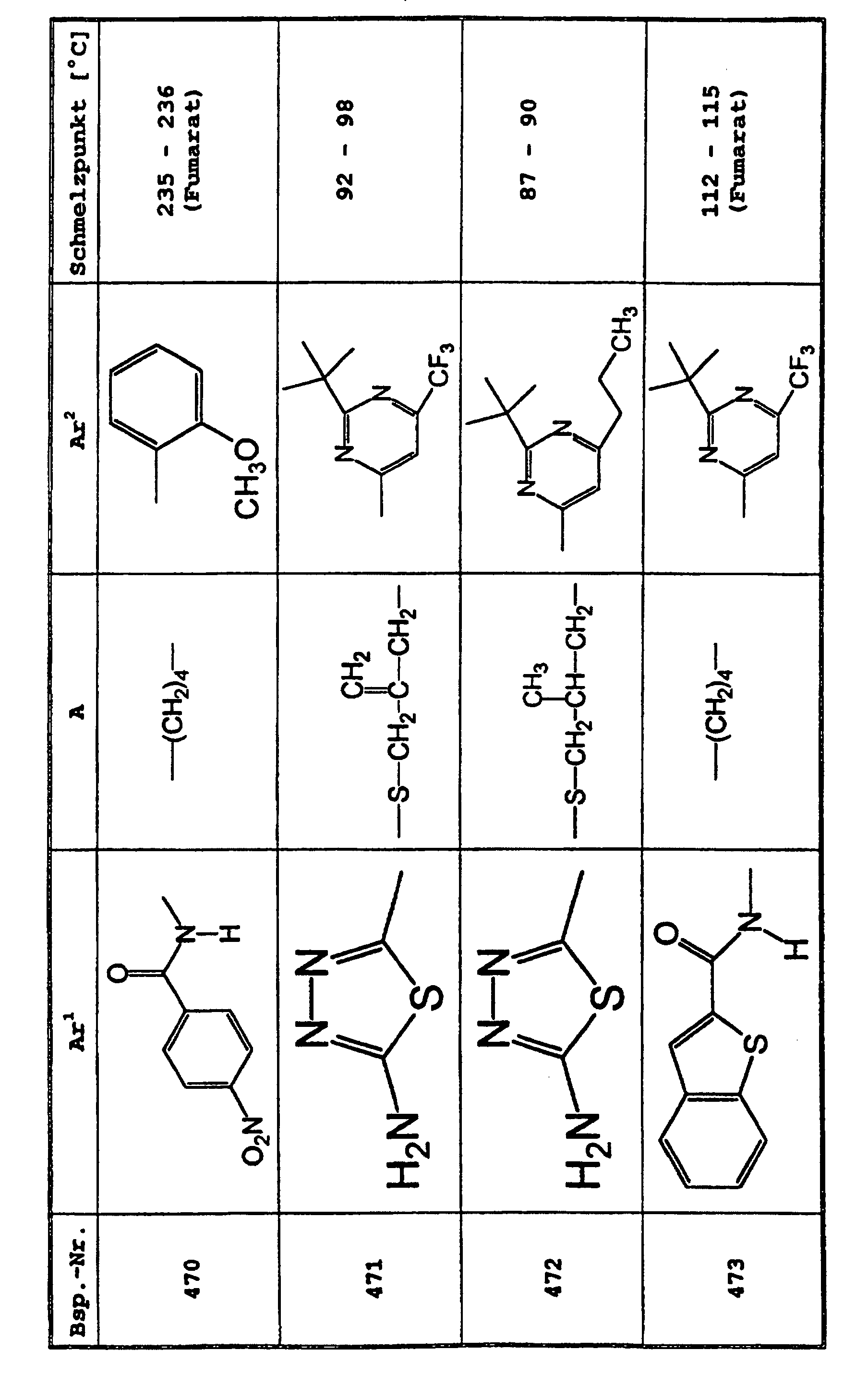

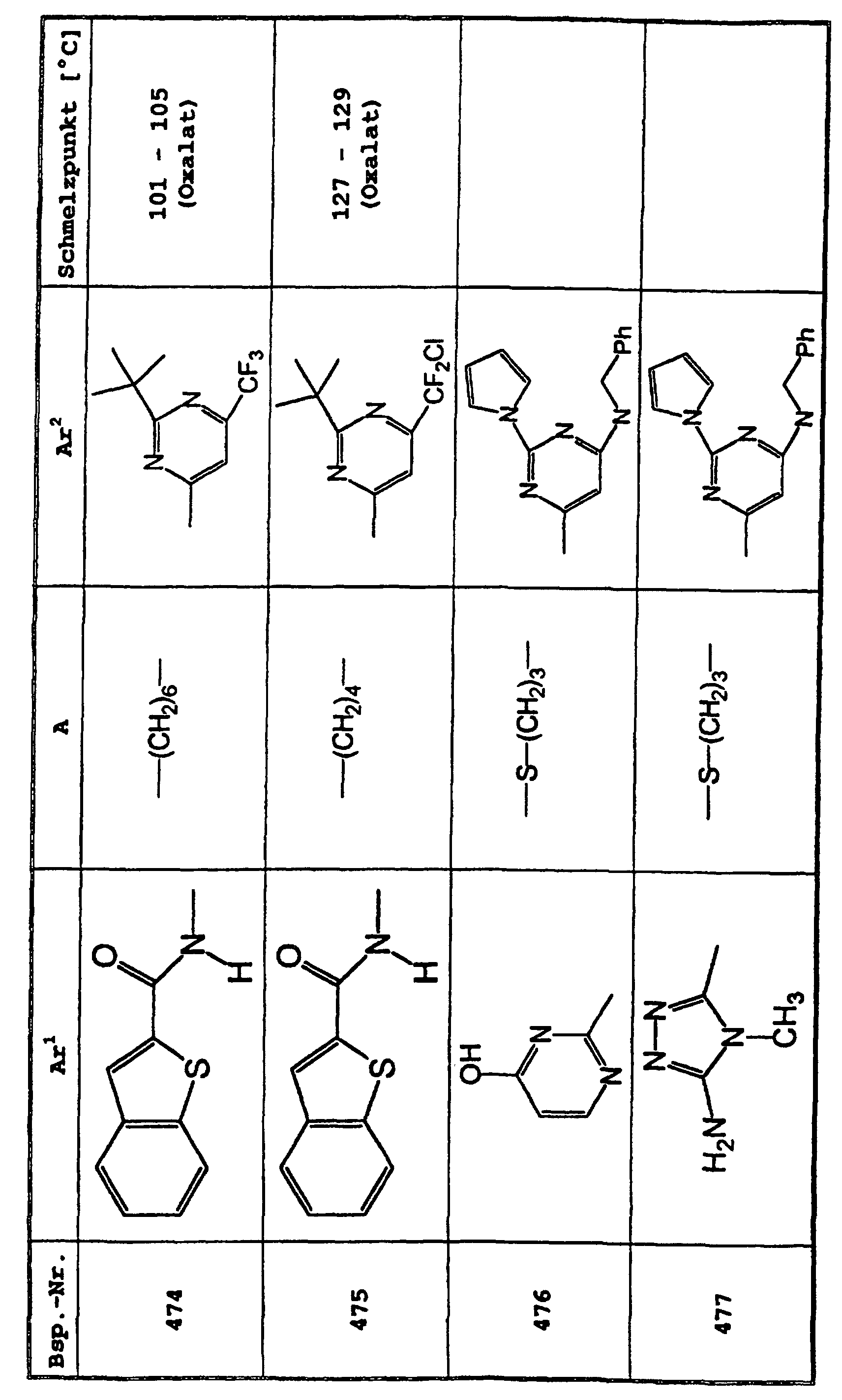

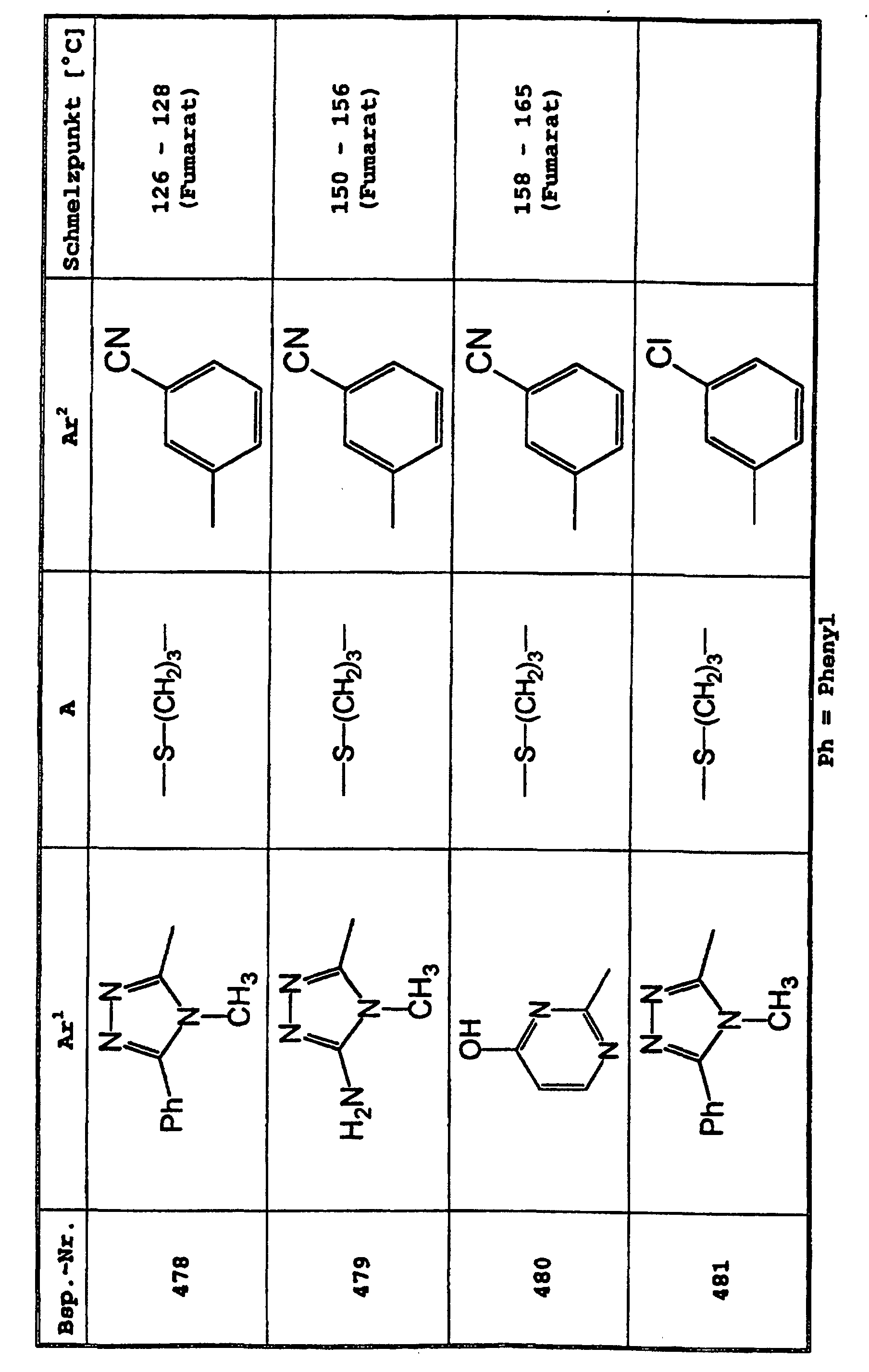

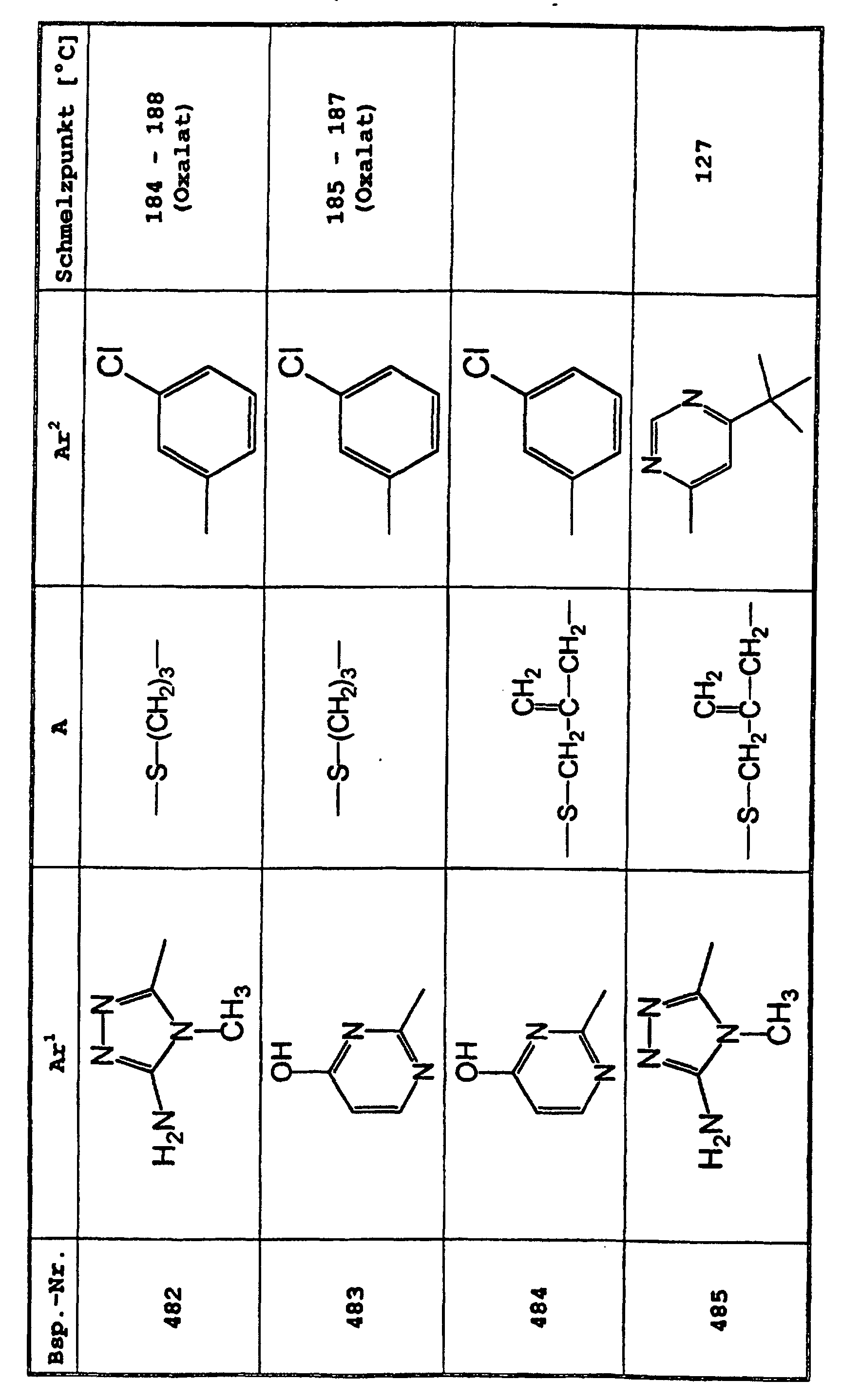

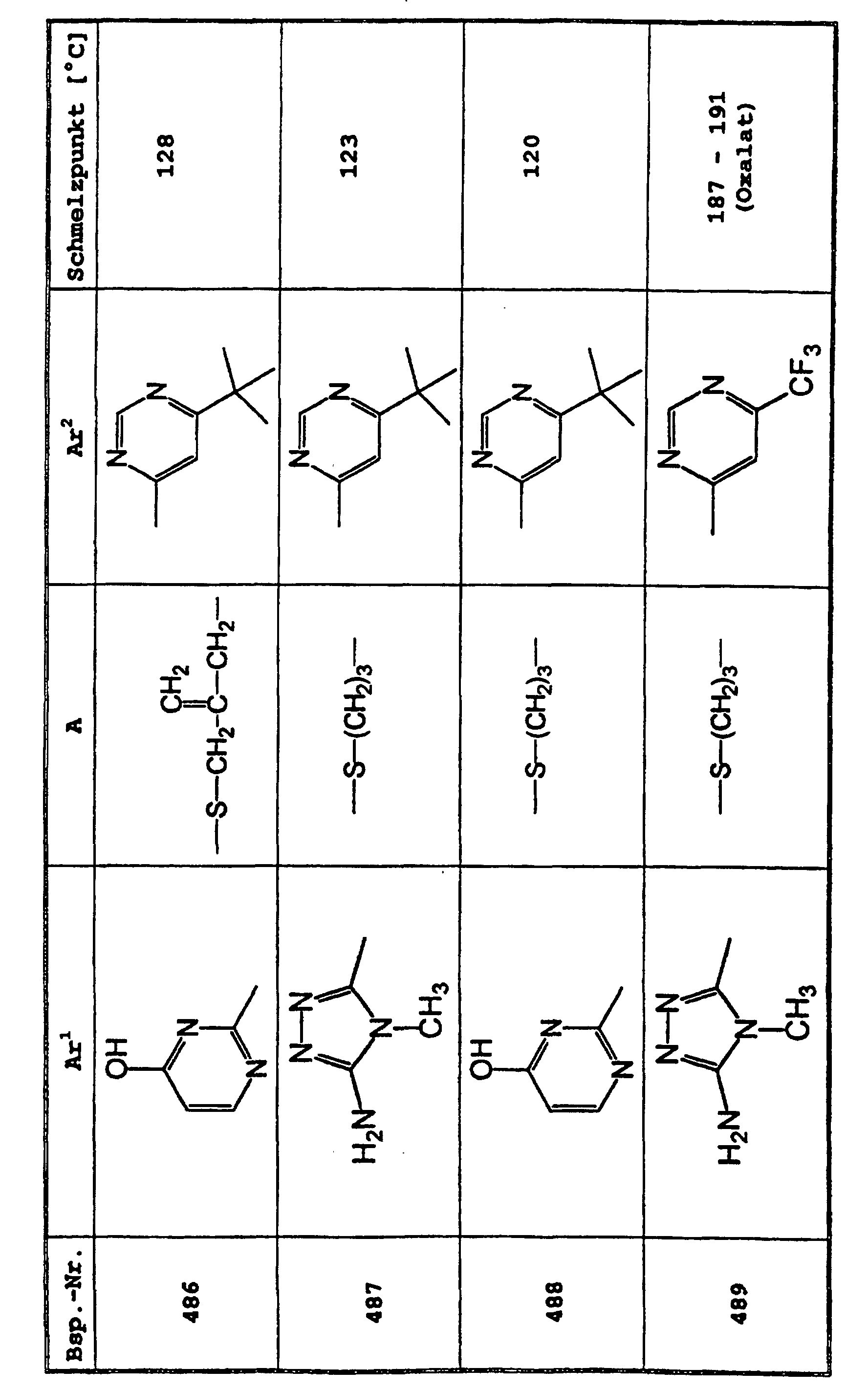

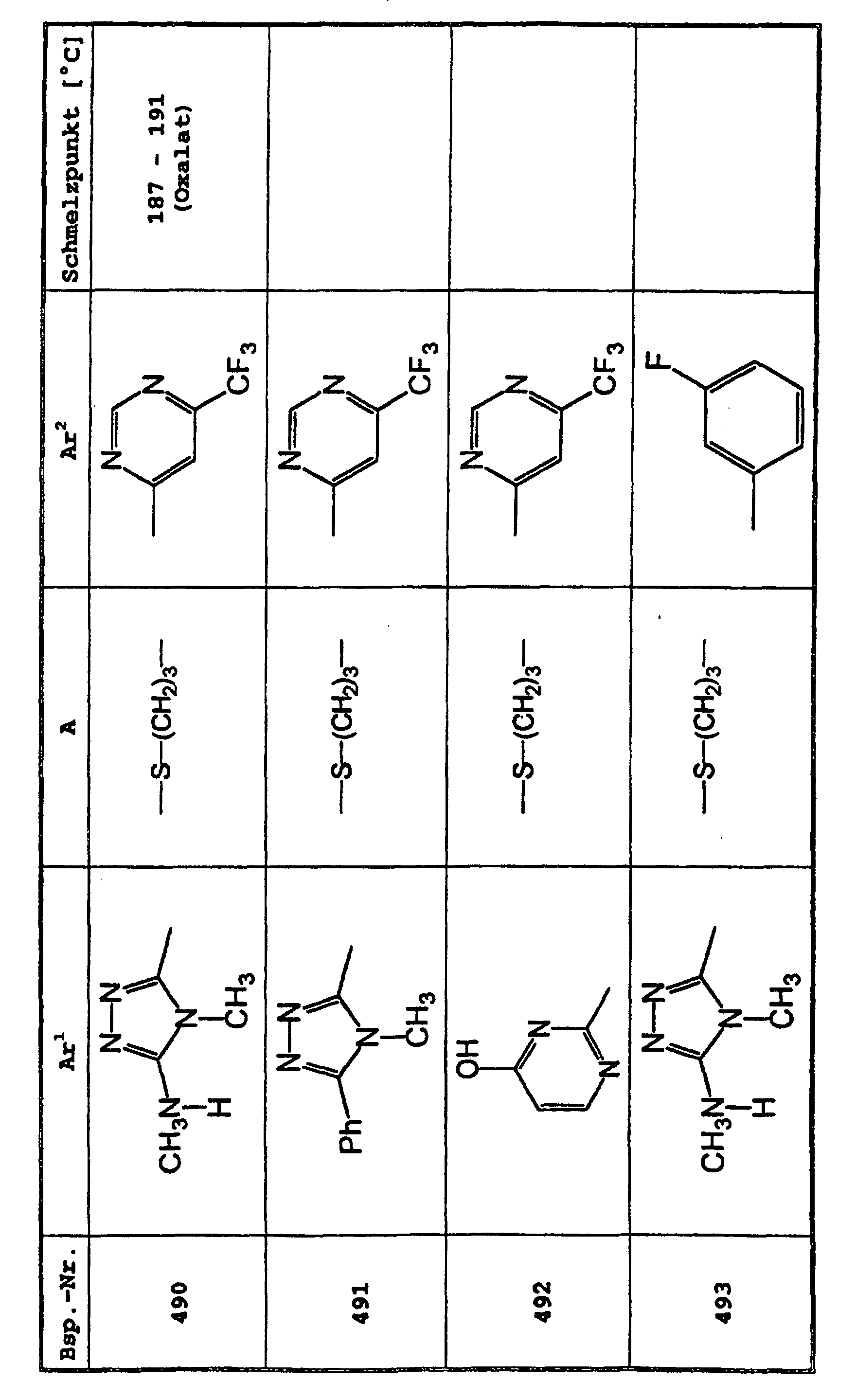

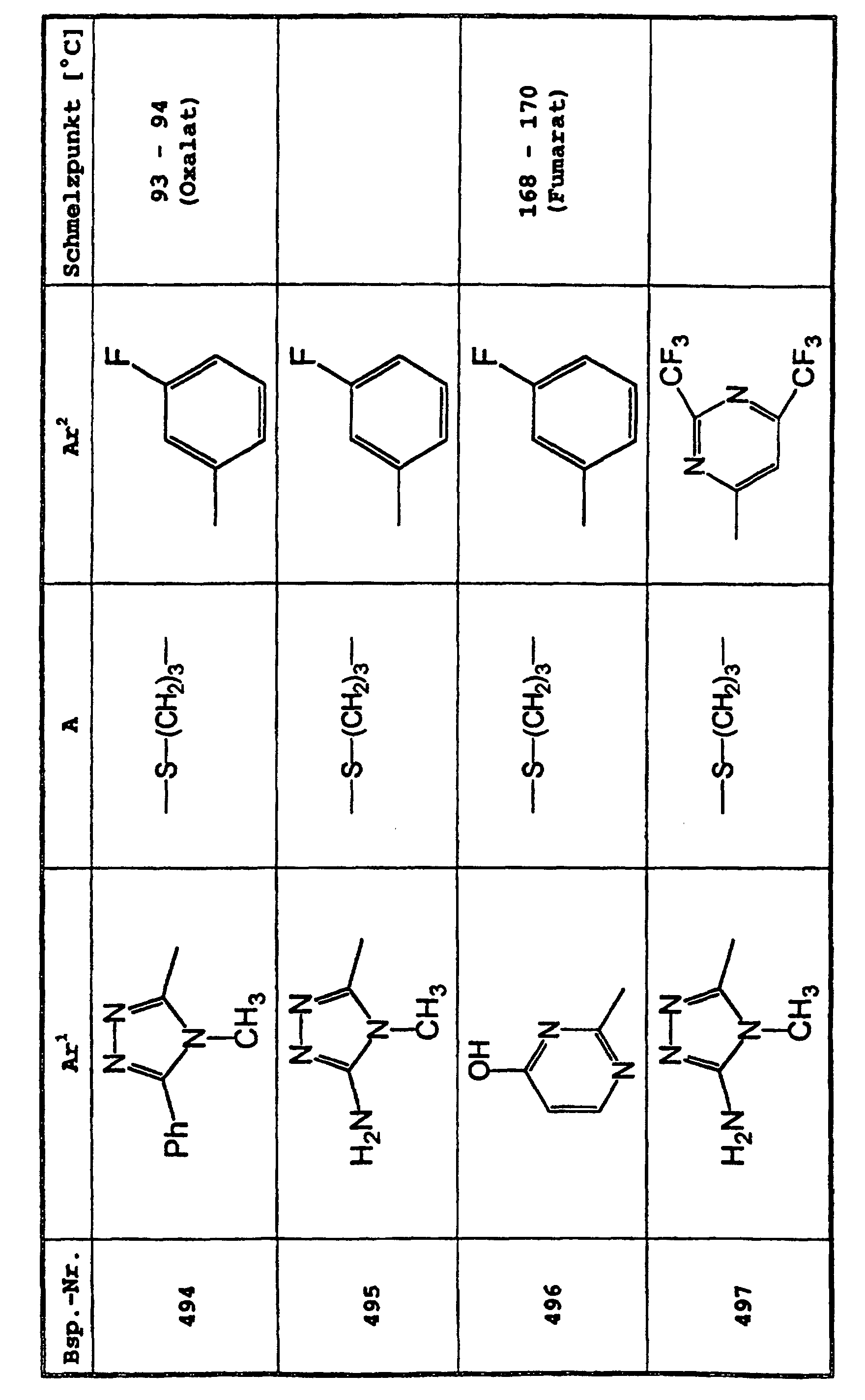

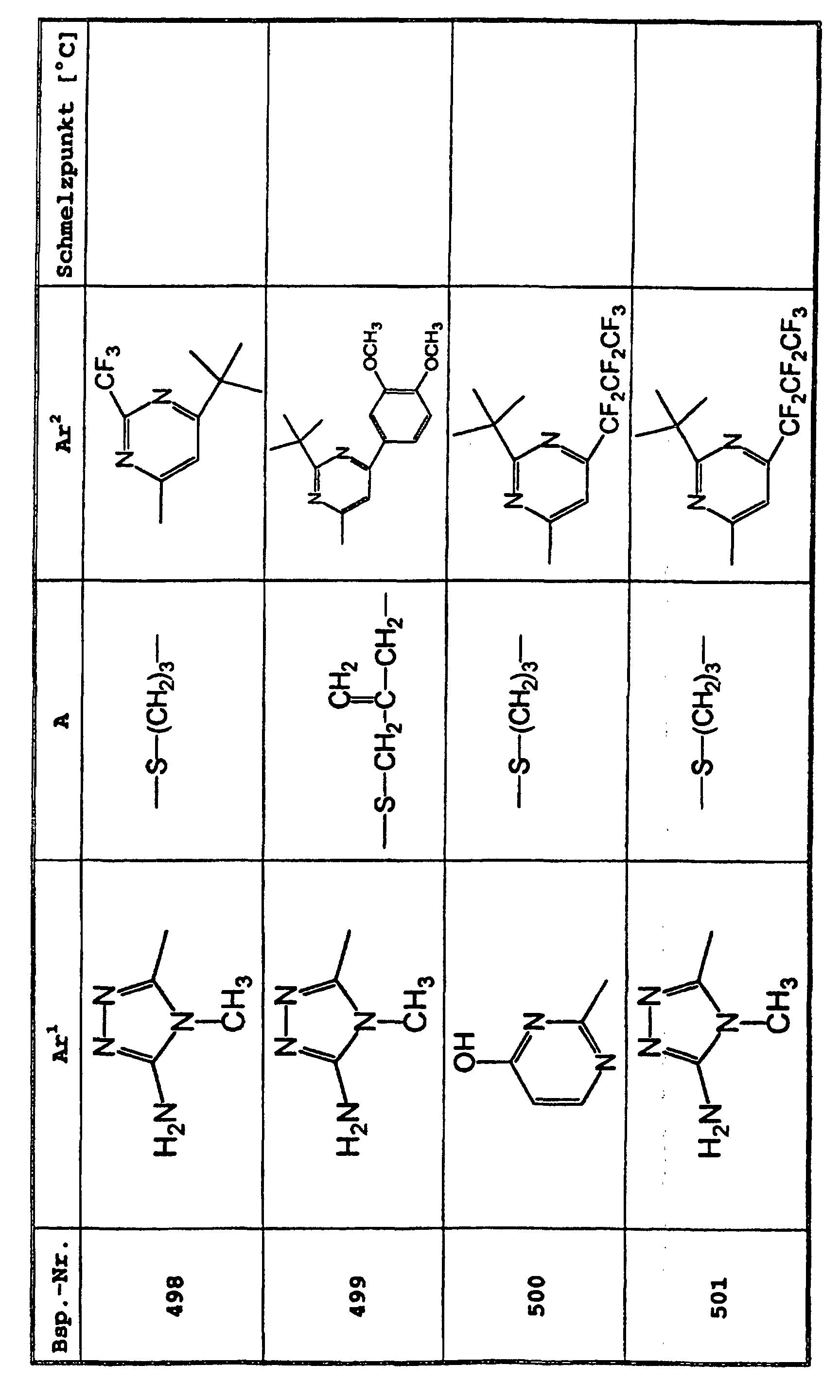

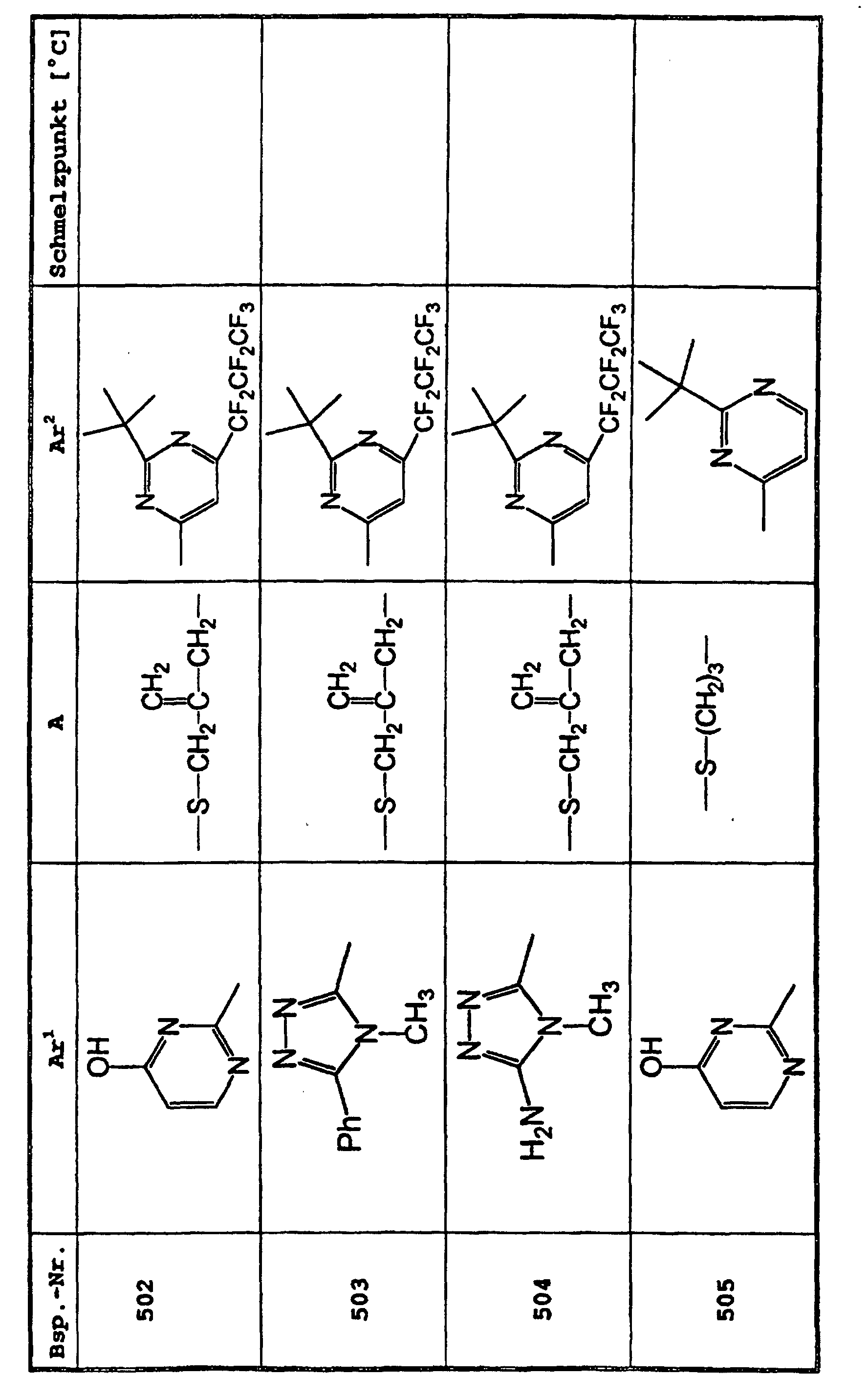

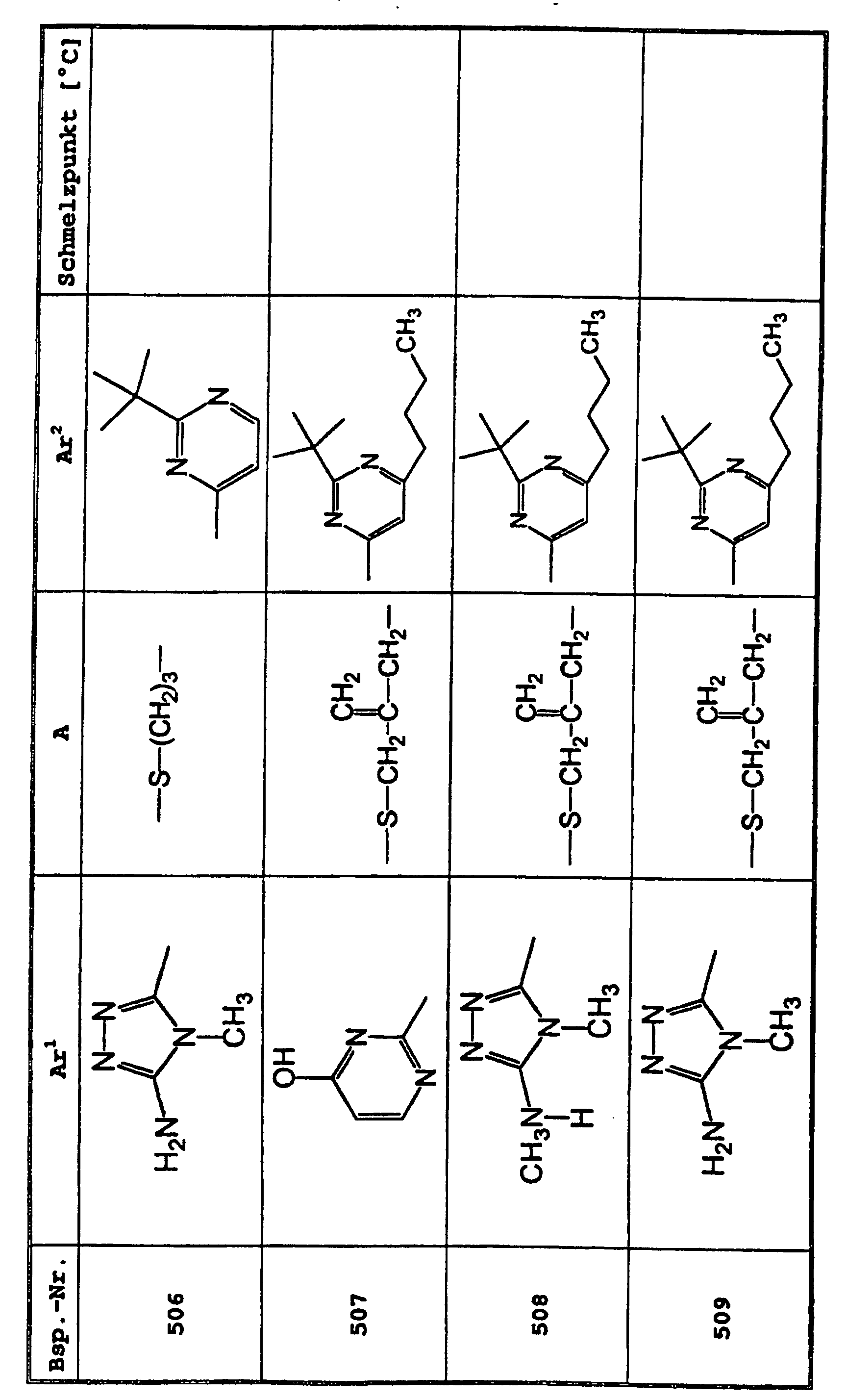

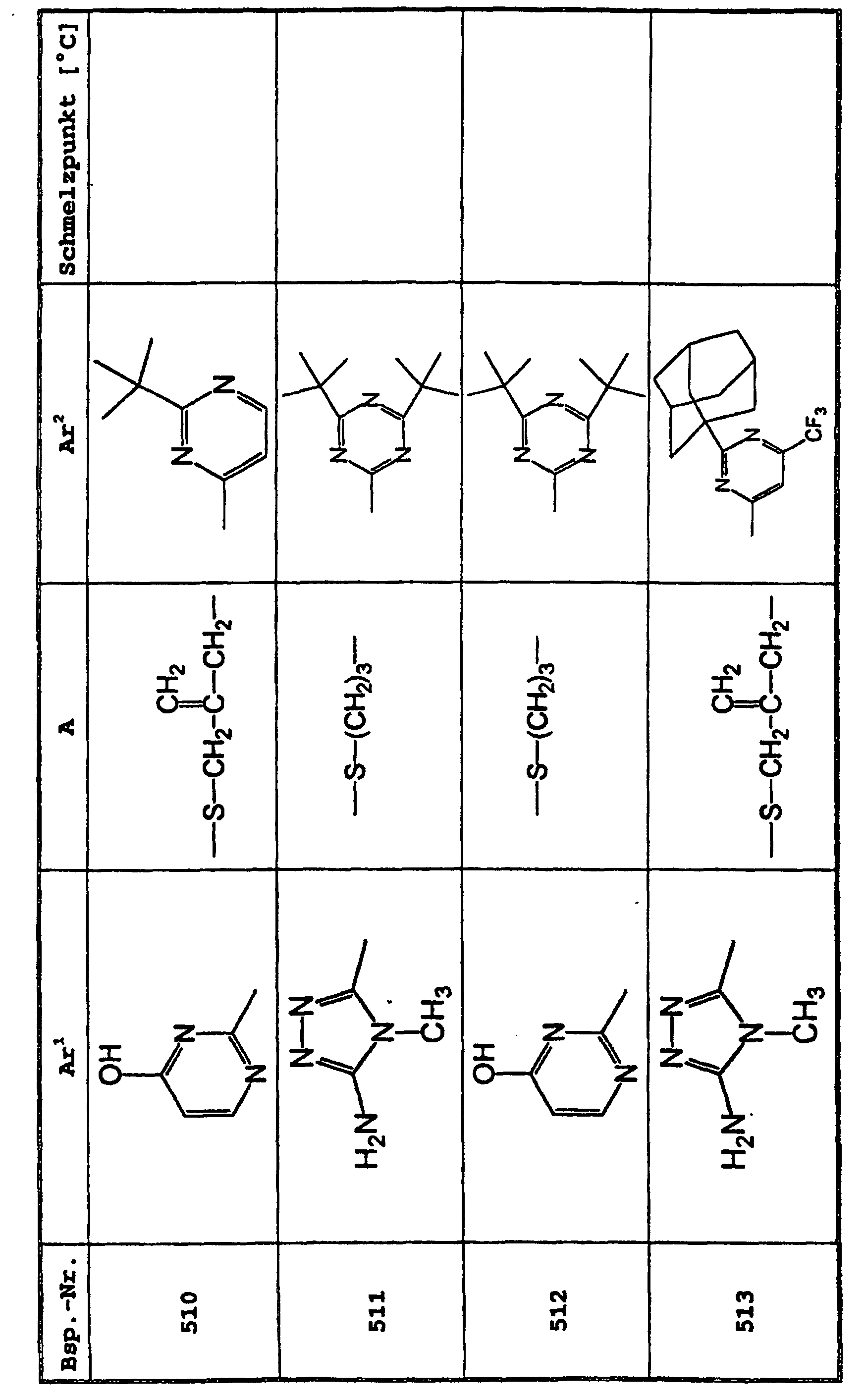

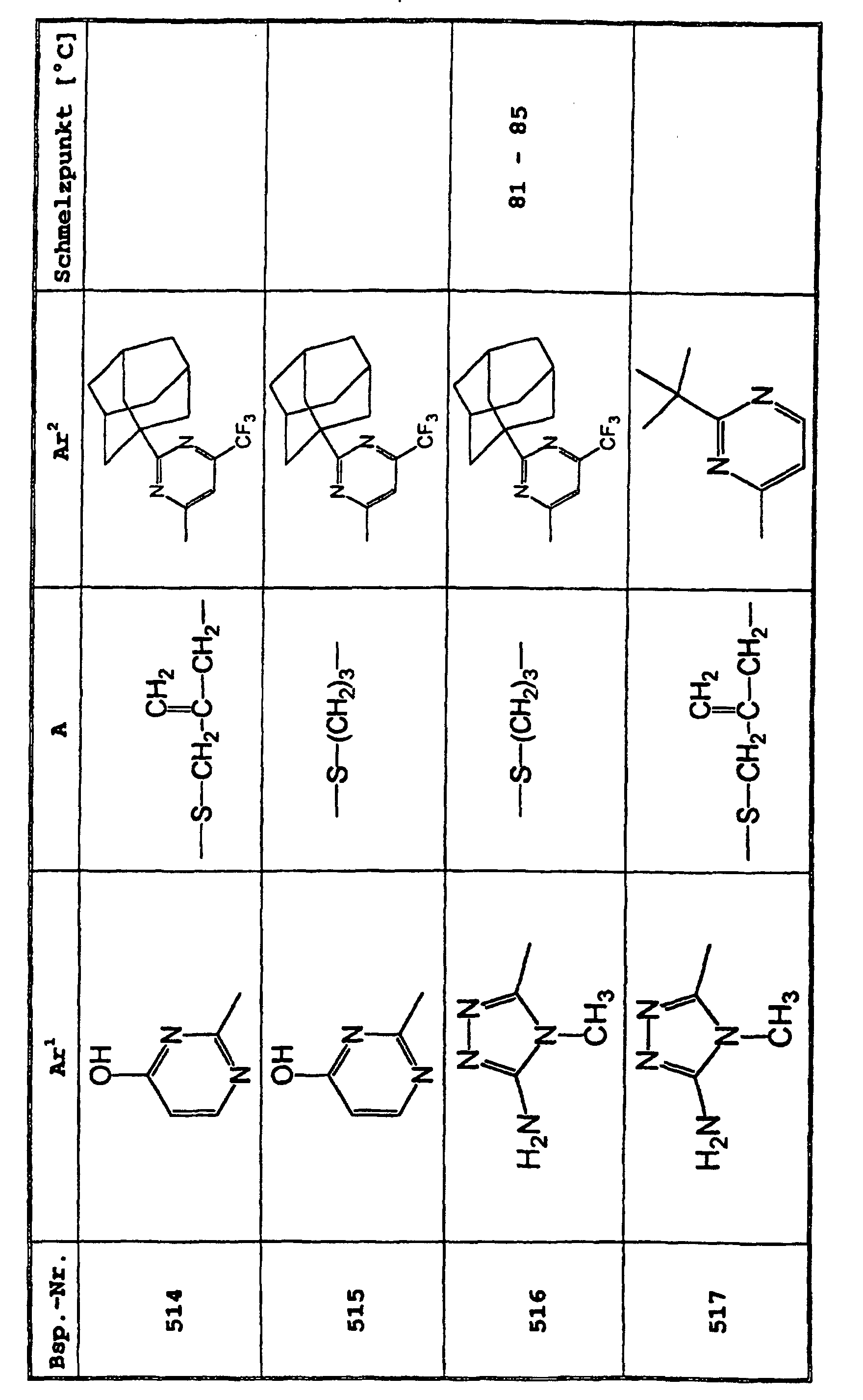

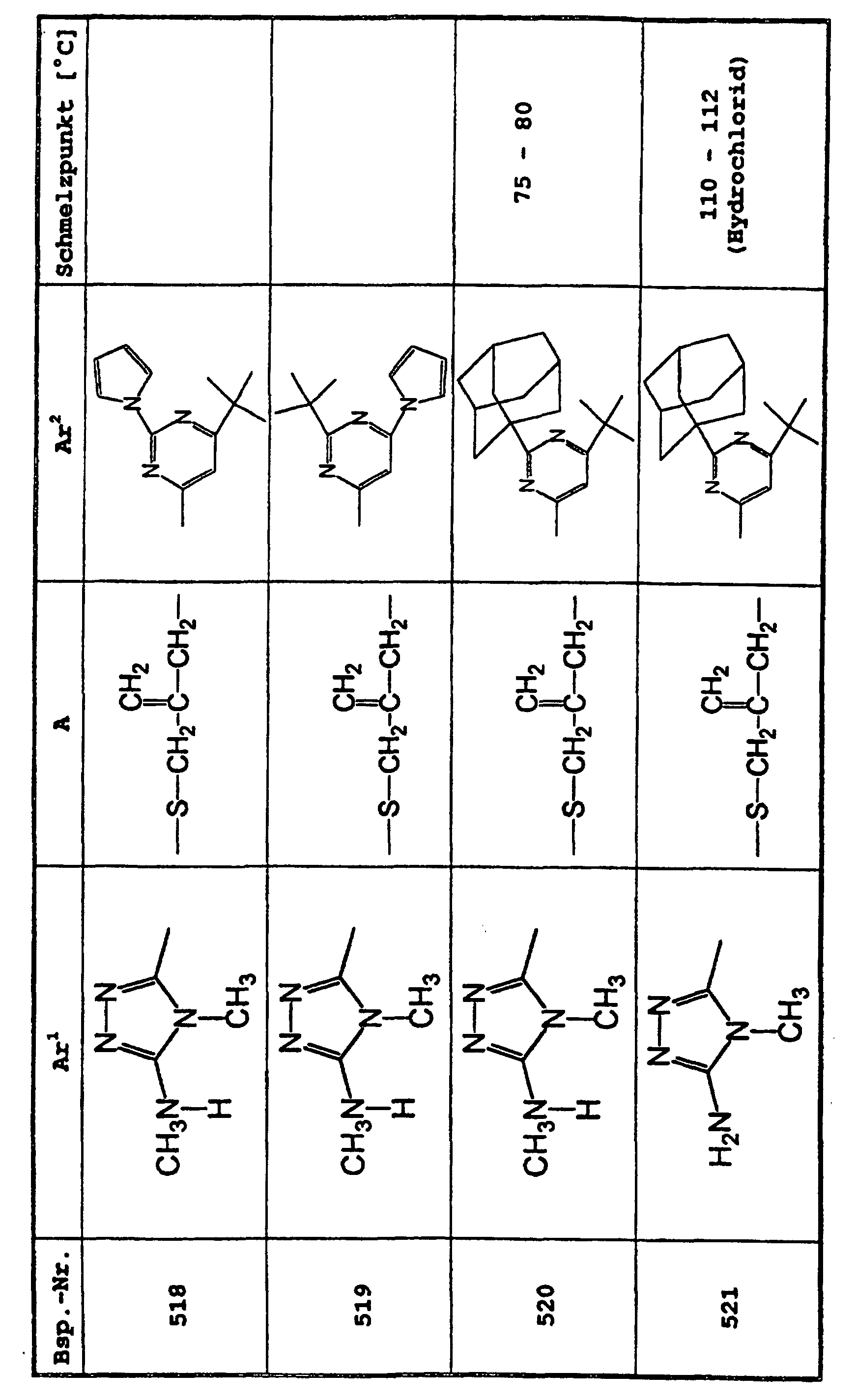

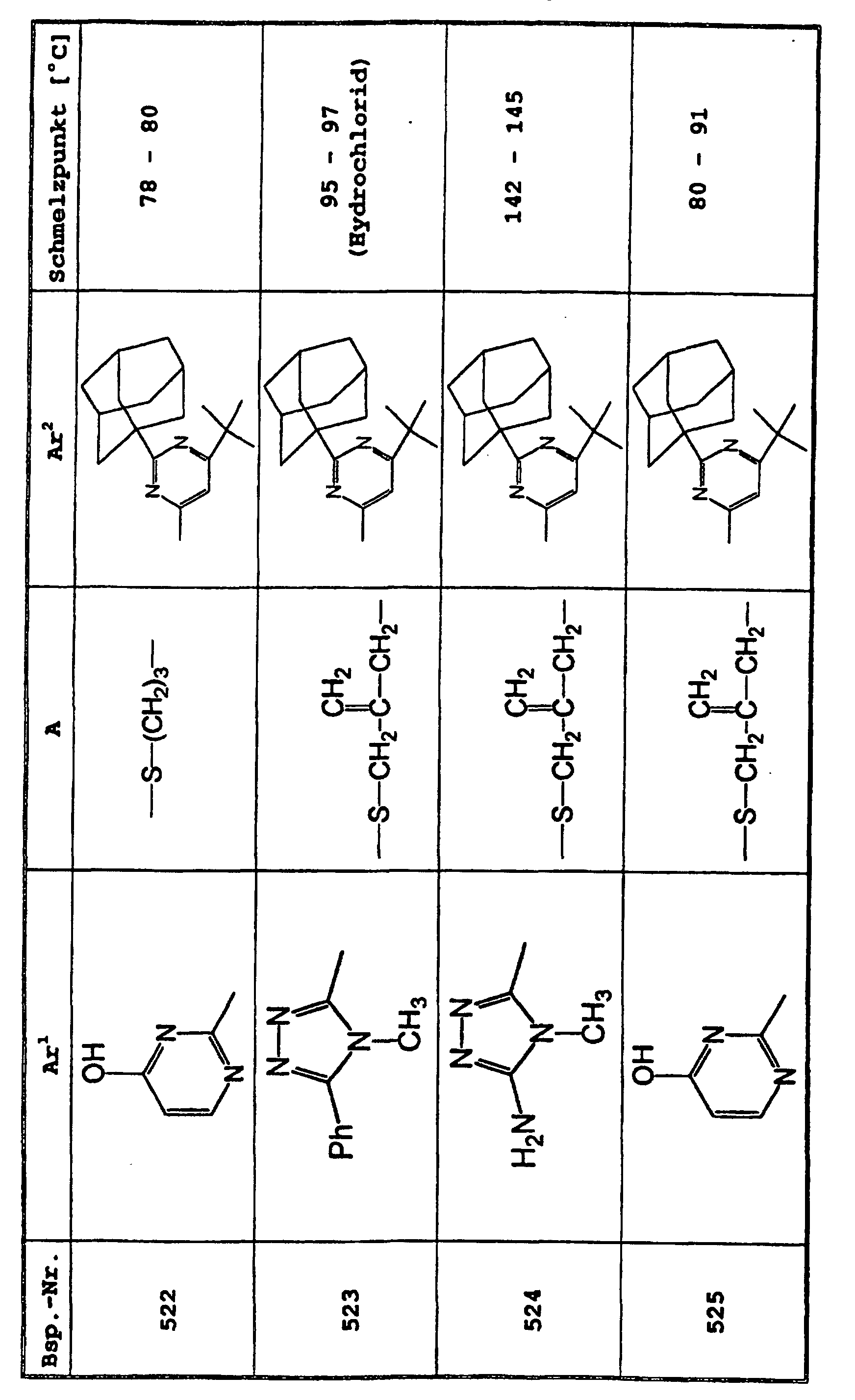

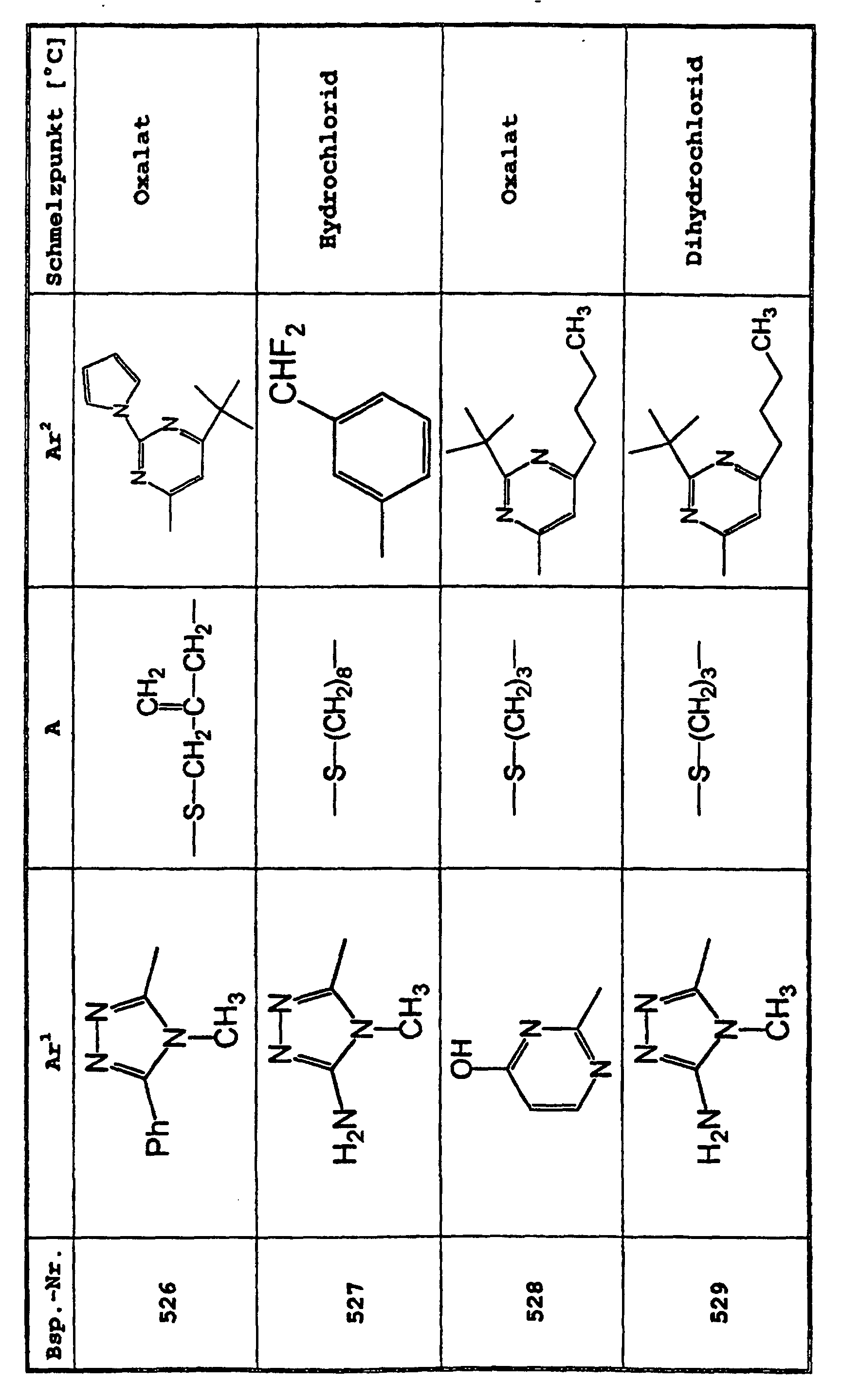

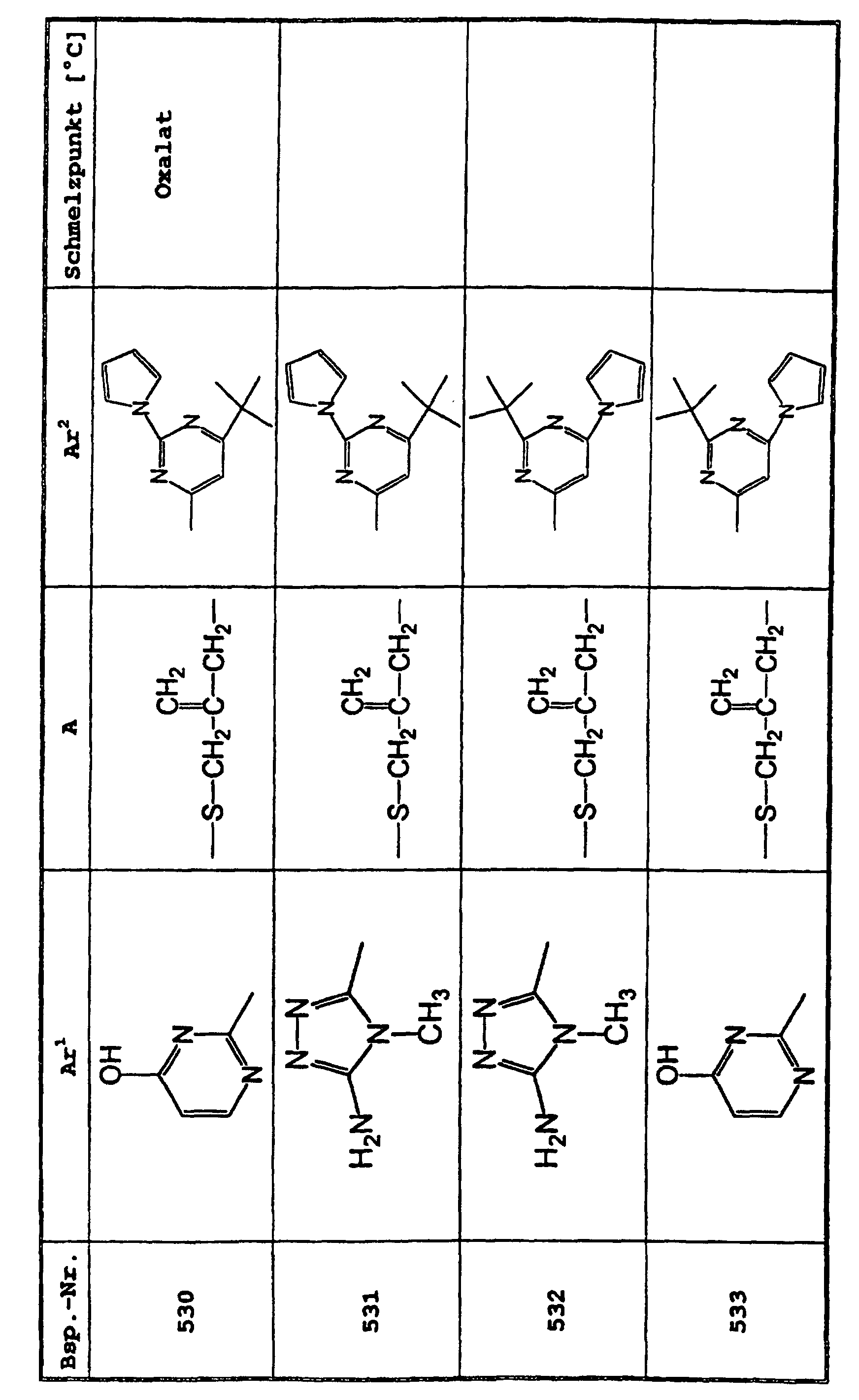

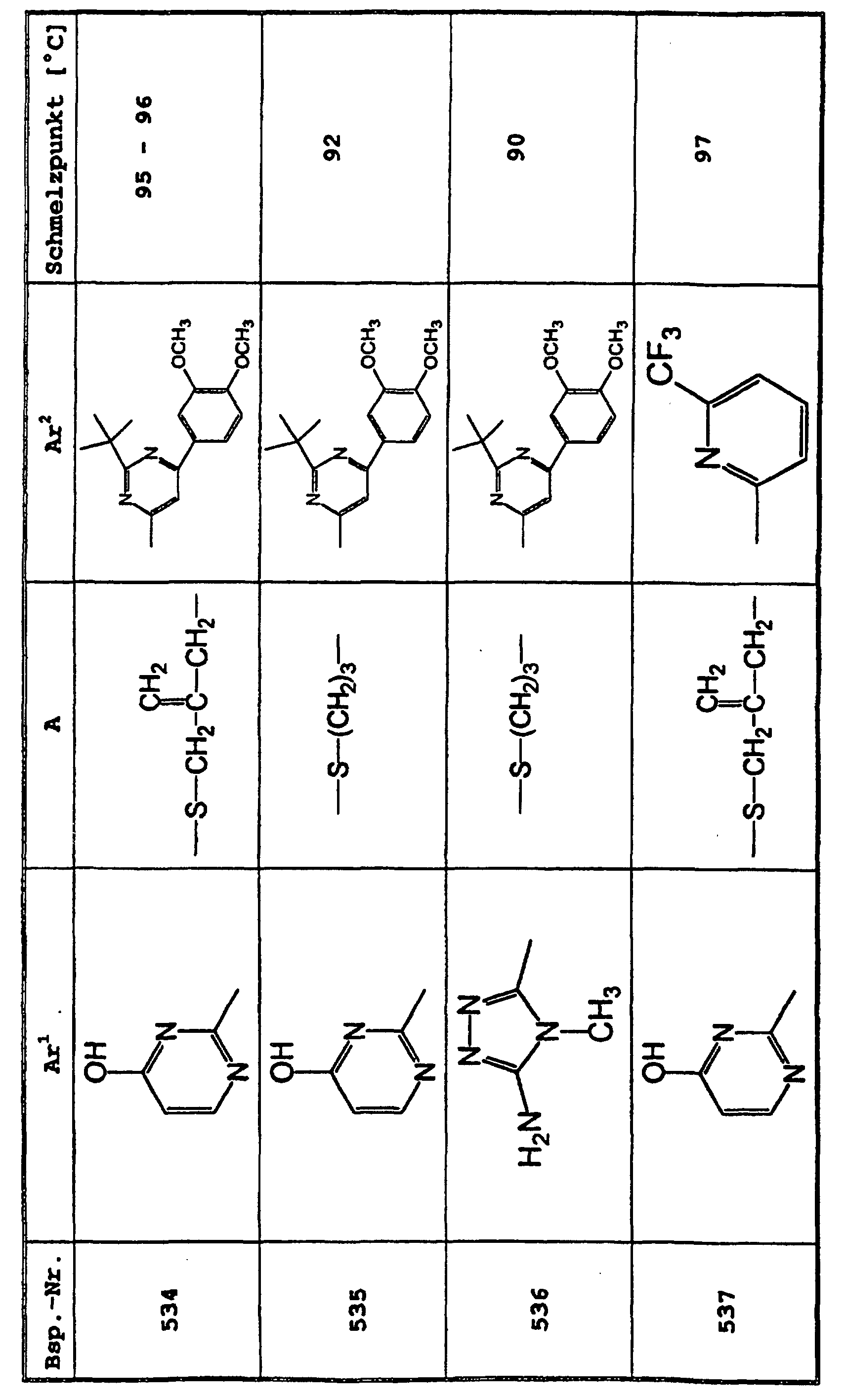

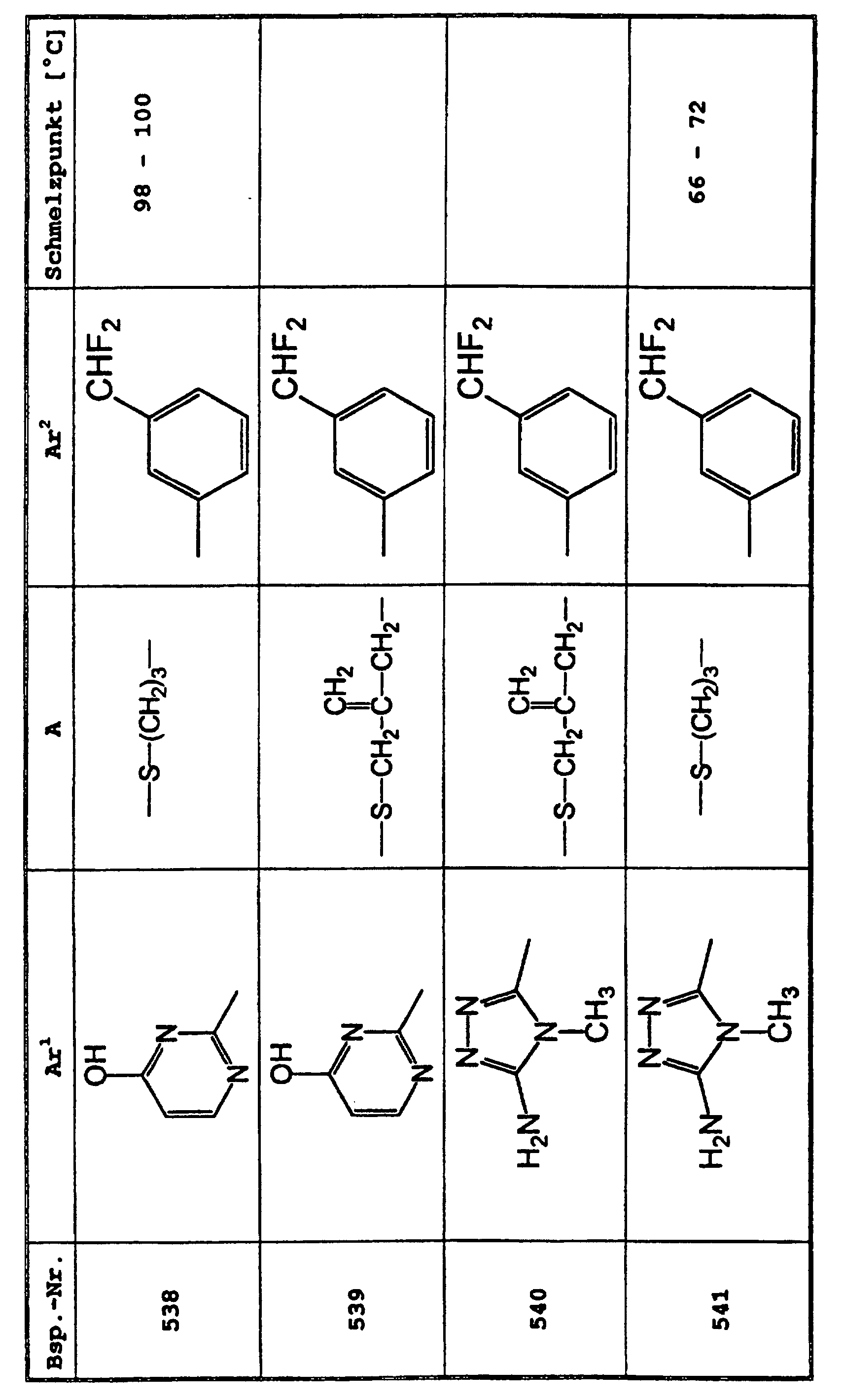

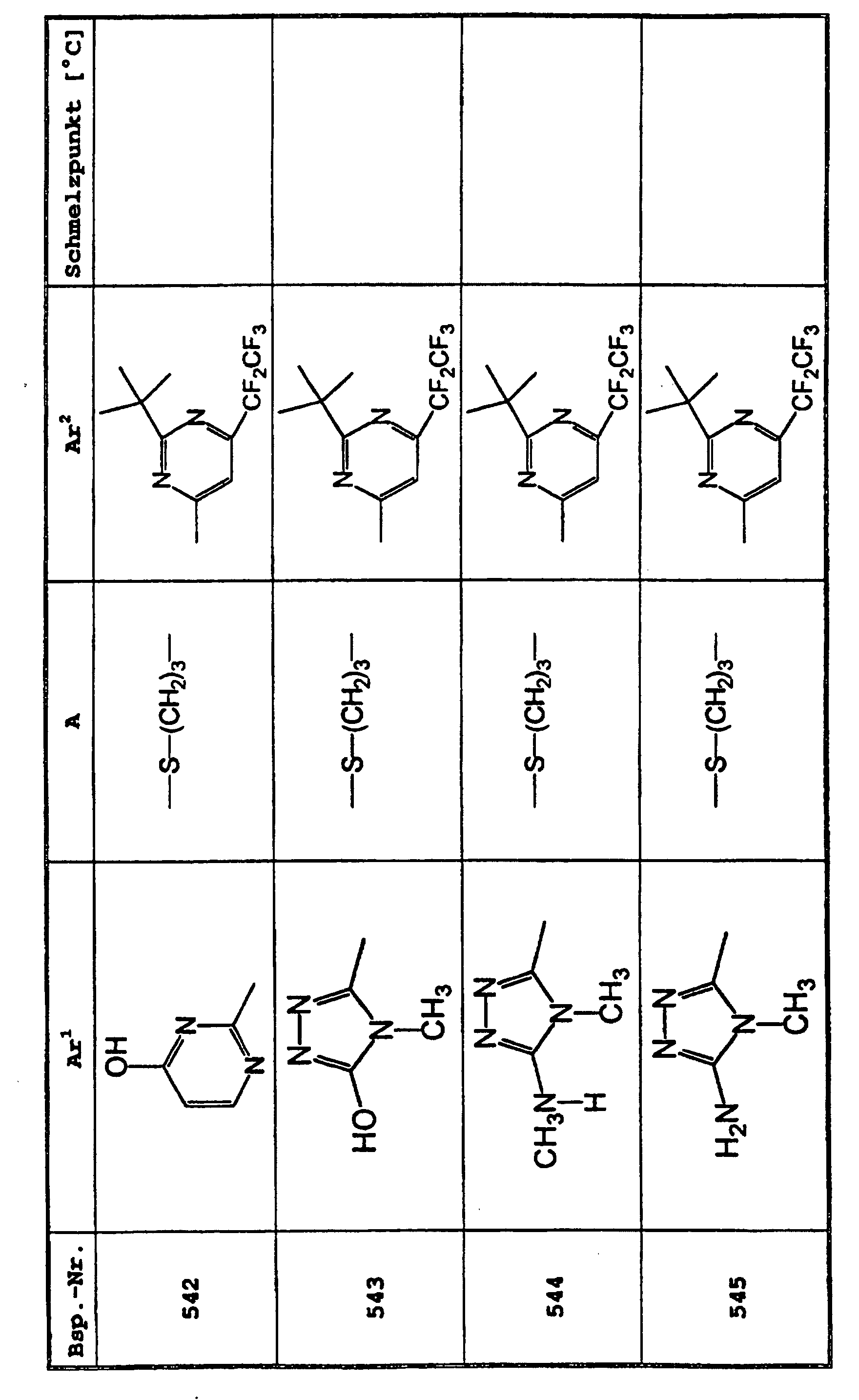

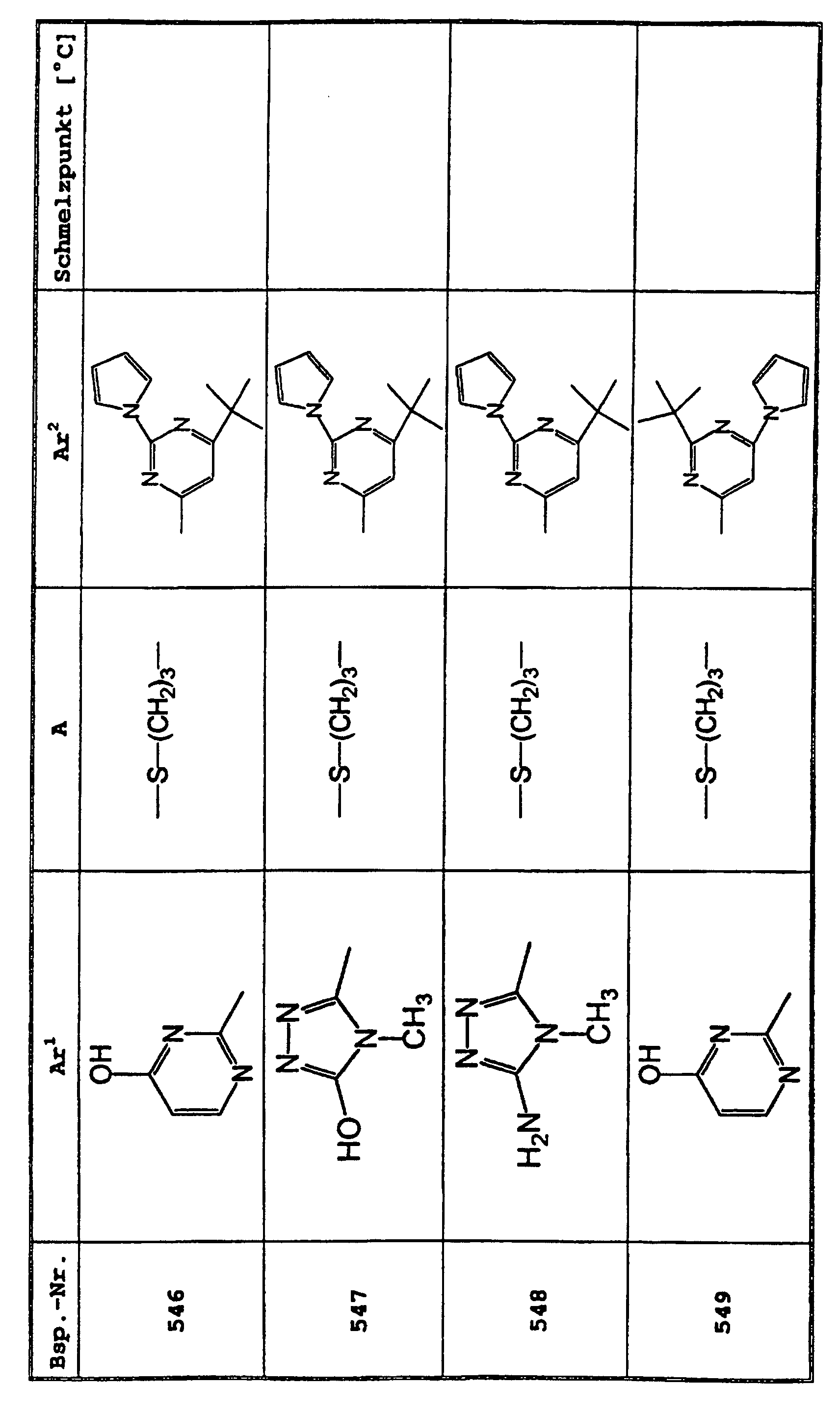

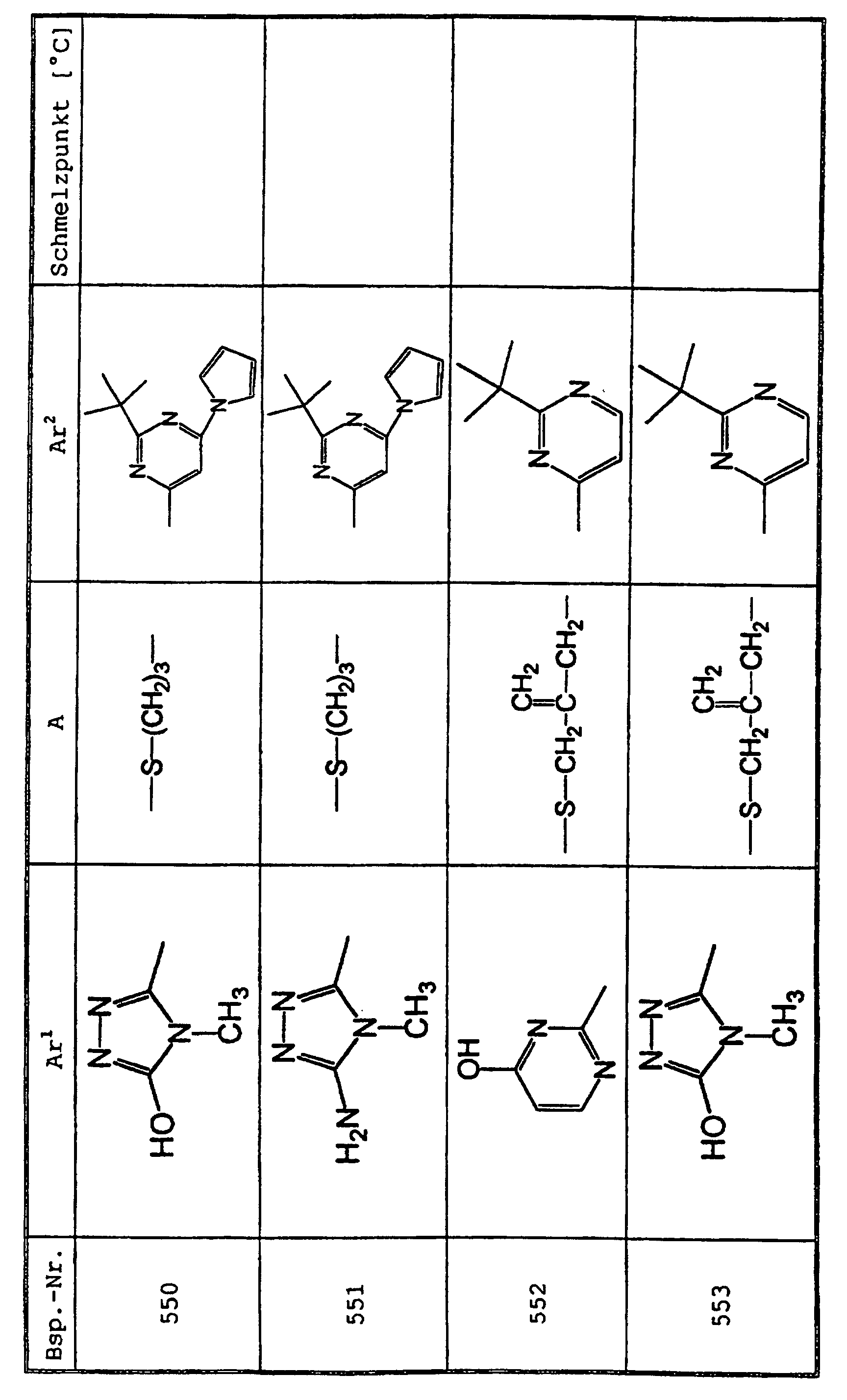

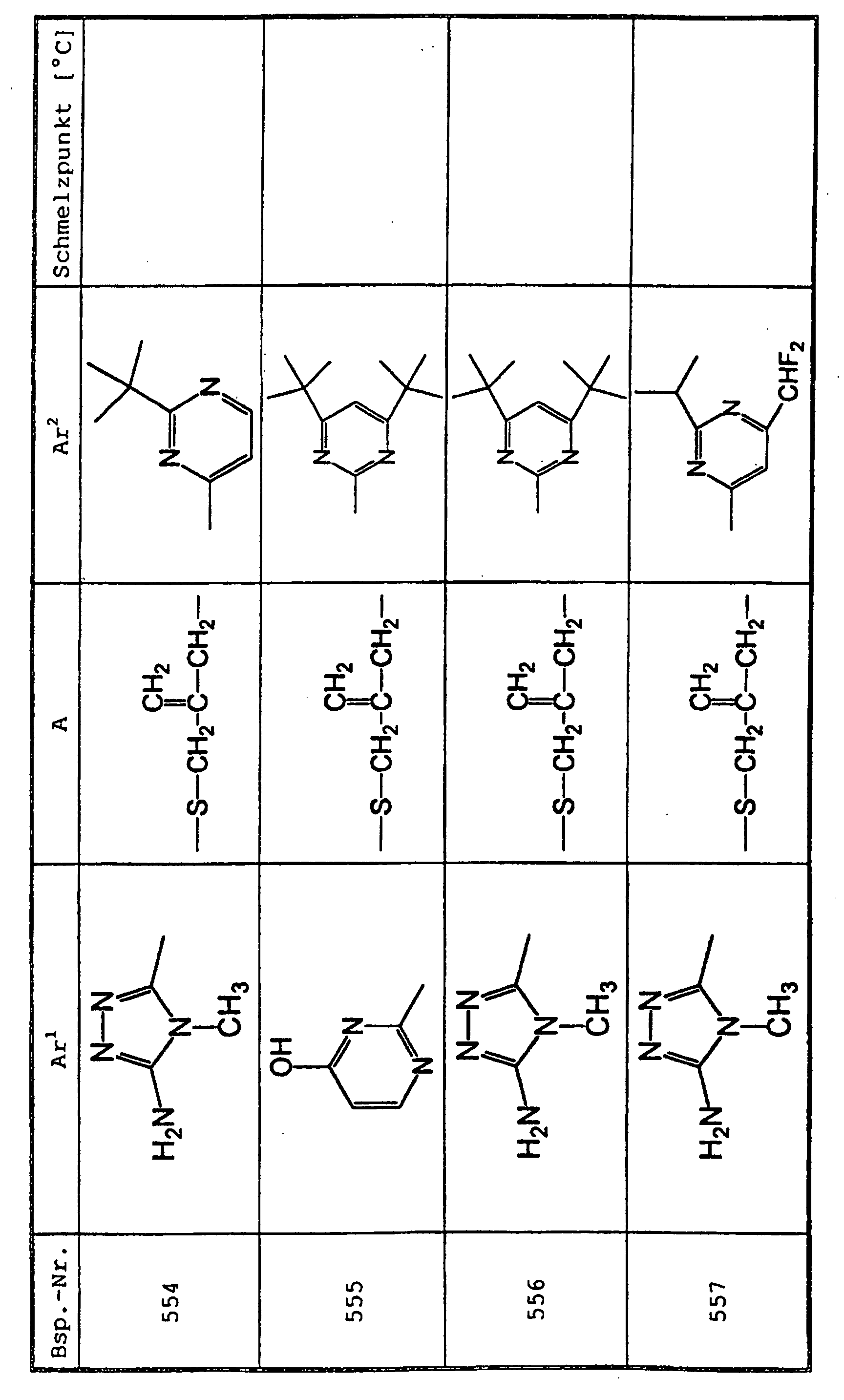

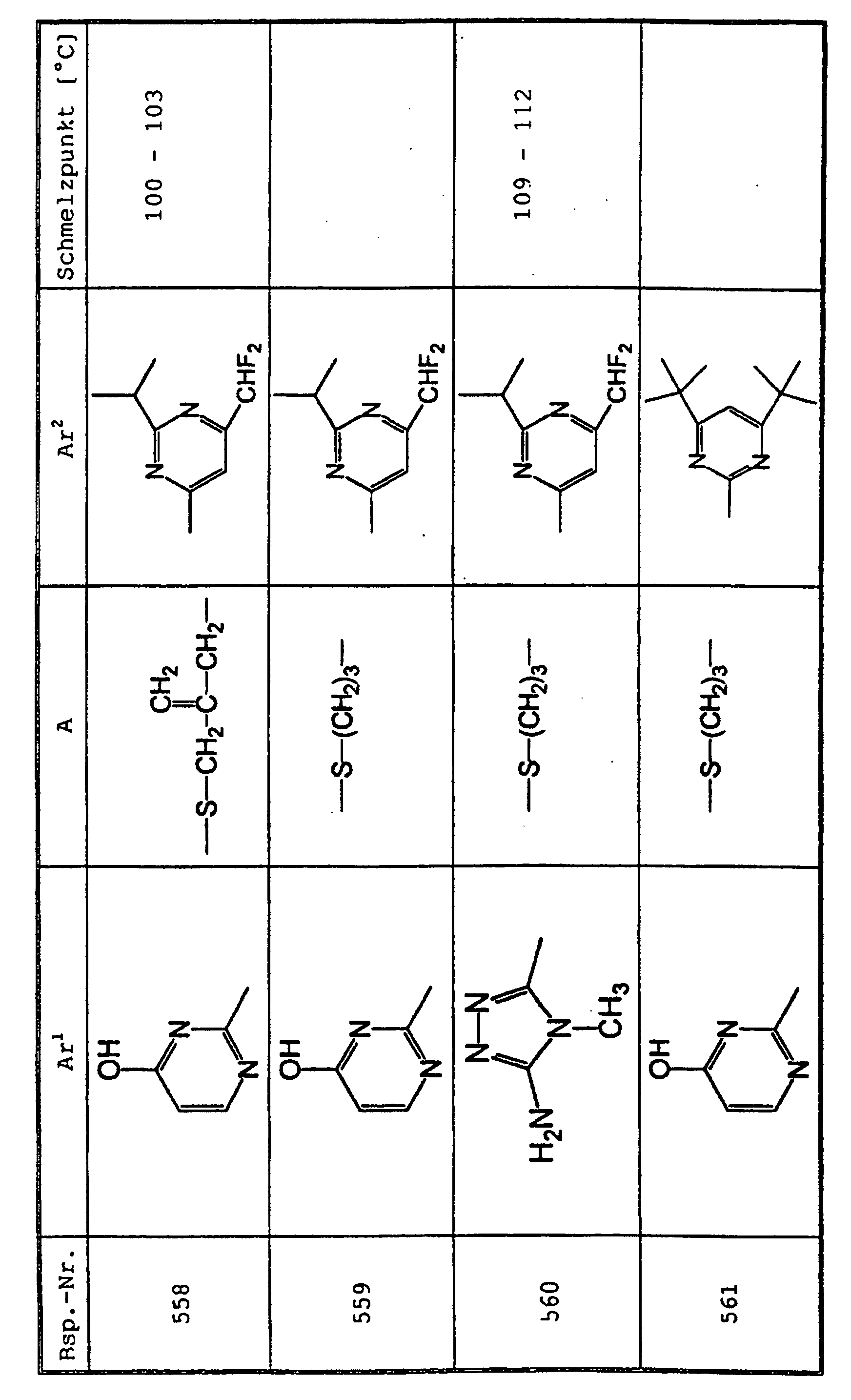

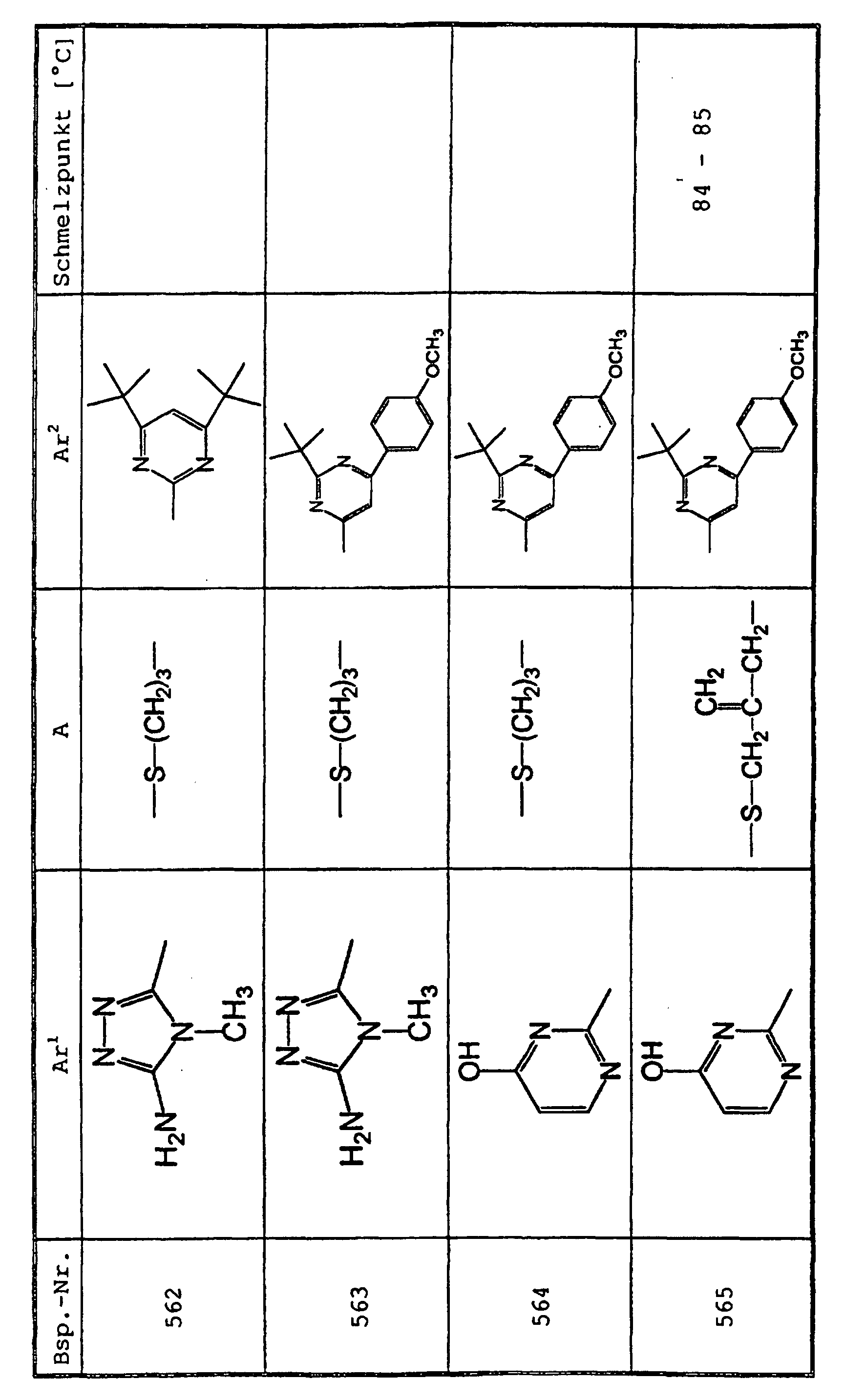

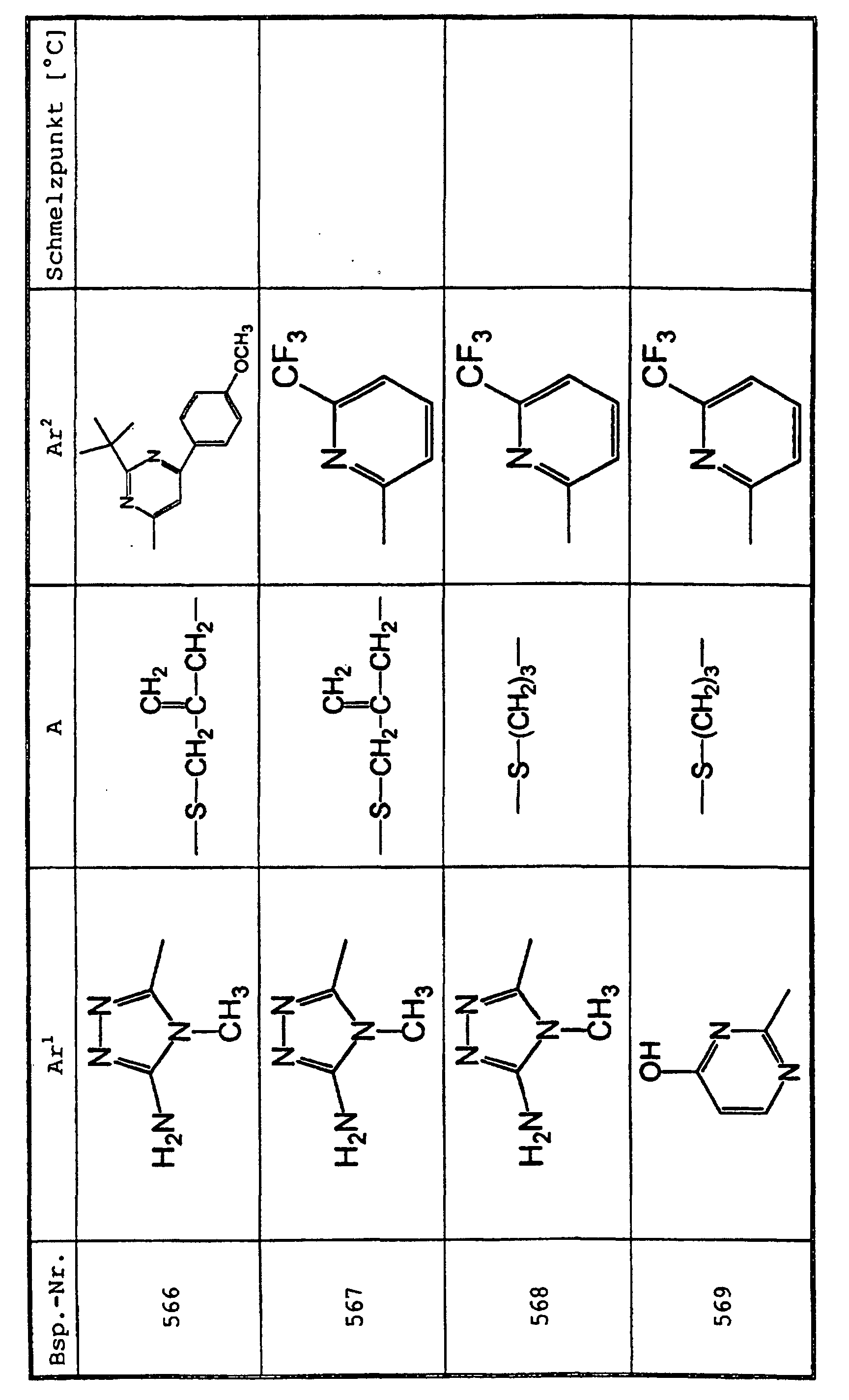

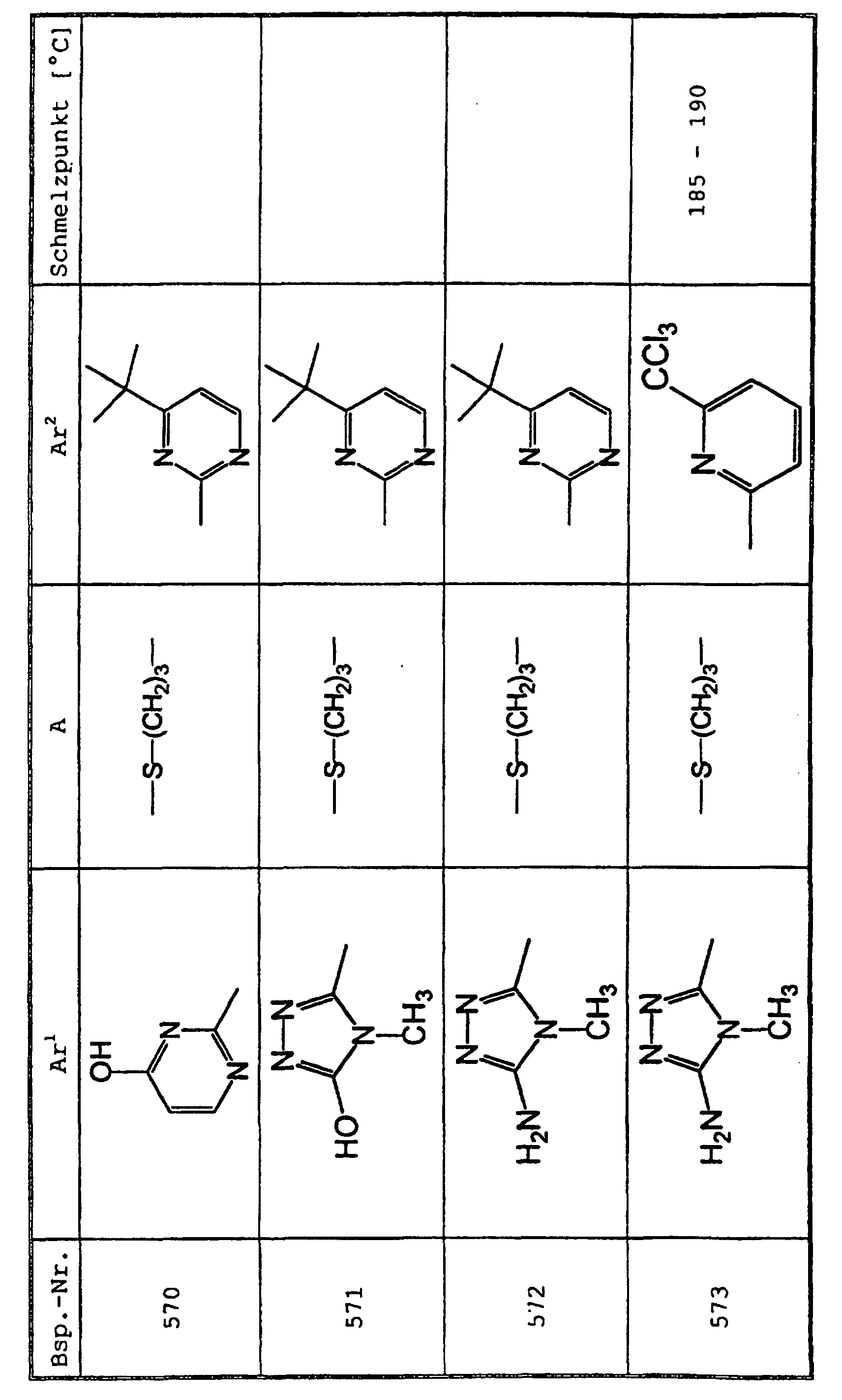

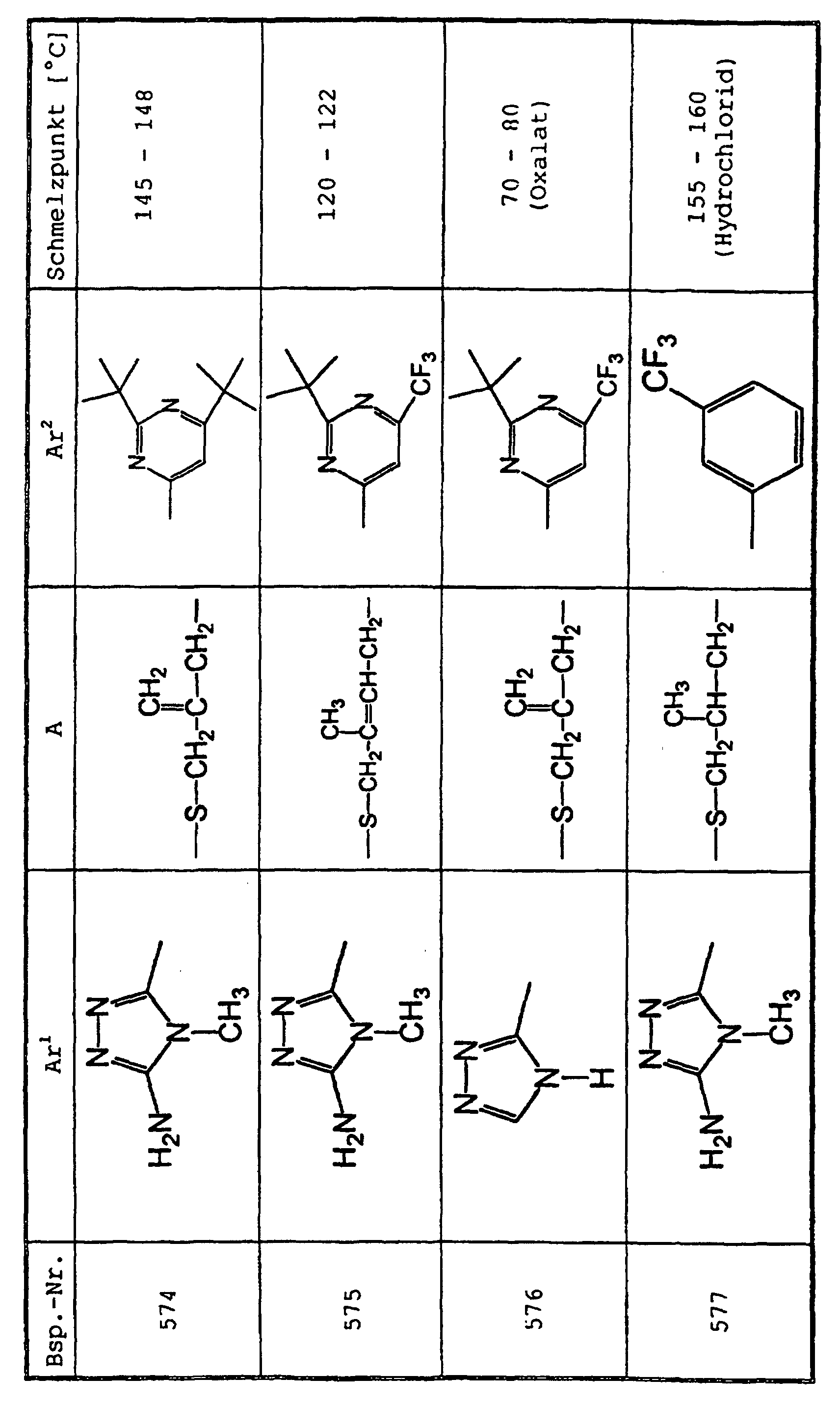

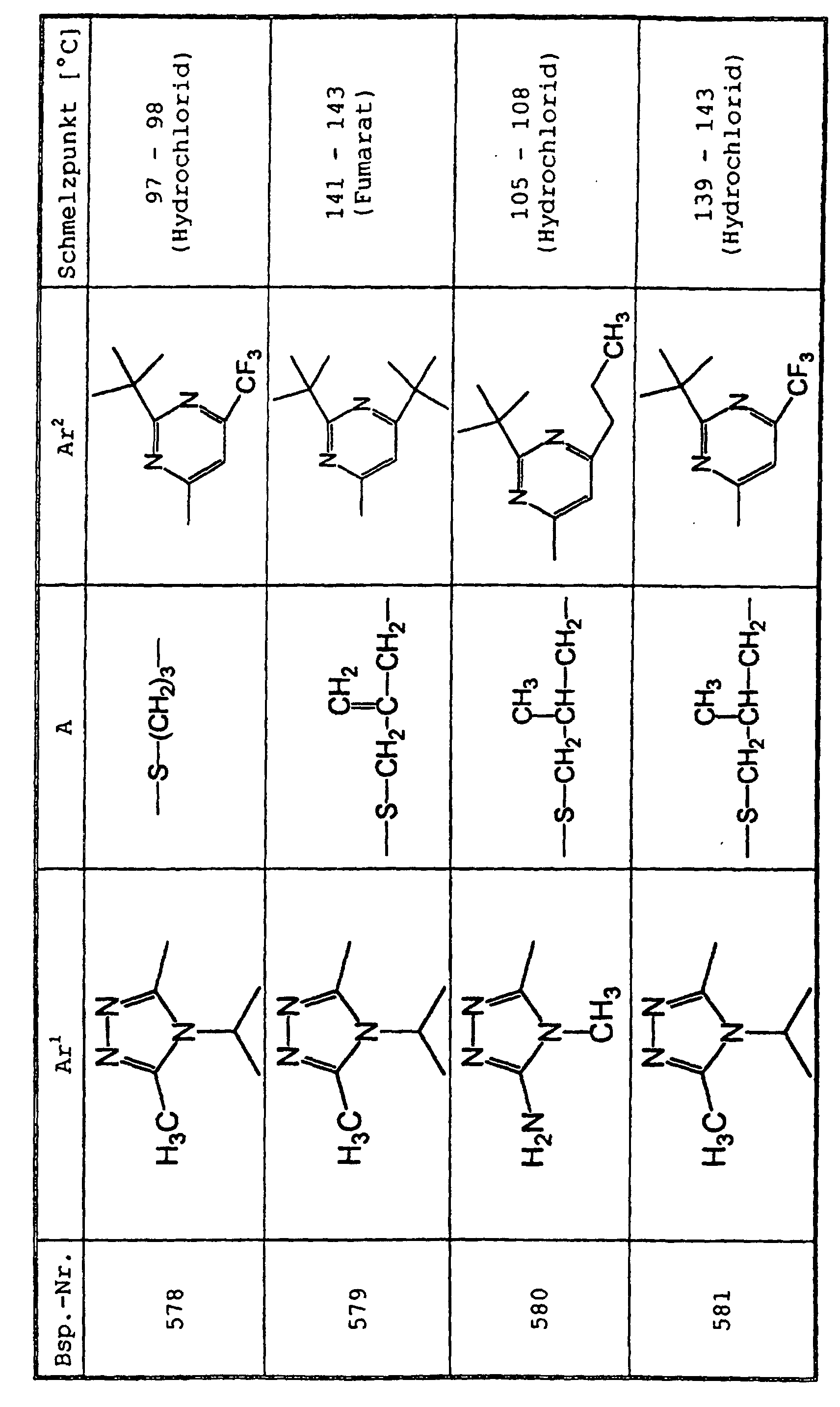

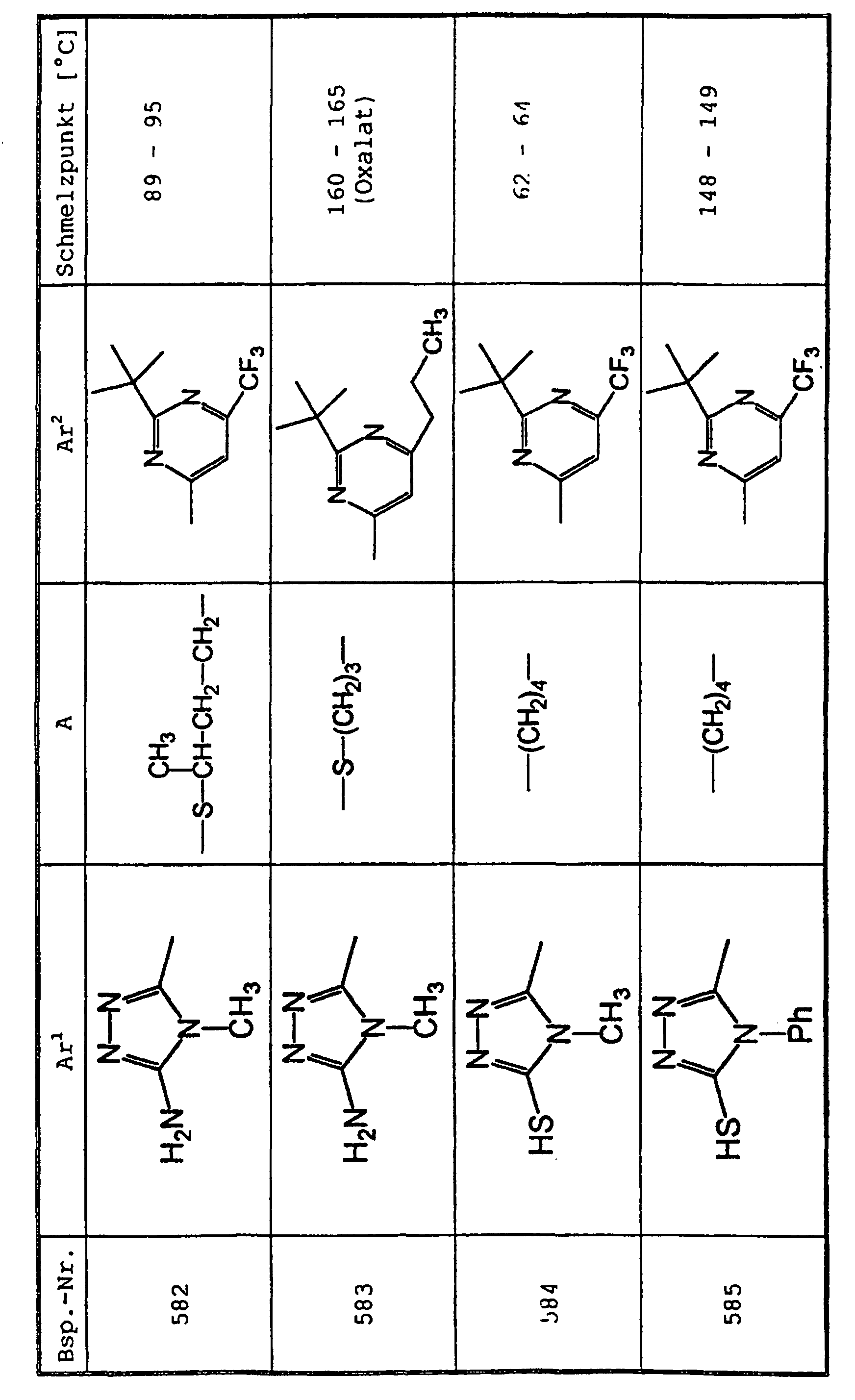

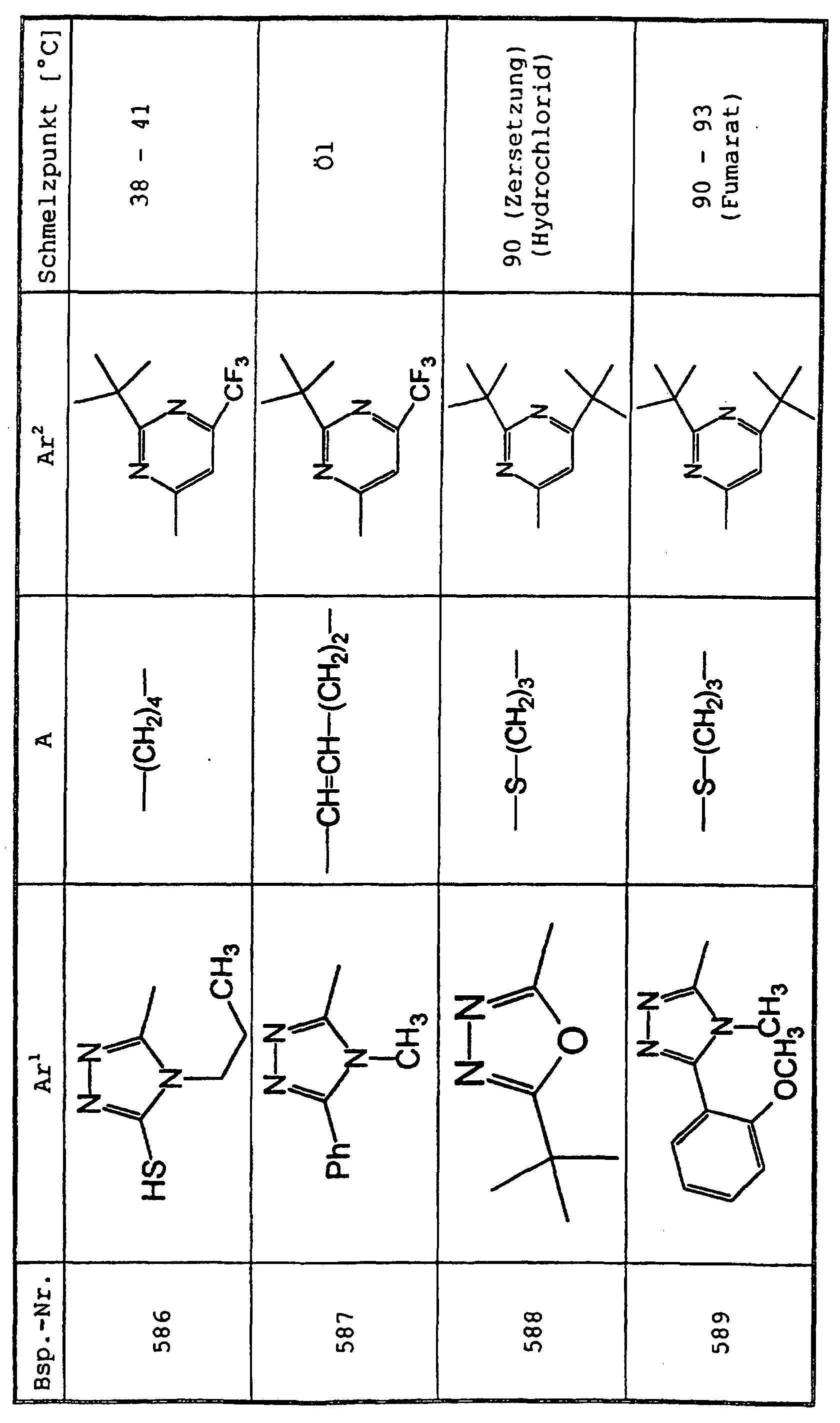

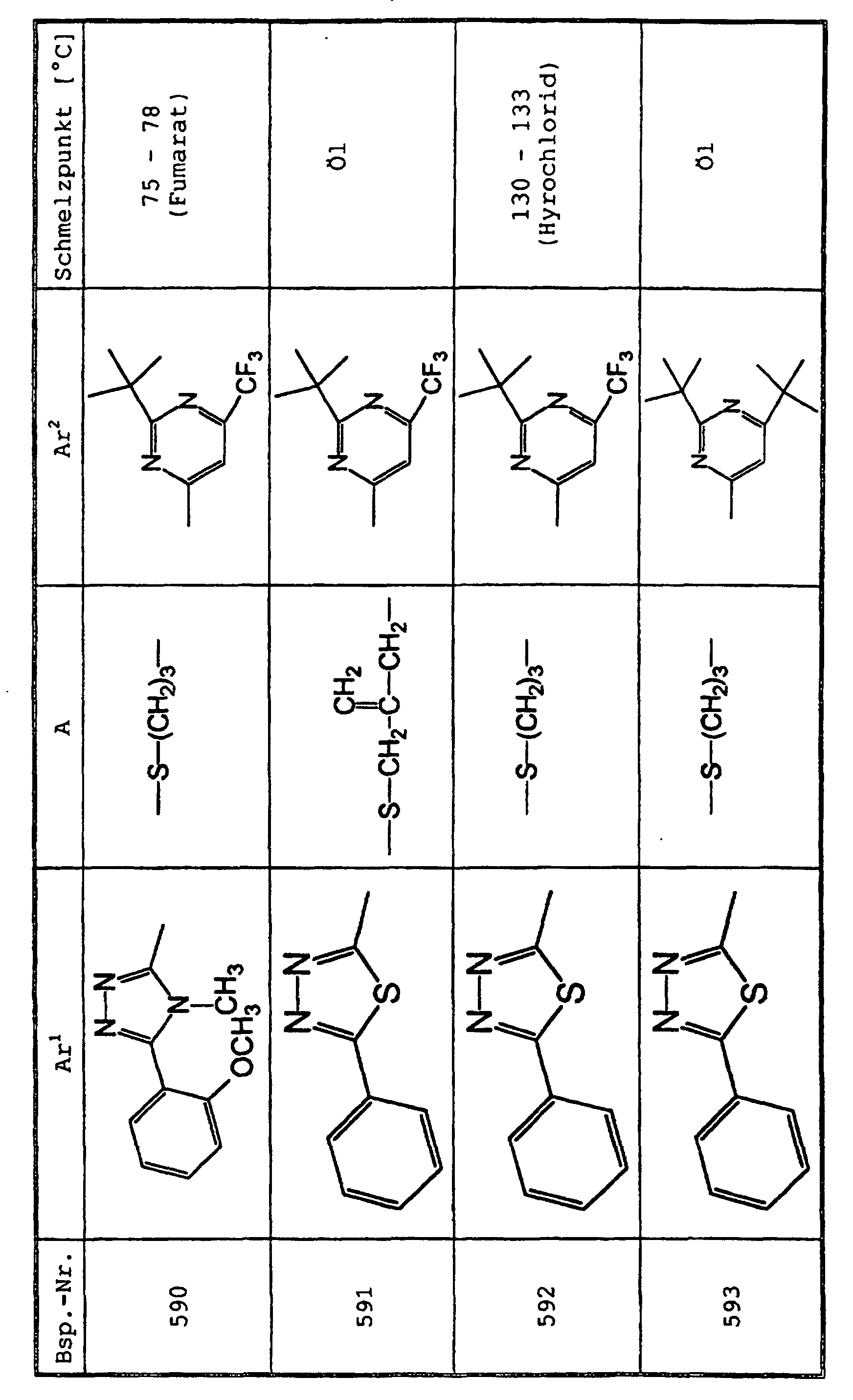

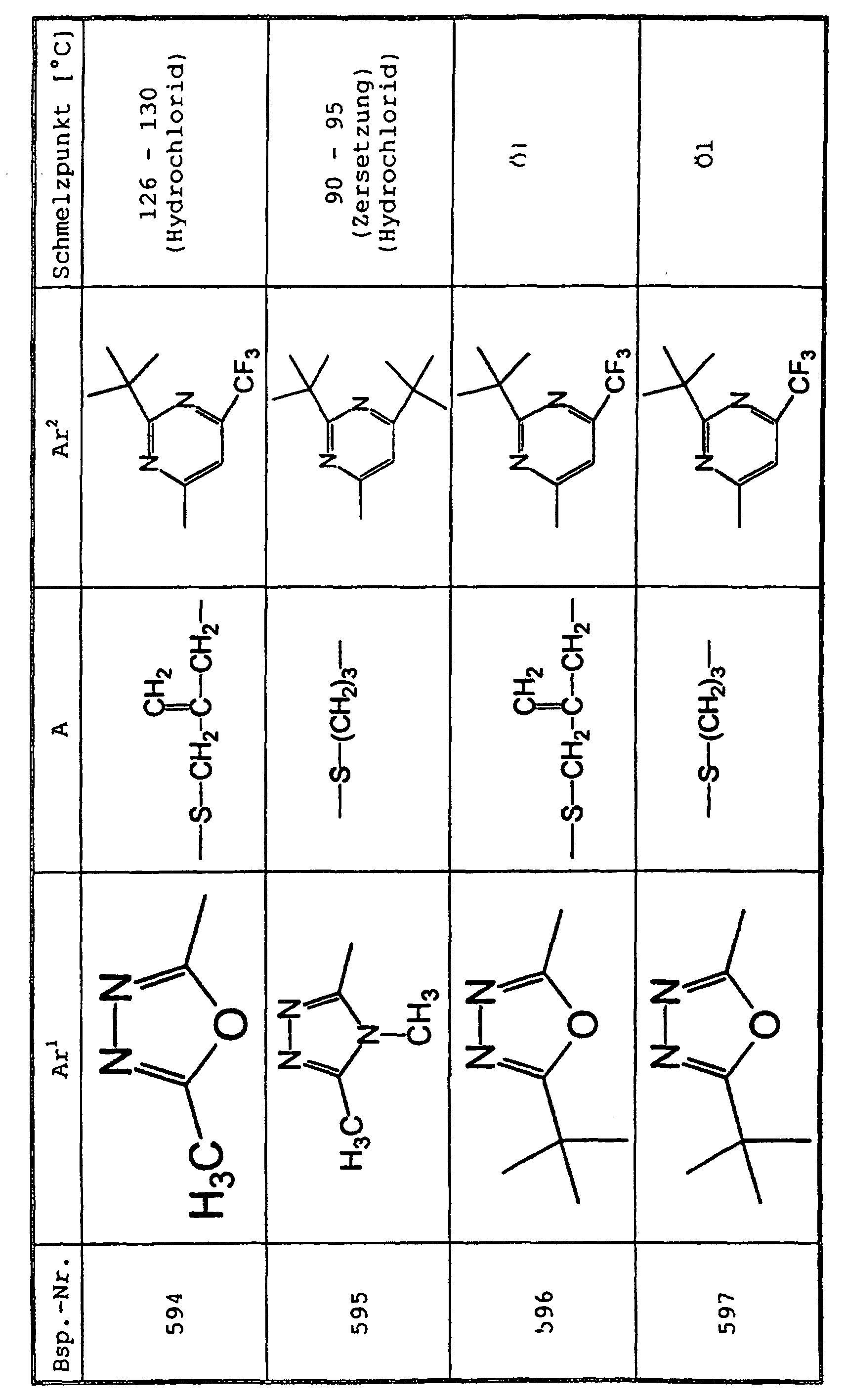

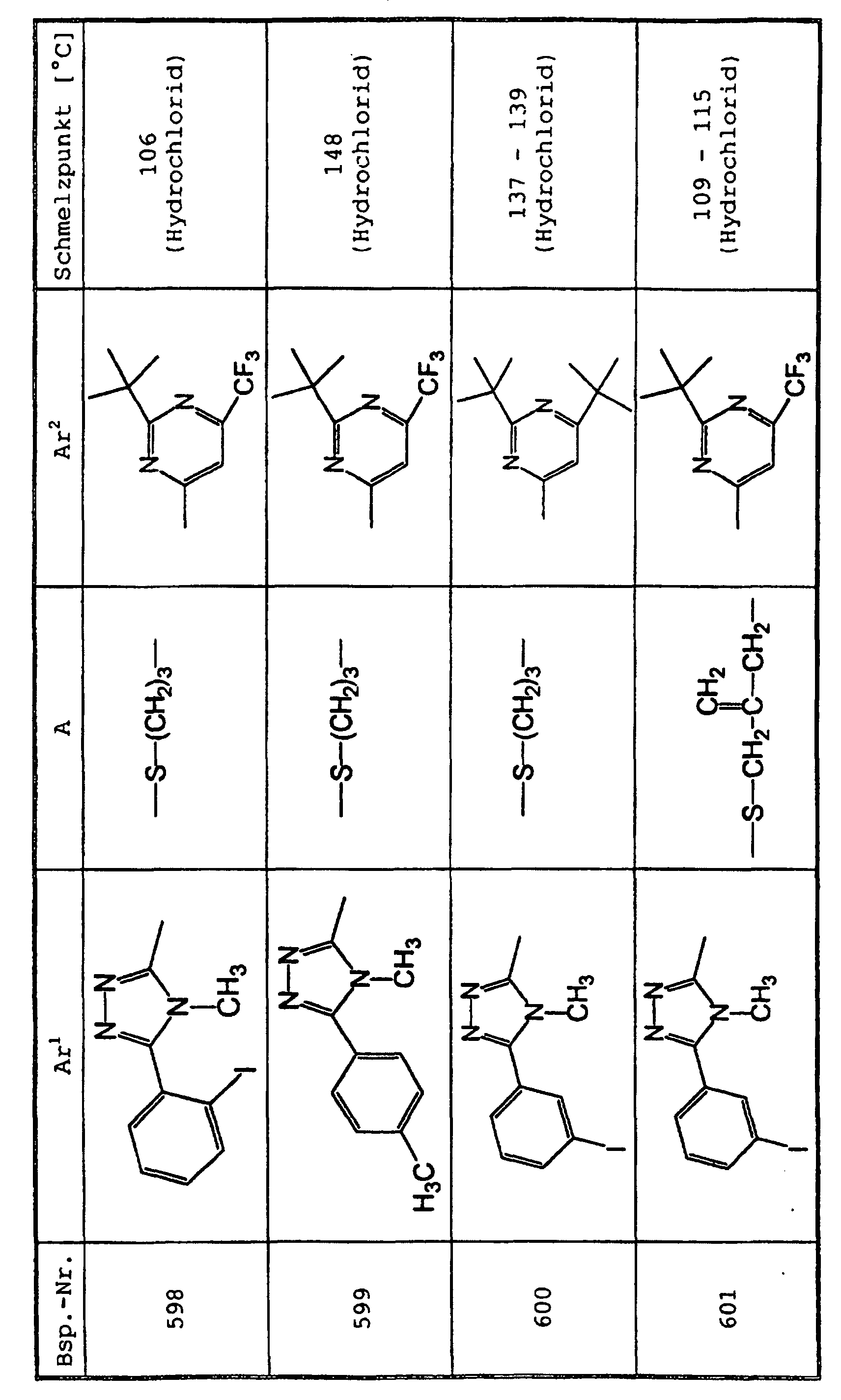

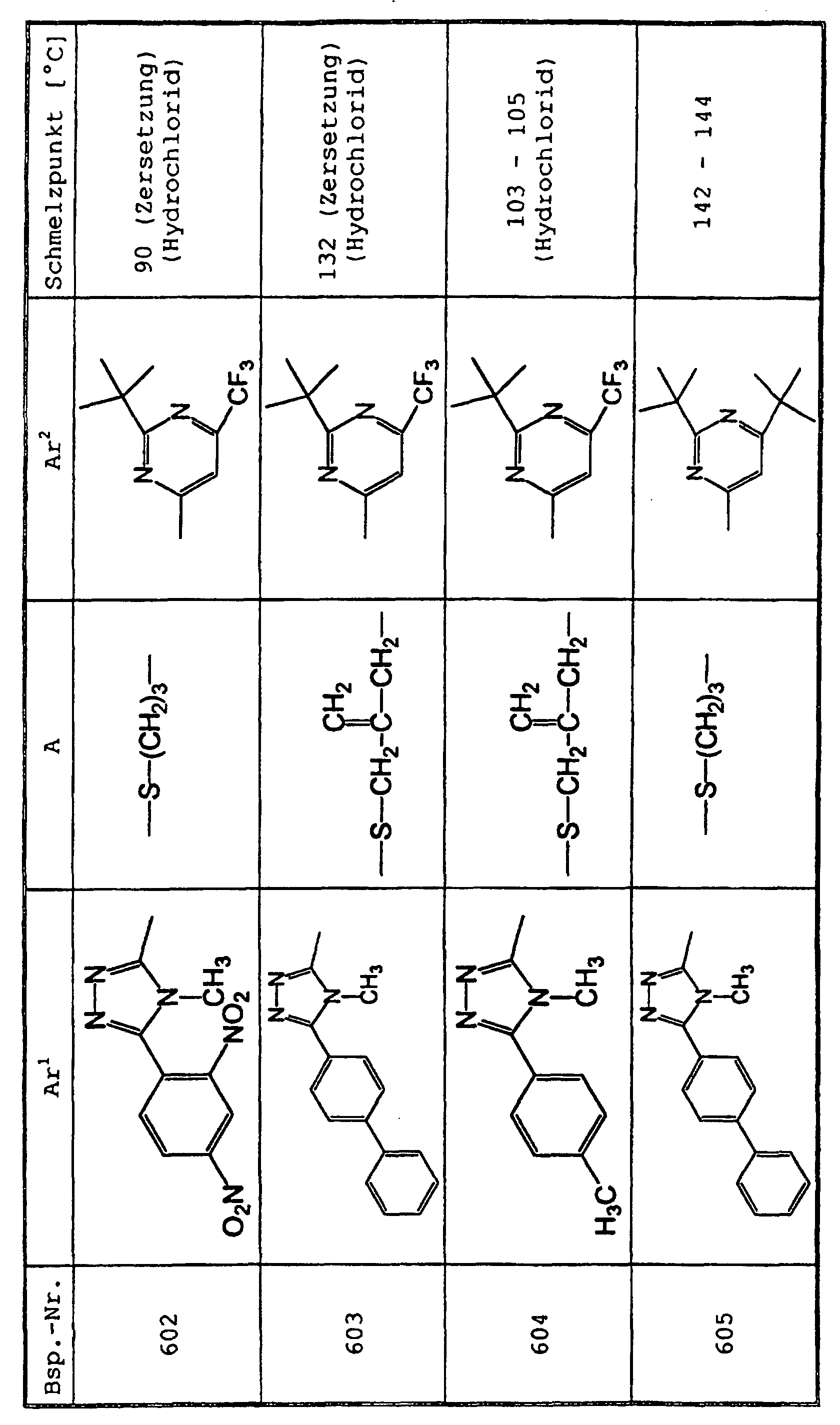

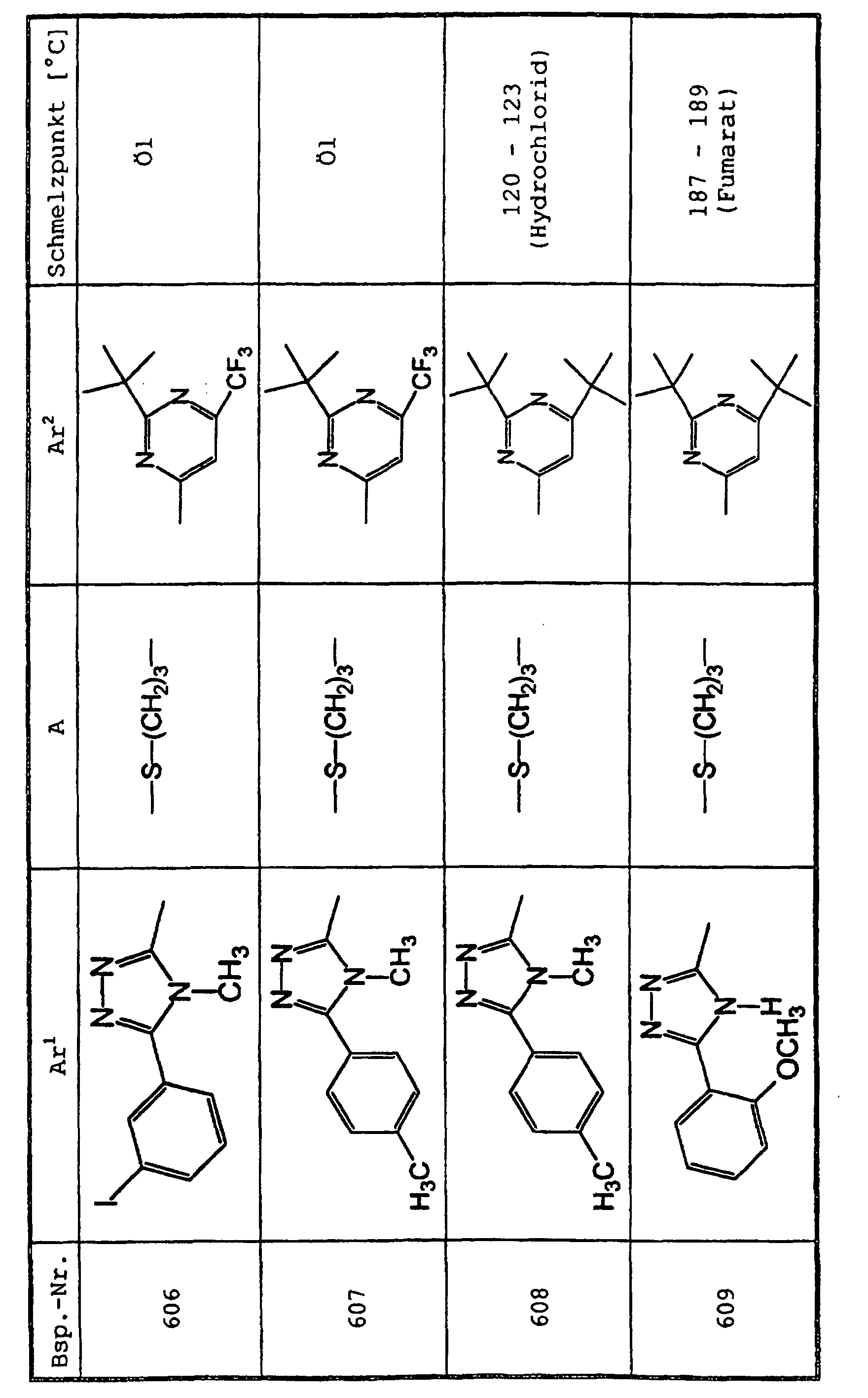

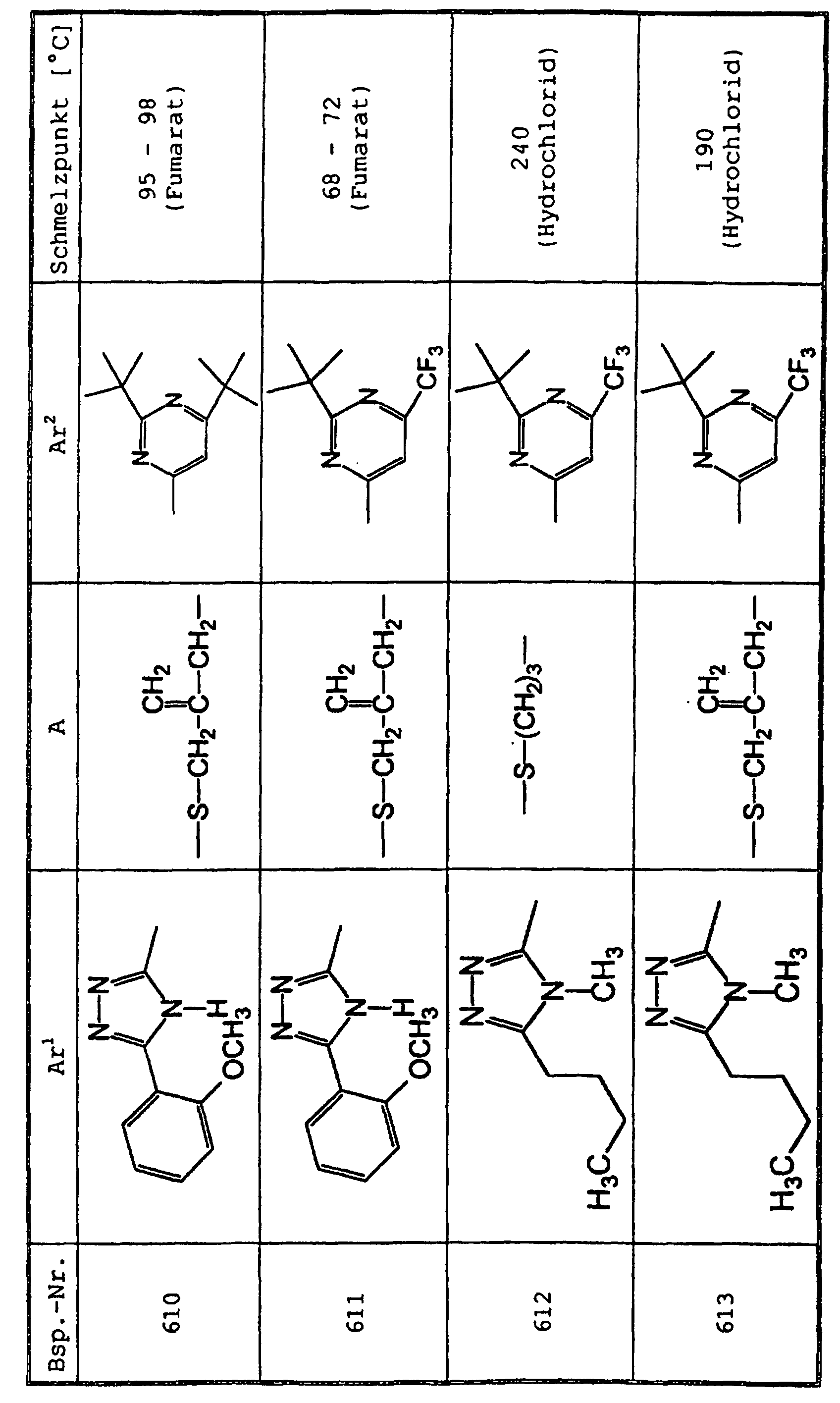

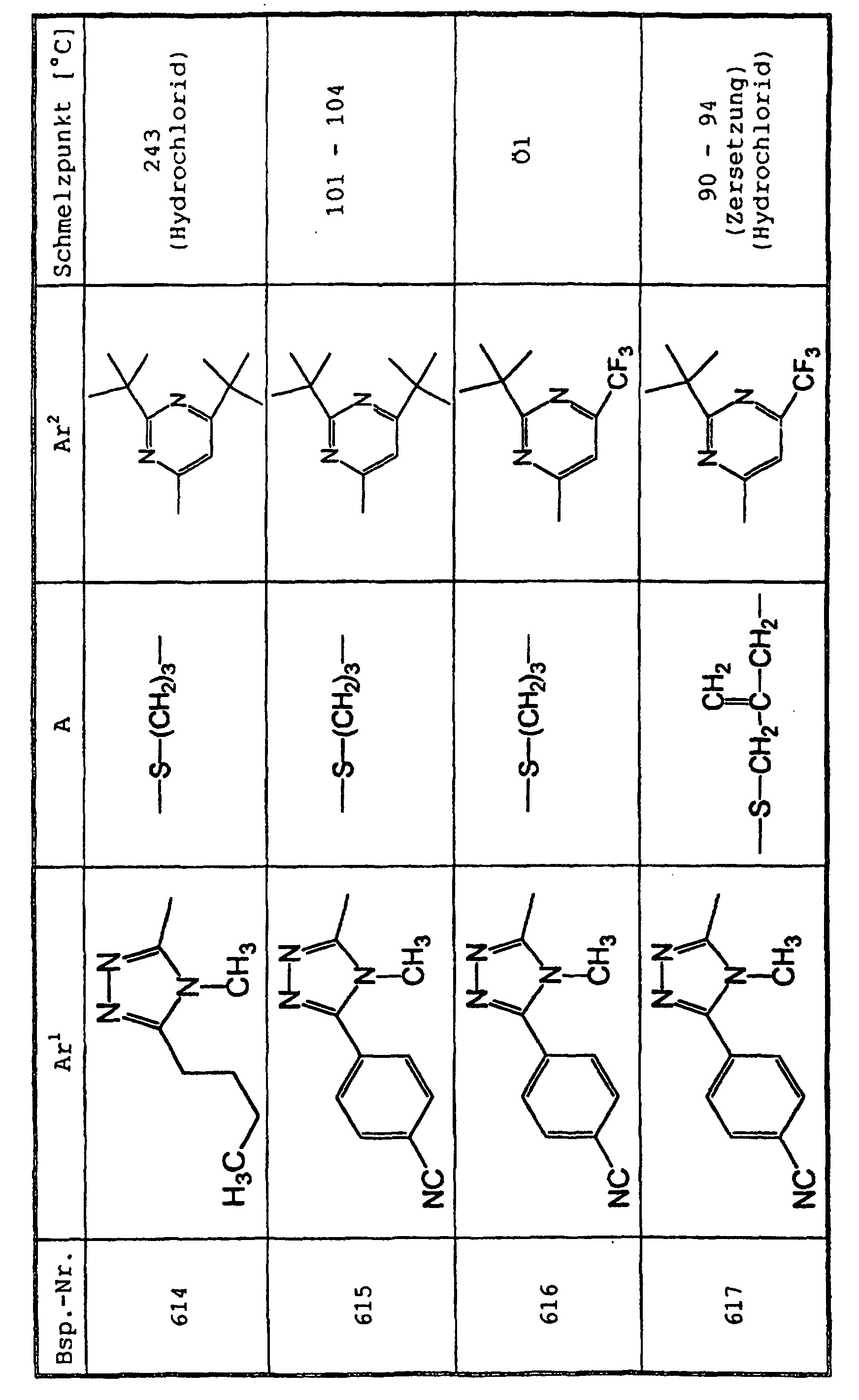

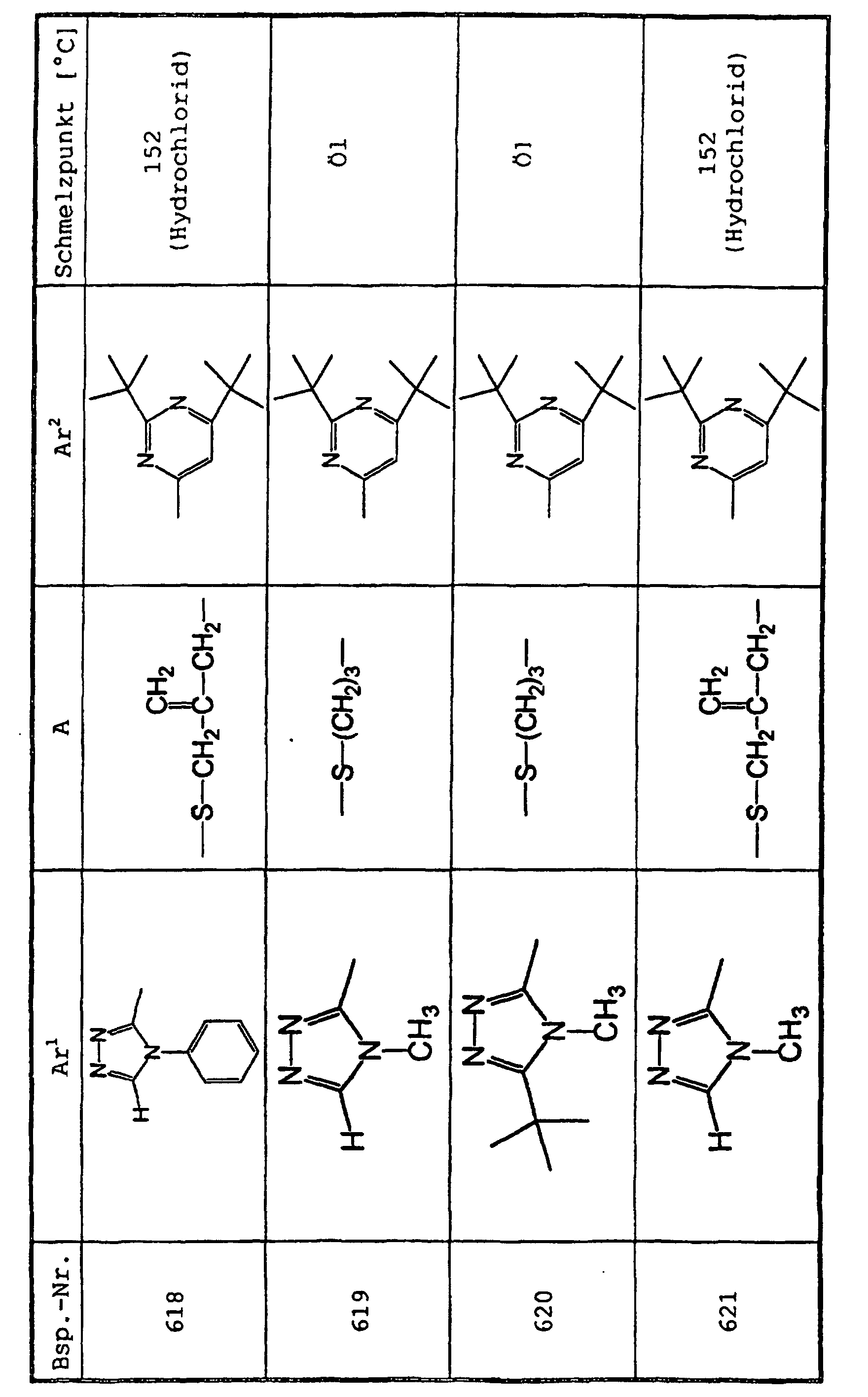

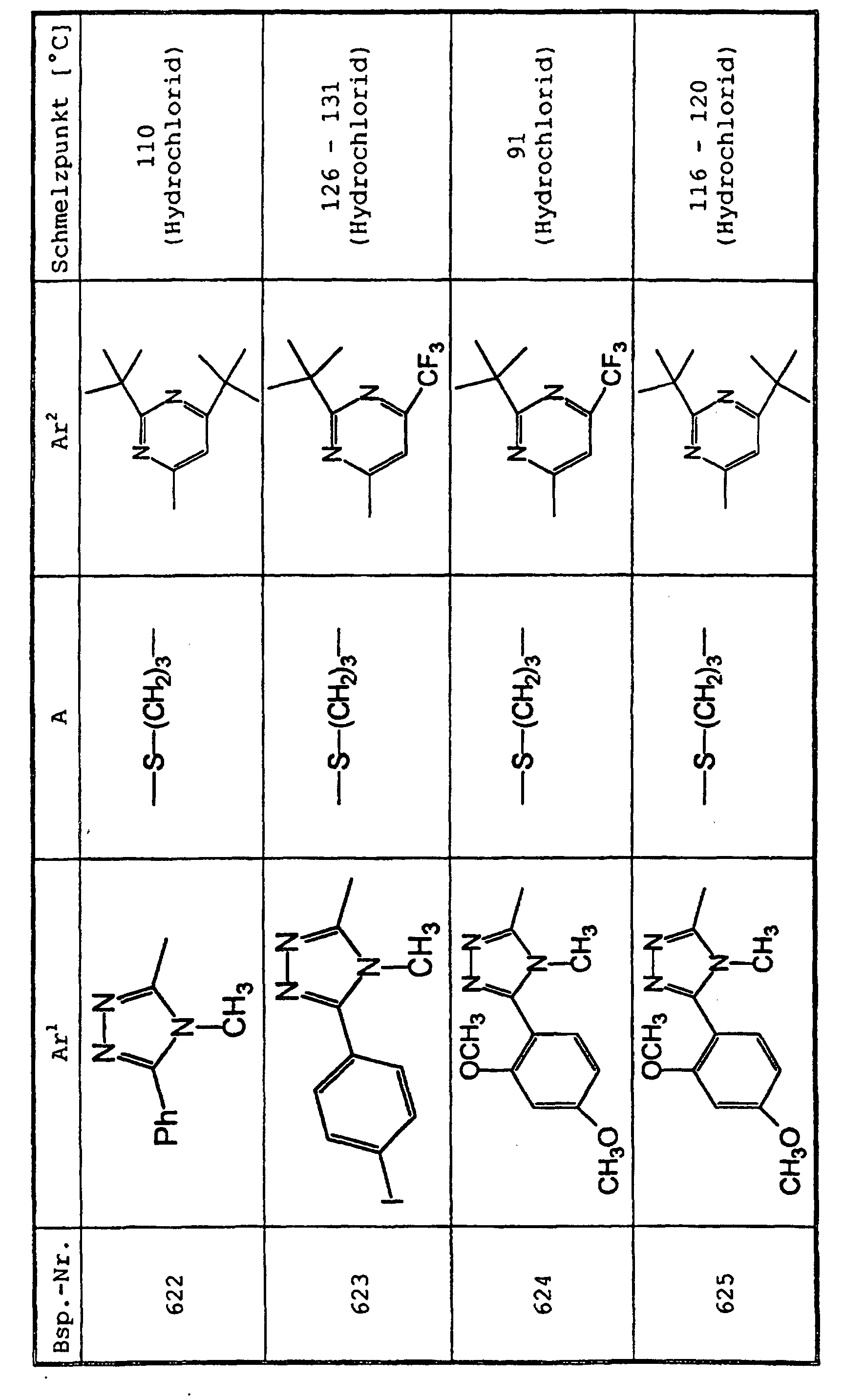

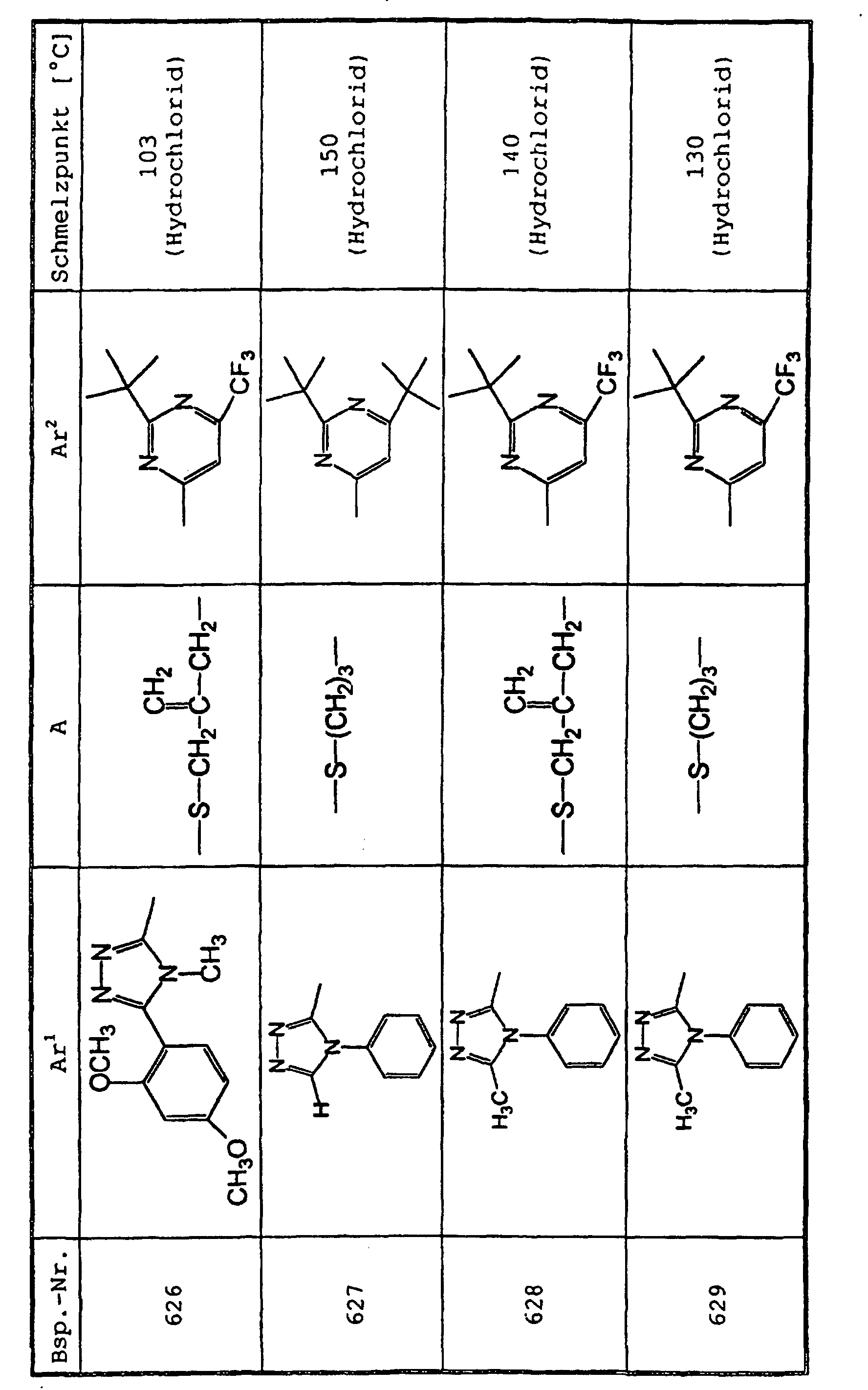

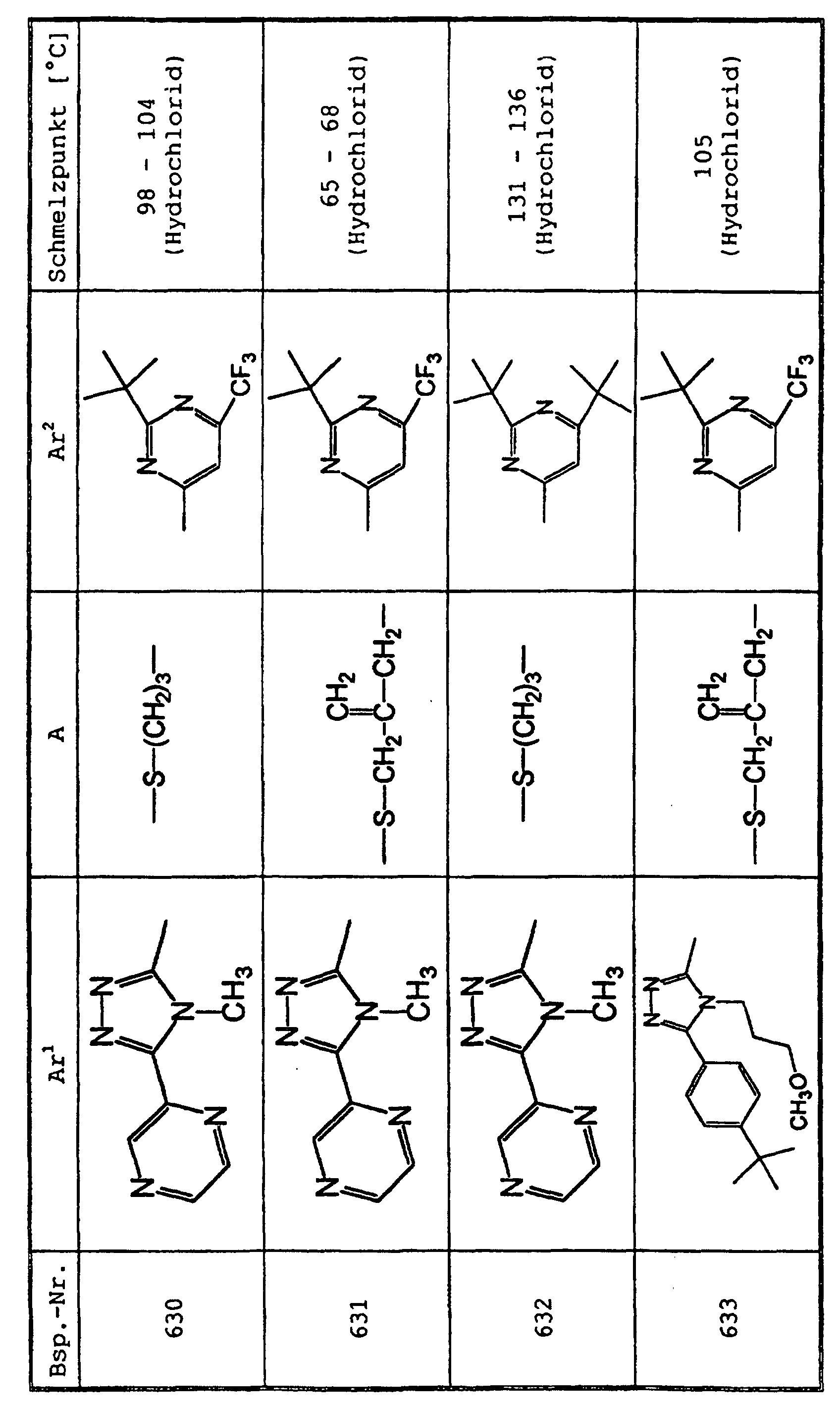

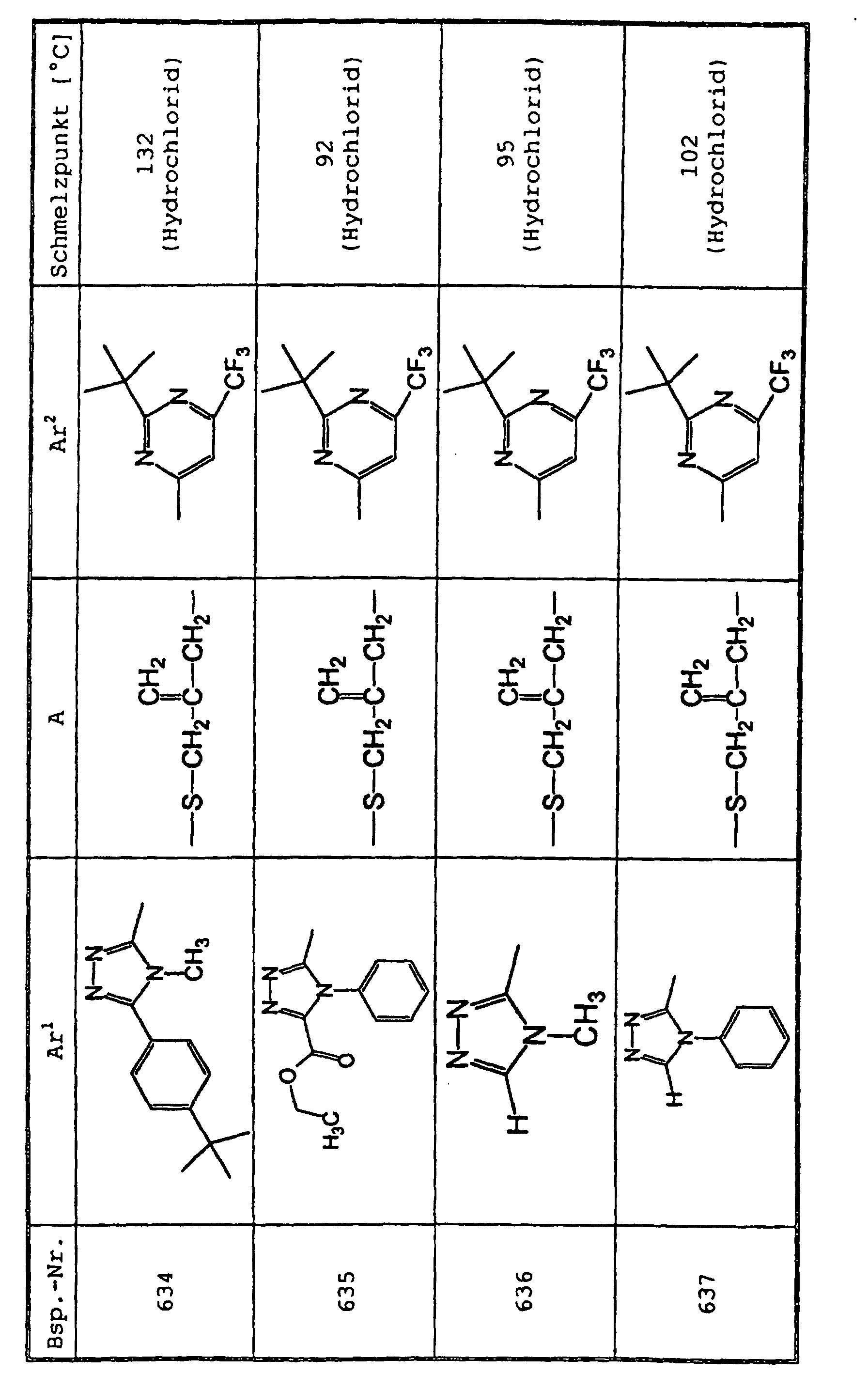

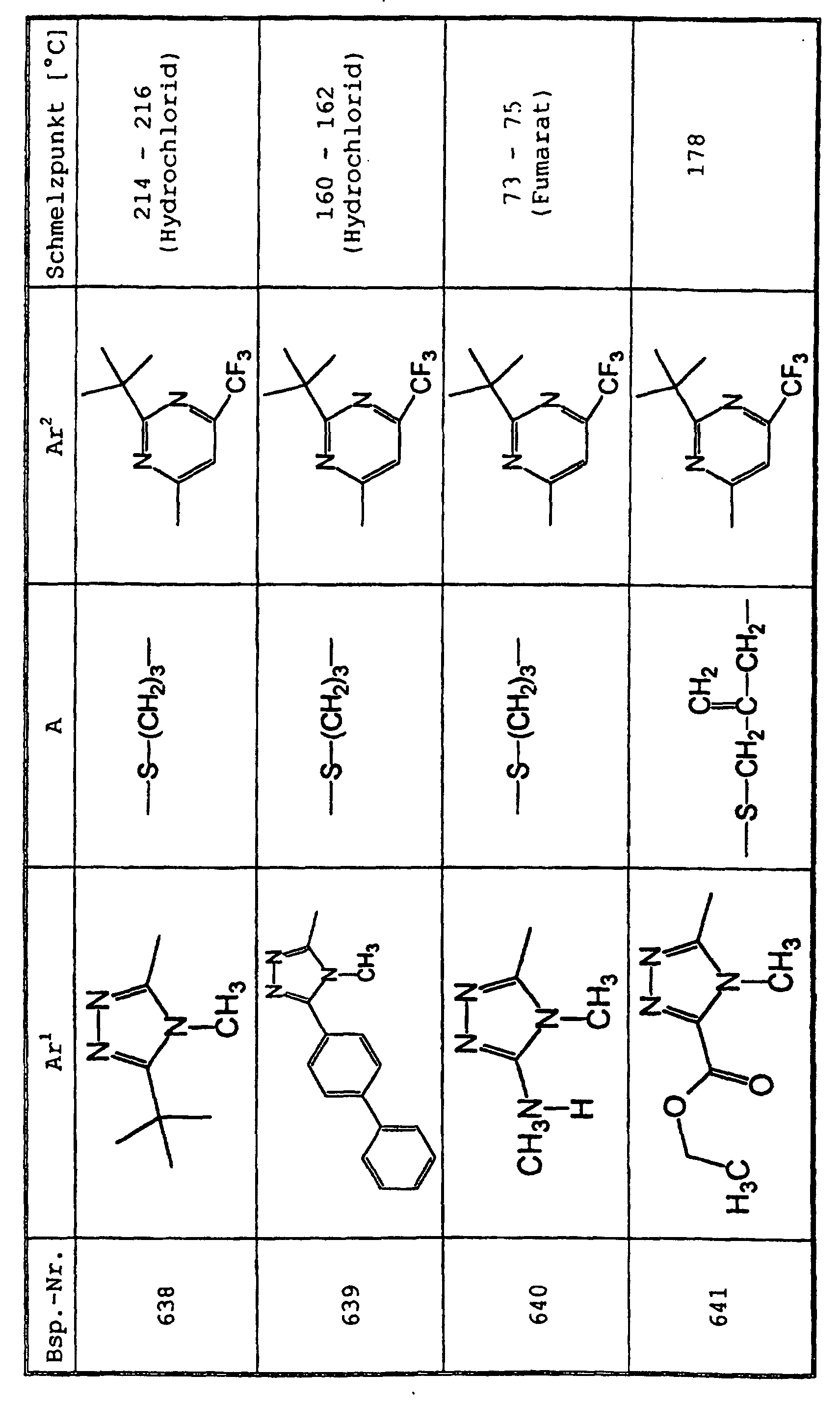

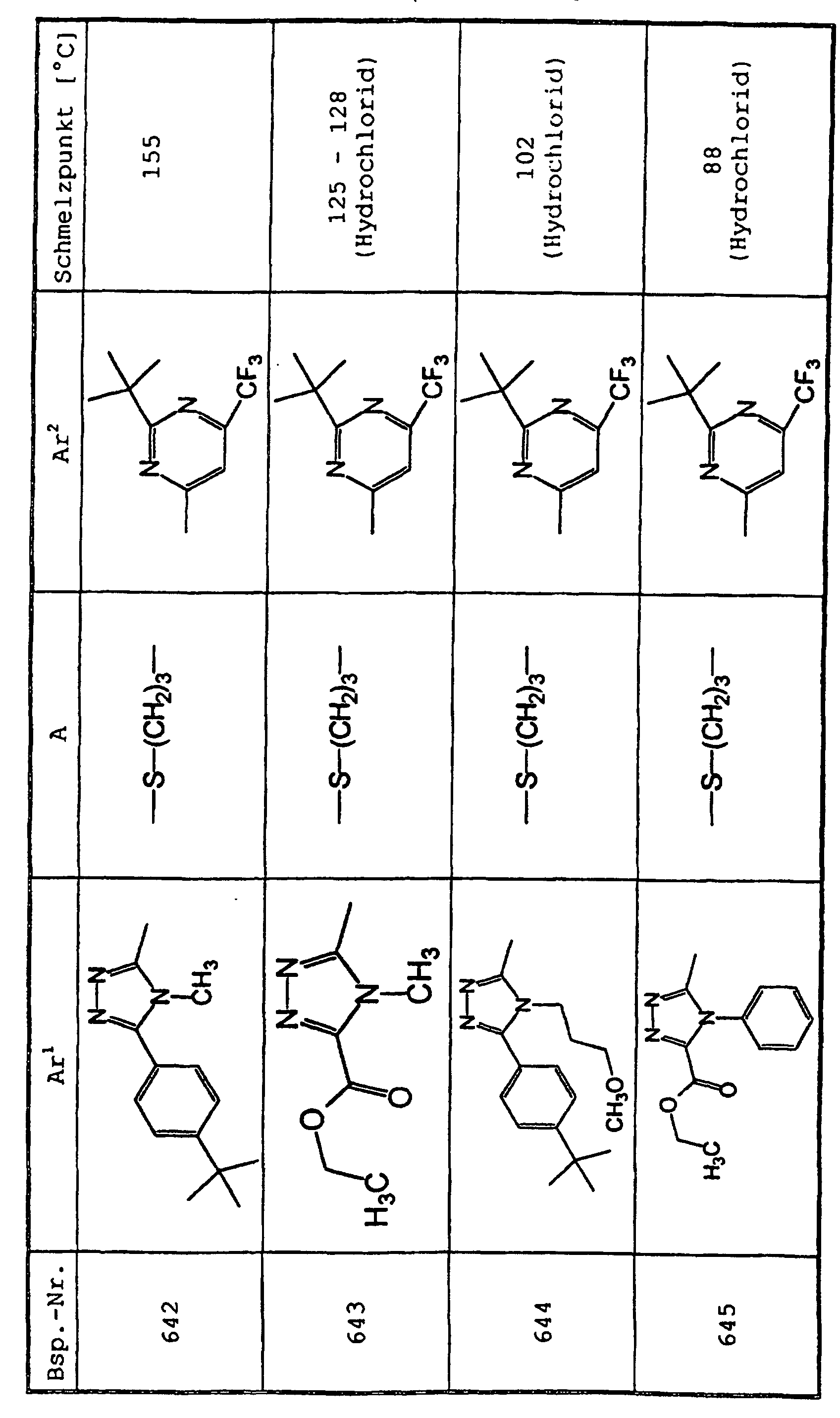

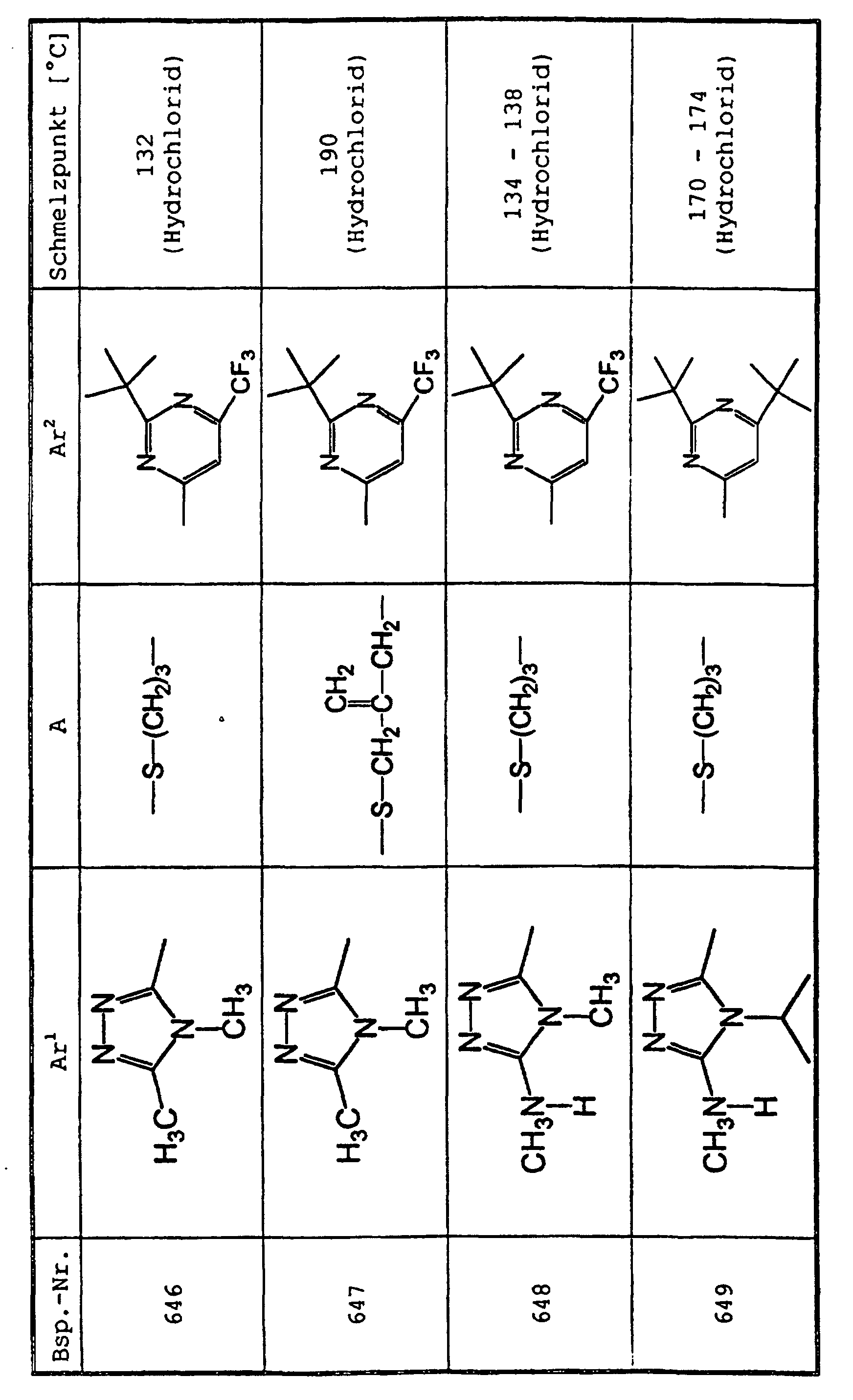

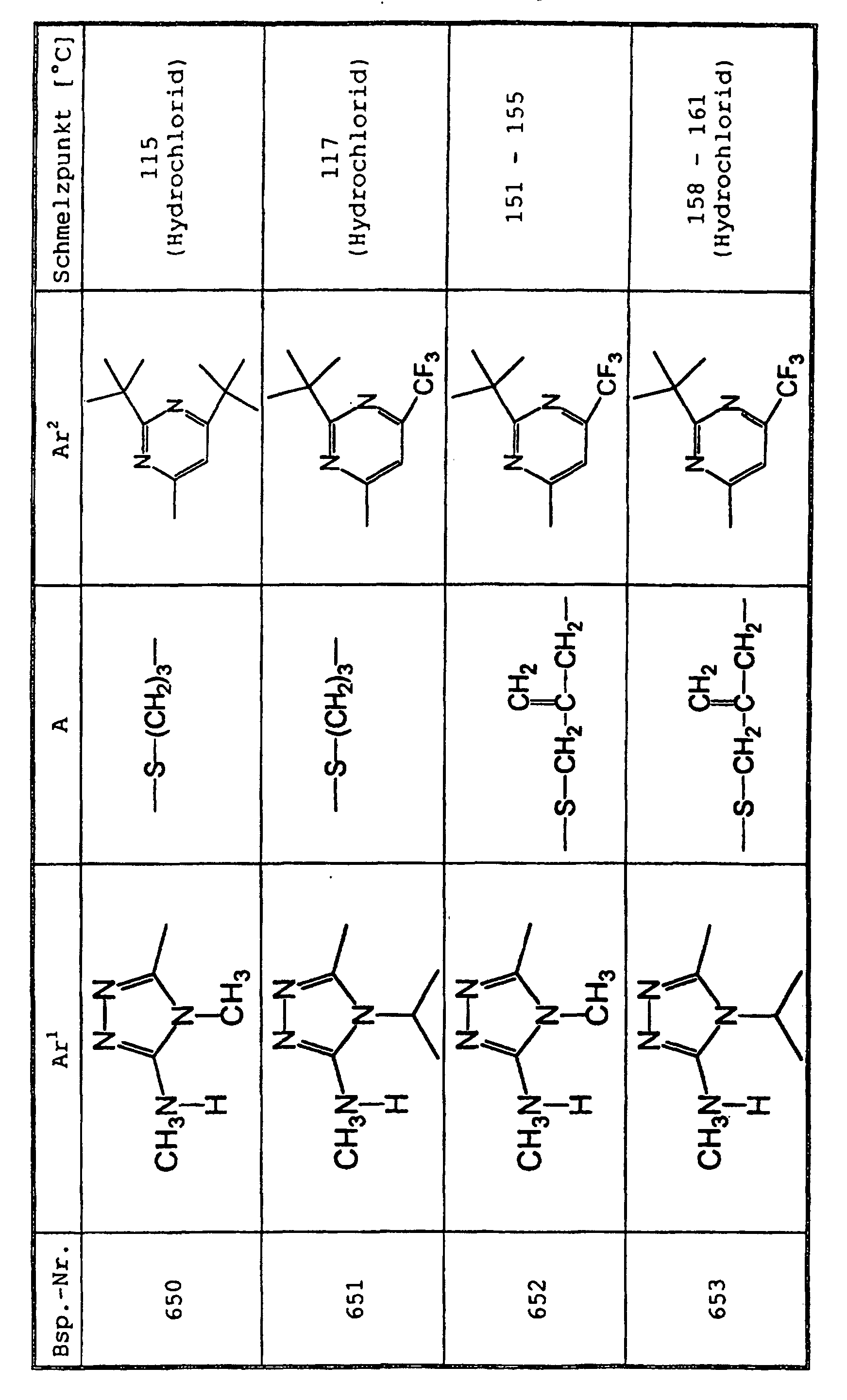

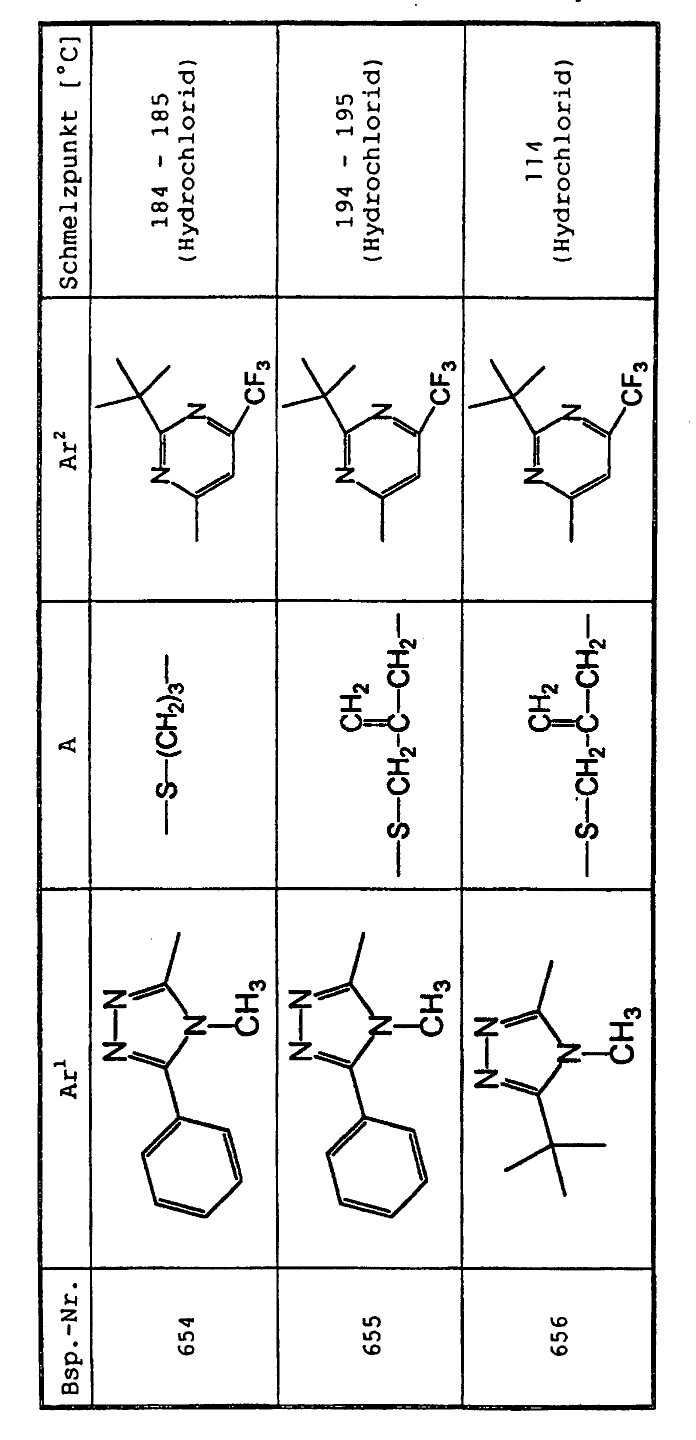

Die obigen Verbindungen, die nicht durch den Schmelzpunkt charakterisiert sind, besitzen die folgenden NMR-Spektren (d₆-DMSO):

| Bsp.-Nr. | | |
|---|---|---|
| 476 | | 1,8-2,1 (m,4H); 2,6-2,7 (m,4H); 2,8 (t,2H); 3,2 (t,2H); 3,5-3,7 (b,2H); 3,7-3,9 (b,2H); 4,5 (d,2H); 5,1 (t,1H); 5,2 (s,1H); 6,1 (d,1H); 6,2 (m,2H); 7,3 (m,5H); 7,7 (m,2H); 7,8 (d,1H) |
| 477 | | 1,8-1,9 (m,4H); 2,5-2,6 (m,4H); 2,7 (t,2H); 3,0 (t,2H); 3,3 (s,3H); 3,5-3,8 (b,4H); 4,2 (s,2H); 4,5 (d,2H); 5,1 (t,1H); 5,2 (s,1H); 6,2 (m,2H); 7,3-7,4 (m,5H); 7,7 (m,2H) |
| 481 | Oxalat | 2,1 (b,4H); 3,2-3,4 (m,8H); 3,6 (s,3H); 3,7 (b,2H); 6,6-6,8 (m,3H); 7,2 (t,1H); 7,6 (m,3H); 7,7 (m,2H) |
| 484 | Oxalat | 2,0 (b,2H); 2,8-3,0 (b,4H); 3,4-3,5 (m,4H); 3,6-3,7 (b,2H); 3,9 (s,2H); 5,3 (d,2H); 6,1 (d,1H); 6,5-6,8 (m,3H); 7,21 (t,1H); 7,9 (d,1H); |
| 491 | Oxalat | 2,0-2,3 (b,4H); 3,0-3,4 (b,8H); 3,6 (s,3H); 3,9 (b,2H); 4,1 (b,2H); 7,1-7,3 (b,1H); 7,5 (m,3H); 7,6 (m,2H); 8,6 (s,1H); |
| 492 | | 1,7-1,9 (m,4H); 2,6 (b,2H); 2,8 (b,2H); 3,1 (t,2H); 3,6-3,9 (b,4H); 6,1 (d,1H); 7,0 (d,1H); 7,8 (d,1H); 8,5 (s,1H); |
| 493 | Oxalat | 1,9-2,1 (b,4H); 2,7 (s,3H); 2,8 (t,2H); 3,0-3,3 (b,6H); 3,3 (s,3H); 3,4 (t,2H); 3,7 (b,2H); 6,3 (b,1H); 6,4-6,5 (m,3H); 7,0 (m,1H); |
| 495 | Fumarat | 1,6-1,8 (b,4H); 2,6 (m,2H); 2,7 (t,2H); 3,2 (s,3H); 3,3-3,5 (m,4H); 5,9 (s,2H); 6,2-6,5 (m,3H); 7,0 (m,1H); |
| 497 | Fumarat | 1,6-1,8 (m,2H); 1,8-2,0 (b,2H); 2,5-2,7 (b,4H); 2,8-2,9 (m,4H); 3,2 (s,3H); 3,7-3,9 (m,4H); 5,9 (b,2H); 6,5 (s,2H); 7,4 (d,1H); |
| 498 | Fumarat | 1,3 (s,9H); 1,8-2,1 (b,4H); 2,7-3,0 (b,6H); 3,3 (s,3H); 3,5-3,8 (b,6H); 6,1 (b,2H); 6,6 (s,2H); 6,7 (s,1H); |
| 499 | Hydrochlorid | 1,3 (s,9H); 1,9 (b2H); 2,2 (s,2H); 2,5 (b,2H); 2,7 (b,2H); 3,1 (s,2H); 3,4 (s,3H); 3,7 (s,2H); 3,8 (s,3H); 3,9 (s,3H); 5,0 (d,2H); 6,5 (s,1H); 6,9 (d,1H); 7,5 (d,1H); 7,7 (s,1H);11,3 (b,1H); |
| 500 | Fumarat | 1,3 (s,9H); 1,7-1,9 (b,4H); 2,5-2,7 (b,4H); 2,8-2,9 (b,2H); 3,1 (t,2H); 3,6-3,7 (b,2H); 3,8-4,0 (b,2H); 6,1 (d,1H); 6,6 (s,2H); 6,9 (d,1H); 7,8 (d,1H) |
| 501 | | 1,3 (s,9H); 1,8-2,0 (m,4H); 2,6 (m,4H); 2,8 (b,2H); 3,1 (t,2H); 3,4 (s,3H); 3,5 (b,2H); 3,9-4,1 (b,2H); 4,4 (b,2H); 6,5 (s,1H) |
| 502 | Fumarat | 1,3 (s,9H); 1,8-1,9 (b,2H); 2,5-2,6 (b,2H); 2,7 (b,2H); 3,1 (s,2H); 3,6-3,7 (b,2H); 3,7-4,0 (m,4H); 5,1 (s,1H); 5,2 (s,1H); 6,1 (d,1H); 6,6 (s,2H); 6,9 (d,1H); 7,8 (d,1H) |
| 503 | Fumarat | 1,3 (s,9H); 1,8-1,9 (b,2H); 2,5-2,6 (b,2H); 2,7-2,8 (b,2H); 3,2 (s,2H); 3,6 (s,3H); 3,6-3,7 (b,2H); 3,7 (s,2H); 3,8-4,0 (b,2H); 5,0 (s,1H); 5,1 (s,1H); 6,6 (s,2H); 6,9 (d,1H); 7,6 (m,3H); 7,7 (m,2H) |
| 504 | Fumarat | 1,3 (s,9H); 1,8-1,9 (b,2H); 2,5 (b,2H); 2,6-2,7 (b,2H); 3,1 (s,2H); 3,3 (s,3H); 3,4 (s,2H); 3,5-3,7 (b,2H); 3,8-4,0 (b,2H); 4,9 (d,2H); 6,0 (s,2H); 6,6 (s,2H); 6,9 (d,1H) |
| 505 | Fumarat | 1,3 (s,9H); 1,7-1,9 (b,4H); 2,5-2,7 (b,4H); 2,8 (t,2H); 3,1 (t,2H); 3,4-4,0 (b,4H); 6,1 (d,1H); 6,5 (d,1H); 6,6 (s,2H); 7,8 (d,1H); 8,2 (d,1H) |
| 506 | | 1,4 (s,9H); 1,8 (m,2H); 2,1-2,2 (b,2H); 2,6 (m,4H); 2,7 (t,2H); 3,0 (t,2H); 3,4 (s,3H); 3,5-4,0 (b,4H); 4,6 (b,2H); 6,2 (d,1H); 8,2 (d,1H) |
| 507 | | 0,9 (t,3H); 1,3 (s,9H); 1,4 (m,2H); 1,7 (m,2H); 2,1 (b,2H); 2,6 (t,2H); 2,7 (t,2H); 2,8 (t,2H); 3,3 (s,2H); 3,6-3,7 (b,2H); 3,8 (s,2H); 3,9-4,0 (b,2H); 5,1 (s,1H); 5,2 (s,1H); 6,1 (s,1H); 6,2 (d,1H); 7,8 (d,1H) |
| 508 | | 0,9 (t,3H); 1,3 (s,9H); 1,4 (m,2H); 1,7 (m,2H); 1,9 (m,2H); 2,6 (m,4H); 2,7 (t,2H); 3,1 (d,3H); 3,2 (s,2H); 3,3 (s,3H); 3,6 (m,1H); 3,7 (s,2H); 3,6-3,8 (b,4H); 5,0 (d,2H); 6,0 (s,1H) |
| 509 | Dihydrochlorid | 0,9 (t,3H); 1,3 (m,2H); 1,4 (s,9H); 1,7 (m,2H); 2,4-2,5 (b,2H); 2,9 (t,2H); 3,2-3,4 (b,4H); 3,5 (s,3H); 3,7-3,8 (b,2H); 3,9 (s,2H); 3,9-4,2 (b,2H); 4,1 (s,2H); 5,4 (s,2H); 6,9 (s,1H);8,5 (s,2H); 11,7 (b,1H); 14,0 (b,1H) |
| 510 | | 1,3 (s,9H); 2,0-2,1 (b,2H); 2,7 (t,2H); 2,8 (t,2H); 3,3 (s,2H); 3,5-3,8 (b,2H); 3,8 (s,2H); 3,8-4,1 (b,2H); 5,1 (s,1H); 5,2 (s,1H); 6,2 (m,2H); 7,8 (d,1H); 8,2 (d,1H) |
| 511 | | 1,3 (s,18H); 1,6-1,9 (b,4H); 2,4 (b,2H); 2,7 (b,2H); 2,8 (t,2H); 3,2 (s,3H); 3,7 (m,4H); 5,8 (s,1H); |
| 512 | | 1,3 (s,18H); 1,7-1,8 (m,4H); 2,5 (b,2H); 2,7 (b,2H); 3,0 (t,2H); 3,7-3,8 (m,4H); 6,0 (d,1H); 7,8 (d,1H); |
| 513 | | 1,6-2,1 (m,17H); 2,7 (s,2H); 3,1 (s,2H): 3,3-3,6 (b,6H); 3,4 (s,3H); 3,8-3,9 (b,2H); 4,8 (b,2H); 6,0 (b,2H); 6,8 (s,1H); |
| 514 | | 1,6-2,0 (m,17H); 2,5 (s,2H); 2,6 (s,2H); 3,0 (s,2H); 3,5-3,9 (b,6H); 5,0 (d,2H); 6,0 (d,1H); 6,8 (d,1H); 7,8 (d,1H); |
| 515 | | 1,7-2,0 (m,19H); 2,5 (b,2H); 2,7 (s,2H); 3,0 (t,2H); 3,5-3,8 (b,4H); 6,0 (d,1H); 6,8 (d,1H); 7,8 (d,1H); |
| 517 | Dihydrochlorid | 1,4 (s,9H); 2,4-2,5 (b,2H); 3,3-3,6 (b,4H); 3,5 (s,3H); 3,7-3,8 (b,2H); 3,8-3,9 (b,2H); 3,9-4,2 (b,4H); 5,4 (s,2H); 7,1 (d,1H); 8,3 (d,1H); 8,5 (s,2H); 11,7 (b,1H); 14,5 (b,1H) |
| 518 | | 1,3 (s,9H); 1,8-2,0 (b,2H); 2,6 (t,2H); 2,7 (t,2H); 3,0 (d,3H); 3,2 (s,2H); 3,3 (s,3H); 3,6 (s,2H); 3,6-3,9 (b,5H); 5,0 (d,2H); 6,2 (s,1H); 6,3 (m,2H); 7,8 (m,2H) |
| 519 | | 1,4 (s,9H); 1,9 (m,2H); 2,6 (t,2H); 2,7 (t,2H); 3,0 (d,3H); 3,2 (s,2H); 3,3 (s,3H); 3,7 (s,2H); 3,6-3,9 (b,5H); 5,0 (d,2H); 6,1 (s,1H); 6,3 (m,2H); 7,6 (m,2H) |
| 527 | Hydrochlorid | 1,2-1,5 (b,10H); 1,5-1,8 (b,4H); 3,1 (m,4H); 3,5 (m,4H); 3,8-4,0 (b,4H); 6,8 (t,1H); 6,9 (b,3H); 7,1 (t,1H); 10,5 (b,1H); |
| 526 | Oxalat | 1,3 (s,9H); 1,9-2,1 (b,2H); 2,7-3,0 (b,4H); 3,4 (b,2H); 3,6 (s,3H); 3,6-3,8 (b,2H); 3,8 (s,2H); 3,9-4,1 (b,2H); 5,2 (d,2H); 6,2 (m,2H); 6,4 (s,1H); 7,6 (m,3H); 7,7 (m,4H) |
| 528 | Oxalat | 0,9 (t,3H); 1,3 (s,9H); 1,4 (m,2H); 1,6 (m,2H); 1,9-2,2 (b,4H); 2,5 (m,2H); 3,0-3,2 (b,4H); 3,2-3,4 (b,4H); 3,5-3,7 (b,2H); 3,9-4,1 (b,2H); 6,1 (d,1H); 6,4 (s,1H); 7,8 (d,1H) |
| 529 | Dihydrochlorid | 0,9 (t,3H); 1,3 (m,2H); 1,4 (s,9H); 1,7 (m, 2H); 2,2 (b,3H); 3,0 (t,2H); 3,2 (b,5H); 3,5 (s,3H); 3,5-4,2 (b,7H); 4,5-4,7 (b,1H); 7,0 (d,1H); 8,6 (s,2H); 11,7 (b,1H); 14,2 (b,1H) |
| 530 | Oxalat | 1,3 (s,9H); 1,9-2,1 (b,2H); 2,7-3,0 (b,4H); 2,3-2,4 (b,2H); 2,6-4,1 (b,4H); 3,9 (s,2H); 5,2 (s,1H); 5,3 (s,1H); 6,1 (d,1H); 6,2 (m,2H); 6,4 (s,1H); 7,7 (m,2H); 7,8 (d,1H) |
| 531 | | 1,3 (s,9H); 1,9 (m,2H); 2,6 (t,2H); 2,7 (t,2H); 3,2 (s,2H); 3,3 (s,3H); 3,7 (s,2H); 3,6-3,9 (b,4H); 4,3 (s,2H); 5,0 (d,2H); 6,2 (s,1H); 6,3 (m,2H); 7,8 (m,2H) |
| 533 | | 1,3 (s,9H); 2,1 (m,2H); 2,7 (m,2H); 2,9 (m,2H); 3,3 (s,2H); 3,6-3,8 (b,2H); 3,8 (s,2H); 3,9-4,1 (b,2H); 5,1 (s,1H); 5,2 (s,1H); 6,1 (s,1H); 6,2 (d,1H); 6,3 (m,2H); 7,5 (m,2H); 7,8 (d,1H) |
| 532 | | 1,3 (s,9H); 1,9 (m,2H); 2,6 (t,2H); 2,7 (t,2H); 3,2 (s,2H); 3,3 (s,3H); 3, 7 (s,2H); 3,6-3,6 (b,4H); 4,3 (s,2H); 5,0 (s,2H); 6,1 (s,1H); 6,3 (m,2H); 7,6 (m,2H) |
| 539 | Hydrochlorid | 2,2-2,3 (b,2H); 3,0-3,2 (b,2H); 3,5-4,0 (b,8H), 4,2 (s,2H); 5,5 (d,2H); 6,2 (d,1H); 6,9-7,0 (m,3H); 7,0 (t,1H); 7,4 (m,1H); 7,9 (d,1H); 10,9 (b,1H); |
| 540 | Hydrochlorid | 2,4 (b, 2H); 3,2 (b,4H); 3,4 (s,3H); 3,5 (m,2H); 3,7 (b,4H); 4,0 (s,2H); 5,3 (d,2H); 6,8-7,1 (m,4H); 7,2-7,4 (m,3H); |
| 542 | | 1,3 (s,9H); 1,9 (m, 2H) ; 1,9-2,1 (b,2H); 2,6 (m,4H); 2,8 (b,2H); 3,2 (t,2H); 3,5-3,7 (b,2H); 3,9-4,2 (b,2H); 6,2 (d,1H); 6,5 (s,1H); 6,8 (d,1H) |
| 543 | | 1,3 (s,9H); 1,9 (m,2H); 1,9-2,0 (b,2H); 2,6-2,7 (b,4H); 2,8 (b,2H); 3,1 (t,2H); 3,2 (s,3H); 3,5-3,6 (b,2H); 3,9-4,1 (b,2H); 6,5 (s,1H); 10,8 (b,1H) |
| 544 | | 1,3 (s,9H); 1,8-2,0 (b,4H); 2,6 (m,4H); 2,7-2,8 (b,2H); 3,0 (m,5H); 3,3 (s,3H); 3,5-3,6 (b,2H); 3,9-4,1 (b,3H); 6,5 (s,1H) |
| 545 | Hydrochlorid | 1,3 (s,9H); 2,1-2,2 (b,3H); 2,5-2,6 (b,1H); 3,1-3,3 (b,6H); 3,4 (s,3H); 3,4-3,8 (b,4H); 4,0-4,1 (b,1H); 4,6-4,7 (b,1H); 7,0 (s,1H); 8,6 (s,2H); 11,3 (b,1H) |
| 546 | | 1,3 (s,9H); 1,9 (m,2H); 2,1 (m,2H); 2,7 (m,4H); 2,9 (m,2H); 3,2 (t,2H); 3,8-3,9 (b,2H); 3,9-4,0 (b,2H); 6,2 (d,2H); 6,3 (m,2H); 7,8 (m,3H) |
| 547 | | 1,3 (s,9H); 1,9 (m,4H); 2,6 (m,4H); 2,8 (t,2H); 3,1 (t,2H); 3,2 (s,3H); 3,6-4,0 (b,4H); 6,2 (s,1H); 6,3 (m,2H); 7,8 (m,2H); 9,2 (s,1H) |
| 548 | | 1,3 (s,9H); 1,9 (m,4H); 2,6 (t,4H); 2,8 (t,2H); 3,1 (t,2H); 3,3 (s,3H); 3,5-3,9 (b,4H); 4,1 (s,2H); 6,2 (s,1H); 6,3 (m,2H); 7,8 (m,2H) |
| 549 | | 1,4 (s,9H); 1,9 (m,2H); 2,0-2,1 (b,2H); 2,7 (m,4H); 2,9 (m,2H); 3,2 (t,2H); 3,6-3,8 (b,2H); 3,8-4,1 (b,2H); 6,1 (s,1H); 6,2 (d,1H); 6,3 (m,2H); 7,6 (m,2H), 7,8 (d,1H) |
| 550 | | 1,4 (s,9H); 1,9 (m,4H); 2,6 (m,4H); 2,8 (b,2H); 3,1 (t,2H); 3,2 (s,3H); 3,6-4,0 (b,4H); 6,1 (s,1H); 6,3 (m,2H); 7,5 (m,2H); 10,0 (b,1H) |
| 551 | | 1,4 (s,9H); 1,9 (m,4H); 2,6 (m,4H); 2,8 (b,2H); 3,1 (t,2H); 3,4 (s,3H); 3,5-4,0 (b,4H); 4,3 (s,2H); 6,1 (s,1H); 6,3 (m,2H); 7,5 (m,2H) |
| 552 | | 1,3 (s,9H); 2,1 (m,2H); 2,8 (m,2H); 2,9 (m,2H); 3,3 (s,2H); 3,8 (s,2H); 3,9 (t,2H); 4,1 (b,2H); 5,1 (s,1H); 5,2 (s,1H); 6,2 (d,1H); 6,5 (d,1H); 7,8 (d,1H); 8,2 (d,1H) |
| 553 | Hydrochlorid | 1,3 (s,9H); 2,2-2,3 (b,1H); 2,5-2,7 (b,1H); 3,1 (s,3H); 3,0-3,2 (b,2H); 3,5-3,7 (b,3H); 3,8-4,1 (m,6H); 4,4-4,5 (b,1H); 5,4 (d,2H); 6,8 (d,1H); 8,3 (d,1H); 11,2 (b,1H); 11,9 (s,1H) |
| 554 | | 1,3 (s,9H); 1,9 (m,2H); 2,6 (t,2H); 2,7 (t,2H); 3,2 (s,2H); 3,4 (s,3H); 3,7 (s,2H); 3,8 (m,4H); 4,4 (s,2H); 5,0 (s,2H); 6,5 (d,1H); 8,2 (d,1H) |
| 555 | | 1,3 (s,18H); 2,1 (m,2H); 2,8 (t,2H); 3,0 (t,2H); 3,3 (s,2H); 3,8 (s,2H); 3,9 (t,2H); 4,1 (t,2H); 5,1 (s,1H); 5,2 (s,1H); 6,2 (d,1H); 6,5 (s,1H); 7,8 (d,1H) |
| 556 | | 1,3 (s,18H); 1,9 (m,2H); 2,6 (t,2H); 2,7 (t,2H); 3,2 (s,2H); 3,4 (s,3H); 3,6 (s,2H); 3,9 (m,4H); 4,2 (s,2H); 5,0 (s,2H); 6,5 (s,1H) |
| 557 | Hydrochlorid | 1,3 (d,6H); 2,2 (b,1H); 2,6 (b,1H); 3,0 (b,4H); 3,4 (s,3H); 3,5-3,7 (b,4H); 3,8 (s,2H); 4,1 (s,2H); 5,4 (s,2H); 6,8 (t,1H); 6,9 (s,1H); 8,5 (b,2H); 11,6 (b,1H); |
| 559 | Hydrochlorid | 1,2 (d,6H); 2,1 (b,4H); 3,0-3,2 (b,8H); 3,5-4,2 (b,4H); 6,1 (d,1H); 6,8 (t,1H); 6,9 (b,1H); 7,8 (d,1H); 11,3 (b,1H); |
| 561 | | 1,3 (s,18H); 1,9 (m,2H); 2,0 (m,2H); 2,7 (t,2H); 2,8 (t,2H); 3,0 (t,2H); 3,2 (t,2H); 3,9 (t,2H); 4,0 (t,2H); 6,1 (d,1H); 6,5 (s,1H); 7,8 (d,1H) |
| 562 | | 1,3 (s,18H); 1,9 (m,4H); 2,6 (m,4H); 2,8 (t,2H); 3,0 (t,2H); 3,4 (s,3H); 3,8 (t,2H); 3,9 (t,2H); 4,3 (s,2H); 6,5 (s,1H) |
| 564 | Oxalat | 1,3 (s,9H); 2,0 (b,2H); 2,2 (b,2H); 3,1-3,4 (b,8H); 3,3 (b,2H); 3,8 (s,3H); 4,0 (b,2H); 6,1 (d,2H); 6,9 (s,1H); 7,0 (d,1H); 7,8 (d,1H); 8,1 (d,1H); |
| 563 | Oxalat | 1,3 (s,9H); 1,9 (b,2H); 2,2 (b,2H); 2,9 (t,2H); 3,1 (t,2H); 3,3 (b,4H); 3,4 (s,3H); 3,7 (b,2H); 3,8 (s,3H); 4,0 (b,2H); 5,3 (b,2H); 6,8 (s,1H); 6,9 (d,1H); 8,0 (d,1H); |
| 566 | | 1,4 (s,9H); 1,9 (b,2H); 2,5 (b,2H); 2,8 (b,2H); 3,2 (s,2H); 3,3 (s,3H); 3,6 (s,2H); 3,6-3,8 (b,4H); 3,8 (s,3H); 4,8 (b,2H); 5,0 (s,2H); 6,5 (s,1H); 7,0 (d,1H); 8,0 (d,1H); |
| 567 | Oxalat | 2,0 (b,2H); 2,8 (b,2H); 3,0 (b,2H); 3,3 (s,3H); 3,4 (s,2H); 3,6 (b,4H); 3,8 (b,2H); 5,1 (s,2H); 5,5 (b,2H); 7,0 (m,2H); 7,7 (t,1H); |
| 568 | Oxalat | 2,0 (b,2H); 2,2 (b,2H); 3,0 (t,2H); 3,1 (m,2H); 3,2 (b,4H); 3,4 (s,3H); 3,6 (m,2H); 3,9 (b,2H); 6,7 (b,2H); 7,0 (t,2H); 7,7 (t,1H); |
| 569 | Oxalat | 2,0-2,2 (b,4H); 3,0-3,4 (m,8H); 3,5 (m,2H); 3,9 (b,2H); 6,1 (d,1H); 6,5 (b,2H); 7,0 (t,2H); 7,7 (t,1H); 7,8 (d,1H); |
| 570 | Hydrochlorid | 1,3 (s,9H); 2,0-2,2 (b,3H); 2,3-2,4 (b,1H); 3,2 (m, 6H); 3,4-4,0 (b,5H); 4,3-4,5 (b,1H); 6,2 (d,1H); 6,8 (d,1H); 7,9 (d,1H); 8,3 (d,1H); 10,8 (b,1H) |
| 571 | Hydrochlorid | 1,3 (s,9H); 2,0-2,2 (b,3H); 2,3-2,4 (b,1H); 3,1 (s,3H); 3,0-3,2 (m,6H); 3,4-4,0 (b,5H); 4,3-4,5 (b,1H); 6,8 (d,1H); 8,3 (d,1H); 10,9 (b,1H); 11,9 (s,1H) |
| 572 | | 1,3 (s,9H); 1,9-2,0 (m,4H); 2,6 (m,4H); 2,8 (t,2H); 3,0 (t,2H); 3,4 (s,3H); 3,8 (t,2H); 3,9 (b,2H); 4,6 (b,2H); 6,5 (d,1H); 8,2 (d,1H) |

## Patentansprüche

1. Verbindungen der allgemeinen Formel I
Ar¹-A-B-Ar² (I)
worin
Ar¹ für 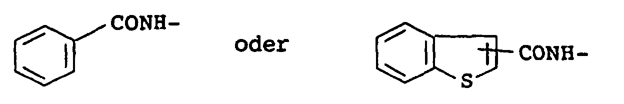 oder einen 5- oder 6-gliedrigen aromatischen Heteromonocyclus mit 1, 2 oder 3 Heteroatomen, die unabhängig voneinander ausgewählt sind unter O, N und S steht, wobei Ar¹ gegebenenfalls 1, 2, 3 oder 4 Substituenten aufweist, die unabhängig voneinander ausgewählt sind unter OR¹, C₁-C₈-Alkyl, das gegebenenfalls durch OH, OC₁-C₈-Alkyl oder Halogen substituiert ist, C₂-C₆-Alkenyl, C₂-C₆-Alkinyl, C₃-C₆-Cycloalkyl, Halogen, CN, CO₂R¹, NO₂, NR¹R², SR¹, CF₃, CHF₂, Phenyl, das gegebenenfalls durch C₁-C₆-Alkyl, OC₁-C₆-Alkyl, HCO, C₁-C₆-Alkyl-CO, Phenyl, Amino, Nitro, Cyano oder Halogen substituiert ist, Phenoxy, das gegebenenfalls durch C₁-C₆-Alkyl, OC₁-C₆-Alkyl oder Halogen substituiert ist, C₁-C₆-Alkanoyl oder Benzoyl;
R¹ für H, C₁-C₈-Alkyl, das gegebenenfalls durch OH, OC₁-C₆-Alkyl, Phenyl oder Halogen substituiert ist, steht;
R² die für R¹ angegebenen Bedeutungen besitzt oder für COR¹ oder CO₂R¹ steht;
A für eine C₃-C₁₅-Alkylengruppe steht, wenn Ar¹ für C₆H₅CONH steht, oder, wenn Ar¹ einen 5- oder 6-gliedrigen aromatischen Heteromonocyclus bedeutet, für eine C₄-C₁₅-Alkylengruppe oder eine C₃-C₁₅-Alkylengruppe steht, die wenigstens eine Gruppe Z umfaßt, die ausgewählt ist unter O, S, NR¹, einer Doppel- und einer Dreifachbindung, wobei R¹ wie oben definiert ist,
B für einen 7- oder 8-gliedrigen gesättigten Ring mit einem oder zwei Stickstoffheteroatomen steht, wobei sich die Stickstoffheteroatome in 1,4- oder 1,5-Position befinden und der Ring in 1-Position an den Rest A und in 4- oder 5-Position an den Rest Ar² gebunden ist und wobei der Ring außerdem eine Doppelbindung in 3- oder 4-Position aufweisen kann;
Ar² für Phenyl, Pyridyl, Pyrimidinyl oder Triazinyl steht, wobei Ar² gegebenenfalls 1, 2, 3 oder 4 Substituenten aufweisen kann, die unabhängig voneinander ausgewählt sind unter OR¹, C₁-C₈-Alkyl, C₂-C₆-Alkenyl, C₂-C₆-Alkinyl, C₁-C₈-Alkoxy-C₁-C₈-alkyl, C₁-C₈-Halogenalkyl, Halogen, CN, CO₂R¹, NO₂, SO₂R¹, NR¹R², SO₂NR¹R², SR¹, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter O, S und N, wobei der carbocyclische oder der heterocyclische Ring gegebenenfalls substituiert ist durch C₁-C₈-Alkyl, Phenyl, Halogen, OC₁-C₈-Alkyl, OH, NO₂ oder CF₃, wobei Ar² gegebenenfalls mit einem carbocyclischen Ring der oben definierten Art kondensiert sein kann und wobei Ar² nicht für einen Pyrimidinylrest stehen kann, der mit 2 Hydroxygruppen substituiert ist,
und die Salze davon mit physiologisch verträglichen Säuren.

2. Verbindungen nach Anspruch 1 der Formel I, worin Ar¹ für 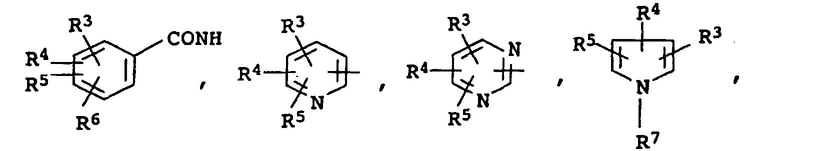 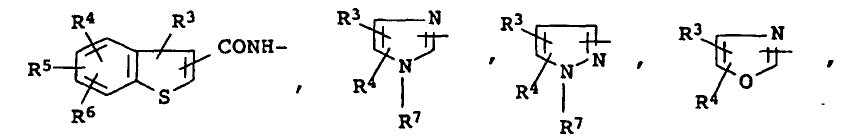 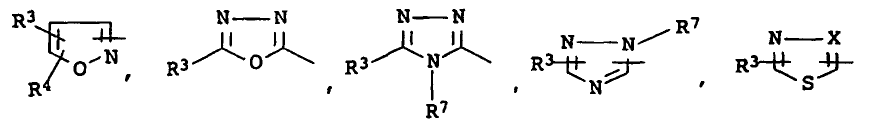 steht, worin
R³ bis R⁶ unabhängig voneinander für H oder die in Anspruch 1 genannten Substituenten des Restes Ar¹ stehen,
R⁷ die in Anspruch 1 für R² angegebenen Bedeutungen besitzt oder für C₃-C₆-Cycloalkyl steht und
X für N oder CH steht.

3. Verbindungen nach Anspruch 1 der Formel I, worin Ar¹ für 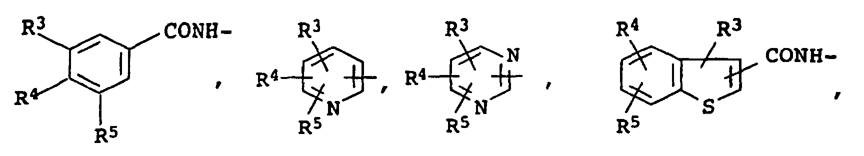 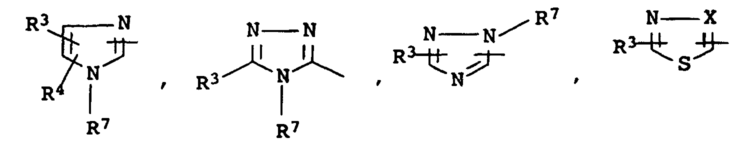 steht, worin R³ bis R⁵, R⁷ und X die in Anspruch 2 angegebenen Bedeutungen besitzen.

4. Verbindungen nach Anspruch 3 der Formel I, worin
Ar¹ für 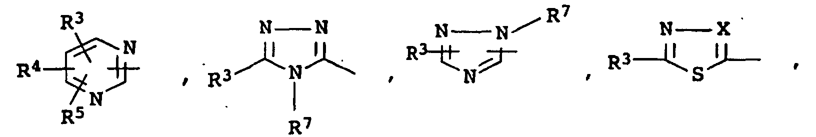 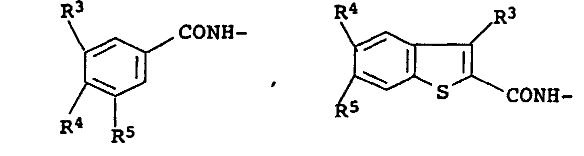 steht,
worin R³ bis R⁵, R⁷ und X die in Anspruch 2 angegebenen Bedeutungen besitzen.

5. Verbindungen nach Anspruch 4 der Formel I, worin R³, R⁴ und R⁵ unabhängig voneinander für H, OR¹, C₁-C₈-Alkyl, NR¹R², Halogen, Phenoxy, CN, Phenyl, das gegebenenfalls durch C₁-C₆-Alkyl, HCO, C₁-C₆-Alkyl-CO oder Halogen substituiert ist, oder COOR¹ steht;
R¹ und R² unabhängig voneinander für H, C₁-C₈-Alkyl oder Benzyl stehen;
R⁷ für H, C₁-C₈-Alkyl oder C₃-C₆-Cycloalkyl steht; und
X für N oder CH steht.

6. Verbindungen nach Anspruch 5, wobei R³ bis R⁵ unabhängig voneinander ausgewählt sind unter H, C₁-C₆-Alkyl, OR¹, NR¹R², Phenyl, das gegebenenfalls durch C₁-C₆-Alkyl, HCO, C₁-C₆-Alkyl-CO oder Halogen substituiert ist, und Halogen, wobei R¹ und R² die oben angegebenen Bedeutungen besitzen, R⁷ für H oder C₁-C₈-Alkyl steht und X für N steht.

7. Verbindungen nach Anspruch 6, wobei Ar¹ für Pyrimidinyl steht, das gegebenenfalls durch OH, OC₁-C₈-Alkyl oder OBenzyl substituiert ist.

8. Verbindungen nach Anspruch 6, wobei Ar¹ für 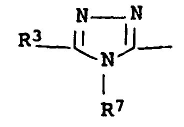 steht, worin R³ für NR¹R² steht, wobei R¹ und R² die in Anspruch 5 angegebenen Bedeutungen besitzen und R⁷ für H oder C₁-C₈-Alkyl steht.

9. Verbindungen nach Anspruch 6, wobei Ar¹ für Thiadiazol steht, das gegebenenfalls durch NR¹R² substituiert ist, wobei R¹ und R² die in Anspruch 5 angegebenen Bedeutungen besitzen.

10. Verbindungen nach Anspruch 6, wobei Ar¹ für 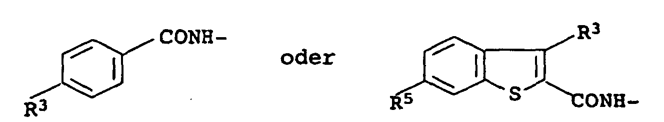 steht, worin R³ und R⁵ unabhängig voneinander für H oder Halogen, C₁-C₈-Alkyl oder Phenyl stehen.

11. Verbindungen nach einem der vorhergehenden Ansprüche der Formel I, worin A für -Z-C₃-C₆-Alkylen, insbesondere -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂C(=CH₂)CH₂-, -Z-CH₂CH(CH₃)CH₂- oder für einen linearen -Z-C₇-C₁₀-Alkylenrest steht, wobei Z an Ar¹ gebunden ist und für CH₂, O oder S steht.

12. Verbindungen nach einem der vorhergehenden Ansprüche der Formel I, worin B für 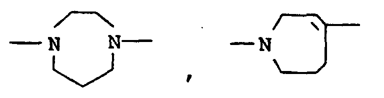 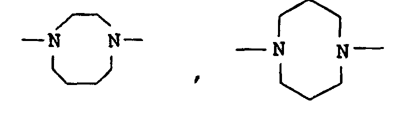 steht.

13. Verbindungen nach einem der vorhergehenden Ansprüche der Formel I, worin Ar² für Phenyl, Pyridinyl oder Pyrimidinyl steht, das gegebenenfalls einen oder zwei Substituenten aufweist, die unabhängig voneinander ausgewählt sind unter C₁-C₆-Alkyl, C₂-C₆-Alkinyl, Halogen, CN, C₁-C₈-Halogenalkyl, OC₁-C₈-Alkyl, NO₂, Phenyl, Pyrrolyl, Imidazolyl, Pyrazolyl, Thienyl, Cyclopentyl und Cyclohexyl.

14. Verbindungen nach Anspruch 13 der Formel I, worin der und die Substituenten unabhängig voneinander ausgewählt sind unter C₁-C₆-Alkyl, NO₂, und C₁-C₈-Halogenalkyl, insbesondere CF₃, CHF₂ und CF₂Cl.

15. Verbindung ausgewählt unter 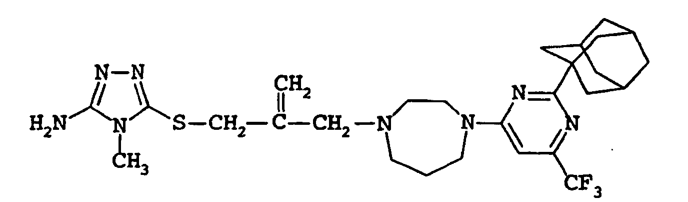 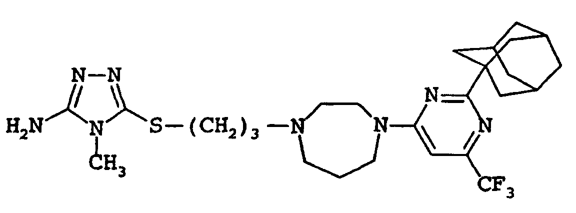 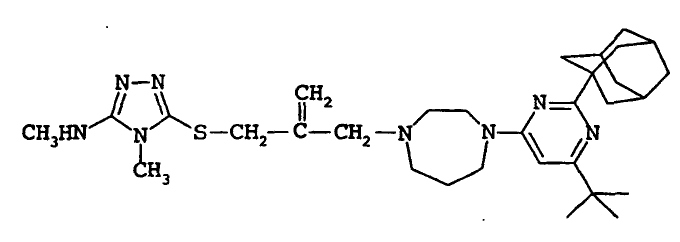 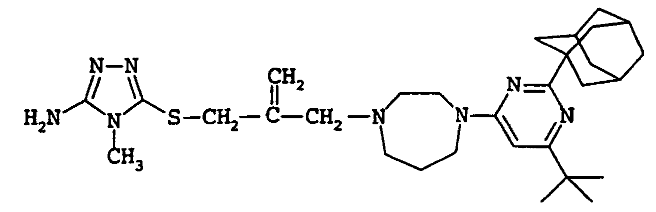 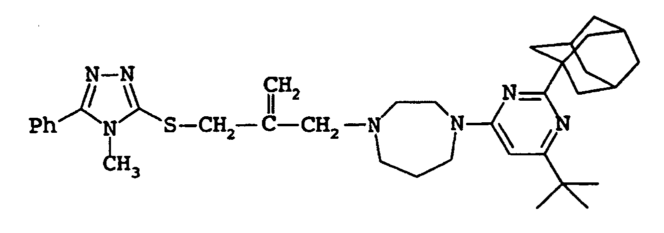 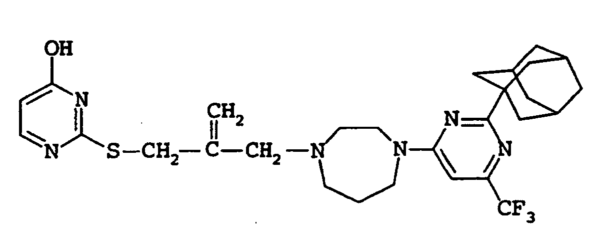 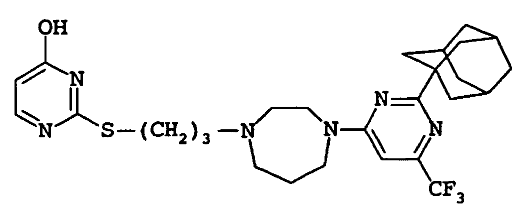 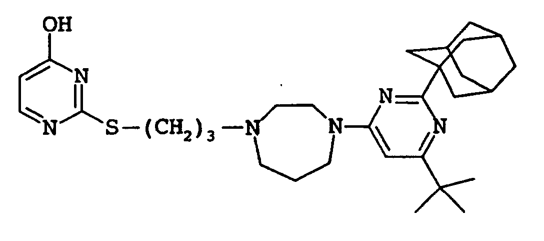 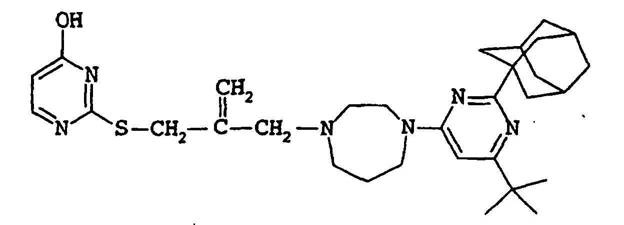 und die Salze davon mit physiologisch verträglichen Säuren.

16. Verbindung ausgewählt unter 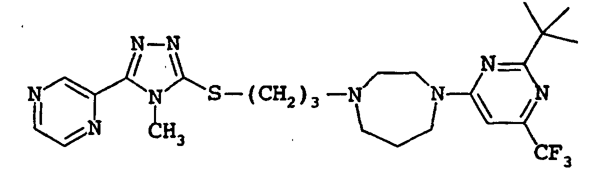 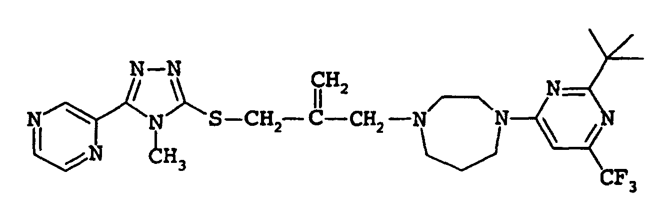 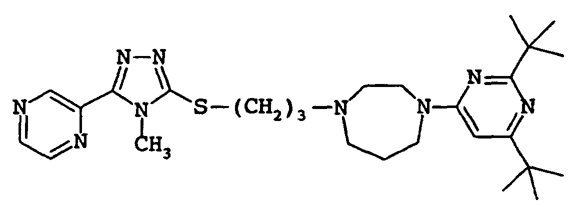 und die Salze davon mit physiologisch verträglichen Säuren..

17. Pharmazeutisches Mittel, enthaltend wenigstens eine Verbindung nach einem der Ansprüche 1 bis 16, gegebenenfalls zusammen mit physiologisch akzeptablen Trägern und/oder Hilfsstoffen.

18. Verwendung wenigstens einer Verbindung nach einem der Ansprüche 1 bis 16 zur Herstellung eines pharmazeutischen Mittels zur Behandlung von Erkrankungen, die auf Dopamin-D₃-Rezeptorantagonisten bzw. -agonisten ansprechen.

## Claims

1. A compound of the formula I
Ar¹-A-B-Ar² (I)
where
Ar¹ is 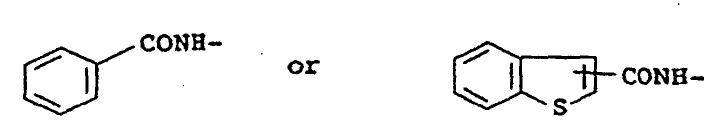 or a 5- or 6-membered heteroaromatic ring with 1, 2 or 3 heteroatoms which are selected, independently of one another, from O, N and S, where Ar¹ may have 1, 2, 3 or 4 substituents which are selected, independently of one another, from OR¹, C₁-C₈-alkyl which is unsubstituted or substituted by OH, OC₁-C₈-alkyl or halogen,
or C₂-C₆-alkenyl, C₂-C₆-alkynyl, C₃-C₆-cycloalkyl, halogen, CN, CO₂R¹, NO₂, NR¹R², SR¹, CF₃, CHF₂, phenyl which is unsubstituted or substituted by C₁-C₆-alkyl, OC₁-C₆-alkyl, HCO, C₁-C₆-alkyl-CO, phenyl, amino, nitro, cyano or halogen, or phenoxy which is unsubstituted or substituted by C₁-C₆-alkyl, OC₁-C₆-alkyl or halogen,
or C₁-C₆-alkanoyl or benzoyl;
R¹ is H, C₁-C₈-alkyl which is unsubstituted or substituted by OH, OC₁-C₆-alkyl, phenyl or halogen;
R² has the meanings stated for R¹ or is COR¹ or CO₂R¹;
A is a C₃-C₁₅-alkylene group when Ar¹ is C₆H₅CONH, or, when Ar¹ is a 5- or 6-membered heteroaromatic ring, is a C₄-C₁₅-alkylene group or a C₃-C₁₅-alkylene group which comprises at least one group Z which is selected from O, S, NR¹, a double and a triple bond, where R¹ is as defined above,
B is a 7- or 8-membered saturated ring with one or two nitrogen heteroatoms, the nitrogen heteroatoms being located in the 1,4 or 1,5 position and the ring being bonded in position 1 to the radical A and in position 4 or 5 to the radical Ar², and it additionally being possible for the ring to have a double bond in position 3 or 4;
Ar² is phenyl, pyridyl, pyrimidinyl or triazinyl, it being possible for Ar² to have 1, 2, 3 or 4 substituents which are selected, independently of one another, from OR¹, C₁-C₈-alkyl, C₂-C₆-alkenyl, C₂-C₆-alkynyl, C₁-C₈-alkoxy-C₁-C₈-alkyl, C₁-C₈-haloalkyl, halogen, CN, CO₂R¹, NO₂, SO₂R¹, NR¹R², SO₂NR¹R², SR¹, a 5- or 6-membered carbocyclic, aromatic or non-aromatic ring and a 5- or 6-membered heterocyclic aromatic or non-aromatic ring with 1 to 3 heteroatoms which are selected from O, S and N, the carbocyclic or heterocyclic ring being unsubstituted or substituted by C₁-C₈-alkyl, phenyl, halogen, OC₁-C₈-alkyl, OH, NO₂ or CF₃, where Ar² may also be fused to a carbocyclic ring of the type defined above, and where Ar² cannot be a pyrimidinyl radical substituted by 2 hydroxyl groups,
and the salts thereof with physiologically tolerated acids.

2. A compound as claimed in claim 1 of the formula I
where Ar¹ is 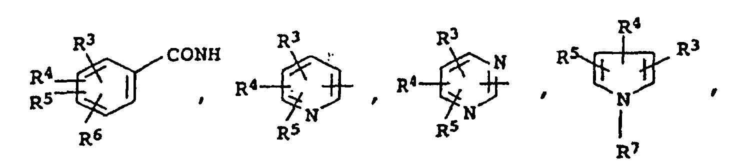 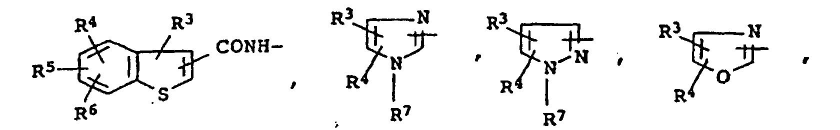 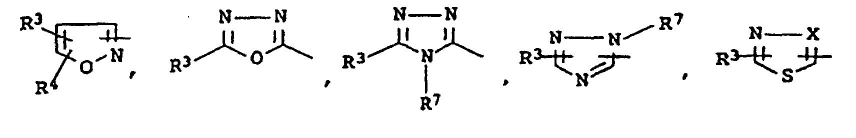 where
R³ to R⁶ are, independently of one another, H or the substituents mentioned in claim 1 for the radical
Ar¹,
R⁷ has the meanings stated for R² in claim 1 or is C₃-C₆-cycloalkyl, and
X is N or CH.

3. A compound as claimed in claim 1 of the formula I
where Ar¹ is 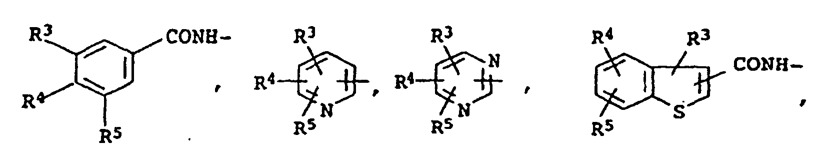 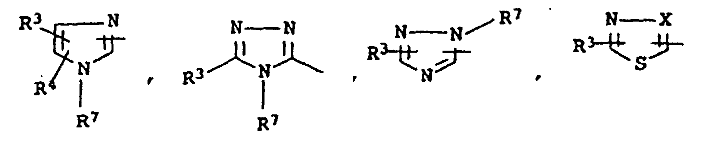 where R³ to R⁵, R⁷ and X have the meanings stated in claim 2.

4. A compound as claimed in claim 3 of the formula I
where Ar¹ is 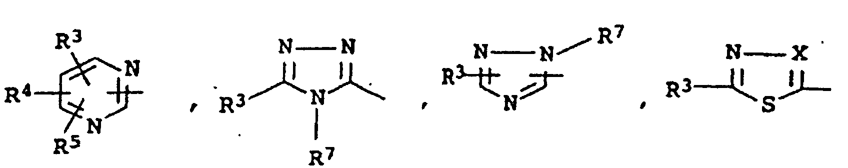 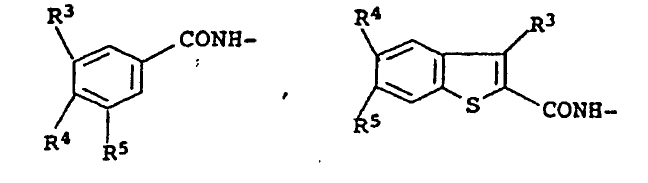 where R³ to R⁵, R⁷ and X have the meanings stated in claim 2.

5. A compound as claimed in claim 4 of the formula I where R³, R⁴ and R⁵ are, independently of one another, H, OR¹, C₁-C₈-alkyl, NR¹R², halogen, phenoxy, CN, phenyl which is unsubstituted or substituted by C₁-C₆-alkyl, HCO, C₁-C₆-alkyl-CO or halogen, or COOR¹;
R¹ and R² are, independently of one another, H, C₁-C₈-alkyl or benzyl;
R⁷ is H, C₁-C₈-alkyl or C₃-C₆-cycloalkyl; and
X is N or CH.

6. A compound as claimed in claim 5, where R³ to R⁵ are selected, independently of one another, from H, C₁-C₆-alkyl, OR¹, NR¹R², phenyl which is unsubstituted or substituted by C₁-C₆-alkyl, HCO, C₁-C₆-alkyl-CO or halogen, and halogen, where R¹ and R² have the abovementioned meanings, R⁷ is H or C₁-C₈-alkyl, and X is N.

7. A compound as claimed in claim 6, where Ar¹ is pyrimidinyl which is unsubstituted or substituted by OH, OC₁-C₈-alkyl or Obenzyl.

8. A compound as claimed in claim 6, where Ar¹ is 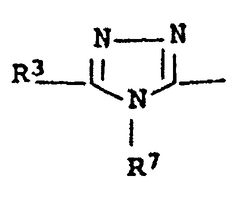 where R³ is NR¹R², where R¹ and R² have the meanings stated in claim 5, and R⁷ is H or C₁-C₈-alkyl.

9. A compound as claimed in claim 6, where Ar¹ is thiadiazole which is unsubstituted or substituted by NR¹R², where R¹ and R² have the meanings stated in claim 5.

10. A compound as claimed in claim 6, where Ar¹ is 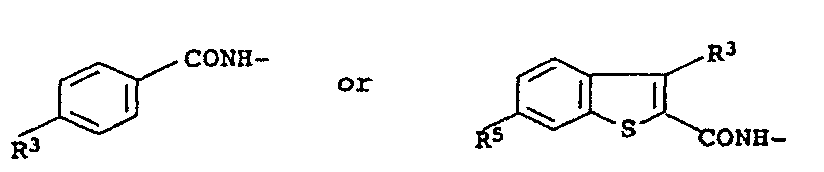 where R³ and R⁵ are, independently of one another, H or halogen, C₁-C₈-alkyl or phenyl.

11. A compound as claimed in any of the preceding claims of the formula I, where A is -Z-C₃-C₆-alkylene, in particular -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂C(=CH₂)CH₂-, -Z-CH₂CH(CH₃)CH₂- or a linear -Z-C₇-C₁₀-alkylene radical, where Z is bonded to Ar¹ and is CH₂, O or S.

12. A compound as claimed in any of the preceding claims of the formula I, where B is

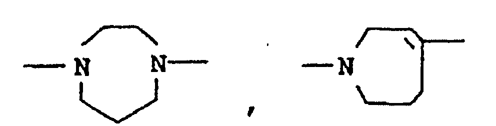

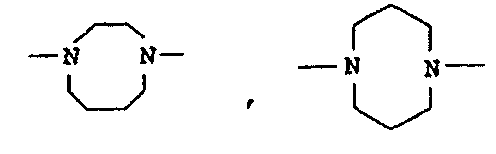

13. A compound as claimed in any of the preceding claims of the formula I, where Ar² is phenyl, pyridinyl or pyrimidinyl, which may have one or two substituents which are selected, independently of one another, from C₁-C₆-alkyl, C₂-C₆-alkynyl, halogen, CN, C₁-C₈-haloalkyl, OC₁-C₈-alkyl, NO₂, phenyl, pyrrolyl, imidazolyl, pyrazolyl, thienyl, cyclopentyl and cyclohexyl.

14. A compound as claimed in claim 13 of the formula I, where the substituent(s) are selected, independently of one another, from C₁-C₆-alkyl, NO₂ and C₁-C₈-haloalkyl, in particular CF₃, CHF₂ and CF₂Cl.

15. A compound selected from 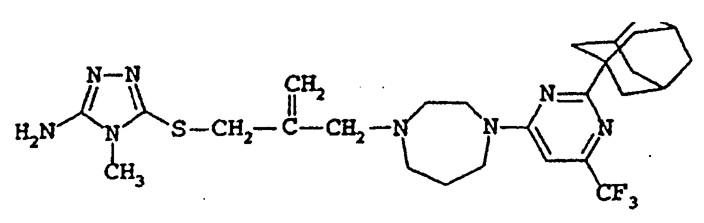 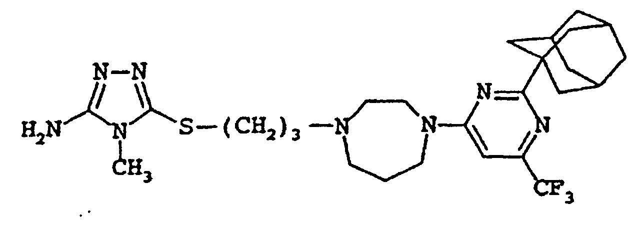 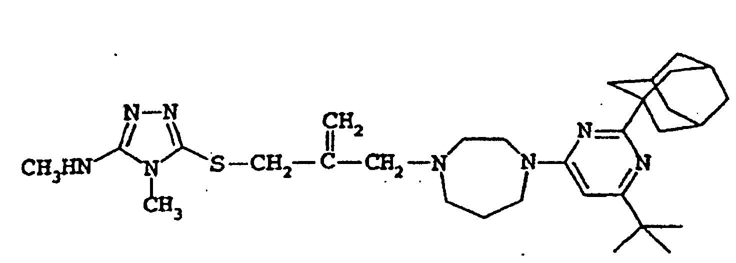 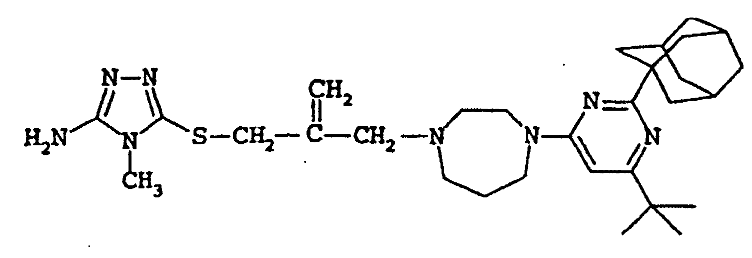 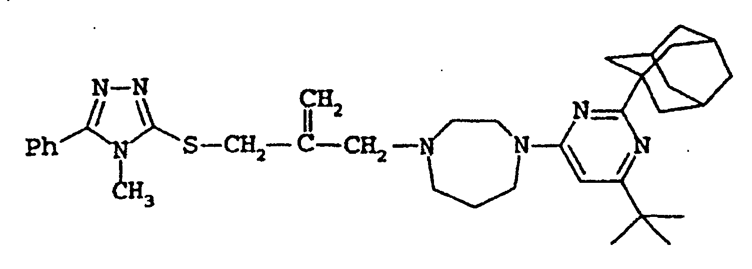 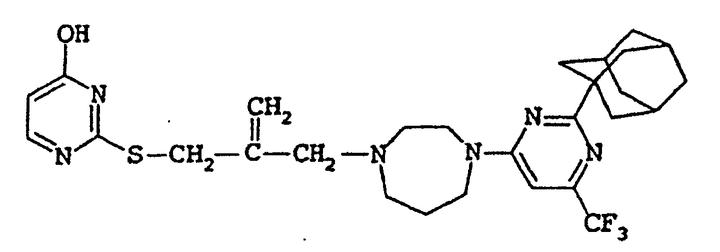 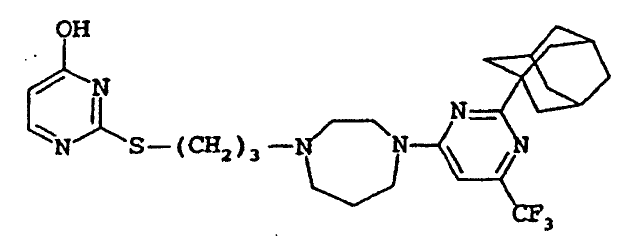 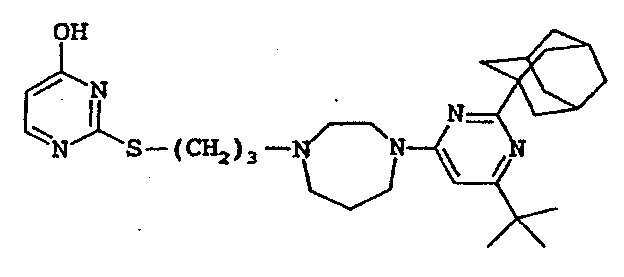 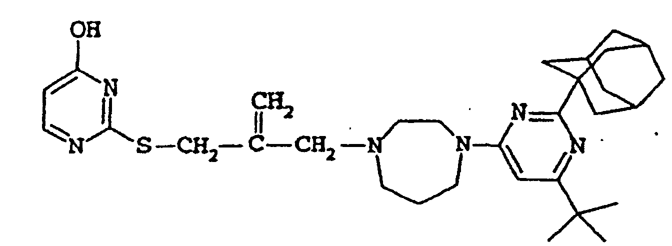 and the salts thereof with physiologically tolerated acids.

16. A compound selected from 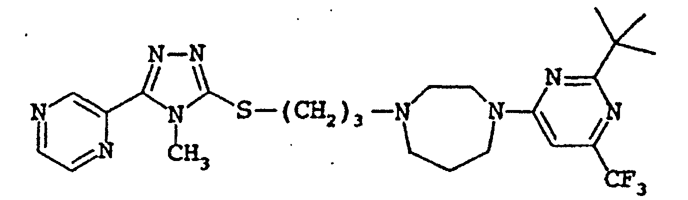 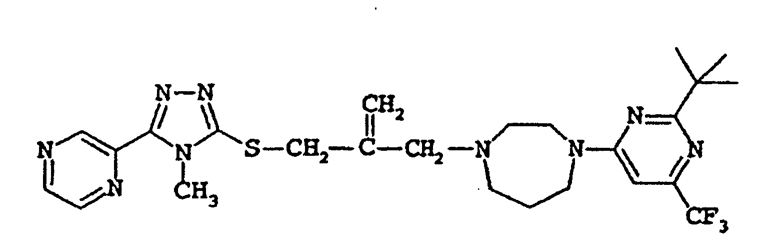 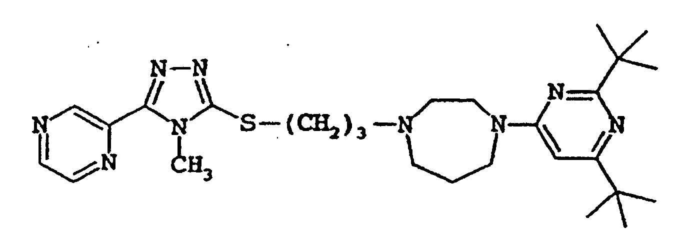 and the salts thereof with physiologically tolerated salts.

17. A pharmaceutical composition comprising at least one compound as claimed in any of claims 1 to 16, with or without physiologically acceptable vehicles and/or ancillary substances.

18. The use of at least one compound as claimed in any of claims 1 to 16 for producing a pharmaceutical composition for the treatment of disorders which respond to dopamine D₃ receptor antagonists or agonists.

## Revendications

1. Composés répondant à la formule générale I
Ar¹-A-B-Ar² (I)
dans laquelle
Ar¹ représente: 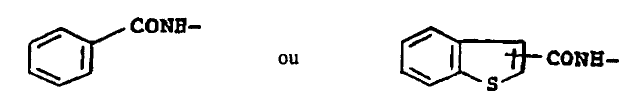 ou un hétéromonocycle aromatique à 5 ou 6 chaînons, comportant 1, 2, ou 3 hétéroatomes, qui sont choisis indépendamment les uns des autres parmi un atome d'oxygène, un atome d'azote et un atome de soufre, Ar¹ comportant le cas échéant 1, 2, 3 ou 4 substituant(s) qui est (sont) choisi(s) indépendamment les uns des autres parmi un groupement OR¹, un groupement alkyle en C₁-C₈, qui est, le cas échéant substitué par un groupement OH, un groupement O-alkyle en C₁-C₈ ou un atome d'halogène, un groupement alcényle en C₂-C₆, un groupement alcynyle en C₂-C₆, un groupement cycloalkyle en C₃-C₆, un atome d'halogène, un groupement CN, un groupement CO₂R¹, un groupement NO₂, un groupement NR¹R², un groupement SR¹, un groupement CF₃, un groupement CHF₂, un groupement phényle, qui est substitué le cas échéant par un groupement alkyle en C₁-C₆, un groupement O-alkyle en C₁-C₆, un groupement HCO, un groupement CO-alkyle en C₁-C₆, un groupement phényle, un groupement amino, un groupement nitro, un groupement cyano ou un atome d'halogène, un groupement phénoxy, qui est le cas échéant substitué par un groupement alkyle en C₁-C₆, un groupement O-alkyle en C₁-C₆ ou un atome d'halogène, un groupement alcanoyle en C₁-C₆, ou un groupement benzoyle;
R¹ représente un atome d'hydrogène, un groupement alkyle en C₁-C₈, qui est le cas échéant substitué par un groupement OH, un groupement O-alkyle en C₁-C₆, un groupement phényle ou un atome d'halogène;
R² possède les significations indiquées pour R¹ ou représente COR¹ ou CO₂R¹;
A représente un groupement alkylène en C₃-C₁₅, lorsque Ar¹ représente C₆H₅CONH, ou lorsque Ar¹ représente un hétéromonocycle aromatique à 5 ou 6 chaînons, un groupement alkylène en C₄-C₁₅, ou un groupement alkylène en C₃-C₁₅, qui comporte au moins un groupement Z, qui est choisi parmi un atome d'oxygène, un atome de soufre, un groupement NR¹, une double liaison et une triple liaison, R¹ étant comme défini ci-dessus,
B représente un noyau saturé à 7 ou 8 chaînons, comportant un ou deux hétéroatomes azote, les hétéroatomes azote se trouvant en position 1,4 ou 1,5 et le noyau étant relié en position 1 au reste A et en position 4 ou 5 au reste Ar², et le noyau pouvant comporter en outre une double liaison dans la position 3 ou 4;
Ar² représente un groupement phényle, un groupement pyridyle, un groupement pyrimidinyle ou un groupement triazinyle, Ar² pouvant comporter le cas échéant 1, 2, 3 ou 4 substituant(s), qui sont choisis indépendamment les uns des autres parmi un groupement OR¹, un groupement alkyle en C₁-C₈, un groupement alcényle en C₂-C₆, un groupement alcynyle en C₂-C₆, un groupement alcoxy en C₁-C₈-alkyle en C₁-C₈, un groupement halogénalkyle en C₁-C₈, un atome d'halogène, un groupement CN, un groupement CO₂R¹, un groupement NO₂, un groupement SO₂R¹, un groupement NR¹R², un groupement SO₂NR¹R², un groupement SR¹, un noyau aromatique ou non aromatique, carbocyclique, à 5 ou 6 chaînons et un noyau aromatique ou non aromatique hétérocyclique à 5 ou 6 chaînons comportant 1 à 3 hétéroatome(s), qui est (sont) choisi(s) parmi un atome d'oxygène, un atome de soufre et un atome d'azote, le noyau carbocyclique ou hétérocyclique étant le cas échéant substitué par un groupement alkyle en C₁-C₈, un groupement phényle, un atome d'halogène, un groupement O-alkyle en C₁-C₈, un groupement OH, un groupement NO₂ ou un groupement CF₃, Ar² pouvant être, le cas échéant, condensé de la manière ci-dessus définie avec un noyau carbocyclique et Ar² ne pouvant pas représenter un reste pyrimidinyle qui est substitué par 2 groupements hydroxy,
et leurs sels avec des acides physiologiquement compatibles.

2. Composés selon la revendication 1 répondant à la formule I où Ar¹ représente : 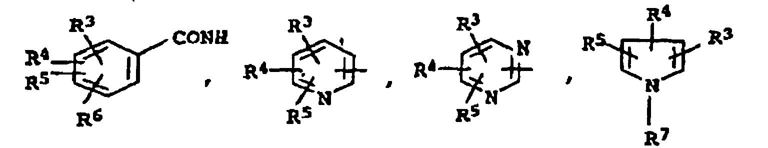 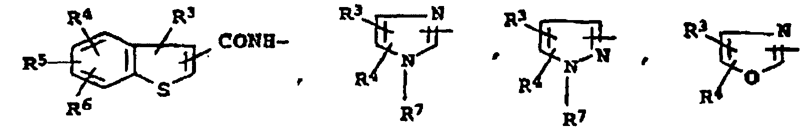 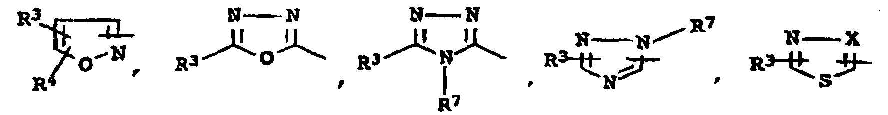 dans laquelle
R³ à R⁶ représentent, indépendamment les uns des autres, un atome d'hydrogène ou les substituants du reste Ar¹ cités dans la revendication 1,
R⁷ possède les significations indiquées dans la revendication 1 pour R² ou représente un groupement cycloalkyle en C₃-C₆, et
x représente un atome d'azote ou un groupement CH.

3. Composés selon la revendication 1 répondant à la formule I, où Ar¹ représente 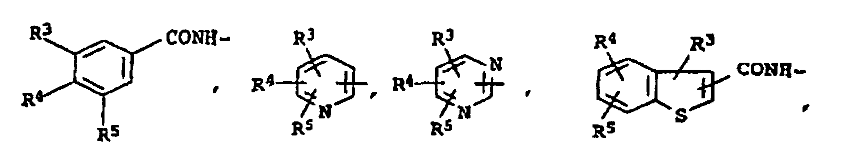 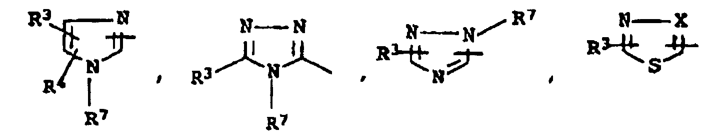 où R³ à R⁵, R⁷ et x possèdent les significations indiquées dans la revendication 2.

4. Composés selon la revendication 3 répondant à la formule I, où Ar¹ représente 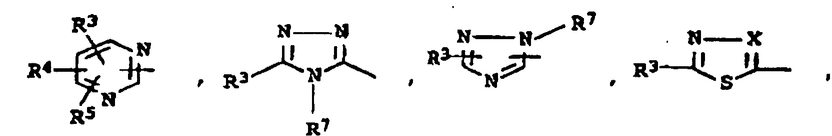 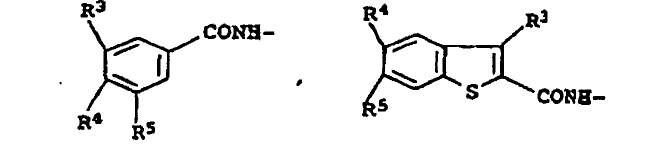 dans laquelle
R³ à R⁵, R⁷ et X possèdent les significations indiquées dans la revendication 2.

5. Composés selon la revendication 4 répondant à la formule I dans laquelle R³, R⁴ et R⁵ représentent, indépendamment les uns des autres, un atome d'hydrogène, un groupement OR¹, un groupement alkyle en C₁-C₈, un groupement NR¹R², un atome d'halogène, un groupement phénoxy, un groupement CN, un groupement phényle qui est substitué le cas échéant par un groupement alkyle en C₁-C₆, un groupement HCO, un groupement CO-alkyle en C₁-C₆ ou un atome d'halogène, ou un groupement COOR¹;
R¹ et R² représentent, indépendamment l'un de l'autre, un atome d'hydrogène, un groupement alkyle en C₁-C₈, ou un groupement benzyle;
R⁷ représente un atome d'hydrogène, un groupement alkyle en C₁-C₈ ou un groupement cycloalkyle en C₃-C₆; et
X représente un atome d'azote ou un groupement CH.

6. Composés selon la revendication 5, R³ à R⁵ étant choisis indépendamment les uns des autres parmi un atome d'hydrogène, un groupement alkyle en C₁-C₆, un groupement OR¹, un groupement NR¹R², un groupement phényle, qui est substitué le cas échéant par un groupement alkyle en C₁-C₆, un groupement HCO, un groupement CO-alkyle en C₁-C₆ ou un atome d'halogène, et un atome d'halogène, R¹ et R² possédant les significations ci-dessus indiquées, R⁷ représentant un atome d'hydrogène ou un groupement alkyle en C₁-C₈, et X représentant un atome d'azote.

7. Composés selon la revendication 6, Ar¹ représentant un groupement pyrimidinyle, qui est le cas échéant substitué par un groupement OH, un groupement O-alkyle en C₁-C₈, ou un groupement O-benzyle.

8. Composés selon la revendication 6, Ar¹ représentant : 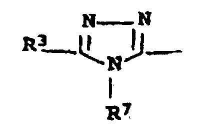 formule dans laquelle R³ représente un groupement NR¹R², R¹ et R² possédant les significations indiquées dans la revendication 5, et R⁷ représentant un atome d'hydrogène ou un groupement alkyle en C₁-C₈.

9. Composés selon la revendication 6, Ar¹ représentant un groupement thiadiazole, qui est le cas échéant substitué par un groupement NR¹R², R¹ et R² possédant les significations indiquées dans la revendication 5.

10. Composés selon la revendication 6, Ar¹ représentant 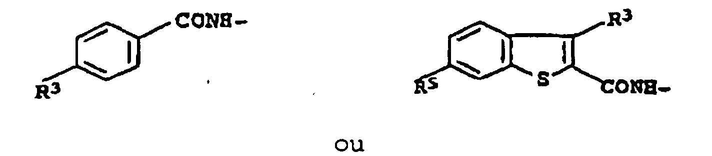 formules dans lesquelles
R³ et R⁵ représentent, indépendamment l'un de l'autre, un atome d'hydrogène ou un atome d'halogène, un groupement alkyle en C₁-C₈, ou un groupement phényle.

11. Composés selon l'une quelconque des revendication précédentes, répondant à la formule I dans laquelle A représente un groupement Z-alkylène en C₃-C₆, en particulier -Z-CH₂CH₂CH₂-, -Z-CH₂CH₂CH₂CH₂-, -Z-CH₂CH=CHCH₂-, -Z-CH₂C(CH₃)=CHCH₂-, -Z-CH₂C(=CH₂)CH₂-, -Z-CH₂CH(CH₃)CH₂-, ou un reste Z-alkylène en C₇-C₁₀, linéaire, Z étant lié à Ar¹ et représentant un groupement CH₂, un atome d'oxygène ou un atome de soufre.

12. Composés selon l'une quelconque des revendication précédentes répondant à la formule I, dans laquelle B représente :

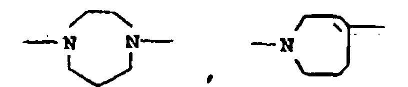

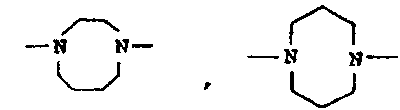

13. Composés selon l'une quelconque des revendication précédentes répondant à la formule I dans laquelle Ar² représente un groupement phényle, un groupement pyridinyle ou un groupement pyrimidinyle, qui comporte le cas échéant un ou deux substituant(s) qui est (sont) choisi(s) indépendamment parmi un groupement alkyle en C₁-C₆, un groupement alcynyle en C₂-C₆, un atome d'halogène, un groupement CN, un groupement halogènalkyle en C₁-C₈, un groupement O-alkyle en C₁-C₈, un groupement NO₂, un groupement phényle, un groupement pyrrolyle, un groupement imidazolyle, un groupement pyrazolyle, un groupement thiényle, un groupement cyclopentyle et un groupement cyclohexyle.

14. Composés selon la revendication 13 répondant à la formule I, dans lesquels le ou les substituant(s) est (sont) choisi(s) indépendamment les uns des autres parmi un groupement alkyle en C₁-C₆, un groupement NO₂ et un groupement halogénoalkyle en C₁-C₈, en particulier un groupement CF₃, un groupement CHF₂ et un groupement CF₂Cl.

15. Composé choisi parmi 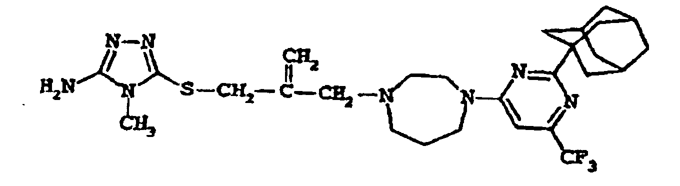 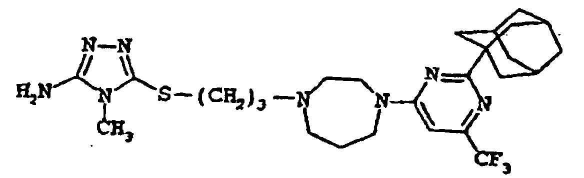 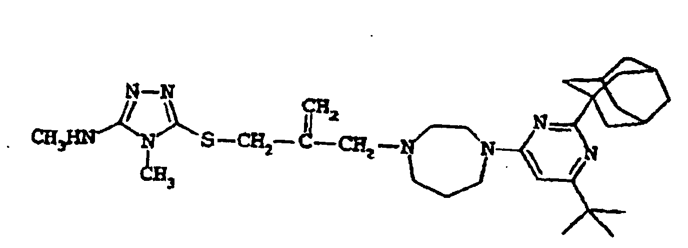 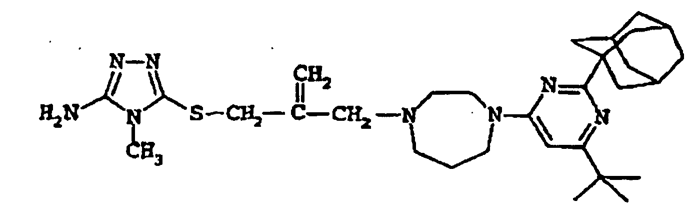 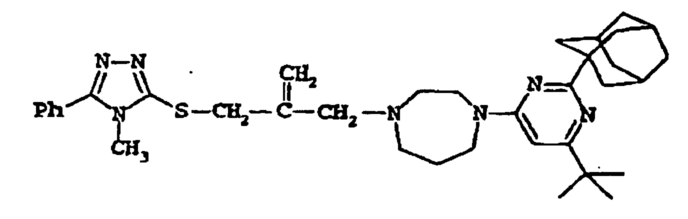 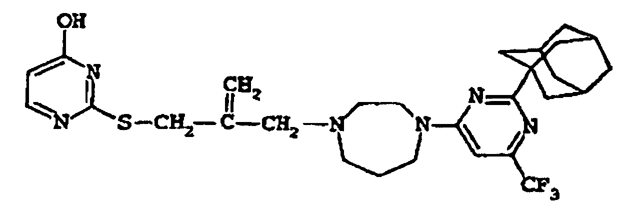 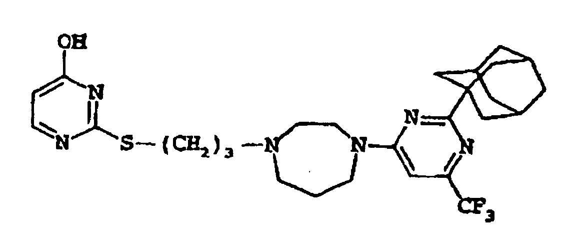 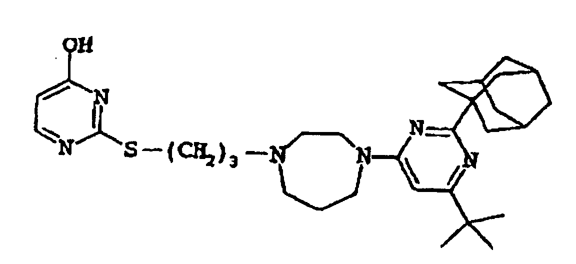 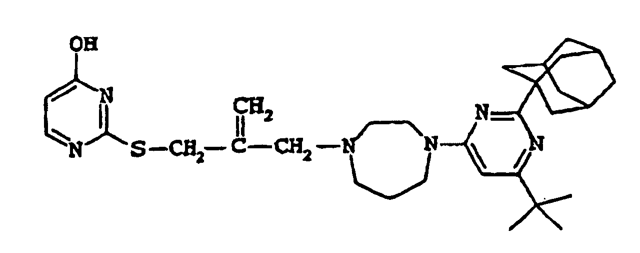 et leurs sels avec des acides physiologiquement compatibles.

16. Composé choisi parmi: 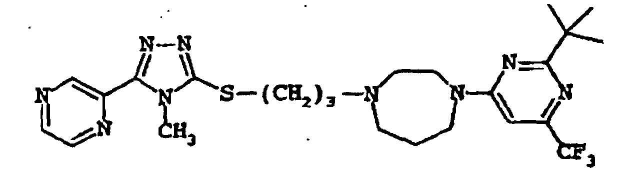 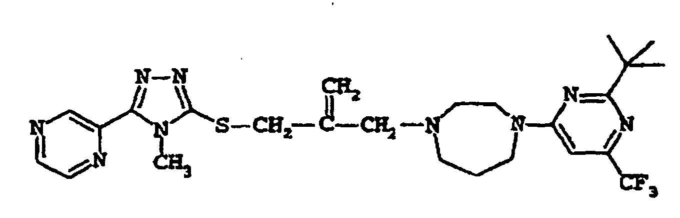 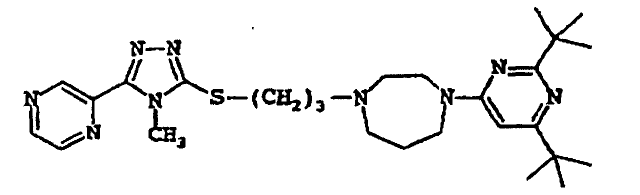 et leurs sels avec des acides physiologiquement compatibles.

17. Produit pharmaceutique contenant au moins un composé selon l'une quelconque des revendications 1 à 16, le cas échéant avec des supports et/ou adjuvants physiologiquement acceptables.

18. Utilisation d'au moins un composé selon l'une quelconque des revendications 1 à 16 pour la fabrication d'un produit pharmaceutique pour le traitement de maladies qui réagissent avec les antagonistes et agonistes du récepteur D₃ de la dopamine.
